# EUROPEAN PATENT SPECIFICATION

(11) **EP 1 395 612 B1**
(45) Date of publication and mention of the grant of the patent: **31.08.2005**
(21) Application number: 01993622.8
(22) Date of filing: 09.11.2001
(51) Int. Cl.: C07K 19/00, C07K 14/005, C12N 5/10, C12N 15/33

(54) **MODIFIED RECOMBINASE**
MODIFIZIERTE REKOMBINASE
RECOMBINASE MODIFIEE

(30) Priority: 10.11.2000 EP 00124629; 17.04.2001 EP 01109543; 13.08.2001 US 311876 P
(43) Date of publication of application: 10.03.2004
(73) Proprietor: ARTEMIS Pharmaceuticals GmbH, 51063 Köln (DE)
(72) Inventor: KÜHN, Ralf, 50937 Köln (DE); FELDER, Susanne, 41469 Neuss (DE); SCHWENK, Frieder, 50999 Köln (DE); KÜTER-LUKS, Birgit, 50733 Köln (DE); FAUST, Nicole, 51503 Rösrath (DE)
(74) Representative: Helbing, Jörg
(86) International application number: PCT/EP2001/012975
(87) International publication number: WO 2002/038613

(56) References cited:
- LE ET AL: "Nuclear targeting determinants of the phage P1 Cre DNA recombinase" NUCLEIC ACIDS RESEARCH, vol. 27, 1999, pages 4703-4709, XP002226963 cited in the application
- GROTH ET AL: "A phage integrase directs efficient site-specific integration in human cells" PROCEEDINGS OF THE NATIONAL ACADEMY OF SCIENCES, U.S.A., vol. 97, May 2000 (2000-05), pages 5995-6000, XP002221880 cited in the application
- THORPE ET AL: "Control of directionality in the site-specific recombination system of the Streptomyces phage phiC31" MOLECULAR MICROBIOLOGY, vol. 38, October 2000 (2000-10), pages 232-241, XP002166454 ISSN: 0950-382X cited in the application
- BOULIKAS: "Nuclear localization signals (NLS)" CRITICAL REVIEWS IN EUKARYOTIC GENE EXPRESSION, vol. 3, 1993, pages 193-227, XP002113642 ISSN: 1045-4403 cited in the application
- ZANTA ET AL: "Gene delivery: a single nuclear localization signal peptide is sufficient to carry DNA to the cell nucleus" PROCEEDINGS OF THE NATIONAL ACADEMY OF SCIENCES, U.S.A., vol. 96, January 1999 (1999-01), pages 91-96, XP002104949 ISSN: 0027-8424
- KANEGAE ET AL: "EFFICIENT GENE ACTIVATION IN MAMMALIAN CELLS BY USING RECOMBINANT ADENOVIRUS EXPRESSING SITE-SPECIFIC CRE RECOMBINASE" NUCLEIC ACIDS RESEARCH, vol. 23, 11 October 1995 (1995-10-11), pages 3816-3821, XP002006337 ISSN: 0305-1048
- DYMECKI : "A modular set of Flp, FRT and lacZ fusion vectors for manipulating genes by site-specific recombination" GENE, vol. 171, 1996, pages 197-201, XP004042794 ISSN: 0378-1119
- JO ET AL: "Epigenetic regulation of gene structure and function with a cell-permeable Cre recombinase" NATURE BIOTECHNOLOGY, vol. 19, October 2001 (2001-10), pages 929-933, XP002200323 ISSN: 1087-0156

## Description

The present invention concerns a fusion protein comprising the site-specific DNA recombinase C31-Int of phage ΦC31, and a peptide sequence which directs the nuclear uptake of the fusion protein in eucaryotic cells, and the use of this fusion protein to recombine, invert or delete DNA molecules containing recognition sequences for said recombinase in eucaryotic cells at high efficiency. In addition the invention relates to a cell, preferably a mammalian cell which contains recognition sequences for said recombinase in its genome and wherein the genome is recombined by the action of said fusion protein. Moreover, the invention relates to the use of said cell to study the function of genes and for the creation of non-human transgenic organisms to study gene function at various developmental stages, including the adult. In conclusion, the present invention provides a process which enables the highly efficient modification of the genome of mammalian cells by site-specific recombination.

### Background of the invention

The controlled and permanent modification of the genome of eucaryotic cells and organisms is an important method for research applications, e.g. for studying gene function, for medical applications like gene therapy and the creation of disease models and for the design of economically important animals and crops. The basic methods for genome manipulations by the engineering of endogenous genes through gene targeting in murine embryonic stem (ES) cells are well established and used since many years (Capecchi, Trends in Genetics, 5, 70-76 (1989)). Since ES cells can pass mutations induced in vitro to transgenic offspring in vivo it is possible to analyse the consequences of gene disruption in the context of the entire organism. Thus, numerous mouse strains with functionally inactivated genes ("knock-out mice") have been created by this technology and utilised to study the biological function of a variety of genes (Koller et al., Ann. Rev. Immunol., 10, 705 - 730 (1992)). More importantly, mouse mutants created by this procedure (also known as "conventional, complete or classical mutants"), contain the inactivated gene in all cells and tissues throughout life. Thus, classical mouse mutants represent the best animal model for inherited human diseases as the mutation is introduced into the germline but are not the optimal model to study gene function in adults, e.g. to validate potential drug target genes.
A refined method of targeted mutagenesis, referred to as conditional mutagenesis, employs the Cre/loxP site-specific recombination system which enables the temporally and/or spatially restricted inactivation of target genes in cells or mice (Rajewsky et al., J. Clin. Invest., 98, 600 - 603 (1996)). The phage P1 derived Cre recombinase recognises a 34 bp sequence referred to as IoxP site which is structured as an inverted repeat of 13 bp separated by an asymmetric 8 bp sequence which defines the direction of the loxP site. If two loxP sites are located on a DNA molecule in the same orientation the intervening DNA sequence is excised by Cre recombinase from the parental molecule as a closed circle leaving one loxP site on each of the reaction products (Kilby et al., TIG, 9, 413-421 (1993)). The creation of conditional mouse mutants initially requires the generation of two mouse strains, one containing two or more Cre recombinase recognition (loxP) sites in its genome while the other harbours a Cre transgene. The former strain is generated by homologous recombination in ES cells as described above, except that the exon(s) of the target gene is (are) flanked by two loxP sites which reside in introns and do not interfere with gene expression. The Cre transgenic strain contains a transgene whose expression is either constitutively active in certain cells and tissues or is inducible by external agents, depending on the promoter region used. Crossing of the loxP-flanked mouse strain with the Cre recombinase expressing strain enables the deletion of the loxP-flanked exons in the genome of doubly transgenic offspring in a prespecified temporally and/or spatially restricted manner. Thus, the method allows the analysis of gene function in particular cell types and tissues of otherwise widely expressed genes. Moreover, it enables the analysis of gene function in the adult organism by circumventing embryonic lethality which is often the consequence of complete (germline) gene inactivation. For pharmaceutical research, aiming to validate the utility of genes and their products for drug development, gene inactivation which is inducible in adults provides an excellent genetic tool as this mimicks the biological effects of target inhibition upon drug application.

Since the first description of the concept of conditional gene targeting using the Cre/IoxP system in mice in 1994 (Gu et al., Science 265, 103-106 (1994)) this method became increasingly popular among the research community and resulted in a broad collection of genetic tools for biological research in the mouse. More than 30 Cre transgenic mouse strains with various tissue specificities for gene inactivation have been created, including several "deleter" strains which allow to remove the loxP-flanked target gene segment in the male or female germline (Cohen-Tannoudji et al., Mol. Hum. Reprod. 4, 929-938 (1998); Metzger et al., Curr. Op. Biotech., 10, 470-476 (1999)). The need to characterise the expression pattern of Cre mediated recombination in newly generated strains stimulated the construction of a number of "Cre-reporter" strains which harbour a silent reporter gene the expression of which is activated upon Cre-mediated deletion (Nagy, Genesis, 26, 99-109 (2000)). Conditional mouse mutants have been reported for about 20 different genes, many of them could not be studied in adults as their complete inactivation leads to embryonic lethality (Cohen-Tannoudji et al., Mol. Hum. Reprod. 4, 929-938 (1998)).

Great efforts have also been made to control the expression of Cre recombinase in an inducible fashion in mice. After the first demonstration that inducible gene knock-out is feasible in adult mice using an interferon controlled promoter (Kühn et al., Science, 269, 1427-1429 (1995)), mainly two methods were applied to control the activity of Cre recombinase. First, it has been demonstrated that the fusion of Cre with the ligand binding domain of a mutant estrogen receptor allows to control recombinase activity by a specific steroid-like inducer. Several transgenic mouse strains expressing such a fusion protein have been generated and allow to induce gene inactivation in specific tissues (Metzger et al., Curr. Op. Biotech., 10, 470-476 (1999)). Furthermore, the tetracycline-regulated gene expression system has been successfully used to control the expression of Cre in transgenic mice and thus provides a second system for inducible gene inactivation using doxycycline as inducer (Saam et al., J. Biol. Chem. 274, 38071-38082 (1999)).

In addition to the application of Cre/loxP for gene inactivation by deletion of a gene segment this recombination system has been proved to be useful also for a number of other genomic manipulations in ES cells or mice. These include the conditional activation of transgenes in mice, chromosome engineering to obtain deletion, translocation or inversion, the simple removal of selection marker genes, gene replacement, the targeted insertion of transgenes and the (in)activation of genes by inversion (Nagy, Genesis, 26, 99-109 (2000); Cohen-Tannoudji et al., Mol. Hum. Reprod. 4, 929-938 (1998)). In conclusion, the Cre/IoxP recombination system has been proven to be extremely useful for the analysis of gene function in mice by broadening the methodological spectrum for genome engineering. It can be expected that many of the protocols now established for the mouse may be applied in future also to other animals or plants.

In contrast to the huge diversity of genome manipulations which have been developed for the Cre/loxP system, very limited efforts have been made to develop further site-specific recombination systems for the use in mammalian cells. Alternative recombination systems of different specificity but with an efficiency comparable to Cre/loxP could further enhance the flexibility of genome engineering by the side to side use of two or more systems in the same cell or organism. Furthermore, unidirectional recombination systems which follow a different mechanism than the reversible Cre/loxP-mediated recombination should allow to develop new applications for genome engineering which cannot be performed with the current systems.

The reasons for the almost exclusive use of the Cre/IoxP system for site-specific recombination in mammalian cells are readily explained by a number of requirements which must be fulfilled for the efficient use of a recombinase in mammalian cells:
i) the recombinase should act independent of cofactors like helper proteins,
ii) it should act independent of the supercoiling status of the target DNA and also on mammalian chromatin,
iii) it should be efficiently active and stable at a temperature of 37°C, and
iv) it should recognize a target sequence which is sufficiently long to be unique among large genomes, and it should exhibit a very high affinity to its target site for efficient action (Kilby et al., TIG, 9, 413-421 (1993)).

Among the more than 200 described members of the integrase and resolvase/invertase recombinase families only the Cre/IoxP system is presently known to fulfill all of these requirements (Nunes-Düby et al., Nucleic Acids Res., 26, 391-406 (1998); Kilby et al., TIG, 9, 413-421 (1993); Ringrose et al., J. Mol. Biol., 284, 363 - 384 (1998)). Besides Cre/loxP a few recombinases have been shown to exhibit some activity in mammalian cells but their practical value is presently unclear as their efficieny has not been compared to the Cre/IoxP system on the same genomic recombination substrate and in some cases it is known that one or more of the criteria listed above are not met. The best characterised examples are the yeast derived FLP and Kw recombinases which exhibit a temperature optimum at 30°C but which are unstable at 37°C (Buchholz et al., Nature Biotech., 16, 657 - 662 (1998); Ringrose et al., Eur. J. Biochem., 248, 903 - 912). For FLP it has been shown in addition that its affinity to the FRT target site is much lower as compared to the affinity of Cre to loxP sites (Ringrose et al., J. Mol. Biol., 284, 363 - 384 (1998)). Other recombinases which show in principle some activity in mammalian cells are a mutant integrase of phage λ, the integrases of phages ΦC31 and HK022, mutant γδ-resolvase and p-recombinase (Lorbach et al., J. Mol. Biol., 296, 1175 - 81 (2000); Groth et al., Proc. Natl. Acad. Sci. USA, 97, 5995 - 6000 (2000); Kolot et al., Mol. Biol. Rep. 26, 207 - 213 (1999); Schwikardi et al., FEBS Lett., 471, 147 - 150 (2000); Diaz et al., J. Biol. Chem., 274, 6634 - 6640 (1999)). Other phage integrase systems include coliphage P4 recombinase, Listeria phage recombinase, bacteriophage R4 Sre recombinase, CisA recombinase, XisF recombinase and transposon Tn*4451* TnpX recombinase (Stark et al. Trends in Genetics 8, 432-439 (1992); Hatfull & Gridley, in Genetic Recombination. Eds. Kucherlipati & Smith, Am. Soc. Microbiol., Washington DC, 357-396 (1988)).

However, the practical value of these recombinases and integrases for use in mammalian cells is limited as their efficiency to recombine mammalian genomic DNA has not been tested or compared with the Cre/IoxP system. From the data available it can be assumed that these recombinases are much less effective than the Cre/loxP system.

In a few cases attempts have been made to improve the performance of recombinases in mammalian cells: for FLP a mutant showing improved thermostability and acticity at 37°C has been isolated but this mutant is still considerably more heat labile as compared to Cre (Buchholz et al., Nature Biotech., 16, 657 - 662 (1998)). In the case of λ-integrase and γδ-resolvase the absolute requirement for coproteins and supercoiled DNA could be eliminated by the introduction of specific point mutations (Schwikardi et al. FEBS Lett 471, pp147-50 (2000)).

The import of cytoplasmic proteins into the nucleus of eucaryotic cells through nuclear pores is a regulated, energy dependent process mediated by specific receptors (Görlich et al., Science, 271, 1513 - 1518 (1996)). Proteins which do not posses a signal sequence recognised by the nuclear import machinery are excluded from the nucleus and remain in the cytoplasm. Numerous of such nuclear localisation signal sequences (NLS), which share a high proportion of basic amino acids in common, have been characterised (Boulikas, Crit. Rev. Eucar. Gene Expression, 3, 193 - 227 (1993)), the prototype of which is the 7 amino acid NLS derived from the T-antigen of the SV40 virus (Kalderon et. al, Cell, 39, 499 - 509 (1984)).

Introduction of exogenous DNA into the cell nucleus by coupling an NLS peptide to a DNA fragment to facilitate translocation of the DNA through the nuclear membrane has already been shown (Zanta et al., Proc. Natl. Acad. Sci. USA, 96, 91-96 (1999)). NLS can also confer nuclear localization on β-Gal in mammalian cells when plasmids with lacZ disrupted by directly repeated FRTs and an SV40-NLS fused to the FRT-domain are activated by Flp-mediated excisional recombination (Dymecki, Gene, 171, 197-201 (1996)).

It was believed that the fusion of such an NLS peptide to a recombinase possibly would enhance the efficiency of the recombinase by mediating its import into the nucleus and therewith increasing the concentration of the recombinase inside the nucleus. However, for Cre recombinase it has been shown that the addition of the SV-40 T-antigen NLS does not improve its recombination efficiency in mammalian cells (Le et al., Nucleic Acid Res., 27, 4703 -4709 (1999)). Nevertheless, both Cre and a Cre-NLS-fusion protein are widely used. Schwikardi (Schwikardi et al., FEBS Lett. 471, pp147-50 (2000)) reported a γδ-resolvase-SV-40 T-antigen NLS fusion protein, which also did not enhance the recombination efficiency.

The level of activity exhibited by recombinases of diverse prokaryotic origin in mammalian cells may be the result of the intrinsic properties of an enzyme depending on parameters like its temperature optimum, its target site affinity, protein structure and stability, the degree of cooperativity, the stability of the synaptic complex and the dependence on coproteins or supercoiled DNA. Within the specific environment of mammalian cells the activity of a prokaryotic recombinase could be limited by additional factors such as a short half-life of the recombinase transcript, a short half-life of its protein, its inability to act on histone-complexed and higher order structured mammalian genomic DNA, exclusion from the nucleus or the recognition of cryptic splice sites within its mRNA resulting in a nonfunctional transcript. Due to the lack of information on the parameters listed above for almost all recombinases it is presently not possible to rationally optimise their performance in mammalian cells.

### Summary of the Invention

The object to be solved by the invention of the present application is the provision of a recombination system alternative to the Cre/IoxP system, which has a different specificity but an efficiency comparable to Cre/IoxP. Such an alternative recombination system is particularly desirable for all those applications which require more than one potent recombination system for being successfully carried out (e.g. the methods disclosed in PCT/EP01/00060 and PCT/EP00/10162 = WO 01/49832 and WO 01/29208). Most surprisingly, it was found that the above object can be solved by fusing a signal peptide capable directing the nuclear import (hereinafter shortly referred to as nuclear localisation signal sequences (NLS)) to specific recombinases.

In contrast to the wildtype recombinases, the resulting modified recombinases allow a highly efficient recombination of extrachromosomal and chromosomal DNA in mammalian cells, and a highly efficient excision of extrachromosomal and chromosomal DNA-stretches, which are flanked by suitable recognition sites for said modified recombinases.

The present invention thus provides:
(1) A fusion protein (hereinafter also referred to as "modified recombinase") comprising
   (a) a recombinase domain comprising a recombinase protein being a bacteriophage ΦC31 integrase (C31-Int) protein or a mutant thereof having a recombinase activity similar to that of the corresponding wild-type recombinase and
   (b) a signal peptide domain being linked to (a) and directing the nuclear import of said fusion protein in eucaryotic cells;
(2) a DNA coding for the fusion protein as defined in (1) above;
(3) a vector containing the DNA as defined in (2) above;
(4) a microorganism containing the DNA of (2) above and/or the vector of (3) above;
(5) a process for preparing the fusion protein as defined in (1) above which comprises culturing a microorganism as defined in (4) above;
(6) the use of the fusion protein as defined in (1) above to recombine DNA molecules, which contain recombinase recognition sequences for the recombinase protein of the recombinase domain, in eucaryotic cells;
(7) a cell, preferably a mammalian cell containing the DNA sequence of (2) above in its genome;
(8) the use of the cell of (7) above for studying the function of genes and for the creation of transgenic non-human organisms;
(9) a transgenic non-human organism, preferably a transgenic non-human mammal containing the DNA sequence of (2) above in its genome;
(10) the use of the transgenic organism of (9) above for studying gene function at various developmental stages;
(11) an *in vitro* method for recombining DNA molecules of cells containing recognition sequences for the recombinase protein of the recombinase domain as defined in (1) above, which method comprises supplying the cells with a fusion protein as defined in (1) above, or with a DNA sequence of (2) above and/or a vector of (3) above which are capable of expressing said fusion protein in the cell or organism;
(12) the use of a fusion protein as defined in (1) above, or a DNA sequence of (2) above and/or a vector of (3) above which are capable of expressing said fusion DNA protein in an organism, for preparing an agent for recombining DNA molecules in organisms containing recombinase recognition sequences for said recombinase protein;
(13) an *in vitro* method for recombining a DNA molecule containing recognition sequences for a recombinase protein in a eukaryotic cell, said method comprising contacting the cell with a fusion protein as defined in (1) above that recognizes said recognition sequences, wherein the fusion protein catalyzes recombination of the DNA molecule;
(14) the fusion protein as defined in (1) above which catalyzes recombination at recognition sequences for the recombinase protein; and
(15) a transgenic non-human organism, preferably a transgenic non-human mammal, comprising a cell containing a DNA sequence coding for a recombinase fusion protein as defined in (1) or (14) above in its genome.

The present invention combines the use of C31-Int with a eukaryotic signal sequence which increases its efficiency in mammalian cells such that it is equal to the widely used Cre/IoxP recombination system. The improved recombination system of the present invention provides an alternative recombination system for use in mammalian cells and organisms which allows to perform the same types of genomic modifications as shown for Cre/IoxP, including conditional gene inactivation by recombinase-mediated deletion, the conditional activation of transgenes in mice, chromosome engineering to obtain deletion, translocation or inversion, the simple removal of selection marker genes, gene replacement, the targeted insertion of transgenes and the (in)activation of genes by inversion.

### Short Description of Figures

Fig. 1: C31-Int and Cre recombinase expression vectors and a recombinase reporter vector used for transient and stable transfections
Fig. 2: Results of transient transfections of C31 Int and Cre expression vectors and reporter vectors into CHO cells.
Fig. 3: Results of transient transfections of XisA and Ssv recombinase expression vectors with and without nuclear localisation signals and reporter vectors into CHO cells.
Fig. 4: Results of transient transfections of C31-Int and Cre recombinase vectors into a stable reporter cell line.
Fig. 5: In situ detection of β-galactosidase in 3T3(pRK64)-3 cells transfected with recombinase expression vectors
Fig. 6: Test vector for C31-Int mediated deletion, pRK64, and the expected deletion product.
Fig. 7: PCR products generated with the primers P64-1 and P64-4 and sequence comparison.
Fig. 8: ROSA26 locus of the C31 reporter mice carrying a C31 reporter construct.
Fig. 9: *In situ* detection of β-galactosidase in a cryosection of the testis of: (A) a double transgenic mouse carrying both the recombinase and the reporter; and (B) a transgenic mouse carrying only the reporter as a control.

### Detailed Description of the Invention

The "organisms" according to the present invention are multi-cell organisms and can be vertebrates such as mammals (humans and non-human animals including rodents such as mice or rats) or non-mammals (e.g. fish), or can be invertebrates such as insects or worms, or can be plants (higher plants, algi or fungi). Most preferred living organisms are mice and fish.

"Cells" and "eucaryotic cells" according to the present invention include cells isolated from the above defined living organism and cultured *in vitro.* These cells can be transformed (immortalized) or untransformed (directly derived from the living organism; primary cell culture).

"Microorganism" according to the present invention relates to procaryotes (e.g. *E. coli*) and eucaryotic microorganisms (e.g. yeasts).

The activity of the fusion protein of the present invention in eucaryotic cells is significantly higher as compared to the acitivity of the wildtype recombinase corresponding to the recombinase of the recombinase domain. An increase in activity of at least 50%, preferably at least 75%, more preferably at least 100% was found relative to the corresponding wildtyp recombinase in eucaryotic cells. The fusion protein of the present invention has at least 25%, preferably at least 50% and more preferably at least 75%, of the activity of Cre/IoxP in 3T3 cells with a stably integrated target sequence.

Recombinase proteins which can be used in the recombinase domain of the fusion protein of the present invention are bacteriophage ΦC31 integrase ("C31-Int"; the amino acid sequence of said integrase and a DNA sequence coding therefor are shown in SEQ ID NOs:21 and 20, respectively), or mutants thereof.

A "mutant" of the above recombinase in accordance with the present invention relates to a mutant of the respective original (viz. wild-type) recombinase having a recombinase activity similar (e.g. at least about 90%) to that of said wild-type recombinase. Mutants include truncated forms of the recombinase (such as N- or C-terminal truncated recombinase proteins), deletion-type mutants (where one or more amino acid residues or segments having more than one continuous amino acid residue have been deleted from the primary sequence of the wildtyp recombinase), replacement-type mutants (where one or more amino acid residues or segments of the primary sequence of the wildtyp recombinase have been replaced with alternative amino acid residues or segments), or combinations thereof.

According to embodiment (1) of the invention, the recombinase domain comprises a phage ΦC31 integrase (C31-Int) protein or a mutant thereof. Thus, the present invention provides a fusion protein comprising
(a) an integrase domain being a C31-Int protein or a mutant thereof, and
(b) a signal peptide domain being linked to (a) and directing the nuclear import of said fusion protein into eucaryotic cells.

In the fusion protein of embodiment (1), the integrase domain is preferably a C31-Int having the amino acid sequence shown in SEQ ID NO:21 or a C-terminal truncated form thereof. Suitable truncated forms of the C31-Int comprise amino acid residues 306 to 613 of SEQ ID NO:21.

The signal peptide domain (hereinafter also referred to as "NLS") is preferably derived from yeast GAL4, SKI3, L29 or histone H2B proteins, polyoma virus large T protein, VP1 or VP2 capsid protein, SV40 VP1 or VP2 capsid protein, Adenovirus E1a or DBP protein, influenza virus NS1 protein, hepatitis virus core antigen or the mammalian lamin, c-myc, max, c-myb, p53, c-erbA, jun, Tax, steroid receptor or Mx proteins (see Boulikas, Crit. Rev. Eucar. Gene Expression, 3, 193 - 227 (1993)), simian virus 40 ("SV40") T-antigen (Kalderon et. al, Cell, 39, 499 - 509 (1984)) or other proteins with known nuclear localisation. The NLS is preferably derived from the SV40 T-antigen.

Furthermore, the signal peptide domain preferably has a length of 5 to 74, preferably 7 to 15 amino acid residues. More preferred is that the signal peptide domain comprises a segment of 6 amino acid residues wherein at least 2 amino acid residues, preferably at least 3 amino acid residues are positively charged basic amino acids. Basic amino acids include, but are not limited to, Lysin, Arginin and Histidine. Particularly preferred signal peptides are show in the following table.

| Organism | Sequence/(SEQ ID NO:) | |
|---|---|---|
| yeast GAL4 | MKx11CRLKKLKCSKEKPKCAKCLKx5Rx3KTKR | (24) |
| yeast SKI3 | IKYFKKFPKD | (25) |
| yeast L29 | MTGSKTRKHRGSGA | (26) |
| | (MTGSKHRKHPGSGA) | (27) |
| yeast histone H2B | (GKKRSKA) | (28) |
| polyoma virus large T protein | (PKKAREDVSRKRPR) | (29) |
| polyoma virus VP1 capsid protein | (APKRKSGVSKC) | (30) |
| polyoma virus VP2 capsid protein | (EEDGPQKKKRRL) | (31) |
| SV40 VP1 capsid protein | (APTKRKGS) | (32) |
| SV40 VP2 capsid protein, | (PNKKKRK) | (33) |
| Adenovirus E1a protein | (KRPRP) | (34) |
| | (CGGLSSKRPRP) | (35) |
| Adenovirus DBP protein | (PPKKRMRRRIEPKKKKKRP) | (36) |
| influenza virus NS1 protein | (PFLDRLRRDQK) | (37) |
| | (PKQKRKMAR) | (38) |
| human laminA | (SVTKKRKLE) | (39) |
| human c-myc | (CGGAAKRVKLD) | (40) |
| | (PAAKRVKLD) | (41) |
| | (RQRRNELKRSP) | (42) |
| HUMAN max | (PQSRKKLR) | (43) |
| HUMAN c-myb | (PLLKKIKQ) | (44) |
| HUMAN p53 | (PQPKKKP) | (45) |
| HUMAN c-erbA | (SKRVAKRKL) | (46) |
| VIRAL jun | (ASKSRKRKL) | (47) |
| HUMAN Tax | (GGLCSARLHRHALLAT) | (48) |
| Mammalian glucocorticoid receptor | (RKTKKKIK) | (49) |
| HUMAN ANDROGEN RECEPTOR | (RKLKKLGN) | (50) |
| MAMMALIAN ESTROGEN RECEPTOR | (RKDRRGGR) | (51) |
| Mx proteins | (DTREKKKFLKRRLLRLDE) | (52) |
| SV40 T-antigen | (PKKKRKV) | (53) |

The most preferred signal peptide domain is that of SV40 T-antigen having the sequence Pro-Lys-Lys-Lys-Arg-Lys-Val.

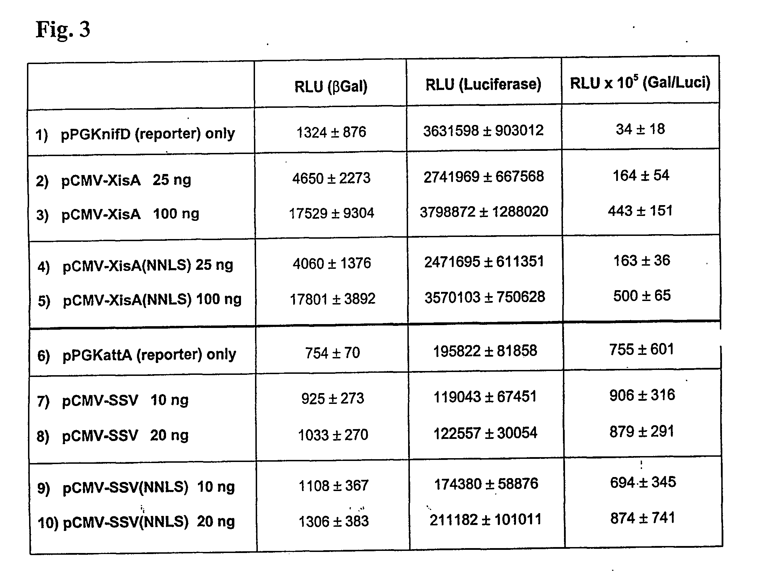

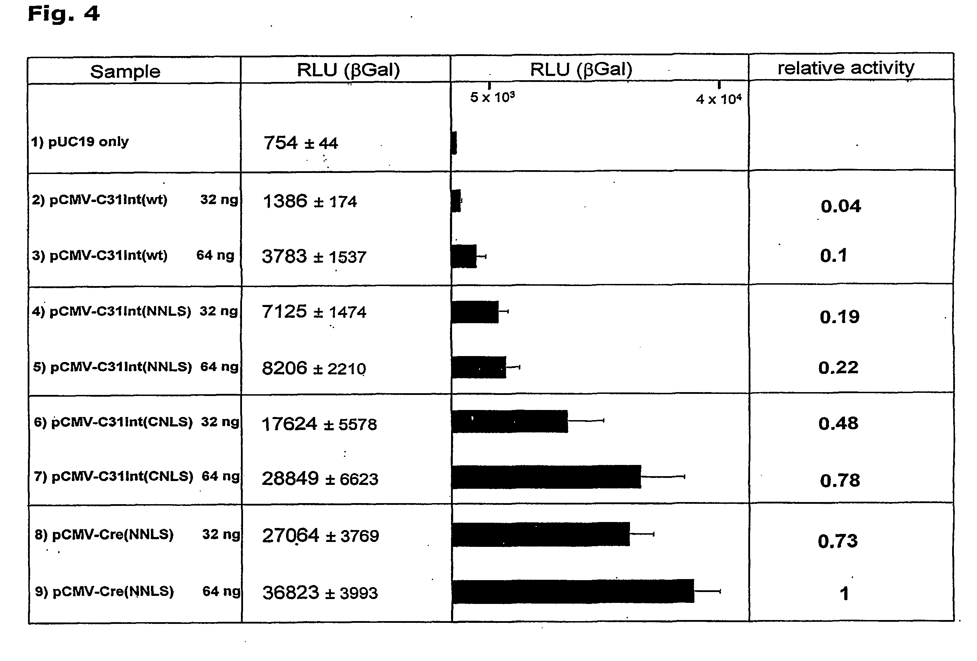

The signal peptide domain may be linked to the N-terminal or C-terminal of the integrase domain or may be integrated into the integrase domain, preferably the signal peptide domain is linked to the C-terminal of the integrase domain. With regard to phage ΦC31 integrase protein of embodiment (1) of the invention it was found that the fusion of an NLS-peptide to the C-terminus of the integrase provided a much higher increase of activity as compared to the fusion of the same NLS-peptide to the N-terminus of the integrase (see Example 1, figures 3 and 4).

According to the present invention, the signal peptide domain may be linked to the integrase domain directly or through a linker peptide. Suitable linkers include peptides having from 1 to 30, preferably 1 to 15 amino acid residues, said amino acid residues being essentially neutral amino acids such as Gly, Ala and Val.

The most preferred fusion protein of the present invention comprises the amino acid sequence shown in SEQ ID NO:23 (a suitable DNA sequence coding for said fusion protein being shown in SEQ ID NO:22).

Fusion proteins used for comparison in present invention are "NLS-XisA" and "NLS-SSV" (having the NLS-peptide fused to the N-terminus of the recombinases) as shown in SEQ ID NO:67 and 69, respectively (suitable DNA sequences coding for said fusion proteins being shown in SEQ ID NO:66 and 68, respectively).

In embodiments (6), (7), (9) and (11) of the invention the DNA molecules, the cell or transgenic organism may also contain recognition sequences for the recombinase protein of the recombinase domain. Thus, when utilizing the fusion protein of embodiment (1), the C31-Int recognition sequences attP and attB are present in DNA molecules, the cell or transgenic organism.

The term "mammal" as used in embodiment (9) of the invention includes non-human mammals (viz. animals as defined above).

Since the modified C31-Int of the invention, acts in mammalian cells as efficient (or at least almost as efficient) as the widely used Cre/IoxP system it can be used for a large variety of genomic modifications (including the methods disclosed in PCT/EP01/00060 and PCT/EP00/10162 = WO 01/49832 and WO 01/29208). Concerning embodiment (10) it is to be noted that the mammals of embodiment (9) can be used to study the function of genes, e.g. in mice, by conditional gene targeting. For this purpose suitable recognition sequences - when utilizing the fusion protein of embodiment (1), one attP and one attB site (C31-Int recognition sequences) in the same orientation - can be introduced into introns of a gene by homologous recombination of a gene targeting vector in ES cells such that the two sites flank one or more exons of the gene to be studied but do not interfere with gene expression. A selection marker gene, needed to isolate recombinant ES cell clones, can be flanked by two recognition sites of another recombinase such as IoxP or FRT sites to enable deletion of the marker gene upon transient expression of the respective recombinase in ES cells. These ES cells can be used to generate germline chimaeric mice which transmit the target gene modified by att sites to their offspring and allow to establish a modified mouse strain. The crossing of this strain with a C31-Int recombinase transgenic line or the application of C31-Int protein will result in the deletion of the att-flanked gene segment from the genome of doubly transgenic offspring and the inactivation of the target gene in doubly transgenic offspring in a prespecified temporally and/or spatially restricted manner. The C31-Int transgenic strain contains a transgene whose expression is either constitutively active in certain cells and tissues or is inducible by external agents, depending on the promoter region used. If an attB and an attP site are placed into the genome in opposite orientation C31-Int mediated recombination results in the irreversible inversion of the flanked gene segment leading the functional loss of on or more exons of the target gene. Thus, the method allows the analysis of gene function in particular cell types and tissues of otherwise widely expressed genes and circumvents embryonic lethality which is often the consequence of complete (germline) gene inactivation. For the validation of genes and their products for drug development, gene inactivation which is inducible in adults provides an excellent genetic tool as this mimicks the biological effects of target inhibition upon drug application. If a pair of attB/P sites is placed in the same or opposite orientation into a chromosome at large distance using two gene targeting vectors, C31-Int recombination allows to delete or invert chromosome segments containing one or more genes, or chromosomal translocations if the two sites are located on different chromosomes. In another application of the method a pair of attB/P sites is placed in the same orientation within a transgene such that the deletion of the att-flanked DNA segment results in gene expression, e.g. of a toxin or reporter gene for cell lineage studies, or in the inactivation of the transgene.

In addition, according with embodiment (11) of the invention, the recombination system of embodiment (1) can also be used for the site specific integration of foreign DNA into the genome of mammalian cells, e.g. for gene therapy. For this purpose, and if the C31-Int recombination system of embodiment (1) is utilized, only one attB (or attP) site is initially introduced into the genome by homologous recombination, or an endogenous genomic sequence which resembles attB or attP is used. The application of a vector containing an attP (or attB) site to such cells or mice in conjunction with the expression of C31-Int recombinase will lead to the site specific integration of the vector into the genomic att site.

Thus, the present invention provides a process which enables the highly efficient modification of the genome of mammalian cells by site-specific recombination. Said process possesses the following advantages over current technology:
(i) the modified C31-Integrase allows to recombine extrachromosomal and genomic DNA in mammalian cells at much higher efficiency as compared to the use of its wildtype form;
(ii) the modified C31-Integrase is the first described alternative recombination system with equal efficiency to Cre/loxP for the recombination of chromosomal DNA in mammalian cells.

The appended figures further explain the present invention:

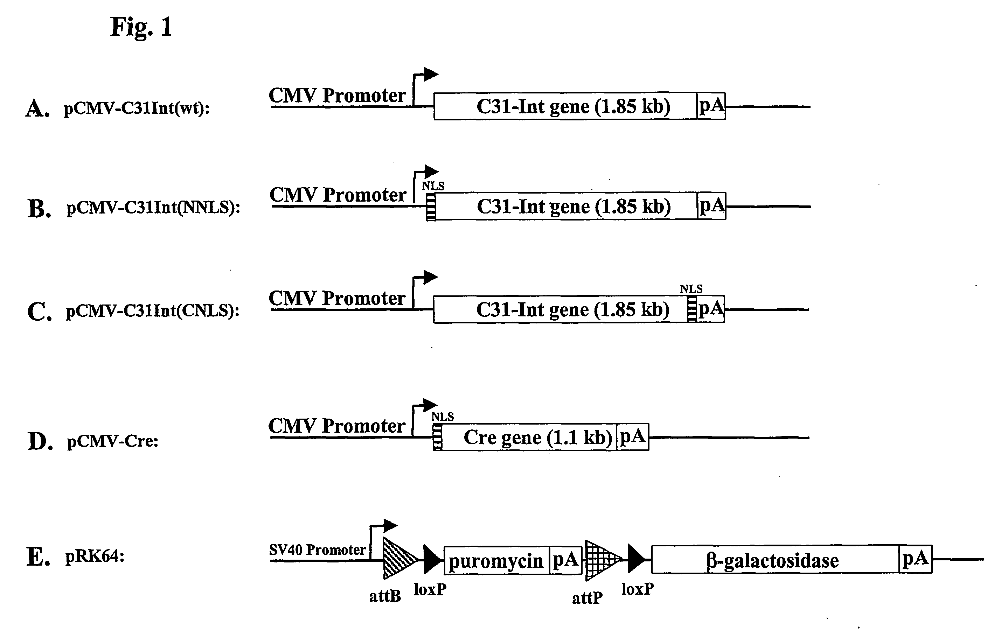

Figure 1 shows C31-Int and Cre recombinase expression vectors and a recombinase reporter vector used for transient and stable transfections.
   A-D: Mammalian expression vectors for recombinases which contain the CMV immediate early promoter followed by a hybrid intron, the coding region of the recombinase to be tested, and an artificial polyadenylation signal sequence (pA).
   A: pCMV-C31Int(wt) containing the nonmodified (wildtype) 1.85 kb coding region of C31-Int as found in the genome of phage ΦX31.
   B: pCMV-C31Int(NNLS) containing a modified C31-Int gene coding for the full length C31-Int protein with a N-terminal fusion to the SV40 virus large T antigen nuclear localisation signal (NLS).
   C: pCMV-C31Int(CNLS) containing a modified C31-Int gene coding for the full length C31-Int protein with a C-terminal fusion to the SV40 virus large T antigen nuclear localisation signal (NLS).
   D: pCMV-Cre contains the 1.1 kb Cre coding region with an N-terminal fusion to the SV40 T antigen NLS.
   E: Recombination substrate vector pRK64 contains a SV40 promoter region followed by a 1.1 kb cassette consisting of the coding region of the puromycin resistance gene and a polyadenylation signal sequence, flanked 5' by the 84 bp attB and 3' by the 84 bp attP recognition site of C31-Int. pRK64 contains in addition two Cre recognition (IoxP) sites in direct orientation next to the att sites.

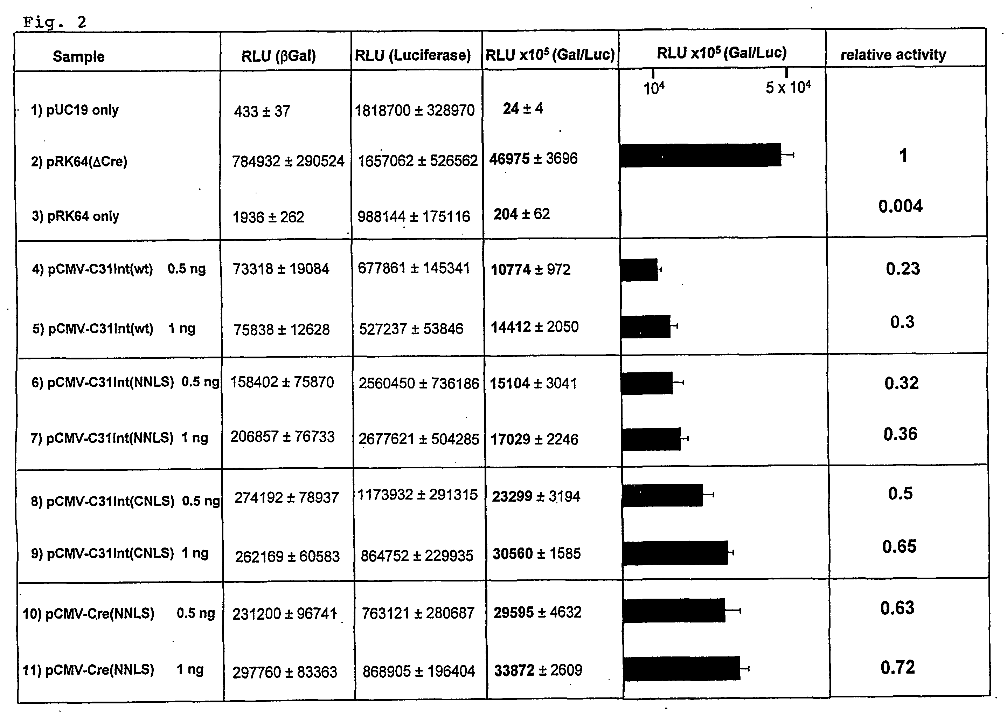

Figure 2 shows results of transient transfections of C31-Int and Cre recombinase and reporter vectors into CHO cells.
   All transfections were performed with a fixed amount of the reporter plasmid pRK64 and 0.5 ng or 1 ng of the recombinase expression plasmids pCMV-C31-Int(wt) (samples 4-5), pCMV-C31-Int(NNLS) (samples 6-7), pCMV-C31-Int(CNLS) (samples 8-9) or pCMV-Cre (samples 10-11). Negative controls: transfection with pRK64 (sample 3) or pUC19 alone (sample 1). Positive control: transfection with the Cre-recombined reporter pRK64(ΔCre) (sample 2).
   The vertical rows show the mean values and standard deviation of "Relative Light Units" obtained from lysates with the assay for β-galactosidase (RLU (β-Gal)), the RLU from the assay for Luciferase, the ratio of the β-galactosidase and Luciferase values with standard deviation (RLU x 10⁵ (Gal/Luc)), and the relative activity of the various recombinases as compared to the positive control defined as 1.
Figure 3 shows results of transient transfections of XisA and Ssv recombinases and reporter vectors into CHO cells.
   All transfections were performed with fixed amounts of the reporter plasmids pPGKnif (for XisA) and pPGKattA (for SSV) and 25 ng or 100 ng of the recombinase expression plasmids pCMV-XisA, pCMV-XisA(NNLS) and 10 ng or 20 ng of the expression plasmids pCMV-Ssv and pCMV-Ssv(NNLS). Negative controls: transfection with pPGKnif or pPGKattA alone.
   The vertical rows show the mean values and standard deviation of "Relative Light Units" obtained from lysates with the assay for β-galactosidase (RLU (β-Gal)), the RLU from the assay for Luciferase, the ratio of the β-galactosidase and "Luciferase" values with standard deviation (RLU x 10⁵ (Gal/Luc)).
Figure 4 shows results of transient transfections of recombinase vectors into a stable reporter cell line.
   All transfections were performed with a NIH 3T3 derived clone containing stably integrated copies of the pRK64 recombination substrate vector. Either 32 ng or 64 ng of the recombinase expression plasmids pCMV-C31-Int(wt) (samples 2-3), pCMV-C31-Int(NNLS) (samples 4-5), pCMV-C31-Int(CNLS) (samples 6-7) or pCMV-Cre(NNLS) (samples 8-9). Negative control: transfection with pUC19 alone (sample 1).
   The vertical rows show the mean values and standard deviation of "Relative Light Units" obtained from lysates with the assay for β-galactosidase (RLU (β-Gal)) and the relative activity of the various recombinases as compared to the value obtained with pCMV-Cre(NNLS) defined as 1.

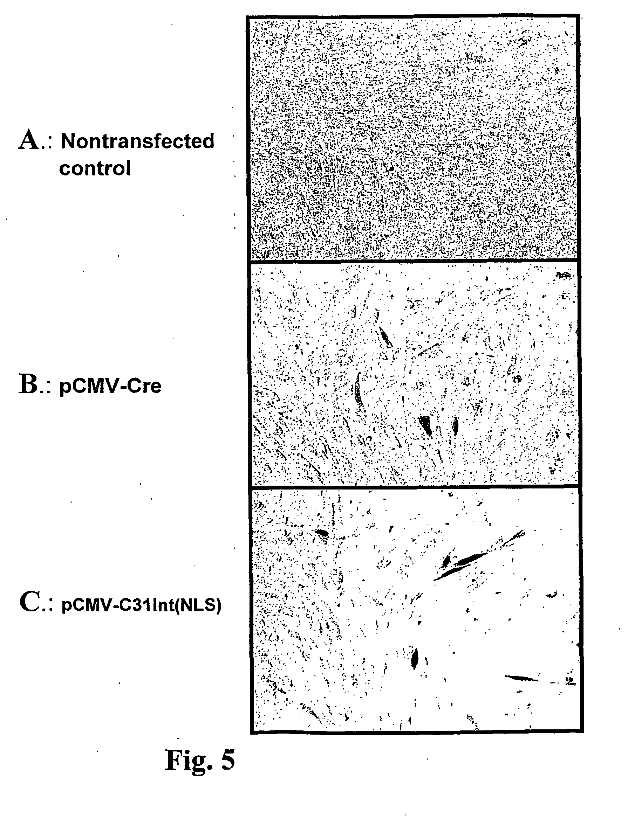

Figure 5 shows the in situ detection of β-galactosidase in 3T3(pRK64)-3 cells transfected with recombinase expression vectors.
   The Cre and C31-Int recombinase reporter cell line 3T3(pRK64)-3 was either not transfected with DNA (A), transfected with the Cre expression vector pCMV-Cre (B) or with the C31-Int expression vector pCMV-C31-Int(CNLS). Two days after tranfection the cells were fixed and incubated with the histochemical X-Gal assay which develops a blue stain in β-galactosidase positive cells indicating recombinase mediated activation of the reporter gene.

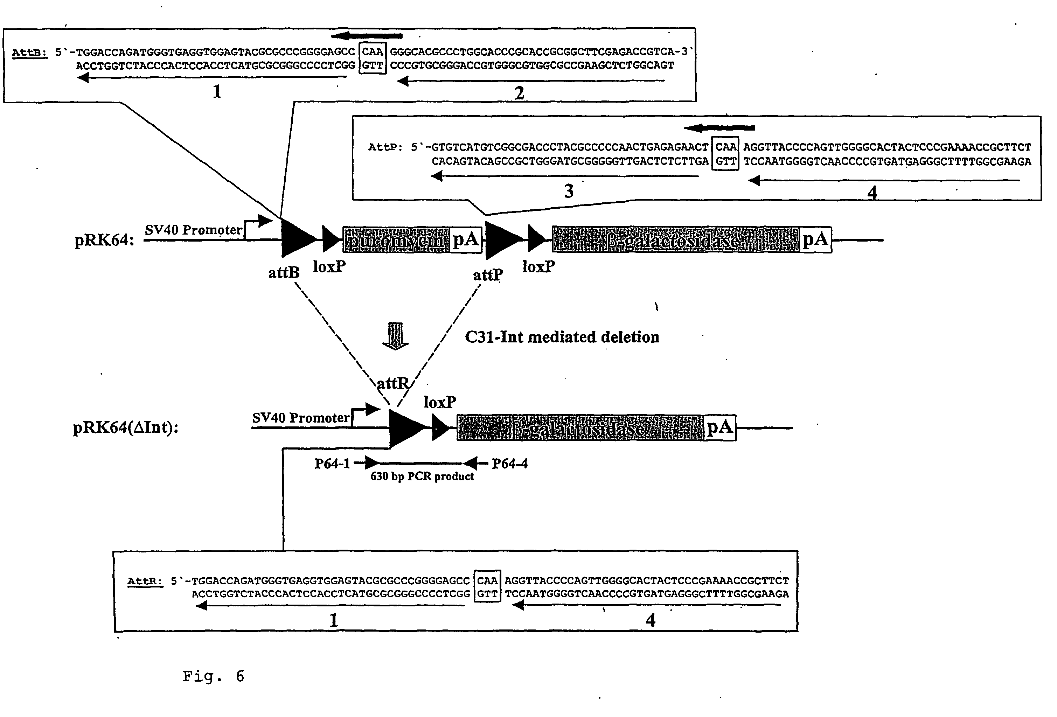

Figure 6 shows the test vector for C31-Int mediated deletion, pRK64, and the expected product of deletion, pRK64(ΔInt).
   Plasmid pRK64 contains the 1.1 kb cassette of the coding region of the puromycin resistance gene and a polyadenylation signal, which is flanked 5' by the 84 bp attB and 3' by the 84 bp attP recognition site (large triangles) of C31-Int. These attB and attP sites are oriented in the same way to each other (thick black arrows) which is used by the ΦX31 phage to integrate into the bacterial genome. In addition, the cassette is flanked by two Cre recombinase recognition (IoxP) sites in the same orientation (black small triangles). For better orientation the half sites of the att sequences are labelled by a direction (thin arrow) and numbered 1-4. The 3 bp sequence within the att sites at which recombination occurs is framed by a box. The positions at which the PCR primers P64-1 and P64-4 hybridise to the pRK64 vector are indicated by arrows, pointing into the 3'direction of both oligonucleotides.
   PRK64(ΔInt) depicts the deletion product expected from the C31-Int mediated recombination between the att sites of pRK64. The recombination between a pair of attB/attP sites generates an attR site remaining on the parental DNA molecule while the puromycin cassette is excised. In this configuration the primers P64-1 and P64-4 will amplify a PCR product of 630 bp from pRK64(ΔInt).

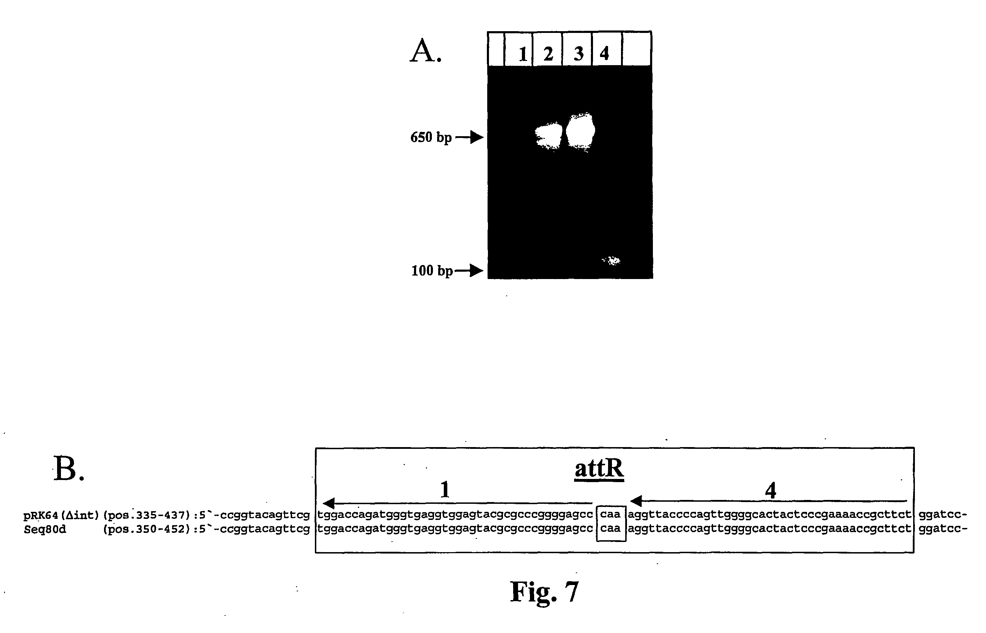

Figure 7 shows PCR products generated with the primers P64-1 and P64-4 and a sequence comparison of the PCR product.
   A: Analysis of PCR products on an agarose gel from PCR reactions using the Primers P64-1 and P64-4 on DNA extracted from MEF5-5 cells transfected 2 days before with plasmid pRK64 alone (lane 4), with pRK64 + CMV-Cre (lane 3), with pRK64 + pCMV-C31-Int(wt) (lane 2), and from a control reaction which did not contain cellular DNA (lane 1). The product with an apparent size around 650 bp, as compared to the size marker used, from lane 2 was excised from the agarose gel and purified. The PCR product was cloned into a sequencing plasmid vector and gave rise to the plasmid pRK80d. The insert of this plasmid was sequenced using reverse primer (seq80d) and compared to the predicted sequence of the pRK64 vector after C31-Int mediated deletion of the att flanked cassette, pRK64(ΔInt). The cloned PCR product shows a 100% identity with the predicted attR sequence after deletion. The generated attR site is shown in a box, with the same sequence designation used in Figure 5. The nucleotide positions (pos.) of the compared sequences pRK64(ΔInt) and Seq80d are indicated.

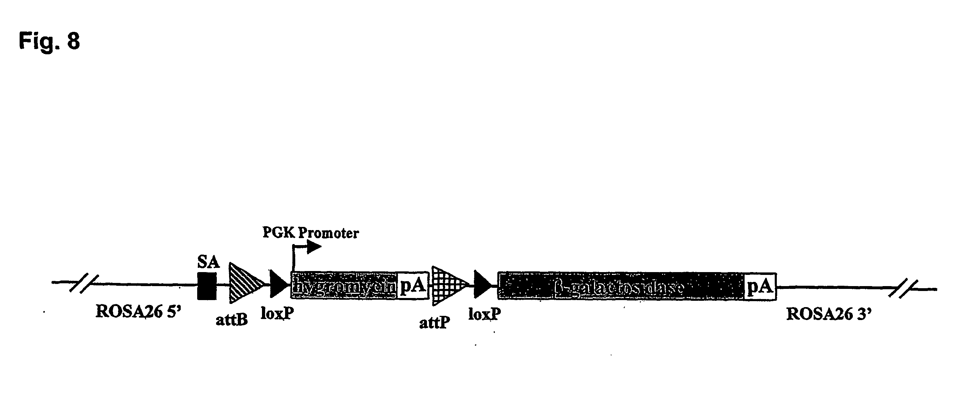

Figure 8 shows the modified ROSA26 locus of C31 reporter mice (Seq ID NO:106). A recombination substrate has been inserted in the ROSA26 locus. The substate consists of a splice acceptor (SA) followed by a cassette consisting of the hygromycin resistance gene driven by a PGK promoter and flanked by the recombination sites attB and attP. In addition the reporter contains two Cre recognition sites (IoxP) in direct orientation next to the att sites. This cassette is followed by the coding region for β-galactosidase, which is only expressed when the hygromycin resistance gene has been deleted by recombination.

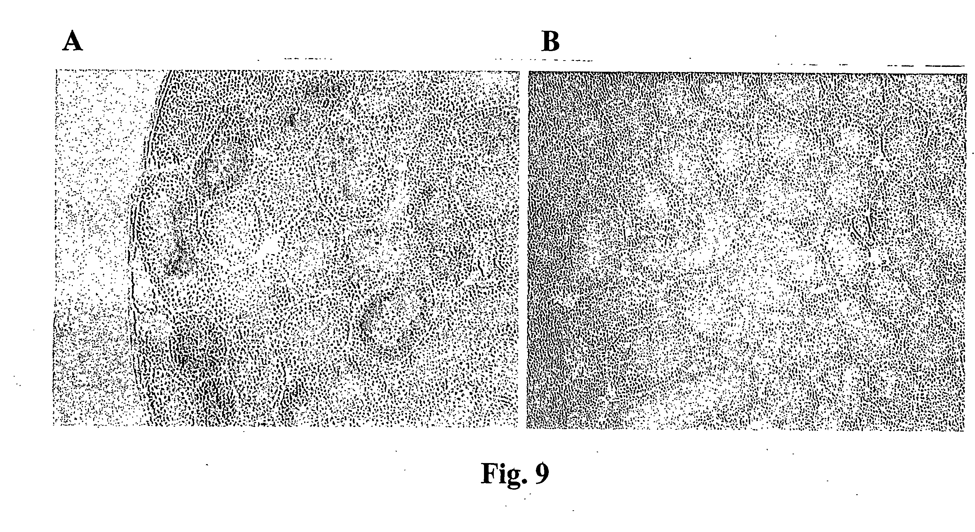

Figure 9 shows the in situ detection of β-galactosidase activity. A cryosection of the testis of a double transgenic mouse carrying both the C31-int recombinase and the recombination substrate was stained with X-Gal (A). The blue colour indicates recombination of the substrate, which leads to the expression of β-galactosidase. As a control a cryosection of testis of a transgenic mouse carrying only the recombination substrate was stained with X-Gal (B).

The present invention is further illustrated by the following Examples.

### Examples

### Example 1

As compared to Cre recombinase the wildtype form of C31-Int exhibits a significantly lower recombination activity in mammalian cells which falls in the range of 10 - 40% of Cre, depending on the assay system used (see below). As a measure which may increase C31-Int efficiency in eukaryotic cells we designed mammalian expression vectors for N- or C-terminal fusion proteins of C31-Int with a peptide was designed which is recognised by the nuclear import machinery. The recombination efficiency obtained by this modified C31-Int recombinase in mammalian cells was compared side by side to the unmodified (wildtype) form of C31-Int and to Cre recombinase. For the quantification of recombinase activities the expression vectors were transiently introduced into a mammalian cell line together with a reporter vector which contains C31-Int and Cre target sites and leads to the expression of β-galactosidase upon recombinase mediated deletion of a vector segment flanked by recombinase recognition sites.

### A. Plasmid constructions:

Construction of the recombination test vectors pPGKnif and pPGKattA: first a nifD site (Haselkorn, Annu Rev.Genet. 26, 113-130 (1992)) generated by the annealing of the two synthetic oligonucleotides nifD3 (SEQ ID NO:89) and nifD4 (SEQ ID NO:90), was ligated into the BamHI restriction site of the vector PSV-Pax1 (Buchholz et al., Nucleic Acids Res., 24, 4256-4262 (1996)), 3' of its puromycin resistance gene and loxP site, giving rise to plasmid pPGKnifD3' (SEQ ID NO:79). Next, another nifD site, generated by the annealing of the two synthetic oligonucleotides nifD1 (SEQ ID NO:87) and nifD2 (SEQ ID NO:88), was ligated into the BstBI restriction site of plasmid pPGKnifD3', upstream of the puromycin resistance gene and loxP site, giving rise to plasmid pPGKnifD (SEQ ID NO:78). For pPGKattA (Muskhelishvili et al., Mol.Gen.Genet. 237, 334-342 (1993)) first a 352bp-fragment was amplified from genomic DNA from the thermophilic bacterium Sulfolobus shibatae (DSM-5389, DSMZ Braunschweig-Deutsche Sammlung von Mikroorganismen und Zellkulturen GmbH, Mascheroder Weg 1b, D-38124 Braunschweig, Germany) with oligonucleotides SSV5 (SEQ ID NO:96) and SSV6 (SEQ ID NO:97) including restriction sites for BamHI and BstBI. The amplified fragment was cloned into the BamHI site of the vector PSV-Pax1 giving rise to plasmid pPGKattA1 (SEQ ID NO:82), subsequently the same 352 bp-fragment was cloned into the BstBI site of pPGKattA1 giving rise to the plasmid pPGKattA2 (SEQ ID NO:83). The sequence and orientation of both nifD sites and attA sites was confirmed by DNA sequence analysis. In pPGKnifD/pPGKattA2 the newly cloned nifD/attA sites (positions 535-619 and 1722-1787/ positions 6718-7081 and 12-363) are in the same orientation flanking the puromycin resistance gene and the SV40 early polyadenylation sequence. The nifD/attA sites are followed by loxP sites in the same orientation (positions 623 - 656 and 1794 - 1827/ positions 7085-7118 and 369-402). The puromycin cassette is transcribed from the SV40 early enhancer/promoter region and followed by the coding region for E. coli β-galactosidase and the SV40 late region polyadenylation sequence.

Construction of XisA and SSV expression vectors: First the XisA gene of cyanobacterium PCC7120 was amplified by PCR from genomic DNA from Nostoc strain PCC7120 (CNCM-Collection Nationale de Cultures de Microorganismes, Institut Pasteur, Paris) using the primers XisA1 (SEQ ID NO:84) and XisA3 (SEQ ID NO:86), and XisA1 (SEQ ID NO:84) and XisA2 (SEQ ID NO:85) (with NLS). The ends of the PCR product were digested with NotI and the product was ligated into plasmid pBluescript II KS, opened with NotI, giving rise to plasmids pRK42a and pRK43 (with NNLS). The DNA sequence of the insert was determined and found to be identical to the published XisA sequence (Genbank GI:3953452) apart from four silent point mutations. The XisA gene was isolated as a 1.4 kb fragment from pRK42a and pRK43 by digestion with NotI and ligated into the generic mammalian expression vector pRK50 (see below), opened with NotI, giving rise to the XisA expression vectors pCMV-XisA (SEQ ID NO:76) and pCMV-XisA(NNLS) (SEQ ID NO:77). pCMV-XisA(wt) contains a Cytomegalovirus immediated early gene promoter (position 1 - 616), a 240 bp hybrid intron (position 716 - 953), the XisA gene (position 974 - 2392), and a synthetic polyadenylation sequence (position 2413 - 2591).
The SSV gene was amplified from genomic DNA from the thermophilic bacterium Sulfolobus shibatae (DSM-5389, DSMZ Braunschweig- Deutsche Sammlung von Mikroorganismen und Zellkulturen GmbH, Mascheroder Weg 1b, D-38124 Braunschweig, Germany) in two PCR steps because of an internal attP sequence. First, two overlapping PCR fragments were created with the oligonucleotides SSV1-1 (SEQ ID NO:91) (or SSV1-2 (SEQ ID NO:92) for the SSV(NNLS) gene) and SSV2 (SEQ ID NO:93) and oligonucleotides SSV3 (SEQ ID NO:94) and SSV4 (SEQ ID NO:95). Using these overlapping fragments as template, a 1000bp fragment containing the complete SSV coding sequence was amplified with primers SSV1-1 (or SSV1-2 for the SSV(NNLS) gene) and SSV4. The 5' 620 bp-fragments of these PCR products were isolated by digestion with NotI-XhoI and cloned into vector pBluescript II KS giving rise to plasmids pRK47 and pRK48 (with NLS). The 3' 380 bp fragment generated by XhoI-digestion was cloned into the XhoI restriction site of vector pBluescript II KS giving rise to the plasmid pBS-SSVs (SEQ ID NO:72). The 380bp SSV-fragment was then isolated by digestion of pBS-SSVs with XhoI and ligated into pRK47 and pRK48 opened by XhoI giving rise to plasmids pBS-SSV3 (SEQ ID NO:70) and pBS-SSV4 (SEQ ID NO:71) (with NLS) containing the complete SSV gene. Sequencing of the plasmids confirmed one point mutation in both plasmids. Therefore 312 bp/ 91 bp fragments generated by digestion with EcoRV-SmaI/ EcoRV-XhoI of another clone of pRK47 were exchanged in plasmids pBS-SSV3/ pBS-SSV4. Sequences were confirmed by sequencing. The SSV gene was isolated from pRK47 and pRK48 by digestion with NotI and KpnI and ligated into the generic mammalian expression vector pRK50 (see below), opened with NotI and SalI, giving rise to the SSV expression vectors pCMV-SSV(wt) (SEQ ID NO:74) and pCMV-SSV(NNLS) (SEQ ID NO:75).

Construction of the recombination test vector pRK64: first an attB site (Thorpe et al. Proc. Natl. Acad. Sci. USA, 95, 5505 - 5510 (1998)), generated by the annealing of the two synthetic oligonucleotides C31-4 (SEQ ID NO:1) and C31-5 (SEQ ID NO:2), was ligated into the BstBI restriction site of the vector PSV-Pax1 (Buchholz et al., Nucleic Acids Res., 24, 4256-4262 (1996)), 5' of its puromycin resistance gene and Ioxp site, giving rise to plasmid pRK52. The sequence and orientation of the cloned attB site was confirmed by DNA sequence analysis. Next, an attP site site (Thorpe et al. Proc. Natl. Acad. Sci. USA, 95, 5505 - 5510 (1998)), generated by the annealing of the two synthetic oligonucleotides C31-6 (SEQ ID NO:3) and C31-7-2 (SEQ ID NO:4), was ligated into the BamHI restriction site of plasmid pRK52, downstream of the puromycin resistance gene and loxP site, giving rise to plasmid pRK64 (SEQ ID NO:5). The sequence and orientation of the attP site was confirmed by DNA sequence analysis. In pRK64 the newly cloned attB (position 348 - 431) and attP (position 1534 - 1617) sites are in the same orientation flanking the puromycin resistance gene and the SV40 early polyadenylation sequence. The attB and attP sites are followed by IoxP sites in the same orientation (positions 435 - 469 and 1624 - 1658). The puromycin cassette is transcribed from the SV40 early enhancer/promoter region and followed by the coding region for E. coli β-galactosidase and the SV40 late region polyadenylation sequence.

Construction of C31-Int expression vectors: First the C31-Int gene of phage ΦC31 was amplified by PCR from phage DNA (DSM-49156, DSMZ-Deutsche Sammlung von Mikroorganismen und Zellkulturen GmbH, Mascheroder Weg 1b, D-38124 Braunschweig, Germany) using the primers C31-1 (SEQ ID NO:6) and C31-3 (SEQ ID NO:7). The ends of the PCR product were digested with NotI and the product was ligated into plasmid pBluescript II KS, opened with NotI, giving rise to plasmid pRK40. The DNA sequence of the 1.85 kb insert was determined and found to be identical to the published C31-Int gene (Kuhstoss et al., J. Mol. Biol. 222, 897-908 (1991)), except for an error in the stop codon. This error was repaired by PCR amplification of a 300 bp fragment from plasmid pRK40 using the primers C31-8 (SEQ ID NO:8) and C31-9 (SEQ ID NO:9), which provide a corrected Stop codon. The ends of this PCR fragment were digested with Eco47III and XhoI, the fragment was ligated into plasmid pRK40 and opened with Eco47III and XhoI to remove the fragment containing the defective stop codon. The resulting plasmid pRK55 contains the correct C31-Int gene as confirmed by DNA sequence analysis.
The C31-Int gene was isolated from pRK55 as 1.85 kb fragment by digestion with NotI and XhoI and ligated into the generic mammalian expression vector pRK50 (see below), opened with NotI and XhoI, giving rise to the C31-Int expression vector pCMV-C31-Int(wt). pCMV-C31-Int(wt) (SEQ ID NO:10) contains a 700 bp cytomegalovirus immediated early gene promoter (position 1 - 700), a 270 bp hybrid intron (position 701 - 970), the C31-Int gene (position 978 - 2819), and a 189 bp synthetic polyadenylation sequence (position 2831 - 3020).
For the construction of pCMV-C31-Int(NNLS) a 1.5 kb fragment was amplified by PCR from phage DNA using oligonucleotides C31-2 (SEQ ID NO:98) and C31-3 (SEQ ID NO:7). The ends of the PCR product were digested with NotI and the product was ligated into plasmid pBluescript II KS, opened with NotI, giving rise to plasmid pRK41 (SEQ ID NO: 99). A 1100 bp fragment generated by digestion of plasmid pRK41 with NcoI and NotI was then ligated into plasmid pRK55 (SEQ ID NO:80), opened with NcoI and NotI, giving rise to the plasmid pRK63 (SEQ ID NO:81). The C31-Int gene with N-terminal NLS was isolated as a 1.8 kb fragment from pRK63 by digestion with NotI and XhoI and ligated into the mammalian expression vector pRK50, opened with NotI and XhoI, giving rise to the C31-Int expression vector pCMV-C31-Int(NNLS). pCMV-C31-Int(NNLS) (SEQ ID NO:73) contains a 700 bp Cytomegalovirus immediated early gene promoter (position 1 - 700), a 270 bp hybrid intron (position 701 - 970), the C31-Int gene with N-terminal NLS (position 976 - 2838), and a 189 bp synthetic polyadenylation sequence (position 2851 - 3040).
For the construction of pCMV-C31-Int(CNLS), the 3'-end of the C31-Int gene was amplified from pCMV-C31-Int(wt) as a 300 bp PCR fragment using the primers C31-8 (SEQ ID NO:8) and C31-2-2 (SEQ ID NO:11). Primer C31-2-2 modifies the 3'-end of the wildtype C31-Int gene such that the stop codon is replaced by a sequence of 21 basepairs coding for the SV40 T-antigen nuclear localisation sequence of 7 amino acids (Prolin-Lysin-Lysin-Lysin-Arginin-Lysin-Valin) (Kalderon et. al, Cell, 39, 499 - 509 (1984)), followed by a new stop codon. The ends of this 300 bp PCR fragment were digested with with Eco47III and XhoI, the fragment was ligated into plasmid pCMV-C31-Int(wt) and opened with Eco47III and XhoI to replace the 3'-end of the wildtype C31-Int gene resulting in the plasmid pCMV-C31-Int(CNLS). The identity of the new gene segment was verified by DNA sequence analysis. pCMV-C31-Int(CNLS) (SEQ ID NO:12) contains a 700 bp cytomegalovirus immediated early gene promoter (position 12 - 711), a 270 bp hybrid intron (position 712 - 981), the modified C31-Int gene (position 989 - 2851), and a 189 bp synthetic polyadenylation sequence (position 2854 - 3043).

To generate the Cre expression plasmid pCMV-Cre (SEQ ID NO:13), the coding sequence of Cre recombinase (Sternberg et al., J. Mol. Biol., 187, 197 - 212 (1986)) with a N-terminal fusion of the 7 amino acid SV40 T-antigen NLS (see above) was recovered from plasmid pgk-Cre and cloned into the NotI and XhoI sites of plasmid pRK50. PRK50 (SEQ ID NO:14) is a generic expression vector for mammalian cells based on the cloning vector pNEB193 (New England Biolabs Inc, Beverly, MA, USA). PRK50 was built by insertion into pNEB193 of a 700 bp cytomegalovirus immediated early gene (CMV-IE) promoter (position 1-700) from plasmid pIREShyg (GenBank#U89672; Clontech Laboratories Inc, Palo Alto, CA, USA), a synthetic 270 bp hybrid intron (position 701-970), consisting of a adenovirus derived splice donor and an IgG derived splice acceptor sequence (Choi et al., Mol. Cell. Biol., 11, 3070 - 3074 (1991)), and a 189 bp synthetic polyadenylation sequence (position 1000-1188) build from the polyadenylation consensus sequence and 4 MAZ polymerase pause sites (Levitt et al., Genes&Dev., 3, 1019 - 1025 (1989); The EMBO J. 13, 5656 - 5667 (1994)). The positive control plasmid pRK64(ΔCre) (SEQ ID NO:15) was generated from pRK64 by transformation into the Cre expressing E. coli strain 294-Cre (Buchholz et al., Nucleic Acids Res., 24, 3118 - 3119 (1996)).

One of the transformed subclones was confirmed for the Cre mediated deletion of the IoxP-flanked cassette by restriction mapping and further expanded. Plasmid pUC19 is a cloning vector without eukaryotic control elements used to equalise DNA amounts for transfections (GenBank#X02514; New England Biolabs Inc, Beverly, MA, USA). All plasmids were propagated in DH5α E. coli cells (Life Technologies GmbH, Karlsruhe, Germany) grown in Luria-Bertani medium and purified with the plasmid DNA purification reagents "Plasmid-Maxi-Kit" (Quiagen GmbH, Hilden, Germany) or "Concert high purity plasmid purification system" (Life Technologies GmbH, Karlsruhe, Germany). Following purification, the plasmid DNA concentrations were determined by absorption at 260 nm and 280 nm in UVette cuvettes (Eppendorf-Netheler-Hinz GmbH, Hamburg, Germany) using a BioPhotometer (Eppendorf-Netheler-Hinz GmbH, Hamburg, Germany) and the plasmids were diluted to the same concentration; finally these were confirmed by separation of 10 ng of each plasmid on an ethidiumbromide-stained agarose gel.
B. Cell culture and transfections: Chinese hamster ovary (CHO) cells (Puck et al., J. Exp. Med., 108, 945 (1958)) were obtained from the Institute for Genetics (University of Cologne, Germany) as a population adapted to growth in DMEM medium. The cells were grown in DMEM/Glutamax medium (Life Technologies) supplemented with 10% fetal calf serum at 37°C, 10% CO₂ in humid atmosphere and passaged upon trypsinisation. One day before transfection 10⁶ cells were plated into a 48-well plate (Falcon). For the transient transfection of cells with plasmids each well received into 250 ml of medium a total amount of 300 ng supercoiled plasmid DNA complexed before with the FuGene6 transfection reagent (Roche Diagnostics GmbH, Mannheim, Germany) according to the manufacturers protocol. Each 300 ng DNA preparation (Fig.2 sample 4 to 11) contained 50 ng of the luciferase expression vector pUHC13-1 (Gossen et al., Proc Natl Acad Sci USA., 89 5547-5551 (1992)), 50 ng of the substrate vector pRK64, 0.5 ng or 1 ng of one of the recombinase expression vectors pCMV-C31Int(wt), pCMV-C31Int(NNLS), pCMV-C31Int(CNLS) or pCMV-Cre and 199 ng or 199.5 ng of pUC19 plasmid, except for the controls which received 50 ng of pUHC13-1 together with 50 ng of pRK64 (sample 3) or pRK64(Δcre) (sample 2) and 200 ng pUC19, or 50 ng pUHC13-1 with 250 ng pUC19 (sample 1). Transfections of Ssv and XisA recombinases (Fig.3) also contained 50 ng of the luciferase expression vector pUHC13-1, 50 ng of substrate vectors pPGKattA and pPGKnif and 10 ng or 20 ng of recombinase expression vector pCMV-SSV or pCMV-SSV(NNLS) or 25 ng or 100 ng of expression vectors pCMV-XisA/ pCMV-XisA(NNLS). Plasmid pUC19 was added to a total amount of 300 ng plasmid DNA. As the C31-Int expression vectors are 15% larger in size than pCMV-Cre and the same amounts of DNA of the three plasmids were used for transfection, the samples with C31-Int vectors received 15% less plasmid molecules as compared to the samples with Cre expression vector. The β-galactosidase values from C31-Int transfected samples by this value were not corrected and thus is a slight underestimation of the calculated C31-Int activities. For each sample to be tested four individual wells were transfected. One day after the addition of the DNA preparations each well received additional 250 ml of growth medium. The cells of each well were lysed 48 hours after transfection with 100 ml lysate reagent supplemented with protease inhibitors (Roche Diagnostics). The lysates were centrifuged and 20 ml were used to determine the β-galactosidase activities using the β-galactosidase reporter gene assay (Roche Diagnostics) according to the manufacturers protocol in a Lumat LB 9507 luminometer (Berthold). To measure luciferase activity, 20ml lysate was diluted into 250ml assay buffer (50mM glycylglycin, 5mM MgCl₂, 5mM ATP) and the "Relative Light Units" (RLU) were counted in a Lumat LB 9507 luminometer after addition of 100 ml of a 1 mM luciferin (Roche Diagnostics) solution. The mean value and standard deviation of the samples was calculated from the β-galactosidase and luciferase RLU values obtained from the four transfected wells of each sample.
C. Results: To set up an assay system for the measurement of C31-Int and Cre recombinase efficiency in mammalian cells the recombination substrate vector pRK64 shown in Figure 1E was first constructed. pRK64 contains a SV40 promoter region for expression in mammalian cells followed by a 1.1 kb cassette which consists of the coding region of the puromycin resistance gene and a polyadenylation signal sequence. This cassette is flanked at the 5'-end by the 84 bp attB and at the 3'-end by the 84 bp attP recognition site of C31-Int (Fig.1 and 6). These attB and attP sites are located on the same DNA molecule and oriented in a way to each other which allows the deletion of the flanked DNA segment. The same orientation of attB and attP sites is used naturally by the ΦC31 phage and the bacterial genome, leading to the integration of the phage genome when both sites are located on different DNA molecules (Thorpe et al., Proc. Natl. Acad. Sci. USA, 95, 5505 - 5510 (1998)). To measure C31-Int and Cre recombinase activities with the same substrate vector, pRK64 contains in addition two Cre recognition (loxP) sites in direct orientation next to the att sites. Since the att/lox-flanked cassette in plasmid pRK64 is inserted between the SV40 promoter and the coding region of the β-galactosidase gene, its presence inhibits β-galactosidase expression as the SV40 promoter derived transcripts are terminated at the polyadenylation signal of the puromycin gene. Plasmid pRK64 is turned into a β-galactosidase expression vector upon C31-Int or Cre mediated deletion of the att/lox-flanked puromycin cassette since the remaining single att and loxP site do not substantially interfere with gene expression.

For the expression of recombinases a mammalian expression vector was designed which contains the CMV immediate early promoter followed by a hybrid intron, the coding region of the recombinase to be tested, and an artificial polyadenylation signal sequence. The backbone sequence of the four recombinase expression vectors shown in Figure 1A-D is identical to each other except for the recombinase coding region. Plasmid pCMV-C31Int(wt) (Fig. 1A) contains the nonmodified (wildtype) 1.85 kb coding region of C31-Int as found in the genome of phage ΦC31 (Kuhstoss, et al., J. Mol. Biol. 222, 897-908 (1991)). Plasmid pCMV-C31Int(NNLS) (Fig. 1B) contains a modified C31-Int gene coding for the full length C31-Int protein with a N-terminal extension of 7 amino acids derived from the SV40 virus large T antigen which serves as a nuclear localisation signal (NLS). Plasmid pCMV-C31Int(CNLS) (Fig. 1C) contains a C-terminal extension of 7 amino acids derived from the SV40 virus large T antigen which serves as a nuclear localisation signal (NLS). Plasmid pCMV-Cre (Fig. 1D) contains the 1.1 kb Cre coding region with an N-terminal fusion of the 7 amino acid NLS of the SV40 T-antigen. For Cre recombinase it has been shown that the N-terminal addition of the SV40 T-antigen NLS does not increase its recombination efficiency in mammalian cells (Le et al., Nucleic Acids Res., 27, 4703 - 4709 (1999)).

As a test system to compare the efficiency of the 4 recombinases the same amount of plasmid DNA of each of the recombinase expression vectors together with a fixed amount of the reporter plasmid pRK64 was transiently introduced into Chinese Hamster Ovary (CHO) cells. Thus, in this assay design the efficiency of the various recombinases on an extrachromosomal substrate introduced into the CHO cells was compared as a circular plasmid. Two days after transfection the cells from the various samples were lysed and the activity of β-galactosidase in the lysates was determined by a specific chemiluminescense assay and expressed in "Relative Light Units" (RLU (β-Gal)) (Fig. 2). In addition all samples contained a fixed amount of a luciferase expression vector to control for the experimental variation of cell transfection and lysis. For this purpose the lysates of each sample were also tested for luciferase activity with a specific chemiluminescense assay and the values expressed as "Relative Light Units" (RLU (Luciferase)) (Fig. 2). All transfection samples contained in addition varying amounts of the unrelated cloning plasmid pUC19 so that all samples were equalised to the same amount of plasmid DNA. As a positive control for β-galactosidase a derivative of the recombination reporter pRK64 was used in which the IoxP flanked 1.1 kb cassette has been removed through Cre mediated recombination in E. coli giving rise to plasmid pRK64(ΔCre). As negative controls served samples which received the unrecombined reporter plasmid pRK64 but no recombinase expression vector as well as samples set up with the pUC19 plasmid alone.

To determine the relative efficiency of the tested recombinases the RLU values of β-galactosidase were divided individually for each sample by the RLU values obtained for luciferase and multiplied with 10⁵. From the values of the four data points of each sample the mean value and standard deviation was calculated as an indicator of recombinase activity (Gal/Luc) (Fig. 2). The relative activity of the tested recombinases was then compared to the positive control defined as an activity of 1.

As shown in Fig. 2, the expression of Cre recombinase (samples 10 and 11) resulted in a 150 to 170-fold increase of β-galactosidase activity as compared to the negative control (sample 3), demonstrating the wide dynamic range of our test system. Each recombinase vector was tested using two different amounts of DNA for transfection (0.5 and 1ng/sample), which in the case of Cre resulted in 63% and 72% recombinase activity (samples 10 and 11 as compared to the positive control). These two values establish that the DNA amounts used are close to the test systems saturation for recombinase expression as the doubling of DNA amounts resulted only in a minor increase of recombinase activity.

In comparison to Cre, the expression of wildtype C31-Int resulted in a considerably lower recombinase activity of 23% and 30% (Fig. 2, samples 4 and 5) as compared to the positive control. These values represent 37% and 42% recombinase activity for wildtype C31-Int as compared to Cre recombinase (compare samples 4 and 5 with 10 and 11). Upon the expression of C31-Int fused with the N-terminal NLS (C31-Int(NNLS)) values of 32% and 36% recombinase activity (samples 6 and 7) were obtained as compared to the positive control. The C31-Int(NNLS) values represent 51% and 50% recombinase activity as compared to Cre (compare samples 6 and 7 to 10 and 11). Thus, the activity of C31-Int in mammalian cells is just moderately enhanced by the addition of a NLS signal.
Surprisingly, upon the expression of C31-Int fused with the C-terminal NLS (C31-Int(CNLS)) values of 50% and 65% recombinase activity (samples 8 and 9) were obtained as compared to the positive control. The C31-Int(CNLS) values represent 79% and 90% recombinase activity as compared to Cre recombinase (compare samples 84 and 9 to 10 and 11). Unexpectedly,C31-Int(CNLS) exhibits a dramatic, more than twofold increase of recombinase activity in comparison to C31-Int(wt) (compare samples 8 and9 to 4 and 5).

In order to test whether the addition of a NLS sequence may be a general, simple method to enhance recombinase activity in mammalian cells we extended our studies by two additional recombinases: XisA recombinase (XisA) derived from the cyanobacterium Anabaena, and SSV-Integrase (SSV-Int) derived from the SSV1 virus of the thermophilic bacterium Sulfolobus shibatae. To this end we constructed mammalian expression vectors for the wildtype forms of XisA and SSV recombinases and compared their activity to versions which were modified by the N-terminal addition of the 7 amino acid NLS of the SV40 T-antigen. These recombinases were compared by the use of the reporter vector shown in Fig.1E, except that the att elements of C31-Int were replaced by the nif recognition sequences for XisA or the att sequences for SSV-Int. As described above for C31-Int, recombinase activities were tested by transient transfection into CHO cells using the reporter vector derived β-galactosidase activity as readout and cotransfected luciferase as internal control.
As shown in Fig.3 for both, XisA and SSV recombinases the addition of a NLS sequence did not improve their activity in a mammalian cell line as compared to the wildtype forms. At both DNA concentrations tested wildtype XisA exhibits a significant recombination activity as compared to the reporter vector alone (compare samples 2 and 3 to sample 1), but this activity is not altered by the addition of an NLS (compare samples 2 and 3 to samples 4 and 5). SSV-Int exhibits only a low recombination activity (compare samples 7 and 8 with sample 6) which is also not enhanced by the addition of a NLS (compare samples 9 and 10 with samples 7 and 8). From these results we conclude that the addition of a NLS to an inefficient recombinase is not a general, simple method to improve its performance in mammalian cells.

Taken together, in the transient transfection test system shown in Figure 2 a more than twofold activity increase of the ΦC31 Integrase could be achieved by the C-terminal, but not the N-terminal addition of the SV40 T antigen NLS signal. As this signal sequence has been characterised to act as a nuclear localisation signal (Kalderon et. al, Cell, 39, 499 - 509 (1984)) we conclude that the efficiency increase of C31-Int(CNLS) is the result of the improved nuclear accumulation of this recombinase. The relative inefficiency of C31-Int (NNLS) may be explained by the inaccessibility of the NLS peptide to the nuclear import machinery at the N-terminal position of the C31-Int protein.
In particular, it could be shown that C31-Int(CNLS) recombines extrachromosomal DNA in mammalian cells almost as efficient as the widely used Cre recombinase and thus provides an additional or alternative recombination system of highest activity. The efficiency increase of C31-Int(CNLS) as compared to its wildtype form is regarded as an invention of substantial use for biotechnology.

### Example 2

As demonstrated in example 1 C31-Int recombinase with the C-terminal fusion of the SV40 T-antigen NLS (C31-Int(CNLS)) shows in mammalian cells a recombination activity comparable to Cre recombinase on an extrachromosomal plasmid vector. It was further tried to test whether C31-Int(CNLS) exhibits a similar activity on a recombination substrate which is chromosomally integrated into the genome of mammalian cells. This question is critical for the use of a recombination system for genome engineering as it is possible that a recombinase may act efficiently on extrachromosomal substrates but is impaired if the recognition sites are part of the mammalian chromatin. To characterise the recombination activity of C31-Int(CNLS) and C31-Int(NNLS) on a chromosomal substrate the pRK64 reporter plasmid (Fig. 1E) was stably integrated, containing a pair of IoxP and att sites, into the genome of a mammalian cell line. One of the stable transfected clones was chosen for further analysis and was transiently transfected with recombinase expression vectors coding for C31-Int(CNLS), C31-Int(NNLS), C31-Int(wt) or Cre recombinase. The activity of β-galactosidase derived from the Cre expression vector recombined in these cells was taken as a measure of recombination efficiency.
A. Plasmid constructions: all plasmids used and their purification are described in example 1.
B. Cell culture and transfections: To generate a stably transfected C31-Int reporter cell line 2.5 x 10⁶ NIH-3T3 cells (Andersson et al., Cell, 16, 63-75 (1979); DSMZ#ACC59; DSMZ-Deutsche Sammlung von Mikroorganismen und Zellkulturen GmbH, Mascheroder Weg 1b, D-38124 Braunschweig, Germany) were electroporated with 5 µg pRK64 plasmid DNA linearised with ScaI and plated into 10cm petri dishes. The cells were grown in DMEM/Glutamax medium (Life Technologies) supplemented with 10% fetal calf serum at 37°C, 10% CO₂ in humid atmosphere, and passaged upon trypsinisation. Two days after tranfection the medium was supplemented with 1mg/ml of puromycin (Calbiochem) for the selection of stable integrants. Upon the growth of resistant colonies these were isolated under a stereomicroscope and individually expanded in the absence of puromycin. To demonstrate stable integration of the transfected vector, genomic DNA of puromycin resistant clones was prepared according to standard methods and 5-10 µg were digested with EcoRV. Digested DNA was separated in a 0.8% agarose gel and transferred to nylon membranes (GeneScreen Plus, NEN DuPont) under alkaline conditions for 16 hours. The filter was dried and hybridised for 16 hours at 65°C with a probe representing the 5' part of the E. coli β-galactosidase gene (1.25 kb NotI - EcoRV fragment of plasmid CMV-β-pA (R. Kühn, unpublished). The probe was radiolabelled with P32-marked α-dCTP (Amersham) using the Megaprime Kit (Amersham). Hybridisation was performed in a buffer consisting of 10% dextranesulfate, 1% SDS, 50 mM Tris and 100 mM NaCl, pH7.5). After hybridisation the filter was washed with 2x SSC/1%SDS and exposed to BioMax MS1 X-ray films (Kodak) at - 80°C.
   Transfections of the selected clone 3T3(pRK64)-3 with plasmid DNAs and the measurement of β-galactosidase activities in lysates were essentially performed as described in example 1 for CHO cells, except that 32ng or 64ng of the recombinase expression plasmids and 218 or 186 ng of pUC19 plasmid were used and the pRK64 plasmid was omitted from all samples.
C. Histochemical detection of β-galactosidase activity in transfected 3T3(pRK64)-3 cells
   To directly demonstrate β-galactosidase expression in recombinase transfected cells, 10⁴ 3T3(pRK64)-3 cells were plated one day before transfection into each well of a 48-well tissue culture plate (Falcon). For the transient transfection of cells with plasmids each well received into 250 µl of medium a total amount of 150 ng supercoiled plasmid DNA complexed before with the FuGene6 transfection reagent (Roche Diagnostics GmbH, Mannheim, Germany) according to the manufacturers protocol. Each 150 ng DNA preparation contained 50 ng of the recombinase expression vector pCMV-Cre or pCMV-C31Int(CNLS) and 100ng of the pUC19 plasmid. After 2 days the culture medium was removed from the wells, the wells were washed once with phosphate buffered saline (PBS), and the cells were fixed for 5 minutes at room temperature in a solution of 2% formaldehyde and 1% glutaraldehyde in PBS. Next, the cells were washed twice with PBS and finally incubated in X-Gal staining solution for 24 hours at 37°C (staining solution: 5 mM K₃(Fe(CN)₆), 5 mM K₄(Fe(CN)₆), 2 mM MgCl₂, 1mg/ml X-Gal (BioMol) in PBS) until photographs were taken.
D. Results
   To generate a mammalian cell clone with a stable genomic integration of the C31-Int and Cre recombinase reporter plasmid pRK64, the murine fibroblast cell line NIH-3T3 was electroporated with linearised pRK64 DNA (Fig.1D; see also example 1) and subjected to selection in puromycin containing growth medium. Plasmid pRK64 contains in between the pair of loxP and att sites the coding region of the puromycin resistance gene expressed from the SV40-IE promoter. Thirty-six puromycin resistant clones were isolated and the genomic DNA of 19 clones was analysed for the presence and copy number of the pRK64 DNA. Three clones, which apparently contain 2 - 4 copies of pRK64, were further characterised on the single cell level for the expression of β-galactosidase upon transient transfection with the Cre expression vector pCMV-Cre (Fig. 1C). The cell clone with the largest proportion of β-galactosidase positive cells, 3T3(pRK64)-3, was selected as most useful for the planned studies on C31-Int and Cre recombinase efficiency.

To compare the efficiency of wildtype C31-Int (C31-Int(wt)), C31-Int(CNLS), C31-Int(NNLS), and Cre recombinases 32ng or 64 ng of the recombinase expression vectors pCMV-C31Int(wt), pCMV-C31Int(CNLS), pCMV-C31Int(NNLS), or pCMV-Cre (Fig. 1 A-D) together with the unrelated cloning plasmid pUC19 were transiently introduced into 3T3(pRK64)-3 cells, such that all samples contained the same amount of plasmid DNA. As a negative control a sample prepared with the pUC19 plasmid alone was used. Two days after transfection the cells from the various samples were lysed and the activity of β-galactosidase in the lysates was determined by a specific chemiluminescense assay and expressed in "Relative Light Units" (RLU)(β-Gal) (Fig. 4). From the values of the four data points of each sample the mean value and standard deviation was calculated as an indicator of recombinase activity (Fig.4). The relative activity of the tested recombinases was then compared to the highest value obtained with the Cre expression vector, defined as an activity of 1.

As shown in Figure 4 the expression of Cre recombinase (samples 8 and 9) resulted in a 36 to 49-fold increase of β-galactosidase activity as compared to the negative control (sample 1), demonstrating the dynamic range of the test system used. Each recombinase vector was tested using two different amounts of DNA for transfection (32 ng and 64 ng/sample), which in the case of Cre resulted in 73% and 100% recombinase activity (samples 8 and 9). These two values establish that the DNA amounts used are not far from the linear scale of the test systems ability for recombinase expression as the twofold increase of the amount of DNA also resulted in a significant increase of recombinase activity.

The expression of wildtype C31-Int (Fig. 4, samples 2 and 3) resulted in a low recombinase activity of 4% and 10% as compared to the values obtained by Cre transfection (compare samples 2 and 3 with 8 and 9). This activity was only moderately enhanced by the expression of C31-Int(NNLS) to values of 19% and 22% of Cre activity (compare samples 4 and 5 with samples 8 and 9). Upon the expression of C31-Int(CNLS) values of 48% and 78% recombinase activity were obtained as compared to Cre recombinase (compare samples 6 and 7 to 8 and 9). Hence, C31-Int(CNLS) exhibits an 12-fold higher activity than C31-Int(wt) at 32 ng plasmid DNA (Fig.4, compare samples 6 and 2) and an 8-fold higher activity than C31-Int(wt) at 64 ng plasmid DNA (Fig.4, compare samples 7 and 3).

In addition, it was aimed to directly demonstrate in situ the expression of β-galactosidase in 3T3(pRK64)-3 cells after transfection with Cre or C31-Int(CNLS) recombinase plasmid. Two days after transfection the cells were fixed in situ and incubated with the histochemical X-Gal assay which detects β-galactosidase positive cells by a blue precipitate. As shown in Figure 5 stained cells were found at a comparable frequency in the samples transfected with the Cre or C31-Int(CNLS) expression vectors but not in the nontransfected control. This result confirms that the β-galactosidase activities measured by chemiluminescense upon recombinase transfection (Fig. 4) results from a population of individual, recombined reporter cells.

In conclusion, upon the transient transfection of recombinase expression vectors into a cell line with a genomic integration of the recombination substrate vector, a 8 - 12-fold activity increase of the ΦC31 Integrase by the C-terminal fusion with the SV40 T-antigen NLS signal was found. As this signal sequence has been characterised to act as a nuclear localisation signal (Kalderon et. al, Cell, 39, 499 - 509 (1984)), it was concluded that the dramatic efficiency increase of C31-Int(CNLS) is the result of the improved nuclear accumulation of this recombinase. The approximately tenfold activity increase of C31-Int(CNLS) upon expression in a cell line with a genomic integration of the substrate vector is considerably higher than the activity increase found upon the transient expression of both vectors (see example 1). Thus, a substrate vector integrated into the chromatin of a mammalian cell may pose more stringent requirements on recombinase activity to be recombined as compared to an extrachromosomal substrate.

The dramatic activity increase of C31-Int(CNLS), as compared to its wildtype form, on a stable integrated substrate in mammalian cells is an invention of significant practical use as this recombinase is as efficient as the widely used Cre/loxP system; thus, C31-Int(CNLS) provides an additional or alternative recombination system of highest activity.

### Example 3

To demonstrate that the increase in β-galactosidase activity obtained by the cotransfection of a C31-Int expression vector and the reporter vector pRK64 into mammalian cells is in fact the result of recombinase mediated deletion, one of the recombination products was detected by a specific polymerase chain reaction (PCR). The amplified PCR product was cloned and its sequence determined. The obtained sequence confirms that C31-Int mediated deletion of the test vector occurs in a mammalian cell line and that the recombination occurs at the known breakpoint within the attB and attP sites.
A. Plasmid constructions: The construction of plasmids pRK64, pCMV-Cre and pCMV-C31-Int(wt) is described in Example 1. To simulate the recombination of pRK64 by C31-Int, the sequence between the CAA motives of the att sites (boxed in Fig.5) was deleted from the computerfile of pRK64, giving rise to the sequence of pRK64(ΔInt) (SEQ ID NO:16).
B. Transfection of Cells and PCR amplification: MEF5-5 mouse fibroblasts (Schwenk et al., 1998) (20000 cells per well of a 12 well plate (Falcon)) were transfected with 0.5 µg pRK64 alone or together with 250 ng pCMV-Int(wt) or pCMV-Cre using the FuGene6 transfection reagent following the manufacturers protocol (Roche Diagnostics). After 2 days DNA was extracted from these cells according to standard methods and used for PCR amplification with Primers P64-1 (SEQ ID NO:17; complementary to position 111-135 of pRK64(ΔInt)) and P64-4 (SEQ ID NO:18; complementary to position 740-714 of pRK64(ΔInt)) using the Expand High Fidelity PCR kit (Roche Diagnostics). PCR products were separated on a 0.8% agarose gel, extracted with the QuiaEx kit (Quiagen) and cloned into the pCR2.1 vector using the TA cloning kit (Invitrogen) resulting in plasmid pRK80d. The sequence of its insert, seq80d (SEQ ID NO:19), was determined using the reverse sequencing primer and standard sequencing methods (MWG Biotech).
   For the measurement of β-galactosidase activity the cells were lysed 2 days after transfection and the β-galactosidase activities were determined with the β-galactosidase reporter gene assay (Roche Diagnostics) as described in example 1.
C. Results: As a test vector for C31-Int mediated DNA recombination plasmid pRK64 was used, which contains the 1.1 kb coding region of the puromycin resistance gene flanked 5' by the 84 bp attB and 3' by the 84 bp attP recognition site of C31-Int (Fig. 5). These attB and attP sites are located on the same DNA molecule and oriented in a way to each other which allows the deletion of the att-flanked DNA segment. The same orientation of attB and attP sites is used naturally by the ΦC31 phage and the bacterial genome for the integration of the phage genome when both sites are located on different DNA molecules (Thorpe et al., Proc. Natl. Acad. Sci. USA, 95, 5505 - 5510 (1998)). As a positive control, vector pRK64 contains in addition two Cre recombinase recognition (IoxP) sites in direct orientation next to the att sites. Since the att-flanked DNA segment in plasmid pRK64 is inserted between a promoter active in mammalian cells and the β-galactosidase gene, its deletion can be measured by the increase of β-galactosidase activity. The expected product of C31-Int mediated deletion of plasmid pRK64 is shown in Fig. 6, designated as pRK64(ΔInt). If the recombination between attB and attP occurs as described in bacteria (Thorpe et al., Proc. NatI. Acad. Sci. USA, 95, 5505 - 5510 (1998)), a single attR site is generated and left on the parental plasmid (Fig. 6) while the flanked DNA is excised and contains an attL site. Beside the measurement of β-galactosidase activity, C31-Int mediated recombination of pRK64 can be directly detected on the DNA level by a specific polymerase chain reaction (PCR) using the primers P64-1 and P64-4 (Fig. 6). These primers, located 5' of the attB site (P64-1) and 3' of the attP site, are designed to amplify a PCR product of 630 bp lenght upon the C31-Int mediated recombination of pRK64. For the expression of C31-Int in mammalian cells plasmid pCMV-C31(wt) was used, which contains the CMV-IE-Promoter upstream of the C31-Int coding region followed by a synthetic polyadenylation signal (see Example 1 and Fig.1).
   The recombination substrate vector pRK64 was transiently transfected into the murine fibroblast cell line MEF5-5 either alone, or together with the C31-Int expression vector pCMV-C31(wt), or together with an expression vector for Cre recombinase, pCMV-Cre. Two days after transfection half the cells of each sample was lysed and used to measure β-galactosidase activity by chemiluminescense, and the other half was used for the preparation of DNA from the transfected cells for PCR analysis. The β-galactosidase levels of the 3 samples were found as following (expressed as "Relative Light Units" (RLU) with standard deviation (SD) of the β-galactosidase assay):

| Sample | | RLU (SD) |
|---|---|---|
| 1) | pRK64 | 692 ± 5 |
| 2) | pRK64 + pCMV-Cre | 8527 ± 269 |
| 3) | pRK64 + pCMV-C31(wt) | 1288 ± 93 |

As the coexpression of the test vector pRK64 together with the C31-Int expression vector in sample 3 leads to a significant increase of β-galactosidase activity as compared to pRK64 alone, this result suggests that pRK64 is recombined by C31-Int as anticipated in Fig. 6.

Next, cellular DNA was prepared from the three samples and tested for the occurrence of the expected Cre or C31-Int generated deletion product by PCR using primers P64-1 and P64-4 for amplification. As shown in Fig. 7 an amplification product of the expected size was found only in the samples cotransfected with the Cre or C31-Int recombinase expression vectors (Fig. 7A, lane3 and lane 4). The PCR products amplified from pRK64 recombined by C31-Int or Cre are of the same size but should be recombined via the attB/P or loxP sites, respectively.
To prove that the PCR product found after cotransfection of plasmids pRK64 and pCMV-C31(wt) represents in fact the deletion product of C31-Int mediated recombination, this DNA fragment was cloned into a plasmid vector and its DNA sequence determined. One clone, pRK80d, was analysed, and its sequence showed exactly the sequence of an attR site as expected from C31-Int mediated deletion of pRK64 (Fig. 7B, compare to Fig. 6).

In conclusion, this experiment demonstrates that C31-Int mediated deletion of a vector containing a pair of attB/attP sites occurs in a mammalian cell line, and that the recombination occurs within the same 3 bp breakpoint region of attB and attP as found in bacteria (Thorpe et al., Proc. Natl. Acad. Sci. USA, 95, 5505 - 5510 (1998)). Thus, it was concluded that an increase of β-galactosidase activity observed by cotransfection of the pRK64 reporter vector and a C31-Int expression vector in mammalian cells truly reflects C31-Int recombinase activity.

### Example 4

As has been demonstrated in examples 1-3, the C31-Int recombinase with the C-terminal fusion of the SV40 T-antigen NLS (C31-Int(CNLS)) shows a recombination activity comparable to Cre recombinase on an extrachromosomal as well as a chromosomally integrated target in mammalian cells in vitro. To test whether C31-Int(CNLS) exhibits activity in mice, transgenic mice carrying a C31-Int(CNLS) expression vector were generated. These transgenic mice were crossed with reporter mice carrying the recombinase substrate. Recombination-mediated expression of β-galactosidase, which can be measured by staining with the substrate X-Gal, was analyzed in testes of double transgenic progeny carrying both the recombinase and the reporter.
A. Plasmid constructions: For the construction of the C31-Int(CNLS) transgene expression vector, the C31Int gene with C-terminal NLS was isolated as a 2 kb-fragment generated by restriction of pCMV-C31Int(CNLS) (SEQ ID NO: 12) with BglII. The fragment was ligated into the BglII restriction site of the vector pCAGGS-Cre-pA (SEQ ID NO:104) giving rise to the plasmid pCAGGS-C31CNLS-pA (SEQ ID NO:105). In pCAGGS-C31CNLS-pA the C31-Int(CNLS) (position 1891-3753) is transcribed from the CAGGS promoter (position 1-1616) and followed by the SV40 late region polyadenylation sequence (position 3763-3941).
B. Production of transgenic mice: For the embryo injections a 3.95 kb-fragment was generated by restriction of the plasmid pCAGGS-C31CNLS-pA with PstI and AscI. This fragment was purified as follows: DNA bands were separated on an agarose-gel without ethidiumbromide. One part of the gel was stained with ethidiumbromide to locate the band to excise. The DNA was electroeluted from the excised band with S&S Biotrap Elution Chamber in 1x TAE (40 mM Tris-acetate, 1 mM EDTA) overnight. The DNA was precipitated from the eluate with 1/10 volume 3M sodium acetate and 2.5 volumes ethanol at -20 °C for several hours. The DNA was pelleted by centrifugation at 13000 rpm for 30 min and washed twice with 70 % ethanol. The dried DNA pellet was resuspended in TE (10 mM Tris, 1 mM EDTA, pH 8). Subsequently the precipitation procedure was repeated once and the DNA resuspended in injection buffer (10 mM Tris pH 7.2, 0.1 mM EDTA ). The sample was dialysed with Slide-A-Lyse Mini Dialysis Unit (Pierce) in injection buffer with several changes of buffer at 4°C overnight.
   Different amounts of the sample were checked on a gel to determine concentration. To generate transgenic mice, 5-10 fg of the purified fragment were injected into one pronucleus of (B6CBA)F2 mouse one-cell embryos. The injected embryos were subsequently transferred into the oviduct of 0.5 day pseudopregnant NMRI females.
C. Analysis of transgenic mice: Mice were analyzed for the presence of the pCAGGS-C31CNLS-pA transgene by PCR using tail DNA and the primers C31-screen 1 (SEQ ID NO:100) and C31-screen 2 (SEQ ID NO:101) amplifying a fragment of 500 bp. The PCR reaction contained 5 µl PCR buffer (Invitrogen), 2 µl 50 mM MgCl₂, 1.5 µl 10 mM dNTP-mix, 2 µl (10 pmol) of each primer, 0.5 µl Taq-polymerase (5 U/ µl) and water to a volume of 50 µl. The program used for the PCR reactions was: 94 °C for 30 s, 55 °C for 30 s and 72 °C for 1 min in 30 cycles.
D. Analysis of C31-Int(CNLS) activity: Founder mice transgenic for the pCAGGS-C31CNLS-pA transgene were crossed to heterozygous C31 reporter mice carrying the C31 reporter construct in the ROSA26 locus (SEQ ID NO:106) (Fig. 8). Offspring of the crosses were genotyped for the presence of the pCAGGS-C31CNLS-pA transgene by the PCR assay described in section C as well as for the ROSA26-C31 reporter allele by a LacZ-specific PCR assay. The PCR was performed using tail DNA and the primers β-Gal 3 (SEQ ID NO:102) and β-Gal 4 (SEQ ID NO:103) amplifying a fragment of 315 bp. The PCR reaction contained 5 µl PCR buffer (Invitrogen), 2.5 µl 50 mM MgCl₂, 2 µl 10 mM dNTP-mix, 1 µl (10 pmol) of each primer, 0.4 µl Taq-polymerase (5 U/ µl) and water to a volume of 50 µl. The program used for the PCR reactions was: 94 °C for 1 min, 60 °C for 1 min and 72 °C for 1 min in 30 cycles.
   Testes from mice carrying the pCAGGS-C31CNLS-pA transgene as well as the reporter locus and from a control mouse carrying the reporter allele only were dissected. The tissues were imbedded in OCT Tissue freezing medium (Leica/Jung) and frozen in liquid nitrogen. Cryosections were generated from the embedded tissues using a Leica CM3050 cryomicrotome, dried on polylysine-coated slides for 1-4 hours and then stained as follows: Sections were fixed in 0.2 % glutaraldehyde, 5 mM EGTA, 2 mM MgCl₂ in 0.1 M PB (K₂HPO₄/ KH₂PO₄, pH 7.3) for 5 min at room temperature and washed in wash buffer (2 mM MgCl₂, 0.02 % Nonidet-40 in PB in 0.1 M PB) 3 times for 15 min. Then sections were stained in X-Gal-solution (0.6 mg/ ml X-Gal in DMSO, 5 mM potassium hexacyanoferrat III, 5 mM potassium hexacyanoferrat II in LacZ wash buffer) overnigth at 37 °C. After staining sections were washed in 1x PBS twice for 5 min. Dehydration was performed by washing the sections first with 70 %, 96 % and 100 % ethanol for 2 min each, then with a 1:1 mix of ethanol and xylol for 5 min and in the end only with xylol for 5 min. Before taking pictures sections were mounted in Entellan.
E. Results: To identify transgenic founder mice carrying the pCAGGS-C31CNLS-pA transgene, 29 mice born from the injection experiment were analyzed for the presence of the transgene. 5 founder mice (3 females and 2 males) were identified. To analyze the activity of the C31-Int(CNLS) recombinase in transgenic mice, 2 of the female founder mice were crossed to heterozygous C31 reporter mice carrying a C31 reporter construct in the ROSA26 locus (Fig. 8). From each of these crosses, one offspring carrying the pCAGGS-C31CNLS-pA transgene as well as the C31 reporter allele was sacrificed. In oder to determine whether pCAGGS-C31CNLS-pA transgenic mice are able to delete an attB/P flanked DNA sequence in the mouse germline, tissue sections from the testes of the sacrificed animals were prepared and stained for β-galactosidase activity with X-Gal. Fig. 9 shows the result of the staining experiment for one of these mice (A) as well as a control mouse carrying only the reporter allele, but lacking the pCAGGS-C31CNLS-pA transgene (B). Clear staining can be detected in the maturing sperm cells in about 50% of the tubules with the proportion of β-galactosidase expressing cells ranging between 10 and 100. No staining could be detected for the control mouse. This clearly demonstrates that C31-int-mediated recombination has taken place during spermatogenesis in the pCAGGS-C31CNLS-pA transgenic mice. These results show that the C31-int is functional in vivo, in a transgenic mouse system and therefore provides a new tool to introduce specific deletions, inversions or integrations into the mouse germline.

## Claims

1. A fusion protein comprising
(a) a recombinase domain comprising a recombinase protein being a bacteriophage ΦC31 integrase (C31-Int) protein or a mutant thereof having a recombinase activity similar to that of the corresponding wild-type recombinase and
(b) a signal peptide domain linked to said recombinase domain which directs nuclear import of said fusion protein in eukaryotic cells.

2. The fusion protein of claim 1, wherein the recombinase protein comprises a C31-Int having the amino acid sequence shown in SEQ ID NO:21 or a C-terminal truncated form thereof, said truncated form of the C31-Int preferably comprising amino acid residues of 306 to 613 of SEQ ID NO:21.

3. The fusion protein according to claim 1 or 2, wherein the signal peptide domain is derived from yeast GAL4, SKI3, L29 or histone H2B proteins, polyoma virus large T protein, VP1 or VP2 capsid protein, SV40 VP1 or VP2 capsid protein, adenovirus E1a or DBP protein, influenza virus NS1 protein, hepatitis virus core antigen or the mammalian lamin, c-myc, max, c-myb, p53, c-erbA, jun, Tax, steroid receptor or Mx proteins, SV40 T-antigen or other proteins with known nuclear localisation, preferably the signal peptide domain comprises a peptide which is derived from the SV40 T-antigen.

4. The fusion protein according to any one of claims 1 to 3, wherein the signal peptide domain
(i) has a length of 5 to 74, preferably 7 to 15 amino acid residues, and/or
(ii) comprises a segment of 6 amino acid residues having at least 2 positively charged basic amino acid residues, said basic amino acid residues being preferably selected from lysine, arginine and histidine.

5. The fusion protein of claim 3 or 4, wherein the signal peptide domain comprises a peptide selected from a sequence shown in SEQ ID NOs:24 to 53, preferably the signal peptide comprises the amino acid sequence Pro-Lys-Lys-Lys-Arg-Lys-Val (SEQ ID NO:53).

6. The fusion protein according to any one of claims 1 to 5, wherein
(i) the signal peptide domain is linked to the N-terminal or C-terminal of the recombinase domain or is integrated into the recombinase domain, preferably the signal peptide domain is linked to the C-terminal of the recombinase domain; and/or
(ii) the signal peptide domain is linked to the recombinase domain directly or through a linker peptide, said linker preferably having 1 to 30 essentially neutral amino acid residues.

7. The fusion protein of claim 1 comprising the amino acid sequence shown in SEQ ID NO:23.

8. A DNA coding for the fusion protein according to any one of claims 1 to 7.

9. A vector containing the DNA as defined in claim 8.

10. A microorganism containing the DNA of claim 8 and/or the vector of claim 9.

11. A process for preparing the fusion protein as defined in any one of claims 1 to 7 which comprises culturing a microorganism as defined in claim 10 under conditions suitable for expression of said fusion protein and recovering said fusion protein.

12. Use of the fusion protein as defined in any one of claims 1 to 7 to recombine DNA molecules, which contain recombinase recognition sequences for the recombinase protein of the recombinase domain, in eukaryotic cells.

13. A cell, preferably a mammalian cell containing the DNA sequence of claim 8 in its genome.

14. The cell of claim 13, also containing recognition sequences for the recombinase protein of the recombinase domain in its genome.

15. Use of the cell of claim 13 or 14 for studying the function of genes and for the creation of transgenic non-human organisms.

16. A transgenic non-human organism, preferably a transgenic non-human mammal containing the DNA sequence of claim 8 in its genome.

17. The transgenic organism of claim 16 also containing recognition sequences for the recombinase protein of the recombinase domain in its genome.

18. Use of the transgenic organism of claim 16 or 17 for studying gene function at various developmental stages.

19. An *in vitro* method for recombining DNA molecules of cells containing recombinase recognition sequences for the recombinase protein of the recombinase domain as defined in claims 1 to 7, which method comprises supplying the cells with a fusion protein as defined in claims 1 to 7 or with a DNA sequence of claim 8 and/or a vector of claim 9 which are capable of expressing said fusion protein in the cell.

20. Use of a fusion protein as defined in claims 1 to 7, or a DNA sequence of claim 8 and/or a vector of claim 9 which are capable of expressing said fusion DNA protein in an organism, for preparing an agent for recombining DNA molecules in organisms containing recombinase recognition sequences for said recombinase protein.

21. An *in vitro* method for recombining a DNA molecule containing recognition sequences for a recombinase protein in a eukaryotic cell, said method comprising contacting the cell with a fusion protein according to claim 1 that recognizes said recognition sequences, wherein the fusion protein catalyzes recombination of the DNA molecule.

22. The fusion protein according to any one of claims 1 to 7 which catalyzes recombination at recognition sequences for the recombinase protein.

23. A transgenic non-human organism, preferably a transgenic non-human mammal, comprising a cell containing a DNA sequence coding for a recombinase fusion protein as defined in claims 1 to 7 and 22 in its genome.

## Patentansprüche

1. Fusionsprotein, umfassend:
(a) eine Rekombinase-Domäne, die ein Rekombinase-Protein umfasst, bei dem es sich um ein Bakteriophagen-ΦC31-Integrase(C31-Int)-Protein oder eine Mutante davon handelt und das eine ähnliche Rekombinase-Aktivität wie die entsprechende Wildtyp-Rekombinase hat; und
(b) eine Signalpeptiddomäne, die mit der Rekombinase-Domäne verknüpft ist und das Fusionsprotein in eukaryontischen Zellen zum Import in den Zellkern lenkt.

2. Fusionsprotein gemäß Anspruch 1, wobei das Rekombinase-Protein ein C31-Int mit der in SEQ ID Nr. 21 gezeigten Aminosäuresequenz oder eine C-terminal verkürzte Form davon umfasst, wobei die verkürzte Form des C31-Int vorzugsweise die Aminosäurereste 306 bis 613 von SEQ ID Nr. 21 umfasst.

3. Fusionsprotein gemäß Anspruch 1 oder 2, wobei die Signalpeptiddomäne von Hefe-GAL4-, -SKI3-, -L29- oder -Histon-H2B-Proteinen, Polyomvirus-Large-T-Protein, -VP1- oder -VP2-Capsidprotein, SV40-VP1- oder -VP2-Capsidprotein, Adenovirus-E1a- oder -DBP-Protein, Influenzavirus-NS1-Protein, Hepatitisvirus-Kernantigen oder den Säuger-Lamin-, -c-myc-, -max-, -c-myb-, -p53-, -c-erbA-, -jun-, -Tax-, -Steroidrezeptor- oder -Mx-Proteinen, dem SV40-T-Antigen oder anderen Proteinen mit bekannter Lokalisierung im Zellkern abgeleitet ist und die Signalpeptiddomäne vorzugsweise ein Peptid umfasst, das vom SV40-T-Antigen abgeleitet ist.

4. Fusionsprotein gemäß einem der Ansprüche 1 bis 3, wobei die Signalpeptiddomäne:
(i) eine Länge von 5 bis 74, vorzugsweise 7 bis 15, Aminosäureresten hat; und/oder
(ii) ein Segment von 6 Aminosäureresten umfasst, das wenigstens 2 positiv geladene basische Aminosäurereste aufweist, wobei die basischen Aminosäurereste vorzugsweise aus Lysin, Arginin und Histidin ausgewählt sind.

5. Fusionsprotein gemäß Anspruch 3 oder 4, wobei die Signalpeptiddomäne ein Peptid umfasst, das aus einer in SEQ ID Nr. 24 bis 53 gezeigten Sequenz ausgewählt ist, und das Signalpeptid vorzugsweise die Aminosäuresequenz Pro-Lys-Lys-Lys-Arg-Lys-Val (SEQ ID Nr. 53) umfasst.

6. Fusionsprotein gemäß einem der Ansprüche 1 bis 5, wobei:
(i) die Signalpeptiddomäne mit dem N-Terminus oder C-Terminus der Rekombinase-Domäne verknüpft oder in die Rekombinase-Domäne integriert ist und die Signalpeptiddomäne vorzugsweise mit dem C-Terminus der Rekombinase-Domäne verknüpft ist; und/oder
(ii) die Signalpeptiddomäne direkt oder über ein Linkerpeptid mit der Rekombinase-Domäne verknüpft ist, wobei der Linker vorzugsweise 1 bis 30 im Wesentlichen neutrale Aminosäurereste aufweist.

7. Fusionsprotein gemäß Anspruch 1, das die in SEQ ID Nr. 23 gezeigte Aminosäuresequenz umfasst.

8. DNA, die für das Fusionsprotein gemäß einem der Ansprüche 1 bis 7 codiert.

9. Vektor, der die in Anspruch 8 definierte DNA enthält.

10. Mikroorganismus, der die DNA von Anspruch 8 und/oder den Vektor von Anspruch 9 enthält.

11. Verfahren zur Herstellung des Fusionsproteins gemäß einem der Ansprüche 1 bis 7, umfassend das Kultivieren eines Mikroorganismus, wie er in Anspruch 10 definiert ist, unter Bedingungen, die für die Expression des Fusionsproteins geeignet sind, und Gewinnen des Fusionsproteins.

12. Verwendung des Fusionsproteins gemäß einem der Ansprüche 1 bis 7, um DNA-Moleküle, die Rekombinase-Erkennungssequenzen für das Rekombinase-Protein der Rekombinase-Domäne enthalten, in eukaryontischen Zellen zu rekombinieren.

13. Zelle, vorzugsweise eine Säugerzelle, die die DNA-Sequenz von Anspruch 8 in ihrem Genom enthält.

14. Zelle gemäß Anspruch 13, die außerdem Erkennungssequenzen für das Rekombinase-Protein der Rekombinase-Domäne in ihrem Genom enthält.

15. Verwendung der Zelle von Anspruch 13 oder 14 zur Untersuchung der Funktion von Genen und zur Schaffung von transgenen nichthumanen Organismen.

16. Transgener nichthumaner Organismus, vorzugsweise ein transgener nichthumaner Säuger, der die DNA-Sequenz von Anspruch 8 in seinem Genom enthält.

17. Transgener Organismus gemäß Anspruch 16, der außerdem Erkennungssequenzen für das Rekombinase-Protein der Rekombinase-Domäne in seinem Genom enthält.

18. Verwendung des transgenen Organismus von Anspruch 16 oder 17 zur Untersuchung der Genfunktion in verschiedenen Stadien der Entwicklung.

19. In-vitro-Verfahren zum Rekombinieren von DNA-Molekülen von Zellen, die Rekombinase-Erkennungssequenzen für das Rekombinase-Protein der Rekombinase-Domäne, wie es in den Ansprüchen 1 bis 7 definiert ist, enthalten, wobei das Verfahren das Versorgen der Zellen mit einem Fusionsprotein, wie es in den Ansprüchen 1 bis 7 definiert ist, oder mit einer DNA-Sequenz von Anspruch 8 und/oder einem Vektor von Anspruch 9, die in der Lage sind, das Fusionsprotein in der Zelle zu exprimieren, umfasst.

20. Verwendung eines Fusionsproteins, wie es in den Ansprüchen 1 bis 7 definiert ist, oder einer DNA-Sequenz von Anspruch 8 und/oder eines Vektors von Anspruch 9, die in der Lage sind, das Fusionsprotein in einem Organismus zu exprlmieren, zur Herstellung eines Mittels zum Rekombinieren von DNA-Molekülen in Organismen, die Rekombinase-Erkennungssequenzen für das Rekombinase-Protein enthalten.

21. In-vitro-Verfahren zum Rekombinieren eines DNA-Moleküls, das Erkennungssequenzen für ein Rekombinase-Protein enthält, in einer eukaryontischen Zelle, wobei das Verfahren das In-Kontakt-Bringen der Zelle mit einem Fusionsprotein gemäß Anspruch 1, das die Erkennungssequenzen erkennt, umfasst, wobei das Fusionsprotein die Rekombination des DNA-Moleküls katalysiert.

22. Fusionsprotein gemäß einem der Ansprüche 1 bis 7, das die Rekombination an Erkennungssequenzen für das Rekombinase-Protein katalysiert.

23. Transgener nichthumaner Organismus, vorzugsweise ein transgener nichthumaner Säuger, der eine Zelle umfasst, die in ihrem Genom eine DNA-Sequenz enthält, welche für ein Rekombinase-Fusionsprotein codiert, wie es in den Ansprüchen 1 bis 7 und 22 definiert ist.

## Revendications

1. Protéine de fusion comprenant :
(a) un domaine recombinase comprenant une protéine de recombinase étant une protéine intégrase de bactériophage φC31 (C31-Int) ou un mutant de celle-ci ayant une activité de recombinase similaire à la recombinase correspondante de type sauvage et
(b) un domaine peptidique de signal lié audit domaine de recombinase qui dirige l'import nucléaire de ladite protéine de fusion dans des cellules eucaryotes.

2. La protéine de fusion de la revendication 1, dans laquelle la protéine de recombinase comprend un C31-Int ayant la séquence d'acides aminés de la SEQ ID :21 ou une forme tronquée au terminus C de celle-ci, ladite forme tronquée de la C31-Int comprenant de préférence des résidus d'acides aminés de 306 à 613 de la SEQ ID :21.

3. La protéine de fusion selon la revendication 1 ou 2, dans laquelle le domaine peptidique de signal est dérivé de la levure GAL4, SK13, L29 ou des protéines d'histone H2B, la protéine T large du virus de la polyoma, la protéine de capside VP1 ou VP2, la protéine de capside SV40 VP1 ou VP2, la protéine de l'adénovirus E1a ou DBP 37, la protéine du virus de la grippe NS1, l'antigène central du virus de l'hépatite ou lamine de mammifère, c-myc, max, c-myb, p53, c-erbA, jun, Tax, récepteur stéroïde de mammifere ou protéines Mx, ou l'antigène T SV40 ou toutes autres protéines avec une localisation nucléaire connue, de préférence le domaine peptidique de signal comprend un peptide qui est dérivé de l'antigène T SV40.

4. La protéine de fusion selon l'une quelconque des revendications 1 à 3, dans laquelle le domaine peptidique de signal
- a une longueur de 5 à 74, de préférence 7 à 15 acides aminés, et/ou
- comprend un segment de 6 résidus d'acides aminés ayant au moins 2 résidus d'acides aminés basiques positivement chargés, lesdits résidus d'acides aminés basiques étant de préférence sélectionnés dans le groupe consistant en la lysine, l'arginine et l'histidine.

5. La protéine de fusion selon la revendication 3 ou 4, dans laquelle le domaine peptidique de signal comprend un peptide sélectionné dans une séquence selon la SEQ ID :24 à 53, de préférence le peptide de signal comprend la séquence d'acides aminés Pro-Lys-Lys-Lys-Arg-Lys-Val (SEQ ID N° 53).

6. La protéine de fusion selon l'une quelconque des revendications 1 à 5, dans laquelle :
(i) le domaine peptidique de signal est lié au terminus N ou C du domaine recombinase ou est intégré dans le domaine recombinase, de préférence le domaine peptidique de signal est lié au terminus C du domaine recombinase ; et/ou
(ii) le domaine peptidique de signal est lié au domaine recombinase directement ou à travers un peptide de liaison, ledit peptide de liaison ayant de préférence 1 à 30 résidus d'acides aminés essentiellement neutres.

7. La protéine de fusion de la revendication 1, comprenant la séquence d'acides aminés de la SEQ ID N° 23.

8. L'ADN codant pour la protéine de fusion selon l'une quelconque des revendications 1 à 7.

9. Un vecteur contenant l'ADN tel que défini dans la revendication 8.

10. Un microorganisme contenant l'ADN de la revendication 8 et/ou le vecteur de la revendication 9.

11. Un procédé pour préparer la protéine de fusion définie dans l'une quelconque des revendications 1 à 7 qui comprend la culture d'un microorganisme telle que définie dans la revendication 10 dans les conditions adaptées pour l'expression de ladite protéine de fusion et le récupération de ladite protéine de fusion.

12. Utilisation de la protéine de fusion telle que définie dans l'une quelconque des revendications 1 à 7 pour recombiner des molécules d'ADN, qui contiennent des séquences de reconnaissance de recombinase pour la protéine recombinase du domaine recombinase, dans des cellules eucaryotes.

13. Une cellule, de préférence, une cellule de mammifere contenant la séquence d'ADN de la revendication 8 dans son génome.

14. La cellule de la revendication 13, contenant en outre une séquence de reconnaissance pour la protéine de recombinase du domaine recombinase dans son génome.

15. Utilisation de la cellule de la revendication 13 ou 14 pour l'étude de la fonction des gènes et pour la création d'organismes non-humains transgéniques.

16. Un organisme non-humain transgénique de préférence un mammifère non-humain transgénique contenant la séquence d'ADN de la revendication 8 dans son génome.

17. L'organisme transgénique de la revendication 16, contenant aussi des séquences de reconnaissance pour la protéine de recombinase du domaine recombinase dans son génome.

18. Utilisation d'un organisme transgénique de la revendication 16 ou 17 pour étudier la fonction d'un gène aux différentes étapes de développement.

19. Une méthode in vitro pour recombiner des molécules d'ADN de cellules contenant des séquences de reconnaissance de recombinase pour la protéine recombinase du domaine recombinase telle que définie dans les revendications 1 à 7, ladite méthode comprenant la fourniture aux cellules d'une protéine de fusion telle que définie dans les revendications 1 à 7 ou avec une séquence d'ADN selon la revendication 8 et/ou un vecteur selon la revendication 9 qui sont capables d'exprimer ladite protéine de fusion dans la cellule.

20. Utilisation d'une protéine de fusion telle que définie dans les revendications 1 à 7, ou une séquence d'ADN selon la revendication 8 et/ou un vecteur selon la revendication 9 qui sont capables d'exprimer ladite protéine d'ADN de fusion dans un organisme, pour préparer un agent pour recombiner des molécules d'ADN dans des organismes contenant des séquences de reconnaissance de recombinase pour ladite protéine recombinase.

21. Une méthode in vitro pour recombiner une molécule d'ADN contenant des séquences de reconnaissance pour une protéine de recombinase dans une cellule eucaryotique, ladite méthode comprenant le contact de la cellule avec une protéine de fusion selon la revendication 1 qui reconnaît lesdites séquences de reconnaissance, dans lesquelles la protéine de fusion catalyse la recombinaison de la molécule d'ADN.

22. La protéine de fusion selon l'une quelconque des revendications 1 à 7 qui catalyse la recombinaison à des séquences de reconnaissance pour la protéine de recombinase.

23. Un organisme transgénique non-humain de préférence un mammifere non-humain transgénique, comprenant une cellule contenant une séquence d'ADN codant pour la protéine de fusion recombinase telle que définie dans les revendications 1 à 7 et 22 dans son génome.


### SEQUENCE LISTING

<110> Artemis Pharmaceuticals GmbH
<120> Modified Recombinase
<130> 012787wo/JH/ml
<140>
   <141>
<160> 108
<170> PatentIn Ver. 2.1
<210> 1
   <211> 86
   <212> DNA
   <213> Artificial Sequence
<220>
   <223> Description of Artificial Sequence: primer C31-4
<400> 1
   cgtgacggtc tcgaagccgc ggtgcgggtg ccagggcgtg cccttgggct ccccgggcgc 60
   gtactccacc tcacccatct ggtcca 86
<210> 2
   <211> 86
   <212> DNA
   <213> Artificial Sequence
<220>
   <223> Description of Artificial Sequence: primer C31-5
<400> 2
   cgtggaccag atgggtgagg tggagtacgc gcccggggag cccaagggca cgccctggcc 60
   cacgcaccgc ggcttcgaga ccgtca 86
<210> 3
   <211> 90
   <212> DNA
   <213> Artificial Sequence
<220>
   <223> Description of Artificial Sequence: primer C31-6
<400> 3
   gatccagaag cggttttcgg gagtagtgcc ccaactgggg taacctttga gttctctcag 60
   ttgggggcgt agggtcgccg acatgacacg 90
<210> 4
   <211> 90
   <212> DNA
   <213> Artificial Sequence
<220>
   <223> Description of Artificial Sequence: primer C31-7-2
<400> 4
   gatccgtgtc atgtcggcga ccctacgccc ccaactgaga gaactcaaag gttaccccag 60
   ttggggcact actcccgaaa accgcttctg 90
<210> 5
   <211> 7438
   <212> DNA
   <213> Artificial Sequence
<220>
   <223> Description of Artificial Sequence: vector pRK64
<400> 5
   cgtcatcacc gaaacgcgcg aggcagctgt ggaatgtgtg tcagttaggg tgtggaaagt 60
   ccccaggctc cccagcaggc agaagtatgc aaagcatgca tctcaattag tcagcaacca 120
   ggctccccag caggcagaag tgtgcaaagc atgcatctca attagtcagc aaccatagtc 180
   ccgcccctaa ctccgcccat cccgccccta actccgccca gttccgccca ttctccgccc 240
   catggctgac taattttttt tatttatgca gaggccgagg ccgcctcggc ctaggaacag 300
   tcgacgacac tgcagagacc tacttcacta acaaccggta cagttcgtgg accagatggg 360
   tgaggtggag tacgcgcccg gggagcccaa gggcacgccc tggcacccgc accgcggctt 420
   cgagaccgtc acgaataact tcgtatagca tacattatac gaagttataa gcttgcatgc 480
   ctgcaggtcg gccgccacga ccggccggcc ggtgccgcca ccatcccctg acccacgccc 540
   ctgacccctc acaaggagac gaccttccat gaccgagtac aagcccacgg tgcgcctcgc 600
   cacccgcgac gacgtccccc gggccgtacg caccctcgcc gccgcgttcg ccgactaccc 660
   cgccacgcgc cacaccgtcg acccggaccg ccacatcgag cgggtcaccg agctgcaaga 720
   actcttcctc acgcgcgtcg ggctcgacat cggcaaggtg tgggtcgcgg acgacggcgc 780
   cgcggtggcg gtctggacca cgccggagag cgtcgaagcg ggggcggtgt tcgccgagat 840
   cggcccgcgc atggccgagt tgagcggttc ccggctggcc gcgcagcaac agatggaagg 900
   cctcctggcg ccgcaccggc ccaaggagcc cgcgtggttc ctggccaccg tcggcgtctc 960
   gcccgaccac cagggcaagg gtctgggcag cgccgtcgtg ctccccggag tggaggcggc 1020
   cgagcgcgcc ggggtgcccg ccttcctgga gacctccgcg ccccgcaacc tccccttcta 1080
   cgagcggctc ggcttcaccg tcaccgccga cgtcgagtgc ccgaaggacc gcgcgacctg 1140
   gtgcatgacc cgcaagcccg gtgcctgacg cccgccccac gacccgcagc gcccgaccga 1200
   aaggagcgca cgaccccatg gctccgaccg aagccgaccc gggcggcccc gccgaccccg 1260
   cacccgcccc cgaggcccac cgactctaga ggatcataat cagccatacc acatttgtag 1320
   aggttttact tgctttaaaa aacctcccac acctccccct gaacctgaaa cataaaatga 1380
   atgcaattgt tgttgttaac ttgtttattg cagcttataa tggttacaaa taaagcaata 1440
   gcatcacaaa tttcacaaat aaagcatttt tttcactgca ttctagttgt ggtttgtcca 1500
   aactcatcaa tgtatcttat catgtctgga tccgtgtcat gtcggcgacc ctacgccccc 1560
   aactgagaga actcaaaggt taccccagtt ggggcactac tcccgaaaac cgcttctgga 1620
   tccataactt cgtatagcat acattatacg aagttatacc gggccaccat ggtcgcgagt 1680
   agcttggcac tggccgtcgt tttacaacgt cgtgactggg aaaaccctgg cgttacccaa 1740
   cttaatcgcc ttgcagcaca tccccctttc gccagctggc gtaatagcga agaggcccgc 1800
   accgatcgcc cttcccaaca gttgcgcagc ctgaatggcg aatggcgctt tgcctggttt 1860
   ccggcaccag aagcggtgcc ggaaagctgg ctggagtgcg atcttcctga ggccgatact 1920
   gtcgtcgtcc cctcaaactg gcagatgcac ggttacgatg cgcccatcta caccaacgta 1980
   acctatccca ttacggtcaa tccgccgttt gttcccacgg agaatccgac gggttgttac 2040
   tcgctcacat ttaatgttga tgaaagctgg ctacaggaag gccagacgcg aattattttt 2100
   gatggcgtta actcggcgtt tcatctgtgg tgcaacgggc gctgggtcgg ttacggccag 2160
   gacagtcgtt tgccgtctga atttgacctg agcgcatttt tacgcgccgg agaaaaccgc 2220
   ctcgcggtga tggtgctgcg ttggagtgac ggcagttatc tggaagatca ggatatgtgg 2280
   cggatgagcg gcattttccg tgacgtctcg ttgctgcata aaccgactac acaaatcagc 2340
   gatttccatg ttgccactcg ctttaatgat gatttcagcc gcgctgtact ggaggctgaa 2400
   gttcagatgt gcggcgagtt gcgtgactac ctacgggtaa cagtttcttt atggcagggt 2460
   gaaacgcagg tcgccagcgg caccgcgcct ttcggcggtg aaattatcga tgagcgtggt 2520
   ggttatgccg atcgcgtcac actacgtctg aacgtcgaaa acccgaaact gtggagcgcc 2580
   gaaatcccga atctctatcg tgcggtggtt gaactgcaca ccgccgacgg cacgctgatt 2640
   gaagcagaag cctgcgatgt cggtttccgc gaggtgcgga ttgaaaatgg tctgctgctg 2700
   ctgaacggca agccgttgct gattcgaggc gttaaccgtc acgagcatca tcctctgcat 2760
   ggtcaggtca tggatgagca gacgatggtg caggatatcc tgctgatgaa gcagaacaac 2820
   tttaacgccg tgcgctgttc gcattatccg aaccatccgc tgtggtacac gctgtgcgac 2880
   cgctacggcc tgtatgtggt ggatgaagcc aatattgaaa cccacggcat ggtgccaatg 2940
   aatcgtctga ccgatgatcc gcgctggcta ccggcgatga gcgaacgcgt aacgcgaatg 3000
   gtgcagcgcg atcgtaatca cccgagtgtg atcatctggt cgctggggaa tgaatcaggc 3060
   cacggcgcta atcacgacgc gctgtatcgc tggatcaaat ctgtcgatcc ttcccgcccg 3120
   gtgcagtatg aaggcggcgg agccgacacc acggccaccg atattatttg cccgatgtac 3180
   gcgcgcgtgg atgaagacca gcccttcccg gctgtgccga aatggtccat caaaaaatgg 3240
   ctttcgctac ctggagagac gcgcccgctg atcctttgcg aatacgccca cgcgatgggt 3300
   aacagtcttg gcggtttcgc taaatactgg caggcgtttc gtcagtatcc ccgtttacag 3360
   ggcggcttcg tctgggactg ggtggatcag tcgctgatta aatatgatga aaacggcaac 3420
   ccgtggtcgg cttacggcgg tgattttggc gatacgccga acgatcgcca gttctgtatg 3480
   aacggtctgg tctttgccga ccgcacgccg catccagcgc tgacggaagc aaaacaccag 3540
   cagcagtttt tccagttccg tttatccggg caaaccatcg aagtgaccag cgaatacctg 3600
   ttccgtcata gcgataacga gctcctgcac tggatggtgg cgctggatgg taagccgctg 3660
   gcaagcggtg aagtgcctct ggatgtcgct ccacaaggta aacagttgat tgaactgcct 3720
   gaactaccgc agccggagag cgccgggcaa ctctggctca cagtacgcgt agtgcaaccg 3780
   aacgcgaccg catggtcaga agccgggcac atcagcgcct ggcagcagtg gcgtctggcg 3840
   gaaaacctca gtgtgacgct ccccgccgcg tcccacgcca tcccgcatct gaccaccagc 3900
   gaaatggatt tttgcatcga gctgggtaat aagcgttggc aatttaaccg ccagtcaggc 3960
   tttctttcac agatgtggat tggcgataaa aaacaactgc tgacgccgct gcgcgatcag 4020
   ttcacccgtg caccgctgga taacgacatt ggcgtaagtg aagcgacccg cattgaccct 4080
   aacgcctggg tcgaacgctg gaaggcggcg ggccattacc aggccgaagc agcgttgttg 4140
   cagtgcacgg cagatacact tgctgatgcg gtgctgatta cgaccgctca cgcgtggcag 4200
   catcagggga aaaccttatt tatcagccgg aaaacctacc ggattgatgg tagtggtcaa 4260
   atggcgatta ccgttgatgt tgaagtggcg agcgatacac cgcatccggc gcggattggc 4320
   ctgaactgcc agctggcgca ggtagcagag cgggtaaact ggctcggatt agggccgcaa 4380
   gaaaactatc ccgaccgcct tactgccgcc tgttttgacc gctgggatct gccattgtca 4440
   gacatgtata ccccgtacgt cttcccgagc gaaaacggtc tgcgctgcgg gacgcgcgaa 4500
   ttgaattatg gcccacacca gtggcgcggc gacttccagt tcaacatcag ccgctacagt 4560
   caacagcaac tgatggaaac cagccatcgc catctgctgc acgcggaaga aggcacatgg 4620
   ctgaatatcg acggtttcca tatggggatt ggtggcgacg actcctggag cccgtcagta 4680
   tcggcggaat tccagctgag cgccggtcgc taccattacc agttggtctg gtgtcaaaaa 4740
   taataataac cgggcagggg ggatctttgt gaaggaacct tacttctgtg gtgtgacata 4800
   attggacaaa ctacctacag agatttaaag ctctaaggta aatataaaat ttttaagtgt 4860
   ataatgtgtt aaactactga ttctaattgt ttgtgtattt tagattccaa cctatggaac 4920
   tgatgaatgg gagcagtggt ggaatgccag atccagacat gataagatac attgatgagt 4980
   ttggacaaac cacaactaga atgcagtgaa aaaaatgctt tatttgtgaa atttgtgatg 5040
   ctattgcttt atttgtaacc attataagct gcaataaaca agttaacaac aacaattgca 5100
   ttcattttat gtttcaggtt cagggggagg tgtgggaggt tttttaaagc aagtaaaacc 5160
   tctacaaatg tggtatggct gattatgatc tgcggccgca gggcctcgtg atacgcctat 5220
   ttttataggt taatgtcatg ataataatgg tttcttagac gtcaggtggc acttttcggg 5280
   gaaatgtgcg cggaacccct atttgtttat ttttctaaat acattcaaat atgtatccgc 5340
   tcatgagaca ataaccctga taaatgcttc aataatattg aaaaaggaag agtatgagta 5400
   ttcaacattt ccgtgtcgcc cttattccct tttttgcggc attttgcctt cctgtttttg 5460
   ctcacccaga aacgctggtg aaagtaaaag atgctgaaga tcagttgggt gcacgagtgg 5520
   gttacatcga actggatctc aacagcggta agatccttga gagttttcgc cccgaagaac 5580
   gttttccaat gatgagcact tttaaagttc tgctatgtgg cgcggtatta tcccgtattg 5640
   acgccgggca agagcaactc ggtcgccgca tacactattc tcagaatgac ttggttgagt 5700
   actcaccagt cacagaaaag catcttacgg atggcatgac agtaagagaa ttatgcagtg 5760
   ctgccataac catgagtgat aacactgcgg ccaacttact tctgacaacg atcggaggac 5820
   cgaaggagct aaccgctttt ttgcacaaca tgggggatca tgtaactcgc cttgatcgtt 5880
   gggaaccgga gctgaatgaa gccataccaa acgacgagcg tgacaccacg atgcctgtag 5940
   caatggcaac aacgttgcgc aaactattaa ctggcgaact acttactcta gcttcccggc 6000
   aacaattaat agactggatg gaggcggata aagttgcagg accacttctg cgctcggccc 6060
   ttccggctgg ctggtttatt gctgataaat ctggagccgg tgagcgtggg tctcgcggta 6120
   tcattgcagc actggggcca gatggtaagc cctcccgtat cgtagttatc tacacgacgg 6180
   ggagtcaggc aactatggat gaacgaaata gacagatcgc tgagataggt gcctcactga 6240
   ttaagcattg gtaactgtca gaccaagttt actcatatat actttagatt gatttaaaac 6300
   ttcattttta atttaaaagg atctaggtga agatcctttt tgataatctc atgaccaaaa 6360
   tcccttaacg tgagttttcg ttccactgag cgtcagaccc cgtagaaaag atcaaaggat 6420
   cttcttgaga tccttttttt ctgcgcgtaa tctgctgctt gcaaacaaaa aaaccaccgc 6480
   taccagcggt ggtttgtttg ccggatcaag agctaccaac tctttttccg aaggtaactg 6540
   gcttcagcag agcgcagata ccaaatactg tccttctagt gtagccgtag ttaggccacc 6600
   acttcaagaa ctctgtagca ccgcctacat acctcgctct gctaatcctg ttaccagtgg 6660
   ctgctgccag tggcgataag tcgtgtctta ccgggttgga ctcaagacga tagttaccgg 6720
   ataaggcgca gcggtcgggc tgaacggggg gttcgtgcac acagcccagc ttggagcgaa 6780
   cgacctacac cgaactgaga tacctacagc gtgagctatg agaaagcgcc acgcttcccg 6840
   aagggagaaa ggcggacagg tatccggtaa gcggcagggt cggaacagga gagcgcacga 6900
   gggagcttcc agggggaaac gcctggtatc tttatagtcc tgtcgggttt cgccacctct 6960
   gacttgagcg tcgatttttg tgatgctcgt caggggggcg gagcctatgg aaaaacgcca 7020
   gcaacgcggc ctttttacgg ttcctggcct tttgctggcc ttttgctcac atgttctttc 7080
   ctgcgttatc ccctgattct gtggataacc gtattaccgc ctttgagtga gctgataccg 7140
   ctcgccgcag ccgaacgacc gagcgcagcg agtcagtgag cgaggaagcg gaagagcgcc 7200
   caatacgcaa accgcctctc cccgcgcgtt ggccgattca ttaatgcagc tggcacgaca 7260
   ggtttcccga ctggaaagcg ggcagtgagc gcaacgcaat taatgtgagt tagctcactc 7320
   attaggcacc ccaggcttta cactttatgc ttccggctcg tatgttgtgt ggaattgtga 7380
   gcggataaca atttcacaca ggaaacagct atgaccatga ttacgccaag ctggcgcg 7438
<210> 6
   <211> 45
   <212> DNA
   <213> Artificial Sequence
<220>
   <223> Description of Artificial Sequence: primer C31-1
<400> 6
   ataagaatgc ggccgcccga tatgacacaa ggggttgtga ccggg 45
<210> 7
   <211> 40
   <212> DNA
   <213> Artificial Sequence
<220>
   <223> Description of Artificial Sequence: primer C31-3
<400> 7
   ataagaatgc ggccgcatcc gccgctacgt cttccgtgcc 40
<210> 8
   <211> 24
   <212> DNA
   <213> Artificial Sequence
<220>
   <223> Description of Artificial Sequence: primer C31-8
<400> 8
   cccgttggca ggaagcactt ccgg 24
<210> 9
   <211> 55
   <212> DNA
   <213> Artificial Sequence
<220>
   <223> Description of Artificial Sequence: primer C31-9
<400> 9
   ggatcctcga gccgcgggcg gccgcctacg ccgctacgtc ttccgtgccg tcctg 55
<210> 10
   <211> 5711
   <212> DNA
   <213> Artificial Sequence
<220>
   <223> Description of Artificial Sequence: vector pCMV-C31-Int(wt)
<400> 10
   aaacagtccg atgtacgggc cagatatacg cgttgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggac 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catggtgatg 420
   cggttttggc agtacatcaa tgggcgtgga tagcggtttg actcacgggg atttccaagt 480
   ctccacccca ttgacgtcaa tgggagtttg ttttggcacc aaaatcaacg ggactttcca 540
   aaatgtcgta acaactccgc cccattgacg caaatgggcg gtaggcgtgt acggtgggag 600
   gtctatataa gcagagctct ctggctaact agagaaccca ctgcttactg gcttatcgaa 660
   attaatacga ctcactatag ggagacccaa gctgactcta gacttaatta agcgttgggg 720
   tgagtactcc ctctcaaaag cgggcatgac ttctgcgcta agattgtcag tttccaaaaa 780
   cgaggaggat ttgatattca cctggcccgc ggtgatgcct ttgagggtgg ccgcgtccat 840
   ctggtcagaa aagacaatct ttttgttgtc aagcttgagg tgtggcaggc ttgagatctg 900
   gccatacact tgagtgacat tgacatccac tttgcctttc tctccacagg tgtccactcc 960
   cagggcggcc gcccgatatg acacaagggg ttgtgaccgg ggtggacacg tacgcgggtg 1020
   cttacgaccg tcagtcgcgc gagcgcgaga attcgagcgc agcaagccca gcgacacagc 1080
   gtagcgccaa cgaagacaag gcggccgacc ttcagcgcga agtcgagcgc gacgggggcc 1140
   ggttcaggtt cgtcgggcat ttcagcgaag cgccgggcac gtcggcgttc gggacggcgg 1200
   agcgcccgga gttcgaacgc atcctgaacg aatgccgcgc cgggcggctc aacatgatca 1260
   ttgtctatga cgtgtcgcgc ttctcgcgcc tgaaggtcat ggacgcgatt ccgattgtct 1320
   cggaattgct cgccctgggc gtgacgattg tttccactca ggaaggcgtc ttccggcagg 1380
   gaaacgtcat ggacctgatt cacctgatta tgcggctcga cgcgtcgcac aaagaatctt 1440
   cgctgaagtc ggcgaagatt ctcgacacga agaaccttca gcgcgaattg ggcgggtacg 1500
   tcggcgggaa ggcgccttac ggcttcgagc ttgtttcgga gacgaaggag atcacgcgca 1560
   acggccgaat ggtcaatgtc gtcatcaaca agcttgcgca ctcgaccact ccccttaccg 1620
   gacccttcga gttcgagccc gacgtaatcc ggtggtggtg gcgtgagatc aagacgcaca 1680
   aacaccttcc cttcaagccg ggcagtcaag ccgccattca cccgggcagc atcacggggc 1740
   tttgtaagcg catggacgct gacgccgtgc cgacccgggg cgagacgatt gggaagaaga 1800
   ccgcttcaag cgcctgggac ccggcaaccg ttatgcgaat ccttcgggac ccgcgtattg 1860
   cgggcttcgc cgctgaggtg atctacaaga agaagccgga cggcacgccg accacgaaga 1920
   ttgagggtta ccgcattcag cgcgacccga tcacgctccg gccggtcgag cttgattgcg 1980
   gaccgatcat cgagcccgct gagtggtatg agcttcaggc gtggttggac ggcagggggc 2040
   gcggcaaggg gctttcccgg gggcaagcca ttctgtccgc catggacaag ctgtactgcg 2100
   agtgtggcgc cgtcatgact tcgaagcgcg gggaagaatc gatcaaggac tcttaccgct 2160
   gccgtcgccg gaaggtggtc gacccgtccg cacctgggca gcacgaaggc acgtgcaacg 2220
   tcagcatggc ggcactcgac aagttcgttg cggaacgcat cttcaacaag atcaggcacg 2280
   ccgaaggcga cgaagagacg ttggcgcttc tgtgggaagc cgcccgacgc ttcggcaagc 2340
   tcactgaggc gcctgagaag agcggcgaac gggcgaacct tgttgcggag cgcgccgacg 2400
   ccctgaacgc ccttgaagag ctgtacgaag accgcgcggc aggcgcgtac gacggacccg 2460
   ttggcaggaa gcacttccgg aagcaacagg cagcgctgac gctccggcag caaggggcgg 2520
   aagagcggct tgccgaactt gaagccgccg aagccccgaa gcttcccctt gaccaatggt 2580
   tccccgaaga cgccgacgct gacccgaccg gccctaagtc gtggtggggg cgcgcgtcag 2640
   tagacgacaa gcgcgtgttc gtcgggctct tcgtagacaa gatcgttgtc acgaagtcga 2700
   ctacgggcag gggcagggga acgcccatcg agaagcgcgc ttcgatcacg tgggcgaagc 2760
   cgccgaccga cgacgacgaa gacgacgccc aggacggcac ggaagacgta gcggcgtagg 2820
   cggcgcccgg gctcgagatc caggcgcgga tcaataaaag atcattattt tcaatagatc 2880
   tgtgtgttgg ttttttgtgt gccttggggg agggggaggc cagaatgagg cgcggccaag 2940
   ggggaggggg aggccagaat gaccttgggg gagggggagg ccagaatgac cttgggggag 3000
   ggggaggcca gaatgaggcg cgcccccggg taccgagctc gaattcactg gccgtcgttt 3060
   tacaacgtcg tgactgggaa aaccctggcg ttacccaact taatcgcctt gcagcacatc 3120
   cccctttcgc cagctggcgt aatagcgaag aggcccgcac cgatcgccct tcccaacagt 3180
   tgcgcagcct gaatggcgaa tggcgcctga tgcggtattt tctccttacg catctgtgcg 3240
   gtatttcaca ccgcatatgg tgcactctca gtacaatctg ctctgatgcc gcatagttaa 3300
   gccagccccg acacccgcca acacccgctg acgcgccctg acgggcttgt ctgctcccgg 3360
   catccgctta cagacaagct gtgaccgtct ccgggagctg catgtgtcag aggttttcac 3420
   cgtcatcacc gaaacgcgcg agacgaaagg gcctcgtgat acgcctattt ttataggtta 3480
   atgtcatgat aataatggtt tcttagacgt caggtggcac ttttcgggga aatgtgcgcg 3540
   gaacccctat ttgtttattt ttctaaatac attcaaatat gtatccgctc atgagacaat 3600
   aaccctgata aatgcttcaa taatattgaa aaaggaagag tatgagtatt caacatttcc 3660
   gtgtcgccct tattcccttt tttgcggcat tttgccttcc tgtttttgct cacccagaaa 3720
   cgctggtgaa agtaaaagat gctgaagatc agttgggtgc acgagtgggt tacatcgaac 3780
   tggatctcaa cagcggtaag atccttgaga gttttcgccc cgaagaacgt tttccaatga 3840
   tgagcacttt taaagttctg ctatgtggcg cggtattatc ccgtattgac gccgggcaag 3900
   agcaactcgg tcgccgcata cactattctc agaatgactt ggttgagtac tcaccagtca 3960
   cagaaaagca tcttacggat ggcatgacag taagagaatt atgcagtgct gccataacca 4020
   tgagtgataa cactgcggcc aacttacttc tgacaacgat cggaggaccg aaggagctaa 4080
   ccgctttttt gcacaacatg ggggatcatg taactcgcct tgatcgttgg gaaccggagc 4140
   tgaatgaagc cataccaaac gacgagcgtg acaccacgat gcctgtagca atggcaacaa 4200
   cgttgcgcaa actattaact ggcgaactac ttactctagc ttcccggcaa caattaatag 4260
   actggatgga ggcggataaa gttgcaggac cacttctgcg ctcggccctt ccggctggct 4320
   ggtttattgc tgataaatct ggagccggtg agcgtgggtc tcgcggtatc attgcagcac 4380
   tggggccaga tggtaagccc tcccgtatcg tagttatcta cacgacgggg agtcaggcaa 4440
   ctatggatga acgaaataga cagatcgctg agataggtgc ctcactgatt aagcattggt 4500
   aactgtcaga ccaagtttac tcatatatac tttagattga tttaaaactt catttttaat 4560
   ttaaaaggat ctaggtgaag atcctttttg ataatctcat gaccaaaatc ccttaacgtg 4620
   agttttcgtt ccactgagcg tcagaccccg tagaaaagat caaaggatct tcttgagatc 4680
   ctttttttct gcgcgtaatc tgctgcttgc aaacaaaaaa accaccgcta ccagcggtgg 4740
   tttgtttgcc ggatcaagag ctaccaactc tttttccgaa ggtaactggc ttcagcagag 4800
   cgcagatacc aaatactgtc cttctagtgt agccgtagtt aggccaccac ttcaagaact 4860
   ctgtagcacc gcctacatac ctcgctctgc taatcctgtt accagtggct gctgccagtg 4920
   gcgataagtc gtgtcttacc gggttggact caagacgata gttaccggat aaggcgcagc 4980
   ggtcgggctg aacggggggt tcgtgcacac agcccagctt ggagcgaacg acctacaccg 5040
   aactgagata cctacagcgt gagctatgag aaagcgccac gcttcccgaa gggagaaagg 5100
   cggacaggta tccggtaagc ggcagggtcg gaacaggaga gcgcacgagg gagcttccag 5160
   ggggaaacgc ctggtatctt tatagtcctg tcgggtttcg ccacctctga cttgagcgtc 5220
   gatttttgtg atgctcgtca ggggggcgga gcctatggaa aaacgccagc aacgcggcct 5280
   ttttacggtt cctggccttt tgctggcctt ttgctcacat gttctttcct gcgttatccc 5340
   ctgattctgt ggataaccgt attaccgcct ttgagtgagc tgataccgct cgccgcagcc 5400
   gaacgaccga gcgcagcgag tcagtgagcg aggaagcgga agagcgccca atacgcaaac 5460
   cgcctctccc cgcgcgttgg ccgattcatt aatgcagctg gcacgacagg tttcccgact 5520
   ggaaagcggg cagtgagcgc aacgcaatta atgtgagtta gctcactcat taggcacccc 5580
   aggctttaca ctttatgctt ccggctcgta tgttgtgtgg aattgtgagc ggataacaat 5640
   ttcacacagg aaacagctat gaccatgatt acgccaagct agcccgggct agcttgcatg 5700
   cctgcaggtt t 5711
<210> 11
   <211> 69
   <212> DNA
   <213> Artificial Sequence
<220>
   <223> Description of Artificial Sequence: primer C31-2-2
<400> 11
   tagaattccg ctcgagagtc taaaccttcc tcttcttctt aggcgccgct acgtcttccg 60
   tgccgtcct 69
<210> 12
   <211> 5723
   <212> DNA
   <213> Artificial Sequence
<220>
   <223> Description of Artificial Sequence: vector pCMV-C31-Int(CNLS)
<400> 12
   cctgcaggtt taaacagtcc gatgtacggg ccagatatac gcgttgacat tgattattga 60
   ctagttatta atagtaatca attacggggt cattagttca tagcccatat atggagttcc 120
   gcgttacata acttacggta aatggcccgc ctggctgacc gcccaacgac ccccgcccat 180
   tgacgtcaat aatgacgtat gttcccatag taacgccaat agggactttc cattgacgtc 240
   aatgggtgga ctatttacgg taaactgccc acttggcagt acatcaagtg tatcatatgc 300
   caagtacgcc ccctattgac gtcaatgacg gtaaatggcc cgcctggcat tatgcccagt 360
   acatgacctt atgggacttt cctacttggc agtacatcta cgtattagtc atcgctatta 420
   ccatggtgat gcggttttgg cagtacatca atgggcgtgg atagcggttt gactcacggg 480
   gatttccaag tctccacccc attgacgtca atgggagttt gttttggcac caaaatcaac 540
   gggactttcc aaaatgtcgt aacaactccg ccccattgac gcaaatgggc ggtaggcgtg 600
   tacggtggga ggtctatata agcagagctc tctggctaac tagagaaccc actgcttact 660
   ggcttatcga aattaatacg actcactata gggagaccca agctgactct agacttaatt 720
   aagcgttggg gtgagtactc cctctcaaaa gcgggcatga cttctgcgct aagattgtca 780
   gtttccaaaa acgaggagga tttgatattc acctggcccg cggtgatgcc tttgagggtg 840
   gccgcgtcca tctggtcaga aaagacaatc tttttgttgt caagcttgag gtgtggcagg 900
   cttgagatct ggccatacac ttgagtgaca ttgacatcca ctttgccttt ctctccacag 960
   gtgtccactc ccagggcggc cgcccgatat gacacaaggg gttgtgaccg gggtggacac 1020
   gtacgcgggt gcttacgacc gtcagtcgcg cgagcgcgag aattcgagcg cagcaagccc 1080
   agcgacacag cgtagcgcca acgaagacaa ggcggccgac cttcagcgcg aagtcgagcg 1140
   cgacgggggc cggttcaggt tcgtcgggca tttcagcgaa gcgccgggca cgtcggcgtt 1200
   cgggacggcg gagcgcccgg agttcgaacg catcctgaac gaatgccgcg ccgggcggct 1260
   caacatgatc attgtctatg acgtgtcgcg cttctcgcgc ctgaaggtca tggacgcgat 1320
   tccgattgtc tcggaattgc tcgccctggg cgtgacgatt gtttccactc aggaaggcgt 1380
   cttccggcag ggaaacgtca tggacctgat tcacctgatt atgcggctcg acgcgtcgca 1440
   caaagaatct tcgctgaagt cggcgaagat tctcgacacg aagaaccttc agcgcgaatt 1500
   gggcgggtac gtcggcggga aggcgcctta cggcttcgag cttgtttcgg agacgaagga 1560
   gatcacgcgc aacggccgaa tggtcaatgt cgtcatcaac aagcttgcgc actcgaccac 1620
   tccccttacc ggacccttcg agttcgagcc cgacgtaatc cggtggtggt ggcgtgagat 1680
   caagacgcac aaacaccttc ccttcaagcc gggcagtcaa gccgccattc acccgggcag 1740
   catcacgggg ctttgtaagc gcatggacgc tgacgccgtg ccgacccggg gcgagacgat 1800
   tgggaagaag accgcttcaa gcgcctggga cccggcaacc gttatgcgaa tccttcggga 1860
   cccgcgtatt gcgggcttcg ccgctgaggt gatctacaag aagaagccgg acggcacgcc 1920
   gaccacgaag attgagggtt accgcattca gcgcgacccg atcacgctcc ggccggtcga 1980
   gcttgattgc ggaccgatca tcgagcccgc tgagtggtat gagcttcagg cgtggttgga 2040
   cggcaggggg cgcggcaagg ggctttcccg ggggcaagcc attctgtccg ccatggacaa 2100
   gctgtactgc gagtgtggcg ccgtcatgac ttcgaagcgc ggggaagaat cgatcaagga 2160
   ctcttaccgc tgccgtcgcc ggaaggtggt cgacccgtcc gcacctgggc agcacgaagg 2220
   cacgtgcaac gtcagcatgg cggcactcga caagttcgtt gcggaacgca tcttcaacaa 2280
   gatcaggcac gccgaaggcg acgaagagac gttggcgctt ctgtgggaag ccgcccgacg 2340
   cttcggcaag ctcactgagg cgcctgagaa gagcggcgaa cgggcgaacc ttgttgcgga 2400
   gcgcgccgac gccctgaacg cccttgaaga gctgtacgaa gaccgcgcgg caggcgcgta 2460
   cgacggaccc gttggcagga agcacttccg gaagcaacag gcagcgctga cgctccggca 2520
   gcaaggggcg gaagagcggc ttgccgaact tgaagccgcc gaagccccga agcttcccct 2580
   tgaccaatgg ttccccgaag acgccgacgc tgacccgacc ggccctaagt cgtggtgggg 2640
   gcgcgcgtca gtagacgaca agcgcgtgtt cgtcgggctc ttcgtagaca agatcgttgt 2700
   cacgaagtcg actacgggca gggggcaggg aacgcccatc gagaagcgcg cttcgatcac 2760
   gtgggcgaag ccgccgaccg acgacgacga agacgacgcc caggacggca cggaagacgt 2820
   agcggcgcct aagaagaaga ggaaggttta gactctcgag atccaggcgc ggatcaataa 2880
   aagatcatta ttttcaatag atctgtgtgt tggttttttg tgtgccttgg gggaggggga 2940
   ggccagaatg aggcgcggcc aagggggagg gggaggccag aatgaccttg gggagggggg 3000
   aggccagaat gaccttgggg gagggggagg ccagaatgag gcgcgccccc gggtaccgag 3060
   ctcgaattca ctggccgtcg ttttacaacg tcgtgactgg gaaaaccctg gcgttaccca 3120
   acttaatcgc cttgcagcac atcccccttt cgccagctgg cgtaatagcg aagaggcccg 3180
   caccgatcgc ccttcccaac agttgcgcag cctgaatggc gaatggcgcc tgatgcggta 3240
   ttttctcctt acgcatctgt gcggtatttc acaccgcata tggtgcactc tcagtacaat 3300
   ctgctctgat gccgcatagt taagccagcc ccgacacccg ccaacacccg ctgacgcgcc 3360
   ctgacgggct tgtctgctcc cggcatccgc ttacagacaa gctgtgaccg tctccgggag 3420
   ctgcatgtgt cagaggtttt caccgtcatc accgaaacgc gcgagacgaa agggcctcgt 3480
   gatacgccta tttttatagg ttaatgtcat gataataatg gtttcttaga cgtcaggtgg 3540
   cacttttcgg ggaaatgtgc gcggaacccc tatttgttta tttttctaaa tacattcaaa 3600
   tatgtatccg ctcatgagac aataaccctg ataaatgctt caataatatt gaaaaaggaa 3660
   gagtatgagt attcaacatt tccgtgtcgc ccttattccc ttttttgcgg cattttgcct 3720
   tcctgttttt gctcacccag aaacgctggt gaaagtaaaa gatgctgaag atcagttggg 3780
   tgcacgagtg ggttacatcg aactggatct caacagcggt aagatccttg agagttttcg 3840
   ccccgaagaa cgttttccaa tgatgagcac ttttaaagtt ctgctatgtg gcgcggtatt 3900
   atcccgtatt gacgccgggc aagagcaact cggtcgccgc atacactatt ctcagaatga 3960
   cttggttgag tactcaccag tcacagaaaa gcatcttacg gatggcatga cagtaagaga 4020
   attatgcagt gctgccataa ccatgagtga taacactgcg gccaacttac ttctgacaac 4080
   gatcggagga ccgaaggagc taaccgcttt tttgcacaac atgggggatc atgtaactcg 4140
   ccttgatcgt tgggaaccgg agctgaatga agccatacca aacgacgagc gtgacaccac 4200
   gatgcctgta gcaatggcaa caacgttgcg caaactatta actggcgaac tacttactct 4260
   agcttcccgg caacaattaa tagactggat ggaggcggat aaagttgcag gaccacttct 4320
   gcgctcggcc cttccggctg gctggtttat tgctgataaa tctggagccg gtgagcgtgg 4380
   gtctcgcggt atcattgcag cactggggcc agatggtaag ccctcccgta tcgtagttat 4440
   ctacacgacg gggagtcagg caactatgga tgaacgaaat agacagatcg ctgagatagg 4500
   tgcctcactg attaagcatt ggtaactgtc agaccaagtt tactcatata tactttagat 4560
   tgatttaaaa cttcattttt aatttaaaag gatctaggtg aagatccttt ttgataatct 4620
   catgaccaaa atcccttaac gtgagttttc gttccactga gcgtcagacc ccgtagaaaa 4680
   gatcaaagga tcttcttgag atcctttttt tctgcgcgta atctgctgct tgcaaacaaa 4740
   aaaaccaccg ctaccagcgg tggtttgttt gccggatcaa gagctaccaa ctctttttcc 4800
   gaaggtaact ggcttcagca gagcgcagat accaaatact gtccttctag tgtagccgta 4860
   gttaggccac cacttcaaga actctgtagc accgcctaca tacctcgctc tgctaatcct 4920
   gttaccagtg gctgctgcca gtggcgataa gtcgtgtctt accgggttgg actcaagacg 4980
   atagttaccg gataaggcgc agcggtcggg ctgaacgggg ggttcgtgca cacagcccag 5040
   cttggagcga acgacctaca ccgaactgag atacctacag cgtgagctat gagaaagcgc 5100
   cacgcttccc gaagggagaa aggcggacag gtatccggta agcggcaggg tcggaacagg 5160
   agagcgcacg agggagcttc cagggggaaa cgcctggtat ctttatagtc ctgtcgggtt 5220
   tcgccacctc tgacttgagc gtcgattttt gtgatgctcg tcaggggggc ggagcctatg 5280
   gaaaaacgcc agcaacgcgg cctttttacg gttcctggcc ttttgctggc cttttgctca 5340
   catgttcttt cctgcgttat cccctgattc tgtggataac cgtattaccg cctttgagtg 5400
   agctgatacc gctcgccgca gccgaacgac cgagcgcagc gagtcagtga gcgaggaagc 5460
   ggaagagcgc ccaatacgca aaccgcctct ccccgcgcgt tggccgattc attaatgcag 5520
   ctggcacgac aggtttcccg actggaaagc gggcagtgag cgcaacgcaa ttaatgtgag 5580
   ttagctcact cattaggcac cccaggcttt acactttatg cttccggctc gtatgttgtg 5640
   tggaattgtg agcggataac aatttcacac aggaaacagc tatgaccatg attacgccaa 5700
   gctagcccgg gctagcttgc atg                                          5723
<210> 13
   <211> 4960
   <212> DNA
   <213> Artificial Sequence
<220>
   <223> Description of Artificial Sequence: vector pCMV-Cre
<400> 13
   aaacagtccg atgtacgggc cagatatacg cgttgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggac 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catggtgatg 420
   cggttttggc agtacatcaa tgggcgtgga tagcggtttg actcacgggg atttccaagt 480
   ctccacccca ttgacgtcaa tgggagtttg ttttggcacc aaaatcaacg ggactttcca 540
   aaatgtcgta acaactccgc cccattgacg caaatgggcg gtaggcgtgt acggtgggag 600
   gtctatataa gcagagctct ctggctaact agagaaccca ctgcttactg gcttatcgaa 660
   attaatacga ctcactatag ggagacccaa gctgactcta gacttaatta agcgttgggg 720
   tgagtactcc ctctcaaaag cgggcatgac ttctgcgcta agattgtcag tttccaaaaa 780
   cgaggaggat ttgatattca cctggcccgc ggtgatgcct ttgagggtgg ccgcgtccat 840
   ctggtcagaa aagacaatct ttttgttgtc aagcttgagg tgtggcaggc ttgagatctg 900
   gccatacact tgagtgacat tgacatccac tttgcctttc tctccacagg tgtccactcc 960
   cagggcggcc tcgaccatgc ccaagaagaa gaggaaggtg tccaatttac tgaccgtaca 1020
   ccaaaatttg cctgcattac cggtcgatgc aacgagtgat gaggttcgca agaacctgat 1080
   ggacatgttc agggatcgcc aggcgttttc tgagcatacc tggaaaatgc ttctgtccgt 1140
   ttgccggtcg tgggcggcat ggtgcaagtt gaataaccgg aaatggtttc ccgcagaacc 1200
   tgaagatgtt cgcgattatc ttctatatct tcaggcgcgc ggtctggcag taaaaactat 1260
   ccagcaacat ttgggccagc taaacatgct tcatcgtcgg tccgggctgc cacgaccaag 1320
   tgacagcaat gctgtttcac tggttatgcg gcggatccga aaagaaaacg ttgatgccgg 1380
   tgaacgtgca aaacaggctc tagcgttcga acgcactgat ttcgaccagg ttcgttcact 1440
   catggaaaat agcgatcgct gccaggatat acgtaatctg gcatttctgg ggattgctta 1500
   taacaccctg ttacgtatag ccgaaattgc caggatcagg gttaaagata tctcacgtac 1560
   tgacggtggg agaatgttaa tccatattgg cagaacgaaa acgctggtta gcaccgcagg 1620
   tgtagagaag gcacttagcc tgggggtaac taaactggtc gagcgatgga tttccgtctc 1680
   tggtgtagct gatgatccga ataactacct gttttgccgg gtcagaaaaa atggtgttgc 1740
   cgcgccatct gccaccagcc agctatcaac tcgcgccctg gaagggattt ttgaagcaac 1800
   tcatcgattg atttacggcg ctaaggatga ctctggtcag agatacctgg cctggtctgg 1860
   acacagtgcc cgtgtcggag ccgcgcgaga tatggcccgc gctggagttt caataccgga 1920
   gatcatgcaa gctggtggct ggaccaatgt aaatattgtc atgaactata tccgtaacct 1980
   ggatagtgaa acaggggcaa tggtgcgcct gctggaagat ggcgattagc cattaacgcg 2040
   taaatgattg cagatccact agttctaggg ccgcgtcgac ctcgagatcc aggcgcggat 2100
   caataaaaga tcattatttt caatagatct gtgtgttggt tttttgtgtg ccttggggga 2160
   gggggaggcc agaatgaggc gcggccaagg gggaggggga ggccagaatg accttggggg 2220
   agggggaggc cagaatgacc ttgggggagg gggaggccag aatgaggcgc gcccccgggt 2280
   accgagctcg aattcactgg ccgtcgtttt acaacgtcgt gactgggaaa accctggcgt 2340
   tacccaactt aatcgccttg cagcacatcc ccctttcgcc agctggcgta atagcgaaga 2400
   ggcccgcacc gatcgccctt cccaacagtt gcgcagcctg aatggcgaat ggcgcctgat 2460
   gcggtatttt ctccttacgc atctgtgcgg tatttcacac cgcatatggt gcactctcag 2520
   tacaatctgc tctgatgccg catagttaag ccagccccga cacccgccaa cacccgctga 2580
   cgcgccctga cgggcttgtc tgctcccggc atccgcttac agacaagctg tgaccgtctc 2640
   cgggagctgc atgtgtcaga ggttttcacc gtcatcaccg aaacgcgcga gacgaaaggg 2700
   cctcgtgata cgcctatttt tataggttaa tgtcatgata ataatggttt cttagacgtc 2760
   aggtggcact tttcggggaa atgtgcgcgg aacccctatt tgtttatttt tctaaataca 2820
   ttcaaatatg tatccgctca tgagacaata accctgataa atgcttcaat aatattgaaa 2880
   aaggaagagt atgagtattc aacatttccg tgtcgccctt attccctttt ttgcggcatt 2940
   ttgccttcct gtttttgctc acccagaaac gctggtgaaa gtaaaagatg ctgaagatca 3000
   gttgggtgca cgagtgggtt acatcgaact ggatctcaac agcggtaaga tccttgagag 3060
   ttttcgcccc gaagaacgtt ttccaatgat gagcactttt aaagttctgc tatgtggcgc 3120
   ggtattatcc cgtattgacg ccgggcaaga gcaactcggt cgccgcatac actattctca 3180
   gaatgacttg gttgagtact caccagtcac agaaaagcat cttacggatg gcatgacagt 3240
   aagagaatta tgcagtgctg ccataaccat gagtgataac actgcggcca acttacttct 3300
   gacaacgatc ggaggaccga aggagctaac cgcttttttg cacaacatgg gggatcatgt 3360
   aactcgcctt gatcgttggg aaccggagct gaatgaagcc ataccaaacg acgagcgtga 3420
   caccacgatg cctgtagcaa tggcaacaac gttgcgcaaa ctattaactg gcgaactact 3480
   tactctagct tcccggcaac aattaataga ctggatggag gcggataaag ttgcaggacc 3540
   acttctgcgc tcggcccttc cggctggctg gtttattgct gataaatctg gagccggtga 3600
   gcgtgggtct cgcggtatca ttgcagcact ggggccagat ggtaagccct cccgtatcgt 3660
   agttatctac acgacgggga gtcaggcaac tatggatgaa cgaaatagac agatcgctga 3720
   gataggtgcc tcactgatta agcattggta actgtcagac caagtttact catatatact 3780
   ttagattgat ttaaaacttc atttttaatt taaaaggatc taggtgaaga tcctttttga 3840
   taatctcatg accaaaatcc cttaacgtga gttttcgttc cactgagcgt cagaccccgt 3900
   agaaaagatc aaaggatctt cttgagatcc tttttttctg cgcgtaatct gctgcttgca 3960
   aacaaaaaaa ccaccgctac cagcggtggt ttgtttgccg gatcaagagc taccaactct 4020
   ttttccgaag gtaactggct tcagcagagc gcagatacca aatactgtcc ttctagtgta 4080
   gccgtagtta ggccaccact tcaagaactc tgtagcaccg cctacatacc tcgctctgct 4140
   aatcctgtta ccagtggctg ctgccagtgg cgataagtcg tgtcttaccg ggttggactc 4200
   aagacgatag ttaccggata aggcgcagcg gtcgggctga acggggggtt cgtgcacaca 4260
   gcccagcttg gagcgaacga cctacaccga actgagatac ctacagcgtg agctatgaga 4320
   aagcgccacg cttcccgaag ggagaaaggc ggacaggtat ccggtaagcg gcagggtcgg 4380
   aacaggagag cgcacgaggg agcttccagg gggaaacgcc tggtatcttt atagtcctgt 4440
   cgggtttcgc cacctctgac ttgagcgtcg atttttgtga tgctcgtcag gggggcggag 4500
   cctatggaaa aacgccagca acgcggcctt tttacggttc ctggcctttt gctggccttt 4560
   tgctcacatg ttctttcctg cgttatcccc tgattctgtg gataaccgta ttaccgcctt 4620
   tgagtgagct gataccgctc gccgcagccg aacgaccgag cgcagcgagt cagtgagcga 4680
   ggaagcggaa gagcgcccaa tacgcaaacc gcctctcccc gcgcgttggc cgattcatta 4740
   atgcagctgg cacgacaggt ttcccgactg gaaagcgggc agtgagcgca acgcaattaa 4800
   tgtgagttag ctcactcatt aggcacccca ggctttacac tttatgcttc cggctcgtat 4860
   gttgtgtgga attgtgagcg gataacaatt tcacacagga aacagctatg accatgatta 4920
   cgccaagcta gcccgggcta gcttgcatgc ctgcaggttt 4960
<210> 14
   <211> 3858
   <212> DNA
   <213> Artificial Sequence
<220>
   <223> Description of Artificial Sequence: vector pRK50
<400> 14
   aaacagtccg atgtacgggc cagatatacg cgttgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggac 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catggtgatg 420
   cggttttggc agtacatcaa tgggcgtgga tagcggtttg actcacgggg atttccaagt 480
   ctccacccca ttgacgtcaa tgggagtttg ttttggcacc aaaatcaacg ggactttcca 540
   aaatgtcgta acaactccgc cccattgacg caaatgggcg gtaggcgtgt acggtgggag 600
   gtctatataa gcagagctct ctggctaact agagaaccca ctgcttactg gcttatcgaa 660
   attaatacga ctcactatag ggagacccaa gctgactcta gacttaatta agcgttgggg 720
   tgagtactcc ctctcaaaag cgggcatgac ttctgcgcta agattgtcag tttccaaaaa 780
   cgaggaggat ttgatattca cctggcccgc ggtgatgcct ttgagggtgg ccgcgtccat 840
   ctggtcagaa aagacaatct ttttgttgtc aagcttgagg tgtggcaggc ttgagatctg 900
   gccatacact tgagtgacat tgacatccac tttgcctttc tctccacagg tgtccactcc 960
   cagggcggcc gcgtcgacct cgagatccag gcgcggatca ataaaagatc attattttca 1020
   atagatctgt gtgttggttt tttgtgtgcc ttgggggagg gggaggccag aatgaggcgc 1080
   ggccaagggg gagggggagg ccagaatgac cttgggggag ggggaggcca gaatgacctt 1140
   gggggagggg gaggccagaa tgaggcgcgc ccccgggtac cgagctcgaa ttcactggcc 1200
   gtcgttttac aacgtcgtga ctgggaaaac cctggcgtta cccaacttaa tcgccttgca 1260
   gcacatcccc ctttcgccag ctggcgtaat agcgaagagg cccgcaccga tcgcccttcc 1320
   caacagttgc gcagcctgaa tggcgaatgg cgcctgatgc ggtattttct ccttacgcat 1380
   ctgtgcggta tttcacaccg catatggtgc actctcagta caatctgctc tgatgccgca 1440
   tagttaagcc agccccgaca cccgccaaca cccgctgacg cgccctgacg ggcttgtctg 1500
   ctcccggcat ccgcttacag acaagctgtg accgtctccg ggagctgcat gtgtcagagg 1560
   ttttcaccgt catcaccgaa acgcgcgaga cgaaagggcc tcgtgatacg cctattttta 1620
   taggttaatg tcatgataat aatggtttct tagacgtcag gtggcacttt tcggggaaat 1680
   gtgcgcggaa cccctatttg tttatttttc taaatacatt caaatatgta tccgctcatg 1740
   agacaataac cctgataaat gcttcaataa tattgaaaaa ggaagagtat gagtattcaa 1800
   catttccgtg tcgcccttat tccctttttt gcggcatttt gccttcctgt ttttgctcac 1860
   ccagaaacgc tggtgaaagt aaaagatgct gaagatcagt tgggtgcacg agtgggttac 1920
   atcgaactgg atctcaacag cggtaagatc cttgagagtt ttcgccccga agaacgtttt 1980
   ccaatgatga gcacttttaa agttctgcta tgtggcgcgg tattatcccg tattgacgcc 2040
   gggcaagagc aactcggtcg ccgcatacac tattctcaga atgacttggt tgagtactca 2100
   ccagtcacag aaaagcatct tacggatggc atgacagtaa gagaattatg cagtgctgcc 2160
   ataaccatga gtgataacac tgcggccaac ttacttctga caacgatcgg aggaccgaag 2220
   gagctaaccg cttttttgca caacatgggg gatcatgtaa ctcgccttga tcgttgggaa 2280
   ccggagctga atgaagccat accaaacgac gagcgtgaca ccacgatgcc tgtagcaatg 2340
   gcaacaacgt tgcgcaaact attaactggc gaactactta ctctagcttc ccggcaacaa 2400
   ttaatagact ggatggaggc ggataaagtt gcaggaccac ttctgcgctc ggcccttccg 2460
   gctggctggt ttattgctga taaatctgga gccggtgagc gtgggtctcg cggtatcatt 2520
   gcagcactgg ggccagatgg taagccctcc cgtatcgtag ttatctacac gacggggagt 2580
   caggcaacta tggatgaacg aaatagacag atcgctgaga taggtgcctc actgattaag 2640
   cattggtaac tgtcagacca agtttactca tatatacttt agattgattt aaaacttcat 2700
   ttttaattta aaaggatcta ggtgaagatc ctttttgata atctcatgac caaaatccct 2760
   taacgtgagt tttcgttcca ctgagcgtca gaccccgtag aaaagatcaa aggatcttct 2820
   tgagatcctt tttttctgcg cgtaatctgc tgcttgcaaa caaaaaaacc accgctacca 2880
   gcggtggttt gtttgccgga tcaagagcta ccaactcttt ttccgaaggt aactggcttc 2940
   agcagagcgc agataccaaa tactgtcctt ctagtgtagc cgtagttagg ccaccacttc 3000
   aagaactctg tagcaccgcc tacatacctc gctctgctaa tcctgttacc agtggctgct 3060
   gccagtggcg ataagtcgtg tcttaccggg ttggactcaa gacgatagtt accggataag 3120
   gcgcagcggt cgggctgaac ggggggttcg tgcacacagc ccagcttgga gcgaacgacc 3180
   tacaccgaac tgagatacct acagcgtgag ctatgagaaa gcgccacgct tcccgaaggg 3240
   agaaaggcgg acaggtatcc ggtaagcggc agggtcggaa caggagagcg cacgagggag 3300
   cttccagggg gaaacgcctg gtatctttat agtcctgtcg ggtttcgcca cctctgactt 3360
   gagcgtcgat ttttgtgatg ctcgtcaggg gggcggagcc tatggaaaaa cgccagcaac 3420
   gcggcctttt tacggttcct ggccttttgc tggccttttg ctcacatgtt ctttcctgcg 3480
   ttatcccctg attctgtgga taaccgtatt accgcctttg agtgagctga taccgctcgc 3540
   cgcagccgaa cgaccgagcg cagcgagtca gtgagcgagg aagcggaaga gcgcccaata 3600
   cgcaaaccgc ctctccccgc gcgttggccg attcattaat gcagctggca cgacaggttt 3660
   cccgactgga aagcgggcag tgagcgcaac gcaattaatg tgagttagct cactcattag 3720
   gcaccccagg ctttacactt tatgcttccg gctcgtatgt tgtgtggaat tgtgagcgga 3780
   taacaatttc acacaggaaa cagctatgac catgattacg ccaagctagc ccgggctagc 3840
   ttgcatgcct gcaggttt                                              3858
<210> 15
   <211> 6257
   <212> DNA
   <213> Artificial Sequence
<220>
   <223> Description of Artificial Sequence: vector pRK64(deltaCre)
<400> 15
   cgtcatcacc gaaacgcgcg aggcagctgt ggaatgtgtg tcagttaggg tgtggaaagt 60
   ccccaggctc cccagcaggc agaagtatgc aaagcatgca tctcaattag tcagcaacca 120
   ggctccccag caggcagaag tgtgcaaagc atgcatctca attagtcagc aaccatagtc 180
   ccgcccctaa ctccgcccat cccgccccta actccgccca gttccgccca ttctccgccc 240
   catggctgac taattttttt tatttatgca gaggccgagg ccgcctcggc ctaggaacag 300
   tcgacgacac tgcagagacc tacttcacta acaaccggta cagttcgtgg accagatggg 360
   tgaggtggag tacgcgcccg gggagcccaa gggcacgccc tggcacccgc accgcggctt 420
   cgagaccgtc acgaatagat ccataacttc gtatagcata cattatacga agttataccg 480
   ggccaccatg gtcgcgagta gcttggcact ggccgtcgtt ttacaacgtc gtgactggga 540
   aaaccctggc gttacccaac ttaatcgcct tgcagcacat ccccctttcg ccagctggcg 600
   taatagcgaa gaggcccgca ccgatcgccc ttcccaacag ttgcgcagcc tgaatggcga 660
   atggcgcttt gcctggtttc cggcaccaga agcggtgccg gaaagctggc tggagtgcga 720
   tcttcctgag gccgatactg tcgtcgtccc ctcaaactgg cagatgcacg gttacgatgc 780
   gcccatctac accaacgtaa cctatcccat tacggtcaat ccgccgtttg ttcccacgga 840
   gaatccgacg ggttgttact cgctcacatt taatgttgat gaaagctggc tacaggaagg 900
   ccagacgcga attatttttg atggcgttaa ctcggcgttt catctgtggt gcaacgggcg 960
   ctgggtcggt tacggccagg acagtcgttt gccgtctgaa tttgacctga gcgcattttt 1020
   acgcgccgga gaaaaccgcc tcgcggtgat ggtgctgcgt tggagtgacg gcagttatct 1080
   ggaagatcag gatatgtggc ggatgagcgg cattttccgt gacgtctcgt tgctgcataa 1140
   accgactaca caaatcagcg atttccatgt tgccactcgc tttaatgatg atttcagccg 1200
   cgctgtactg gaggctgaag ttcagatgtg cggcgagttg cgtgactacc tacgggtaac 1260
   agtttcttta tggcagggtg aaacgcaggt cgccagcggc accgcgcctt tcggcggtga 1320
   aattatcgat gagcgtggtg gttatgccga tcgcgtcaca ctacgtctga acgtcgaaaa 1380
   cccgaaactg tggagcgccg aaatcccgaa tctctatcgt gcggtggttg aactgcacac 1440
   cgccgacggc acgctgattg aagcagaagc ctgcgatgtc ggtttccgcg aggtgcggat 1500
   tgaaaatggt ctgctgctgc tgaacggcaa gccgttgctg attcgaggcg ttaaccgtca 1560
   cgagcatcat cctctgcatg gtcaggtcat ggatgagcag acgatggtgc aggatatcct 1620
   gctgatgaag cagaacaact ttaacgccgt gcgctgttcg cattatccga accatccgct 1680
   gtggtacacg ctgtgcgacc gctacggcct gtatgtggtg gatgaagcca atattgaaac 1740
   ccacggcatg gtgccaatga atcgtctgac cgatgatccg cgctggctac cggcgatgag 1800
   cgaacgcgta acgcgaatgg tgcagcgcga tcgtaatcac ccgagtgtga tcatctggtc 1860
   gctggggaat gaatcaggcc acggcgctaa tcacgacgcg ctgtatcgct ggatcaaatc 1920
   tgtcgatcct tcccgcccgg tgcagtatga aggcggcgga gccgacacca cggccaccga 1980
   tattatttgc ccgatgtacg cgcgcgtgga tgaagaccag cccttcccgg ctgtgccgaa 2040
   atggtccatc aaaaaatggc tttcgctacc tggagagacg cgcccgctga tcctttgcga 2100
   atacgcccac gcgatgggta acagtcttgg cggtttcgct aaatactggc aggcgtttcg 2160
   tcagtatccc cgtttacagg gcggcttcgt ctgggactgg gtggatcagt cgctgattaa 2220
   atatgatgaa aacggcaacc cgtggtcggc ttacggcggt gattttggcg atacgccgaa 2280
   cgatcgccag ttctgtatga acggtctggt ctttgccgac cgcacgccgc atccagcgct 2340
   gacggaagca aaacaccagc agcagttttt ccagttccgt ttatccgggc aaaccatcga 2400
   agtgaccagc gaatacctgt tccgtcatag cgataacgag ctcctgcact ggatggtggc 2460
   gctggatggt aagccgctgg caagcggtga agtgcctctg gatgtcgctc cacaaggtaa 2520
   acagttgatt gaactgcctg aactaccgca gccggagagc gccgggcaac tctggctcac 2580
   agtacgcgta gtgcaaccga acgcgaccgc atggtcagaa gccgggcaca tcagcgcctg 2640
   gcagcagtgg cgtctggcgg aaaacctcag tgtgacgctc cccgccgcgt cccacgccat 2700
   cccgcatctg accaccagcg aaatggattt ttgcatcgag ctgggtaata agcgttggca 2760
   atttaaccgc cagtcaggct ttctttcaca gatgtggatt ggcgataaaa aacaactgct 2820
   gacgccgctg cgcgatcagt tcacccgtgc accgctggat aacgacattg gcgtaagtga 2880
   agcgacccgc attgacccta acgcctgggt cgaacgctgg aaggcggcgg gccattacca 2940
   ggccgaagca gcgttgttgc agtgcacggc agatacactt gctgatgcgg tgctgattac 3000
   gaccgctcac gcgtggcagc atcaggggaa aaccttattt atcagccgga aaacctaccg 3060
   gattgatggt agtggtcaaa tggcgattac cgttgatgtt gaagtggcga gcgatacacc 3120
   gcatccggcg cggattggcc tgaactgcca gctggcgcag gtagcagagc gggtaaactg 3180
   gctcggatta gggccgcaag aaaactatcc cgaccgcctt actgccgcct gttttgaccg 3240
   ctgggatctg ccattgtcag acatgtatac cccgtacgtc ttcccgagcg aaaacggtct 3300
   gcgctgcggg acgcgcgaat tgaattatgg cccacaccag tggcgcggcg acttccagtt 3360
   caacatcagc cgctacagtc aacagcaact gatggaaacc agccatcgcc atctgctgca 3420
   cgcggaagaa ggcacatggc tgaatatcga cggtttccat atggggattg gtggcgacga 3480
   ctcctggagc ccgtcagtat cggcggaatt ccagctgagc gccggtcgct accattacca 3540
   gttggtctgg tgtcaaaaat aataataacc gggcaggggg gatctttgtg aaggaacctt 3600
   acttctgtgg tgtgacataa ttggacaaac tacctacaga gatttaaagc tctaaggtaa 3660
   atataaaatt tttaagtgta taatgtgtta aactactgat tctaattgtt tgtgtatttt 3720
   agattccaac ctatggaact gatgaatggg agcagtggtg gaatgccaga tccagacatg 3780
   ataagataca ttgatgagtt tggacaaacc acaactagaa tgcagtgaaa aaaatgcttt 3840
   atttgtgaaa tttgtgatgc tattgcttta tttgtaacca ttataagctg caataaacaa 3900
   gttaacaaca acaattgcat tcattttatg tttcaggttc agggggaggt gtgggaggtt 3960
   ttttaaagca agtaaaacct ctacaaatgt ggtatggctg attatgatct gcggccgcag 4020
   ggcctcgtga tacgcctatt tttataggtt aatgtcatga taataatggt ttcttagacg 4080
   tcaggtggca cttttcgggg aaatgtgcgc ggaaccccta tttgtttatt tttctaaata 4140
   cattcaaata tgtatccgct catgagacaa taaccctgat aaatgcttca ataatattga 4200
   aaaaggaaga gtatgagtat tcaacatttc cgtgtcgccc ttattccctt ttttgcggca 4260
   ttttgccttc ctgtttttgc tcacccagaa acgctggtga aagtaaaaga tgctgaagat 4320
   cagttgggtg cacgagtggg ttacatcgaa ctggatctca acagcggtaa gatccttgag 4380
   agttttcgcc ccgaagaacg ttttccaatg atgagcactt ttaaagttct gctatgtggc 4440
   gcggtattat cccgtattga cgccgggcaa gagcaactcg gtcgccgcat acactattct 4500
   cagaatgact tggttgagta ctcaccagtc acagaaaagc atcttacgga tggcatgaca 4560
   gtaagagaat tatgcagtgc tgccataacc atgagtgata acactgcggc caacttactt 4620
   ctgacaacga tcggaggacc gaaggagcta accgcttttt tgcacaacat gggggatcat 4680
   gtaactcgcc ttgatcgttg ggaaccggag ctgaatgaag ccataccaaa cgacgagcgt 4740
   gacaccacga tgcctgtagc aatggcaaca acgttgcgca aactattaac tggcgaacta 4800
   cttactctag cttcccggca acaattaata gactggatgg aggcggataa agttgcagga 4860
   ccacttctgc gctcggccct tccggctggc tggtttattg ctgataaatc tggagccggt 4920
   gagcgtgggt ctcgcggtat cattgcagca ctggggccag atggtaagcc ctcccgtatc 4980
   gtagttatct acacgacggg gagtcaggca actatggatg aacgaaatag acagatcgct 5040
   gagataggtg cctcactgat taagcattgg taactgtcag accaagttta ctcatatata 5100
   ctttagattg atttaaaact tcatttttaa tttaaaagga tctaggtgaa gatccttttt 5160
   gataatctca tgaccaaaat cccttaacgt gagttttcgt tccactgagc gtcagacccc 5220
   gtagaaaaga tcaaaggatc ttcttgagat cctttttttc tgcgcgtaat ctgctgcttg 5280
   caaacaaaaa aaccaccgct accagcggtg gtttgtttgc cggatcaaga gctaccaact 5340
   ctttttccga aggtaactgg cttcagcaga gcgcagatac caaatactgt ccttctagtg 5400
   tagccgtagt taggccacca cttcaagaac tctgtagcac cgcctacata cctcgctctg 5460
   ctaatcctgt taccagtggc tgctgccagt ggcgataagt cgtgtcttac cgggttggac 5520
   tcaagacgat agttaccgga taaggcgcag cggtcgggct gaacgggggg ttcgtgcaca 5580
   cagcccagct tggagcgaac gacctacacc gaactgagat acctacagcg tgagctatga 5640
   gaaagcgcca cgcttcccga agggagaaag gcggacaggt atccggtaag cggcagggtc 5700
   ggaacaggag agcgcacgag ggagcttcca gggggaaacg cctggtatct ttatagtcct 5760
   gtcgggtttc gccacctctg acttgagcgt cgatttttgt gatgctcgtc aggggggcgg 5820
   agcctatgga aaaacgccag caacgcggcc tttttacggt tcctggcctt ttgctggcct 5880
   tttgctcaca tgttctttcc tgcgttatcc cctgattctg tggataaccg tattaccgcc 5940
   tttgagtgag ctgataccgc tcgccgcagc cgaacgaccg agcgcagcga gtcagtgagc 6000
   gaggaagcgg aagagcgccc aatacgcaaa ccgcctctcc ccgcgcgttg gccgattcat 6060
   taatgcagct ggcacgacag gtttcccgac tggaaagcgg gcagtgagcg caacgcaatt 6120
   aatgtgagtt agctcactca ttaggcaccc caggctttac actttatgct tccggctcgt 6180
   atgttgtgtg gaattgtgag cggataacaa tttcacacag gaaacagcta tgaccatgat 6240
   tacgccaagc tggcgcg 6257
<210> 16
   <211> 6252
   <212> DNA
   <213> Artificial Sequence
<220>
   <223> Description of Artificial Sequence: vector pRK64 (deltaInt)
<400> 16
   cgtcatcacc gaaacgcgcg aggcagctgt ggaatgtgtg tcagttaggg tgtggaaagt 60
   ccccaggctc cccagcaggc agaagtatgc aaagcatgca tctcaattag tcagcaacca 120
   ggctccccag caggcagaag tgtgcaaagc atgcatctca attagtcagc aaccatagtc 180
   ccgcccctaa ctccgcccat cccgccccta actccgccca gttccgccca ttctccgccc 240
   catggctgac taattttttt tatttatgca gaggccgagg ccgcctcggc ctaggaacag 300
   tcgacgacac tgcagagacc tacttcacta acaaccggta cagttcgtgg accagatggg 360
   tgaggtggag tacgcgcccg gggagcccaa aggttacccc agttggggca ctactcccga 420
   aaaccgcttc tggatccata acttcgtata gcatacatta tacgaagtta taccgggcca 480
   ccatggtcgc gagtagcttg gcactggccg tcgttttaca acgtcgtgac tgggaaaacc 540
   ctggcgttac ccaacttaat cgccttgcag cacatccccc tttcgccagc tggcgtaata 600
   gcgaagaggc ccgcaccgat cgcccttccc aacagttgcg cagcctgaat ggcgaatggc 660
   gctttgcctg gtttccggca ccagaagcgg tgccggaaag ctggctggag tgcgatcttc 720
   ctgaggccga tactgtcgtc gtcccctcaa actggcagat gcacggttac gatgcgccca 780
   tctacaccaa cgtaacctat cccattacgg tcaatccgcc gtttgttccc acggagaatc 840
   cgacgggttg ttactcgctc acatttaatg ttgatgaaag ctggctacag gaaggccaga 900
   cgcgaattat ttttgatggc gttaactcgg cgtttcatct gtggtgcaac gggcgctggg 960
   tcggttacgg ccaggacagt cgtttgccgt ctgaatttga cctgagcgca tttttacgcg 1020
   ccggagaaaa ccgcctcgcg gtgatggtgc tgcgttggag tgacggcagt tatctggaag 1080
   atcaggatat gtggcggatg agcggcattt tccgtgacgt ctcgttgctg cataaaccga 1140
   ctacacaaat cagcgatttc catgttgcca ctcgctttaa tgatgatttc agccgcgctg 1200
   tactggaggc tgaagttcag atgtgcggcg agttgcgtga ctacctacgg gtaacagttt 1260
   ctttatggca gggtgaaacg caggtcgcca gcggcaccgc gcctttcggc ggtgaaatta 1320
   tcgatgagcg tggtggttat gccgatcgcg tcacactacg tctgaacgtc gaaaacccga 1380
   aactgtggag cgccgaaatc ccgaatctct atcgtgcggt ggttgaactg cacaccgccg 1440
   acggcacgct gattgaagca gaagcctgcg atgtcggttt ccgcgaggtg cggattgaaa 1500
   atggtctgct gctgctgaac ggcaagccgt tgctgattcg aggcgttaac cgtcacgagc 1560
   atcatcctct gcatggtcag gtcatggatg agcagacgat ggtgcaggat atcctgctga 1620
   tgaagcagaa caactttaac gccgtgcgct gttcgcatta tccgaaccat ccgctgtggt 1680
   acacgctgtg cgaccgctac ggcctgtatg tggtggatga agccaatatt gaaacccacg 1740
   gcatggtgcc aatgaatcgt ctgaccgatg atccgcgctg gctaccggcg atgagcgaac 1800
   gcgtaacgcg aatggtgcag cgcgatcgta atcacccgag tgtgatcatc tggtcgctgg 1860
   ggaatgaatc aggccacggc gctaatcacg acgcgctgta tcgctggatc aaatctgtcg 1920
   atccttcccg cccggtgcag tatgaaggcg gcggagccga caccacggcc accgatatta 1980
   tttgcccgat gtacgcgcgc gtggatgaag accagccctt cccggctgtg ccgaaatggt 2040
   ccatcaaaaa atggctttcg ctacctggag agacgcgccc gctgatcctt tgcgaatacg 2100
   cccacgcgat gggtaacagt cttggcggtt tcgctaaata ctggcaggcg tttcgtcagt 2160
   atccccgttt acagggcggc ttcgtctggg actgggtgga tcagtcgctg attaaatatg 2220
   atgaaaacgg caacccgtgg tcggcttacg gcggtgattt tggcgatacg ccgaacgatc 2280
   gccagttctg tatgaacggt ctggtctttg ccgaccgcac gccgcatcca gcgctgacgg 2340
   aagcaaaaca ccagcagcag tttttccagt tccgtttatc cgggcaaacc atcgaagtga 2400
   ccagcgaata cctgttccgt catagcgata acgagctcct gcactggatg gtggcgctgg 2460
   atggtaagcc gctggcaagc ggtgaagtgc ctctggatgt cgctccacaa ggtaaacagt 2520
   tgattgaact gcctgaacta ccgcagccgg agagcgccgg gcaactctgg ctcacagtac 2580
   gcgtagtgca accgaacgcg accgcatggt cagaagccgg gcacatcagc gcctggcagc 2640
   agtggcgtct ggcggaaaac ctcagtgtga cgctccccgc cgcgtcccac gccatcccgc 2700
   atctgaccac cagcgaaatg gatttttgca tcgagctggg taataagcgt tggcaattta 2760
   accgccagtc aggctttctt tcacagatgt ggattggcga taaaaaacaa ctgctgacgc 2820
   cgctgcgcga tcagttcacc cgtgcaccgc tggataacga cattggcgta agtgaagcga 2880
   cccgcattga ccctaacgcc tgggtcgaac gctggaaggc ggcgggccat taccaggccg 2940
   aagcagcgtt gttgcagtgc acggcagata cacttgctga tgcggtgctg attacgaccg 3000
   ctcacgcgtg gcagcatcag gggaaaacct tatttatcag ccggaaaacc taccggattg 3060
   atggtagtgg tcaaatggcg attaccgttg atgttgaagt ggcgagcgat acaccgcatc 3120
   cggcgcggat tggcctgaac tgccagctgg cgcaggtagc agagcgggta aactggctcg 3180
   gattagggcc gcaagaaaac tatcccgacc gccttactgc cgcctgtttt gaccgctggg 3240
   atctgccatt gtcagacatg tataccccgt acgtcttccc gagcgaaaac ggtctgcgct 3300
   gcgggacgcg cgaattgaat tatggcccac accagtggcg cggcgacttc cagttcaaca 3360
   tcagccgcta cagtcaacag caactgatgg aaaccagcca tcgccatctg ctgcacgcgg 3420
   aagaaggcac atggctgaat atcgacggtt tccatatggg gattggtggc gacgactcct 3480
   ggagcccgtc agtatcggcg gaattccagc tgagcgccgg tcgctaccat taccagttgg 3540
   tctggtgtca aaaataataa taaccgggca ggggggatct ttgtgaagga accttacttc 3600
   tgtggtgtga cataattgga caaactacct acagagattt aaagctctaa ggtaaatata 3660
   aaatttttaa gtgtataatg tgttaaacta ctgattctaa ttgtttgtgt attttagatt 3720
   ccaacctatg gaactgatga atgggagcag tggtggaatg ccagatccag acatgataag 3780
   atacattgat gagtttggac aaaccacaac tagaatgcag tgaaaaaaat gctttatttg 3840
   tgaaatttgt gatgctattg ctttatttgt aaccattata agctgcaata aacaagttaa 3900
   caacaacaat tgcattcatt ttatgtttca ggttcagggg gaggtgtggg aggtttttta 3960
   aagcaagtaa aacctctaca aatgtggtat ggctgattat gatctgcggc cgcagggcct 4020
   cgtgatacgc ctatttttat aggttaatgt catgataata atggtttctt agacgtcagg 4080
   tggcactttt cggggaaatg tgcgcggaac ccctatttgt ttatttttct aaatacattc 4140
   aaatatgtat ccgctcatga gacaataacc ctgataaatg cttcaataat attgaaaaag 4200
   gaagagtatg agtattcaac atttccgtgt cgcccttatt cccttttttg cggcattttg 4260
   ccttcctgtt tttgctcacc cagaaacgct ggtgaaagta aaagatgctg aagatcagtt 4320
   gggtgcacga gtgggttaca tcgaactgga tctcaacagc ggtaagatcc ttgagagttt 4380
   tcgccccgaa gaacgttttc caatgatgag cacttttaaa gttctgctat gtggcgcggt 4440
   attatcccgt attgacgccg ggcaagagca actcggtcgc cgcatacact attctcagaa 4500
   tgacttggtt gagtactcac cagtcacaga aaagcatctt acggatggca tgacagtaag 4560
   agaattatgc agtgctgcca taaccatgag tgataacact gcggccaact tacttctgac 4620
   aacgatcgga ggaccgaagg agctaaccgc ttttttgcac aacatggggg atcatgtaac 4680
   tcgccttgat cgttgggaac cggagctgaa tgaagccata ccaaacgacg agcgtgacac 4740
   cacgatgcct gtagcaatgg caacaacgtt gcgcaaacta ttaactggcg aactacttac 4800
   tctagcttcc cggcaacaat taatagactg gatggaggcg gataaagttg caggaccact 4860
   tctgcgctcg gcccttccgg ctggctggtt tattgctgat aaatctggag ccggtgagcg 4920
   tgggtctcgc ggtatcattg cagcactggg gccagatggt aagccctccc gtatcgtagt 4980
   tatctacacg acggggagtc aggcaactat ggatgaacga aatagacaga tcgctgagat 5040
   aggtgcctca ctgattaagc attggtaact gtcagaccaa gtttactcat atatacttta 5100
   gattgattta aaacttcatt tttaatttaa aaggatctag gtgaagatcc tttttgataa 5160
   tctcatgacc aaaatccctt aacgtgagtt ttcgttccac tgagcgtcag accccgtaga 5220
   aaagatcaaa ggatcttctt gagatccttt ttttctgcgc gtaatctgct gcttgcaaac 5280
   aaaaaaacca ccgctaccag cggtggtttg tttgccggat caagagctac caactctttt 5340
   tccgaaggta actggcttca gcagagcgca gataccaaat actgtccttc tagtgtagcc 5400
   gtagttaggc caccacttca agaactctgt agcaccgcct acatacctcg ctctgctaat 5460
   cctgttacca gtggctgctg ccagtggcga taagtcgtgt cttaccgggt tggactcaag 5520
   acgatagtta ccggataagg cgcagcggtc gggctgaacg gggggttcgt gcacacagcc 5580
   cagcttggag cgaacgacct acaccgaact gagataccta cagcgtgagc tatgagaaag 5640
   cgccacgctt cccgaaggga gaaaggcgga caggtatccg gtaagcggca gggtcggaac 5700
   aggagagcgc acgagggagc ttccaggggg aaacgcctgg tatctttata gtcctgtcgg 5760
   gtttcgccac ctctgacttg agcgtcgatt tttgtgatgc tcgtcagggg ggcggagcct 5820
   atggaaaaac gccagcaacg cggccttttt acggttcctg gccttttgct ggccttttgc 5880
   tcacatgttc tttcctgcgt tatcccctga ttctgtggat aaccgtatta ccgcctttga 5940
   gtgagctgat accgctcgcc gcagccgaac gaccgagcgc agcgagtcag tgagcgagga 6000
   agcggaagag cgcccaatac gcaaaccgcc tctccccgcg cgttggccga ttcattaatg 6060
   cagctggcac gacaggtttc ccgactggaa agcgggcagt gagcgcaacg caattaatgt 6120
   gagttagctc actcattagg caccccaggc tttacacttt atgcttccgg ctcgtatgtt 6180
   gtgtggaatt gtgagcggat aacaatttca cacaggaaac agctatgacc atgattacgc 6240
   caagctggcg cg                                                    6252
<210> 17
   <211> 25
   <212> DNA
   <213> Artificial Sequence
<220>
   <223> Description of Artificial Sequence: primer P64-1
<400> 17
   tcagcaacca ggctccccag caggc                                      25
<210> 18
   <211> 27
   <212> DNA
   <213> Artificial Sequence
<220>
   <223> Description of Artificial Sequence: primer 64-4
<400> 18
   gacgacagta tcggcctcag gaagatc                                    27
<210> 19
   <211> 840
   <212> DNA
   <213> Artificial Sequence
<220>
   <223> Description of Artificial Sequence: oligonucleotide 80d
<400> 19
   ggtaccgagc tcggatcctc tagtaacggc cgccagtgtg ctggaattcg gcttcagcaa 60
   ccaggctccc cagcaggcag aagtatgcaa agcatgcatc tcaattagtc agcaaccagg 120
   tgtggaaagt ccccaggctc cccagcaggc agaagtatgc aaagcatgca tctcaattag 180
   tcagcaacca tagtcccgcc cctaactccg cccatcccgc ccctaactcc gcccagttcc 240
   gcccattctc cgccccatgg ctgactaatt ttttttattt atgcagaggc cgaggccgcc 300
   tcggcctagg aacagtcgac gacactgcag agacctactt cactaacaac cggtacagtt 360
   cgtggaccag atgggtgagg tggagtacgc gcccggggag cccaaaggtt accccagttg 420
   gggcactact cccgaaaacc gcttctggat ccataacttc gtatagcata cattatacga 480
   agttataccg ggccaccatg gtcgcgagta gcttggcact ggggttgctt ttgcgnygtc 540
   gtgactggga aaaccctggc gttacccaac ttaatcgcct tgcagcacat ccccctttcg 600
   ccagctggcg taatagcgaa gaggcccgca ccgatcgccc ttcccaacag ttgcgcagct 660
   gaatggcgaa tggcgctttg cctggcttcc ggcaccagaa gcggtgccgg aaagctggct 720
   ggagtgcgat cttcctgagg ccgatactgt cgtcaagccg aattctgcag atatccatca 780
   cactggcggc cgctcgagca tgcatctaga gggccaattc gccctatagt gagtcgtatt 840
<210> 20
   <211> 1842
   <212> DNA
   <213> Bacteriophage phi-C31
<220>
   <221> CDS
   <222> (1)..(1839)
<400> 20
   atg aca caa ggg gtt gtg acc ggg gtg gac acg tac gcg ggt gct tac    48
   Met Thr Gln Gly Val Val Thr Gly Val Asp Thr Tyr Ala Gly Ala Tyr
    1               5                  10                  15
   gac cgt cag tcg cgc gag cgc gag aat tcg agc gca gca agc cca gcg    96
   Asp Arg Gln Ser Arg Glu Arg Glu Asn Ser Ser Ala Ala Ser Pro Ala
               20                  25                  30
   aca cag cgt agc gcc aac gaa gac aag gcg gcc gac ctt cag cgc gaa   144
   Thr Gln Arg Ser Ala Asn Glu Asp Lys Ala Ala Asp Leu Gln Arg Glu
           35                  40                  45
   gtc gag cgc gac ggg ggc cgg ttc agg ttc gtc ggg cat ttc agc gaa   192
   Val Glu Arg Asp Gly Gly Arg Phe Arg Phe Val Gly His Phe Ser Glu
       50                  55                  60
   gcg ccg ggc acg tcg gcg ttc ggg acg gcg gag cgc ccg gag ttc gaa   240
   Ala Pro Gly Thr Ser Ala Phe Gly Thr Ala Glu Arg Pro Glu Phe Glu
    65                  70                  75                  80
   cgc atc ctg aac gaa tgc cgc gcc ggg cgg ctc aac atg atc att gtc   288
   Arg Ile Leu Asn Glu Cys Arg Ala Gly Arg Leu Asn Met Ile Ile Val
                   85                  90                  95
   tat gac gtg tcg cgc ttc tcg cgc ctg aag gtc atg gac gcg att ccg   336
   Tyr Asp Val Ser Arg Phe Ser Arg Leu Lys Val Met Asp Ala Ile Pro
               100                 105                 110
   att gtc tcg gaa ttg ctc gcc ctg ggc gtg acg att gtt tcc act cag   384
   Ile Val Ser Glu Leu Leu Ala Leu Gly Val Thr Ile Val Ser Thr Gln
           115                 120                 125
   gaa ggc gtc ttc cgg cag gga aac gtc atg gac ctg att cac ctg att   432
   Glu Gly Val Phe Arg Gln Gly Asn Val Met Asp Leu Ile His Leu Ile
       130                 135                 140
   atg cgg ctc gac gcg tcg cac aaa gaa tct tcg ctg aag tcg gcg aag   480
   Met Arg Leu Asp Ala Ser His Lys Glu Ser Ser Leu Lys Ser Ala Lys
   145                 150                 155                 160
   att ctc gac acg aag aac ctt cag cgc gaa ttg ggc ggg tac gtc ggc   528
   Ile Leu Asp Thr Lys Asn Leu Gln Arg Glu Leu Gly Gly Tyr Val Gly
                   165                 170                 175
   ggg aag gcg cct tac ggc ttc gag ctt gtt tcg gag acg aag gag atc   576
   Gly Lys Ala Pro Tyr Gly Phe Glu Leu Val Ser Glu Thr Lys Glu Ile
               180                 185                 190
   acg cgc aac ggc cga atg gtc aat gtc gtc atc aac aag ctt gcg cac   624
   Thr Arg Asn Gly Arg Met Val Asn Val Val Ile Asn Lys Leu Ala His
           195                 200                 205
   tcg acc act ccc ctt acc gga ccc ttc gag ttc gag ccc gac gta atc   672
   Ser Thr Thr Pro Leu Thr Gly Pro Phe Glu Phe Glu Pro Asp Val Ile
       210                 215                 220
   cgg tgg tgg tgg cgt gag atc aag acg cac aaa cac ctt ccc ttc aag   720
   Arg Trp Trp Trp Arg Glu Ile Lys Thr His Lys His Leu Pro Phe Lys
   225                 230                 235                 240
   ccg ggc agt caa gcc gcc att cac ccg ggc agc atc acg ggg ctt tgt    768
   Pro Gly Ser Gln Ala Ala Ile His Pro Gly Ser Ile Thr Gly Leu Cys
                   245                 250                 255
   aag cgc atg gac gct gac gcc gtg ccg acc cgg ggc gag acg att ggg    816
   Lys Arg Met Asp Ala Asp Ala Val Pro Thr Arg Gly Glu Thr Ile Gly
               260                 265                 270
   aag aag acc gct tca agc gcc tgg gac ccg gca acc gtt atg cga atc    864
   Lys Lys Thr Ala Ser Ser Ala Trp Asp Pro Ala Thr Val Met Arg Ile
           275                 280                 285
   ctt cgg gac ccg cgt att gcg ggc ttc gcc gct gag gtg atc tac aag    912
   Leu Arg Asp Pro Arg Ile Ala Gly Phe Ala Ala Glu Val Ile Tyr Lys
       290                 295                 300
   aag aag ccg gac ggc acg ccg acc acg aag att gag ggt tac cgc att    960
   Lys Lys Pro Asp Gly Thr Pro Thr Thr Lys Ile Glu Gly Tyr Arg Ile
   305                 310                 315                 320
   cag cgc gac ccg atc acg ctc cgg ccg gtc gag ctt gat tgc gga ccg    1008
   Gln Arg Asp Pro Ile Thr Leu Arg Pro Val Glu Leu Asp Cys Gly Pro
                   325                 330                 335
   atc atc gag ccc gct gag tgg tat gag ctt cag gcg tgg ttg gac ggc    1056
   Ile Ile Glu Pro Ala Glu Trp Tyr Glu Leu Gln Ala Trp Leu Asp Gly
               340                 345                 350
   agg ggg cgc ggc aag ggg ctt tcc cgg ggg caa gcc att ctg tcc gcc    1104
   Arg Gly Arg Gly Lys Gly Leu Ser Arg Gly Gln Ala Ile Leu Ser Ala
           355                 360                 365
   atg gac aag ctg tac tgc gag tgt ggc gcc gtc atg act tcg aag cgc    1152
   Met Asp Lys Leu Tyr Cys Glu Cys Gly Ala Val Met Thr Ser Lys Arg
       370                 375                 380
   ggg gaa gaa tcg atc aag gac tct tac cgc tgc cgt cgc cgg aag gtg    1200
   Gly Glu Glu Ser Ile Lys Asp Ser Tyr Arg Cys Arg Arg Arg Lys Val
   385                 390                 395                 400
   gtc gac ccg tcc gca cct ggg cag cac gaa ggc acg tgc aac gtc agc    1248
   Val Asp Pro Ser Ala Pro Gly Gln His Glu Gly Thr Cys Asn Val Ser
                   405                 410                 415
   atg gcg gca ctc gac aag ttc gtt gcg gaa cgc atc ttc aac aag atc    1296
   Met Ala Ala Leu Asp Lys Phe Val Ala Glu Arg Ile Phe Asn Lys Ile
               420                 425                 430
   agg cac gcc gaa ggc gac gaa gag acg ttg gcg ctt ctg tgg gaa gcc    1344
   Arg His Ala Glu Gly Asp Glu Glu Thr Leu Ala Leu Leu Trp Glu Ala
           435                 440                 445
   gcc cga cgc ttc ggc aag ctc act gag gcg cct gag aag agc ggc gaa    1392
   Ala Arg Arg Phe Gly Lys Leu Thr Glu Ala Pro Glu Lys Ser Gly Glu
       450                 455                 460
   cgg gcg aac ctt gtt gcg gag cgc gcc gac gcc ctg aac gcc ctt gaa    1440
   Arg Ala Asn Leu Val Ala Glu Arg Ala Asp Ala Leu Asn Ala Leu Glu
   465                 470                 475                 480
   gag ctg tac gaa gac cgc gcg gca ggc gcg tac gac gga ccc gtt ggc    1488
   Glu Leu Tyr Glu Asp Arg Ala Ala Gly Ala Tyr Asp Gly Pro Val Gly
                   485                 490                 495
   agg aag cac ttc cgg aag caa cag gca gcg ctg acg ctc cgg cag caa    1536
   Arg Lys His Phe Arg Lys Gln Gln Ala Ala Leu Thr Leu Arg Gln Gln
               500                 505                 510
   ggg gcg gaa gag cgg ctt gcc gaa ctt gaa gcc gcc gaa gcc ccg aag    1584
   Gly Ala Glu Glu Arg Leu Ala Glu Leu Glu Ala Ala Glu Ala Pro Lys
           515                 520                 525
   ctt ccc ctt gac caa tgg ttc ccc gaa gac gcc gac gct gac ccg acc    1632
   Leu Pro Leu Asp Gln Trp Phe Pro Glu Asp Ala Asp Ala Asp Pro Thr
       530                 535                 540
   ggc cct aag tcg tgg tgg ggg cgc gcg tca gta gac gac aag cgc gtg    1680
   Gly Pro Lys Ser Trp Trp Gly Arg Ala Ser Val Asp Asp Lys Arg Val
   545                 550                 555                 560
   ttc gtc ggg ctc ttc gta gac aag atc gtt gtc acg aag tcg act acg    1728
   Phe Val Gly Leu Phe Val Asp Lys Ile Val Val Thr Lys Ser Thr Thr
                   565                 570                 575
   ggc agg ggg cag gga acg ccc atc gag aag cgc gct tcg atc acg tgg    1776
   Gly Arg Gly Gln Gly Thr Pro Ile Glu Lys Arg Ala Ser Ile Thr Trp
               580                 585                 590
   gcg aag ccg ccg acc gac gac gac gaa gac gac gcc cag gac ggc acg    1824
   Ala Lys Pro Pro Thr Asp Asp Asp Glu Asp Asp Ala Gln Asp Gly Thr
           595                 600                 605
   gaa gac gta gcg gcg tag                                            1842
   Glu Asp Val Ala Ala
       610
<210> 21
   <211> 613
   <212> PRT
   <213> Bacteriophage phi-C31
<400> 21
   Met Thr Gln Gly Val Val Thr Gly Val Asp Thr Tyr Ala Gly Ala Tyr
    1               5                  10                  15
   Asp Arg Gln Ser Arg Glu Arg Glu Asn Ser Ser Ala Ala Ser Pro Ala
               20                  25                  30
   Thr Gln Arg Ser Ala Asn Glu Asp Lys Ala Ala Asp Leu Gln Arg Glu
           35                  40                  45
   Val Glu Arg Asp Gly Gly Arg Phe Arg Phe Val Gly His Phe Ser Glu
       50                  55                  60
   Ala Pro Gly Thr Ser Ala Phe Gly Thr Ala Glu Arg Pro Glu Phe Glu
   65                  70                  75                  80
   Arg Ile Leu Asn Glu Cys Arg Ala Gly Arg Leu Asn Met Ile Ile Val
                   85                  90                  95
   Tyr Asp Val Ser Arg Phe Ser Arg Leu Lys Val Met Asp Ala Ile Pro
               100                 105                 110
   Ile Val Ser Glu Leu Leu Ala Leu Gly Val Thr Ile Val Ser Thr Gln
           115                 120                 125
   Glu Gly Val Phe Arg Gln Gly Asn Val Met Asp Leu Ile His Leu Ile
       130                 135                 140
   Met Arg Leu Asp Ala Ser His Lys Glu Ser Ser Leu Lys Ser Ala Lys
   145                 150                 155                 160
   Ile Leu Asp Thr Lys Asn Leu Gln Arg Glu Leu Gly Gly Tyr Val Gly
                   165                 170                 175
   Gly Lys Ala Pro Tyr Gly Phe Glu Leu Val Ser Glu Thr Lys Glu Ile
               180                 185                 190
   Thr Arg Asn Gly Arg Met Val Asn Val Val Ile Asn Lys Leu Ala His
           195                 200                 205
   Ser Thr Thr Pro Leu Thr Gly Pro Phe Glu Phe Glu Pro Asp Val Ile
       210                 215                 220
   Arg Trp Trp Trp Arg Glu Ile Lys Thr His Lys His Leu Pro Phe Lys
   225                 230                 235                 240
   Pro Gly Ser Gln Ala Ala Ile His Pro Gly Ser Ile Thr Gly Leu Cys
                   245                 250                 255
   Lys Arg Met Asp Ala Asp Ala Val Pro Thr Arg Gly Glu Thr Ile Gly
               260                 265                 270
   Lys Lys Thr Ala Ser Ser Ala Trp Asp Pro Ala Thr Val Met Arg Ile
           275                 280                 285
   Leu Arg Asp Pro Arg Ile Ala Gly Phe Ala Ala Glu Val Ile Tyr Lys
       290                 295                 300
   Lys Lys Pro Asp Gly Thr Pro Thr Thr Lys Ile Glu Gly Tyr Arg Ile
   305                 310                 315                 320
   Gln Arg Asp Pro Ile Thr Leu Arg Pro Val Glu Leu Asp Cys Gly Pro
                   325                 330                 335
   Ile Ile Glu Pro Ala Glu Trp Tyr Glu Leu Gln Ala Trp Leu Asp Gly
               340                 345                 350
   Arg Gly Arg Gly Lys Gly Leu Ser Arg Gly Gln Ala Ile Leu Ser Ala
           355                 360                 365
   Met Asp Lys Leu Tyr Cys Glu Cys Gly Ala Val Met Thr Ser Lys Arg
       370                 375                 380
   Gly Glu Glu Ser Ile Lys Asp Ser Tyr Arg Cys Arg Arg Arg Lys Val
   385                 390                 395                 400
   Val Asp Pro Ser Ala Pro Gly Gln His Glu Gly Thr Cys Asn Val Ser
                   405                 410                 415
   Met Ala Ala Leu Asp Lys Phe Val Ala Glu Arg Ile Phe Asn Lys Ile
               420                 425                 430
   Arg His Ala Glu Gly Asp Glu Glu Thr Leu Ala Leu Leu Trp Glu Ala
           435                 440                 445
   Ala Arg Arg Phe Gly Lys Leu Thr Glu Ala Pro Glu Lys Ser Gly Glu
       450                 455                 460
   Arg Ala Asn Leu Val Ala Glu Arg Ala Asp Ala Leu Asn Ala Leu Glu
   465                 470                 475                 480
   Glu Leu Tyr Glu Asp Arg Ala Ala Gly Ala Tyr Asp Gly Pro Val Gly
                   485                 490                 495
   Arg Lys His Phe Arg Lys Gln Gln Ala Ala Leu Thr Leu Arg Gln Gln
               500                 505                 510
   Gly Ala Glu Glu Arg Leu Ala Glu Leu Glu Ala Ala Glu Ala Pro Lys
           515                 520                 525
   Leu Pro Leu Asp Gln Trp Phe Pro Glu Asp Ala Asp Ala Asp Pro Thr
       530                 535                 540
   Gly Pro Lys Ser Trp Trp Gly Arg Ala Ser Val Asp Asp Lys Arg Val
   545                 550                 555                 560
   Phe Val Gly Leu Phe Val Asp Lys Ile Val Val Thr Lys Ser Thr Thr
                   565                 570                 575
   Gly Arg Gly Gln Gly Thr Pro Ile Glu Lys Arg Ala Ser Ile Thr Trp
               580                 585                 590
   Ala Lys Pro Pro Thr Asp Asp Asp Glu Asp Asp Ala Gln Asp Gly Thr
           595                 600                 605
   Glu Asp Val Ala Ala
       610
<210> 22
   <211> 1863
   <212> DNA
   <213> Artificial Sequence
<220>
   <223> Description of Artificial Sequence: DNA sequence coding for fusion protein C31-Int(CNLS)
<220>
   <221> CDS
   <222> (1)..(1860)
<400> 22
   atg aca caa ggg gtt gtg acc ggg gtg gac acg tac gcg ggt gct tac    48
   Met Thr Gln Gly Val Val Thr Gly Val Asp Thr Tyr Ala Gly Ala Tyr
    1               5                  10                  15
   gac cgt cag tcg cgc gag cgc gag aat tcg agc gca gca agc cca gcg    96
   Asp Arg Gln Ser Arg Glu Arg Glu Asn Ser Ser Ala Ala Ser Pro Ala
               20                  25                  30
   aca cag cgt agc gcc aac gaa gac aag gcg gcc gac ctt cag cgc gaa    144
   Thr Gln Arg Ser Ala Asn Glu Asp Lys Ala Ala Asp Leu Gln Arg Glu
           35                  40                  45
   gtc gag cgc gac ggg ggc cgg ttc agg ttc gtc ggg cat ttc agc gaa    192
   Val Glu Arg Asp Gly Gly Arg Phe Arg Phe Val Gly His Phe Ser Glu
       50                  55                  60
   gcg ccg ggc acg tcg gcg ttc ggg acg gcg gag cgc ccg gag ttc gaa    240
   Ala Pro Gly Thr Ser Ala Phe Gly Thr Ala Glu Arg Pro Glu Phe Glu
   65                  70                  75                  80
   cgc atc ctg aac gaa tgc cgc gcc ggg cgg ctc aac atg atc att gtc    288
   Arg Ile Leu Asn Glu Cys Arg Ala Gly Arg Leu Asn Met Ile Ile Val
                   85                  90                  95
   tat gac gtg tcg cgc ttc tcg cgc ctg aag gtc atg gac gcg att ccg    336
   Tyr Asp Val Ser Arg Phe Ser Arg Leu Lys Val Met Asp Ala Ile Pro
               100                 105                 110
   att gtc tcg gaa ttg ctc gcc ctg ggc gtg acg att gtt tcc act cag    384
   Ile Val Ser Glu Leu Leu Ala Leu Gly Val Thr Ile Val Ser Thr Gln
           115                 120                 125
   gaa ggc gtc ttc cgg cag gga aac gtc atg gac ctg att cac ctg att     432
   Glu Gly Val Phe Arg Gln Gly Asn Val Met Asp Leu Ile His Leu Ile
       130                 135                 140
   atg cgg ctc gac gcg tcg cac aaa gaa tct tcg ctg aag tcg gcg aag     480
   Met Arg Leu Asp Ala Ser His Lys Glu Ser Ser Leu Lys Ser Ala Lys
   145                 150                 155                 160
   att ctc gac acg aag aac ctt cag cgc gaa ttg ggc ggg tac gtc ggc     528
   Ile Leu Asp Thr Lys Asn Leu Gln Arg Glu Leu Gly Gly Tyr Val Gly
                   165                 170                 175
   ggg aag gcg cct tac ggc ttc gag ctt gtt tcg gag acg aag gag atc     576
   Gly Lys Ala Pro Tyr Gly Phe Glu Leu Val Ser Glu Thr Lys Glu Ile
               180                 185                 190
   acg cgc aac ggc cga atg gtc aat gtc gtc atc aac aag ctt gcg cac     624
   Thr Arg Asn Gly Arg Met Val Asn Val Val Ile Asn Lys Leu Ala His
           195                 200                 205
   tcg acc act ccc ctt acc gga ccc ttc gag ttc gag ccc gac gta atc     672
   Ser Thr Thr Pro Leu Thr Gly Pro Phe Glu Phe Glu Pro Asp Val Ile
       210                 215                 220
   cgg tgg tgg tgg cgt gag atc aag acg cac aaa cac ctt ccc ttc aag     720
   Arg Trp Trp Trp Arg Glu Ile Lys Thr His Lys His Leu Pro Phe Lys
   225                 230                 235                 240
   ccg ggc agt caa gcc gcc att cac ccg ggc agc atc acg ggg ctt tgt     768
   Pro Gly Ser Gln Ala Ala Ile His Pro Gly Ser Ile Thr Gly Leu Cys
                   245                 250                 255
   aag cgc atg gac gct gac gcc gtg ccg acc cgg ggc gag acg att ggg     816
   Lys Arg Met Asp Ala Asp Ala Val Pro Thr Arg Gly Glu Thr Ile Gly
               260                 265                 270
   aag aag acc gct tca agc gcc tgg gac ccg gca acc gtt atg cga atc     864
   Lys Lys Thr Ala Ser Ser Ala Trp Asp Pro Ala Thr Val Met Arg Ile
           275                 280                 285
   ctt cgg gac ccg cgt att gcg ggc ttc gcc gct gag gtg atc tac aag     912
   Leu Arg Asp Pro Arg Ile Ala Gly Phe Ala Ala Glu Val Ile Tyr Lys
       290                 295                 300
   aag aag ccg gac ggc acg ccg acc acg aag att gag ggt tac cgc att     960
   Lys Lys Pro Asp Gly Thr Pro Thr Thr Lys Ile Glu Gly Tyr Arg Ile
   305                 310                 315                 320
   cag cgc gac ccg atc acg ctc cgg ccg gtc gag ctt gat tgc gga ccg     1008
   Gln Arg Asp Pro Ile Thr Leu Arg Pro Val Glu Leu Asp Cys Gly Pro
                   325                 330                 335
   atc atc gag ccc gct gag tgg tat gag ctt cag gcg tgg ttg gac ggc     1056
   Ile Ile Glu Pro Ala Glu Trp Tyr Glu Leu Gln Ala Trp Leu Asp Gly
               340                 345                 350
   agg ggg cgc ggc aag ggg ctt tcc cgg ggg caa gcc att ctg tcc gcc     1104
   Arg Gly Arg Gly Lys Gly Leu Ser Arg Gly Gln Ala Ile Leu Ser Ala
           355                 360                 365
   atg gac aag ctg tac tgc gag tgt ggc gcc gtc atg act tcg aag cgc     1152
   Met Asp Lys Leu Tyr Cys Glu Cys Gly Ala Val Met Thr Ser Lys Arg
       370                 375                 380
   ggg gaa gaa tcg atc aag gac tct tac cgc tgc cgt cgc cgg aag gtg     1200
   Gly Glu Glu Ser Ile Lys Asp Ser Tyr Arg Cys Arg Arg Arg Lys Val
   385                 390                 395                 400
   gtc gac ccg tcc gca cct ggg cag cac gaa ggc acg tgc aac gtc agc    1248
   Val Asp Pro Ser Ala Pro Gly Gln His Glu Gly Thr Cys Asn Val Ser
                   405                 410                 415
   atg gcg gca ctc gac aag ttc gtt gcg gaa cgc atc ttc aac aag atc    1296
   Met Ala Ala Leu Asp Lys Phe Val Ala Glu Arg Ile Phe Asn Lys Ile
               420                 425                 430
   agg cac gcc gaa ggc gac gaa gag acg ttg gcg ctt ctg tgg gaa gcc    1344
   Arg His Ala Glu Gly Asp Glu Glu Thr Leu Ala Leu Leu Trp Glu Ala
           435                 440                 445
   gcc cga cgc ttc ggc aag ctc act gag gcg cct gag aag agc ggc gaa    1392
   Ala Arg Arg Phe Gly Lys Leu Thr Glu Ala Pro Glu Lys Ser Gly Glu
       450                 455                 460
   cgg gcg aac ctt gtt gcg gag cgc gcc gac gcc ctg aac gcc ctt gaa    1440
   Arg Ala Asn Leu Val Ala Glu Arg Ala Asp Ala Leu Asn Ala Leu Glu
   465                 470                 475                 480
   gag ctg tac gaa gac cgc gcg gca ggc gcg tac gac gga ccc gtt ggc    1488
   Glu Leu Tyr Glu Asp Arg Ala Ala Gly Ala Tyr Asp Gly Pro Val Gly
                   485                 490                 495
   agg aag cac ttc cgg aag caa cag gca gcg ctg acg ctc cgg cag caa    1536
   Arg Lys His Phe Arg Lys Gln Gln Ala Ala Leu Thr Leu Arg Gln Gln
               500                 505                 510
   ggg gcg gaa gag cgg ctt gcc gaa ctt gaa gcc gcc gaa gcc ccg aag    1584
   Gly Ala Glu Glu Arg Leu Ala Glu Leu Glu Ala Ala Glu Ala Pro Lys
           515                 520                 525
   ctt ccc ctt gac caa tgg ttc ccc gaa gac gcc gac gct gac ccg acc    1632
   Leu Pro Leu Asp Gln Trp Phe Pro Glu Asp Ala Asp Ala Asp Pro Thr
       530                 535                 540
   ggc cct aag tcg tgg tgg ggg cgc gcg tca gta gac gac aag cgc gtg    1680
   Gly Pro Lys Ser Trp Trp Gly Arg Ala Ser Val Asp Asp Lys Arg Val
   545                 550                 555                 560
   ttc gtc ggg ctc ttc gta gac aag atc gtt gtc acg aag tcg act acg    1728
   Phe Val Gly Leu Phe Val Asp Lys Ile Val Val Thr Lys Ser Thr Thr
                   565                 570                 575
   ggc agg ggg cag gga acg ccc atc gag aag cgc gct tcg atc acg tgg    1776
   Gly Arg Gly Gln Gly Thr Pro Ile Glu Lys Arg Ala Ser Ile Thr Trp
               580                 585                 590
   gcg aag ccg ccg acc gac gac gac gaa gac gac gcc cag gac ggc acg    1824
   Ala Lys Pro Pro Thr Asp Asp Asp Glu Asp Asp Ala Gln Asp Gly Thr
           595                 600                 605
   gaa gac gta gcg gcg cct aag aag aag agg aag gtt tag                1863
   Glu Asp Val Ala Ala Pro Lys Lys Lys Arg Lys Val
       610                 615                 620
<210> 23
   <211> 620
   <212> PRT
   <213> Artificial Sequence
   <223> Description of Artificial Sequence: DNA sequence coding for fusion protein C31-Int(CNLS)
<400> 23
   Met Thr Gln Gly Val Val Thr Gly Val Asp Thr Tyr Ala Gly Ala Tyr
   1               5                   10                  15
   Asp Arg Gln Ser Arg Glu Arg Glu Asn Ser Ser Ala Ala Ser Pro Ala
               20                  25                  30
   Thr Gln Arg Ser Ala Asn Glu Asp Lys Ala Ala Asp Leu Gln Arg Glu
           35                  40                  45
   Val Glu Arg Asp Gly Gly Arg Phe Arg Phe Val Gly His Phe Ser Glu
       50                  55                  60
   Ala Pro Gly Thr Ser Ala Phe Gly Thr Ala Glu Arg Pro Glu Phe Glu
   65                  70                  75                  80
   Arg Ile Leu Asn Glu Cys Arg Ala Gly Arg Leu Asn Met Ile Ile Val
                   85                  90                  95
   Tyr Asp Val Ser Arg Phe Ser Arg Leu Lys Val Met Asp Ala Ile Pro
               100                 105                 110
   Ile Val Ser Glu Leu Leu Ala Leu Gly Val Thr Ile Val Ser Thr Gln
           115                 120                 125
   Glu Gly Val Phe Arg Gln Gly Asn Val Met Asp Leu Ile His Leu Ile
       130                 135                 140
   Met Arg Leu Asp Ala Ser His Lys Glu Ser Ser Leu Lys Ser Ala Lys
   145                 150                 155                 160
   Ile Leu Asp Thr Lys Asn Leu Gln Arg Glu Leu Gly Gly Tyr Val Gly
                   165                 170                 175
   Gly Lys Ala Pro Tyr Gly Phe Glu Leu Val Ser Glu Thr Lys Glu Ile
               180                 185                 190
   Thr Arg Asn Gly Arg Met Val Asn Val Val Ile Asn Lys Leu Ala His
           195                 200                 205
   Ser Thr Thr Pro Leu Thr Gly Pro Phe Glu Phe Glu Pro Asp Val Ile
       210                 215                 220
   Arg Trp Trp Trp Arg Glu Ile Lys Thr His Lys His Leu Pro Phe Lys
   225                 230                 235                 240
   Pro Gly Ser Gln Ala Ala Ile His Pro Gly Ser Ile Thr Gly Leu Cys
                   245                 250                 255
   Lys Arg Met Asp Ala Asp Ala Val Pro Thr Arg Gly Glu Thr Ile Gly
               260                 265                 270
   Lys Lys Thr Ala Ser Ser Ala Trp Asp Pro Ala Thr Val Met Arg Ile
           275                 280                 285
   Leu Arg Asp Pro Arg Ile Ala Gly Phe Ala Ala Glu Val Ile Tyr Lys
       290                 295                 300
   Lys Lys Pro Asp Gly Thr Pro Thr Thr Lys Ile Glu Gly Tyr Arg Ile
   305                 310                 315                 320
   Gln Arg Asp Pro Ile Thr Leu Arg Pro Val Glu Leu Asp Cys Gly Pro
                   325                 330                 335
   Ile Ile Glu Pro Ala Glu Trp Tyr Glu Leu Gln Ala Trp Leu Asp Gly
               340                 345                 350
   Arg Gly Arg Gly Lys Gly Leu Ser Arg Gly Gln Ala Ile Leu Ser Ala
           355                 360                 365
   Met Asp Lys Leu Tyr Cys Glu Cys Gly Ala Val Met Thr Ser Lys Arg
       370                 375                 380
   Gly Glu Glu Ser Ile Lys Asp Ser Tyr Arg Cys Arg Arg Arg Lys Val
   385                 390                 395                 400
   Val Asp Pro Ser Ala Pro Gly Gln His Glu Gly Thr Cys Asn Val Ser
                   405                 410                 415
   Met Ala Ala Leu Asp Lys Phe Val Ala Glu Arg Ile Phe Asn Lys Ile
               420                 425                 430
   Arg His Ala Glu Gly Asp Glu Glu Thr Leu Ala Leu Leu Trp Glu Ala
           435                 440                 445
   Ala Arg Arg Phe Gly Lys Leu Thr Glu Ala Pro Glu Lys Ser Gly Glu
       450                 455                 460
   Arg Ala Asn Leu Val Ala Glu Arg Ala Asp Ala Leu Asn Ala Leu Glu
   465                 470                 475                 480
   Glu Leu Tyr Glu Asp Arg Ala Ala Gly Ala Tyr Asp Gly Pro Val Gly
                   485                 490                 495
   Arg Lys His Phe Arg Lys Gln Gln Ala Ala Leu Thr Leu Arg Gln Gln
               500                 505                 510
   Gly Ala Glu Glu Arg Leu Ala Glu Leu Glu Ala Ala Glu Ala Pro Lys
           515                 520                 525
   Leu Pro Leu Asp Gln Trp Phe Pro Glu Asp Ala Asp Ala Asp Pro Thr
       530                 535                 540
   Gly Pro Lys Ser Trp Trp Gly Arg Ala Ser Val Asp Asp Lys Arg Val
   545                 550                 555                 560
   Phe Val Gly Leu Phe Val Asp Lys Ile Val Val Thr Lys Ser Thr Thr
                   565                 570                 575
   Gly Arg Gly Gln Gly Thr Pro Ile Glu Lys Arg Ala Ser Ile Thr Trp
               580                 585                 590
   Ala Lys Pro Pro Thr Asp Asp Asp Glu Asp Asp Ala Gln Asp Gly Thr
           595                 600                 605
   Glu Asp Val Ala Ala Pro Lys Lys Lys Arg Lys Val
       610                 615                 620
<210> 24
   <211> 43
   <212> PRT
   <213> Artificial Sequence
<220>
   <223> Description of Artificial Sequence: NLS
<400> 24
   Met Lys Lys Lys Lys Lys Lys Lys Lys Lys Lys Lys Cys Arg Leu Lys
   1 5 10 15
   Lys Leu Lys Cys Ser Lys Glu Lys Pro Lys Cys Ala Lys Cys Leu Lys
   20 25 30
   Lys Lys Lys Lys Arg Arg Arg Lys Thr Lys Arg
   35 40
<210> 25
   <211> 10
   <212> PRT
   <213> Artificial Sequence
<220>
   <223> Description of Artificial Sequence: NLS
<400> 25
   Ile Lys Tyr Phe Lys Lys Phe Pro Lys Asp
   1 5 10
<210> 26
   <211> 14
   <212> PRT
   <213> Artificial Sequence
<220>
   <223> Description of Artificial Sequence: NLS
<400> 26
   Met Thr Gly Ser Lys Thr Arg Lys His Arg Gly Ser Gly Ala
   1 5 10
<210> 27
   <211> 14
   <212> PRT
   <213> Artificial Sequence
<220>
   <223> Description of Artificial Sequence: NLS
<400> 27
   Met Thr Gly Ser Lys His Arg Lys His Pro Gly Ser Gly Ala
   1 5 10
<210> 28
   <211> 7
   <212> PRT
   <213> Artificial Sequence
<220>
   <223> Description of Artificial Sequence: NLS
<400> 28
   Gly Lys Lys Arg Ser Lys Ala
   1 5
<210> 29
   <211> 14
   <212> PRT
   <213> Artificial Sequence
<220>
   <223> Description of Artificial Sequence: NLS
<400> 29
   Pro Lys Lys Ala Arg Glu Asp Val Ser Arg Lys Arg Pro Arg
   1 5 10
<210> 30
   <211> 11
   <212> PRT
   <213> Artificial Sequence
<220>
   <223> Description of Artificial Sequence: NLS
<400> 30
   Ala Pro Lys Arg Lys Ser Gly Val Ser Lys Cys
   1 5 10
<210> 31
   <211> 12
   <212> PRT
   <213> Artificial Sequence
<220>
   <223> Description of Artificial Sequence: NLS
<400> 31
   Glu Glu Asp Gly Pro Gln Lys Lys Lys Arg Arg Leu
   1 5 10
<210> 32
   <211> 8
   <212> PRT
   <213> Artificial Sequence
<220>
   <223> Description of Artificial Sequence: NLS
<400> 32
   Ala Pro Thr Lys Arg Lys Gly Ser
   1 5
<210> 33
   <211> 7
   <212> PRT
   <213> Artificial Sequence
<220>
   <223> Description of Artificial Sequence: NLS
<400> 33
   Pro Asn Lys Lys Lys Arg Lys
   1 5
<210> 34
   <211> 5
   <212> PRT
   <213> Artificial Sequence
<220>
   <223> Description of Artificial Sequence: NLS
<400> 34
   Lys Arg Pro Arg Pro
   1 5
<210> 35
   <211> 11
   <212> PRT
   <213> Artificial Sequence
<220>
   <223> Description of Artificial Sequence: NLS
<400> 35
   Cys Gly Gly Leu Ser Ser Lys Arg Pro Arg Pro
   1 5 10
<210> 36
   <211> 19
   <212> PRT
   <213> Artificial Sequence
<220>
   <223> Description of Artificial Sequence: NLS
<400> 36
   Pro Pro Lys Lys Arg Met Arg Arg Arg Ile Glu Pro Lys Lys Lys Lys
   1 5 10 15
   Lys Arg Pro
<210> 37
   <211> 11
   <212> PRT
   <213> Artificial Sequence
<220>
   <223> Description of Artificial Sequence: NLS
<400> 37
   Pro Phe Leu Asp Arg Leu Arg Arg Asp Gln Lys
   1 5 10
<210> 38
   <211> 9
   <212> PRT
   <213> Artificial Sequence
<220>
   <223> Description of Artificial Sequence: NLS
<400> 38
   Pro Lys Gln Lys Arg Lys Met Ala Arg
   1 5
<210> 39
   <211> 9
   <212> PRT
   <213> Artificial Sequence
<220>
   <223> Description of Artificial Sequence: NLS
<400> 39
   Ser Val Thr Lys Lys Arg Lys Leu Glu
   1 5
<210> 40
   <211> 11
   <212> PRT
   <213> Artificial Sequence
<220>
   <223> Description of Artificial Sequence: NLS
<400> 40
   Cys Gly Gly Ala Ala Lys Arg Val Lys Leu Asp
   1 5 10
<210> 41
   <211> 9
   <212> PRT
   <213> Artificial Sequence
<220>
   <223> Description of Artificial Sequence: NLS
<400> 41
   Pro Ala Ala Lys Arg Val Lys Leu Asp
   1 5
<210> 42
   <211> 11
   <212> PRT
   <213> Artificial Sequence
<220>
   <223> Description of Artificial Sequence: NLS
<400> 42
   Arg Gln Arg Arg Asn Glu Leu Lys Arg Ser Pro
   1 5 10
<210> 43
   <211> 8
   <212> PRT
   <213> Artificial Sequence
<220>
   <223> Description of Artificial Sequence: NLS
<400> 43
   Pro Gln Ser Arg Lys Lys Leu Arg
   1 5
<210> 44
   <211> 8
   <212> PRT
   <213> Artificial Sequence
<220>
   <223> Description of Artificial Sequence: NLS
<400> 44
   Pro Leu Leu Lys Lys Ile Lys Gln
   1 5
<210> 45
   <211> 7
   <212> PRT
   <213> Artificial Sequence
<220>
   <223> Description of Artificial Sequence: NLS
<400> 45
   Pro Gln Pro Lys Lys Lys Pro
   1 5
<210> 46
   <211> 9
   <212> PRT
   <213> Artificial Sequence
<220>
   <223> Description of Artificial Sequence: NLS
<400> 46
   Ser Lys Arg Val Ala Lys Arg Lys Leu
   1 5
<210> 47
   <211> 9
   <212> PRT
   <213> Artificial Sequence
<220>
   <223> Description of Artificial Sequence: NLS
<400> 47
   Ala Ser Lys Ser Arg Lys Arg Lys Leu
   1 5
<210> 48
   <211> 16
   <212> PRT
   <213> Artificial Sequence
<220>
   <223> Description of Artificial Sequence: NLS
<400> 48
   Gly Gly Leu Cys Ser Ala Arg Leu His Arg His Ala Leu Leu Ala Thr
   1 5 10 15
<210> 49
   <211> 8
   <212> PRT
   <213> Artificial Sequence
<220>
   <223> Description of Artificial Sequence: NLS
<400> 49
   Arg Lys Thr Lys Lys Lys Ile Lys
   1 5
<210> 50
   <211> 8
   <212> PRT
   <213> Artificial Sequence
<220>
   <223> Description of Artificial Sequence: NLS
<400> 50
   Arg Lys Leu Lys Lys Leu Gly Asn
   1 5
<210> 51
   <211> 8
   <212> PRT
   <213> Artificial Sequence
<220>
   <223> Description of Artificial Sequence: NLS
<400> 51
   Arg Lys Asp Arg Arg Gly Gly Arg
   1 5
<210> 52
   <211> 18
   <212> PRT
   <213> Artificial Sequence
<220>
   <223> Description of Artificial Sequence: NLS
<400> 52
   Asp Thr Arg Glu Lys Lys Lys Phe Leu Lys Arg Arg Leu Leu Arg Leu
   1               5                   10                  15
   Asp Glu
<210> 53
   <211> 7
   <212> PRT
   <213> Artificial Sequence
<220>
   <223> Description of Artificial Sequence: NLS
<400> 53
   Pro Lys Lys Lys Arg Lys Val
   1               5
<210> 54
   <211> 1410
   <212> DNA
   <213> Bacteriophage R4
<220>
   <221> CDS
   <222> (1)..(1407)
<400> 54
   atg aat cga ggg ggg ccc act gta cgg gcc gac atc tac gtc cga atc    48
   Met Asn Arg Gly Gly Pro Thr Val Arg Ala Asp Ile Tyr Val Arg Ile
   1               5                   10                  15
   agc ctg gac cgc aca ggg gaa gag ctc ggg gtc gag cgc cag gag gag    96
   Ser Leu Asp Arg Thr Gly Glu Glu Leu Gly Val Glu Arg Gln Glu Glu
                   20                  25                  30
   tcg tgt cgc gag ctc tgc aag agc ctc ggc atg gag gtg ggc cag gtg    144
   Ser Cys Arg Glu Leu Cys Lys Ser Leu Gly Met Glu Val Gly Gln Val
               35                  40                  45
   tgg gtc gac aac gac ctg agc gcc acc aag aag aac gtc gtc cgc cct    192
   Trp Val Asp Asn Asp Leu Ser Ala Thr Lys Lys Asn Val Val Arg Pro
       50                  55                  60
   gac ttc gag gcg atg atc gcg agc aac ccg cag gcg atc gtc tgc tgg    240
   Asp Phe Glu Ala Met Ile Ala Ser Asn Pro Gln Ala Ile Val Cys Trp
   65                  70                  75                  80
   cac acc gac cgg ctc atc cgc gtc acg cgg gac ctg gag cgg gtg atc    288
   His Thr Asp Arg Leu Ile Arg Val Thr Arg Asp Leu Glu Arg Val Ile
                   85                  90                  95
   gac ctc gga gtc aac gtc cac gcc gtg atg gcc gga cac ctg gac ctg    336
   Asp Leu Gly Val Asn Val His Ala Val Met Ala Gly His Leu Asp Leu
               100                 105                 110
   tcc acc ccg gcc ggc cga gcc gtc gcc cgc acg gtg acg gcc tgg gcc    384
   Ser Thr Pro Ala Gly Arg Ala Val Ala Arg Thr Val Thr Ala Trp Ala
           115                 120                 125
   acg tac gag ggc gag cag aag gct gag cgc cag aag ctc gcc aac atc    432
   Thr Tyr Glu Gly Glu Gln Lys Ala Glu Arg Gln Lys Leu Ala Asn Ile
       130                 135                 140
   cag aac gcc cgc gcc ggc aag ccg tac acc ccc ggc atc cgc ccc ttc    480
   Gln Asn Ala Arg Ala Gly Lys Pro Tyr Thr Pro Gly Ile Arg Pro Phe
   145                 150                 155                 160
   ggg tac ggc gac gac cac atg acc atc gtg acg gcc gag gcg gac gcc    528
   Gly Tyr Gly Asp Asp His Met Thr Ile Val Thr Ala Glu Ala Asp Ala
                   165                 170                 175
   atc cgc gac ggc gcg aag atg atc ctc gac ggc tgg tcc ctg tcg gcc    576
   Ile Arg Asp Gly Ala Lys Met Ile Leu Asp Gly Trp Ser Leu Ser Ala
               180                 185                 190
   gtg gct cgc tac tgg gag gag ctc aag ctc cag tcg ccc cgg agt atg    624
   Val Ala Arg Tyr Trp Glu Glu Leu Lys Leu Gln Ser Pro Arg Ser Met
           195                 200                 205
   gcc gca ggc ggc aag ggc tgg tct ctg cgg ggc gta aag aag gtg ctg    672
   Ala Ala Gly Gly Lys Gly Trp Ser Leu Arg Gly Val Lys Lys Val Leu
       210                 215                 220
   acc tcc ccg cgc tac gtc ggg cgg tcc agc tac ctc ggg gag gtc gtg    720
   Thr Ser Pro Arg Tyr Val Gly Arg Ser Ser Tyr Leu Gly Glu Val Val
   225                 230                 235                 240
   ggc gat gct cag tgg ccg ccc atc ctc gac ccg gac gtc tac tac ggg    768
   Gly Asp Ala Gln Trp Pro Pro Ile Leu Asp Pro Asp Val Tyr Tyr Gly
                   245                 250                 255
   gtc gtg gcc atc ctg aac aac ccc gac cgc ttc agc ggg ggc cct cgg    816
   Val Val Ala Ile Leu Asn Asn Pro Asp Arg Phe Ser Gly Gly Pro Arg
               260                 265                 270
   acc ggc cgc acc ccc ggc acg ctg ctc gca ggc atc gcc ttg tgc ggt    864
   Thr Gly Arg Thr Pro Gly Thr Leu Leu Ala Gly Ile Ala Leu Cys Gly
           275                 280                 285
   gag tgc ggc aag acg gtc agt gga cgc ggc tac cga ggt gtc ctg gtc    912
   Glu Cys Gly Lys Thr Val Ser Gly Arg Gly Tyr Arg Gly Val Leu Val
       290                 295                 300
   tac gga tgt aag gac acg cac act cgg acg cct cgg agc atc gct gac     960
   Tyr Gly Cys Lys Asp Thr His Thr Arg Thr Pro Arg Ser Ile Ala Asp
   305                 310                 315                 320
   ggc cgc gcg agc agc tcg acc ctc gcc cgg ctc atg ttc ccc gac ttc    1008
   Gly Arg Ala Ser Ser Ser Thr Leu Ala Arg Leu Met Phe Pro Asp Phe
                   325                 330                 335
   ctg ccc ggc ctc ctg gcc tct ggg cag gcc gag gac ggc cag tcg gca    1056
   Leu Pro Gly Leu Leu Ala Ser Gly Gln Ala Glu Asp Gly Gln Ser Ala
               340                 345                 350
   gca tcc aag cac tcg gag gcc cag acg ctg cgc gag cgc ctt gac ggg    1104
   Ala Ser Lys His Ser Glu Ala Gln Thr Leu Arg Glu Arg Leu Asp Gly
           355                 360                 365
   ctg gct acg gcc tac gcg gag ggt gcg atc agc ctg tct cag atg acg    1152
   Leu Ala Thr Ala Tyr Ala Glu Gly Ala Ile Ser Leu Ser Gln Met Thr
       370                 375                 380
   gcc ggc tcg gaa gca ctg cgg aag aag ctg gag gtg atc gaa gcc gac    1200
   Ala Gly Ser Glu Ala Leu Arg Lys Lys Leu Glu Val Ile Glu Ala Asp
   385                 390                 395                 400
   ctc gtg ggc tcg gca ggc atc ccg ccc ttc gat cca gtg gcc gga gtg    1248
   Leu Val Gly Ser Ala Gly Ile Pro Pro Phe Asp Pro Val Ala Gly Val
                   405                 410                 415
   gct ggc ctg atc tcc ggc tgg ccc acc acg cct ctc ccg acg cgt cga    1296
   Ala Gly Leu Ile Ser Gly Trp Pro Thr Thr Pro Leu Pro Thr Arg Arg
               420                 425                 430
   gca tgg gtg gac ttc tgc ctg gtg gtc acg ctg aac acc cag aag ggg    1344
   Ala Trp Val Asp Phe Cys Leu Val Val Thr Leu Asn Thr Gln Lys Gly
           435                 440                 445
   cgc cat gcg tcg agc atg acc gtg gac gac cac gtc acc atc gag tgg    1392
   Arg His Ala Ser Ser Met Thr Val Asp Asp His Val Thr Ile Glu Trp
       450                 455                 460
   cga gac gtg gcc gag tag                                            1410
   Arg Asp Val Ala Glu
   465
<210> 55
   <211> 469
   <212> PRT
   <213> Bacteriophage R4
<400> 55
   Met Asn Arg Gly Gly Pro Thr Val Arg Ala Asp Ile Tyr Val Arg Ile
   1               5                   10                  15
   Ser Leu Asp Arg Thr Gly Glu Glu Leu Gly Val Glu Arg Gln Glu Glu
               20                  25                  30
   Ser Cys Arg Glu Leu Cys Lys Ser Leu Gly Met Glu Val Gly Gln Val
           35                  40                  45
   Trp Val Asp Asn Asp Leu Ser Ala Thr Lys Lys Asn Val Val Arg Pro
       50                  55                  60
   Asp Phe Glu Ala Met Ile Ala Ser Asn Pro Gln Ala Ile Val Cys Trp
   65                  70                  75                  80
   His Thr Asp Arg Leu Ile Arg Val Thr Arg Asp Leu Glu Arg Val Ile
                   85                  90                  95
   Asp Leu Gly Val Asn Val His Ala Val Met Ala Gly His Leu Asp Leu
               100                 105                 110
   Ser Thr Pro Ala Gly Arg Ala Val Ala Arg Thr Val Thr Ala Trp Ala
           115                 120                 125
   Thr Tyr Glu Gly Glu Gln Lys Ala Glu Arg Gln Lys Leu Ala Asn Ile
       130                 135                 140
   Gln Asn Ala Arg Ala Gly Lys Pro Tyr Thr Pro Gly Ile Arg Pro Phe
   145                 150                 155                 160
   Gly Tyr Gly Asp Asp His Met Thr Ile Val Thr Ala Glu Ala Asp Ala
                   165                 170                 175
   Ile Arg Asp Gly Ala Lys Met Ile Leu Asp Gly Trp Ser Leu Ser Ala
               180                 185                 190
   Val Ala Arg Tyr Trp Glu Glu Leu Lys Leu Gln Ser Pro Arg Ser Met
           195                 200                 205
   Ala Ala Gly Gly Lys Gly Trp Ser Leu Arg Gly Val Lys Lys Val Leu
       210                 215                 220
   Thr Ser Pro Arg Tyr Val Gly Arg Ser Ser Tyr Leu Gly Glu Val Val
   225                 230                 235                 240
   Gly Asp Ala Gln Trp Pro Pro Ile Leu Asp Pro Asp Val Tyr Tyr Gly
                   245                 250                 255
   Val Val Ala Ile Leu Asn Asn Pro Asp Arg Phe Ser Gly Gly Pro Arg
               260                 265                 270
   Thr Gly Arg Thr Pro Gly Thr Leu Leu Ala Gly Ile Ala Leu Cys Gly
           275                 280                 285
   Glu Cys Gly Lys Thr Val Ser Gly Arg Gly Tyr Arg Gly Val Leu Val
       290                 295                 300
   Tyr Gly Cys Lys Asp Thr His Thr Arg Thr Pro Arg Ser Ile Ala Asp
   305                 310                 315                 320
   Gly Arg Ala Ser Ser Ser Thr Leu Ala Arg Leu Met Phe Pro Asp Phe
                   325                 330                 335
   Leu Pro Gly Leu Leu Ala Ser Gly Gln Ala Glu Asp Gly Gln Ser Ala
               340                 345                 350
   Ala Ser Lys His Ser Glu Ala Gln Thr Leu Arg Glu Arg Leu Asp Gly
           355                 360                 365
   Leu Ala Thr Ala Tyr Ala Glu Gly Ala Ile Ser Leu Ser Gln Met Thr
       370                 375                 380
   Ala Gly Ser Glu Ala Leu Arg Lys Lys Leu Glu Val Ile Glu Ala Asp
   385                 390                 395                 400
   Leu Val Gly Ser Ala Gly Ile Pro Pro Phe Asp Pro Val Ala Gly Val
                   405                 410                 415
   Ala Gly Leu Ile Ser Gly Trp Pro Thr Thr Pro Leu Pro Thr Arg Arg
               420                 425                 430
   Ala Trp Val Asp Phe Cys Leu Val Val Thr Leu Asn Thr Gln Lys Gly
   435 440 445
   Arg His Ala Ser Ser Met Thr Val Asp Asp His Val Thr Ile Glu Trp
   450 455 460
   Arg Asp Val Ala Glu
   465
<210> 56
   <211> 1503
   <212> DNA
   <213> CisA recombinase
<220>
   <221> CDS
   <222> (1)..(1500)
<400> 56
   gtg ata gca ata tat gta agg gta tcg acc gag gaa caa gcg atc aag    48
   Val Ile Ala Ile Tyr Val Arg Val Ser Thr Glu Glu Gln Ala Ile Lys
    1               5                  10                  15
   gga tcg agc atc gac agc caa atc gag gcc tgt ata aag aaa gca ggg    96
   Gly Ser Ser Ile Asp Ser Gln Ile Glu Ala Cys Ile Lys Lys Ala Gly
               20                  25                  30
   act aaa gat gtg ctg aag tat gca gat gaa gga ttt tca gga gag ctt    144
   Thr Lys Asp Val Leu Lys Tyr Ala Asp Glu Gly Phe Ser Gly Glu Leu
           35                  40                  45
   tta gaa cgt ccg gct ttg aat cgc ttg agg gag gat gca agc aag gga    192
   Leu Glu Arg Pro Ala Leu Asn Arg Leu Arg Glu Asp Ala Ser Lys Gly
       50                  55                  60
   ctt ata agt caa gtc att tgt tac gat cct gac cgt ctt tct cgg aaa    240
   Leu Ile Ser Gln Val Ile Cys Tyr Asp Pro Asp Arg Leu Ser Arg Lys
   65                  70                  75                  80
   tta atg aat cag cta atc att gat gac gaa ttg cga aag cga aac ata    288
   Leu Met Asn Gln Leu Ile Ile Asp Asp Glu Leu Arg Lys Arg Asn Ile
                   85                  90                  95
   cct ttg att ttt gta aat ggt gaa tac gcc aat tct cca gaa ggt caa    336
   Pro Leu Ile Phe Val Asn Gly Glu Tyr Ala Asn Ser Pro Glu Gly Gln
               100                 105                 110
   ttg ttt ttc gca atg cgc ggg gca atc tca gaa ttt gaa aaa gcc aaa    384
   Leu Phe Phe Ala Met Arg Gly Ala Ile Ser Glu Phe Glu Lys Ala Lys
           115                 120                 125
   atc aaa gaa cgg aca tca agc ggc cga ctt caa aaa atg aaa aaa ggc    432
   Ile Lys Glu Arg Thr Ser Ser Gly Arg Leu Gln Lys Met Lys Lys Gly
       130                 135                 140
   atg atc att aaa gat tct aaa cta tat ggc tat aaa ttt gtt aaa gag    480
   Met Ile Ile Lys Asp Ser Lys Leu Tyr Gly Tyr Lys Phe Val Lys Glu
   145                 150                 155                 160
   aaa aga act ctt gag ata tta gaa gag gaa gca aaa atc att cgg atg    528
   Lys Arg Thr Leu Glu Ile Leu Glu Glu Glu Ala Lys Ile Ile Arg Met
                   165                 170                 175
   att ttt aac tat ttc acc gat cat aaa agc cct ttt ttc ggc aga gta    576
   Ile Phe Asn Tyr Phe Thr Asp His Lys Ser Pro Phe Phe Gly Arg Val
               180                 185                 190
   aat ggt att gct cta cat tta act cag atg ggg gtt aaa aca aaa aaa     624
   Asn Gly Ile Ala Leu His Leu Thr Gln Met Gly Val Lys Thr Lys Lys
           195                 200                 205
   ggc gcc aaa gta tgg cac agg cag gtt gtt cgg caa ata tta atg aac     672
   Gly Ala Lys Val Trp His Arg Gln Val Val Arg Gln Ile Leu Met Asn
       210                 215                 220
   tct tcc tat aag ggt gaa cat aga cag tat aaa tat gat aca gag ggt     720
   Ser Ser Tyr Lys Gly Glu His Arg Gln Tyr Lys Tyr Asp Thr Glu Gly
   225                 230                 235                 240
   tcc tat gtt tca aag cag gca ggg aac aaa tct ata att aaa ata agg     768
   Ser Tyr Val Ser Lys Gln Ala Gly Asn Lys Ser Ile Ile Lys Ile Arg
                   245                 250                 255
   cct gaa gaa gaa caa atc act gtg aca att cca gca att gtt cca gct     816
   Pro Glu Glu Glu Gln Ile Thr Val Thr Ile Pro Ala Ile Val Pro Ala
               260                 265                 270
   gaa caa tgg gat tat gct caa gaa ctc tta ggt caa agt aaa aga aaa     864
   Glu Gln Trp Asp Tyr Ala Gln Glu Leu Leu Gly Gln Ser Lys Arg Lys
           275                 280                 285
   cac ttg agt atc agc cct cac aat tac ttg tta tcg ggt ttg gtt aga     912
   His Leu Ser Ile Ser Pro His Asn Tyr Leu Leu Ser Gly Leu Val Arg
       290                 295                 300
   tgc gga aaa tgc gga aat acc atg aca ggg aag aaa aga aaa tca cat     960
   Cys Gly Lys Cys Gly Asn Thr Met Thr Gly Lys Lys Arg Lys Ser His
   305                 310                 315                 320
   ggt aaa gac tac tat gta tat act tgc cgg aaa aat tat tct ggc gca    1008
   Gly Lys Asp Tyr Tyr Val Tyr Thr Cys Arg Lys Asn Tyr Ser Gly Ala
                   325                 330                 335
   aag gac cgc ggc tgc gga aaa gaa atg tct gag aat aaa ttg aac cgg    1056
   Lys Asp Arg Gly Cys Gly Lys Glu Met Ser Glu Asn Lys Leu Asn Arg
               340                 345                 350
   cat gta tgg ggt gaa att ttt aaa ttc atc aca aat cct caa aag tat    1104
   His Val Trp Gly Glu Ile Phe Lys Phe Ile Thr Asn Pro Gln Lys Tyr
           355                 360                 365
   gtt tct ttt aaa gag gct gaa caa tca aat cac ctg tct gat gaa tta    1152
   Val Ser Phe Lys Glu Ala Glu Gln Ser Asn His Leu Ser Asp Glu Leu
       370                 375                 380
   gaa ctt att gaa aaa gag ata gag aaa aca aaa aaa ggc cgc aag cgt    1200
   Glu Leu Ile Glu Lys Glu Ile Glu Lys Thr Lys Lys Gly Arg Lys Arg
   385                 390                 395                 400
   ctt tta acg cta atc agc cta agc gat gac gat gat tta gac ata gat    1248
   Leu Leu Thr Leu Ile Ser Leu Ser Asp Asp Asp Asp Leu Asp Ile Asp
                   405                 410                 415
   gaa atc aaa gca caa att att gaa ctg caa aaa aag caa aat cag ctt    1296
   Glu Ile Lys Ala Gln Ile Ile Glu Leu Gln Lys Lys Gln Asn Gln Leu
               420                 425                 430
   act gaa aag tgt aac aga atc cag tca aaa atg aaa gtc cta gat gat    1344
   Thr Glu Lys Cys Asn Arg Ile Gln Ser Lys Met Lys Val Leu Asp Asp
           435                 440                 445
   acg agc tca agt gaa aat gct cta aaa aga gcc atc gac tat ttt caa    1392
   Thr Ser Ser Ser Glu Asn Ala Leu Lys Arg Ala Ile Asp Tyr Phe Gln
       450                 455                 460
   tca atc ggt gca gat aac tta act ctt gaa gat aaa aaa aca att gtt    1440
   Ser Ile Gly Ala Asp Asn Leu Thr Leu Glu Asp Lys Lys Thr Ile Val
   465                 470                 475                 480
   aac ttt atc gtg aaa gaa gtt acc att gtg gat tct gac acc ata tat    1488
   Asn Phe Ile Val Lys Glu Val Thr Ile Val Asp Ser Asp Thr Ile Tyr
                   485                 490                 495
   att gaa acg tat taa                                                1503
   Ile Glu Thr Tyr
               500
<210> 57
   <211> 500
   <212> PRT
   <213> CisA recombinase
<400> 57
   Val Ile Ala Ile Tyr Val Arg Val Ser Thr Glu Glu Gln Ala Ile Lys
   1 5 10 15
   Gly Ser Ser Ile Asp Ser Gln Ile Glu Ala Cys Ile Lys Lys Ala Gly
   20 25 30
   Thr Lys Asp Val Leu Lys Tyr Ala Asp Glu Gly Phe Ser Gly Glu Leu
   35 40 45
   Leu Glu Arg Pro Ala Leu Asn Arg Leu Arg Glu Asp Ala Ser Lys Gly
   50 55 60
   Leu Ile Ser Gln Val Ile Cys Tyr Asp Pro Asp Arg Leu Ser Arg Lys
   65 70 75 80
   Leu Met Asn Gln Leu Ile Ile Asp Asp Glu Leu Arg Lys Arg Asn Ile
   85 90 95
   Pro Leu Ile Phe Val Asn Gly Glu Tyr Ala Asn Ser Pro Glu Gly Gln
   100 105 110
   Leu Phe Phe Ala Met Arg Gly Ala Ile Ser Glu Phe Glu Lys Ala Lys
   115 120 125
   Ile Lys Glu Arg Thr Ser Ser Gly Arg Leu Gln Lys Met Lys Lys Gly
   130 135 140
   Met Ile Ile Lys Asp Ser Lys Leu Tyr Gly Tyr Lys Phe Val Lys Glu
   145 150 155 160
   Lys Arg Thr Leu Glu Ile Leu Glu Glu Glu Ala Lys Ile Ile Arg Met
   165 170 175
   Ile Phe Asn Tyr Phe Thr Asp His Lys Ser Pro Phe Phe Gly Arg Val
   180 185 190
   Asn Gly Ile Ala Leu His Leu Thr Gln Met Gly Val Lys Thr Lys Lys
   195 200 205
   Gly Ala Lys Val Trp His Arg Gln Val Val Arg Gln Ile Leu Met Asn
   210 215 220
   Ser Ser Tyr Lys Gly Glu His Arg Gln Tyr Lys Tyr Asp Thr Glu Gly
   225 230 235 240
   Ser Tyr Val Ser Lys Gln Ala Gly Asn Lys Ser Ile Ile Lys Ile Arg
                   245                 250                 255
   Pro Glu Glu Glu Gln Ile Thr Val Thr Ile Pro Ala Ile Val Pro Ala
               260                 265                 270
   Glu Gln Trp Asp Tyr Ala Gln Glu Leu Leu Gly Gln Ser Lys Arg Lys
           275                 280                 285
   His Leu Ser Ile Ser Pro His Asn Tyr Leu Leu Ser Gly Leu Val Arg
       290                 295                 300
   Cys Gly Lys Cys Gly Asn Thr Met Thr Gly Lys Lys Arg Lys Ser His
   305                 310                 315                 320
   Gly Lys Asp Tyr Tyr Val Tyr Thr Cys Arg Lys Asn Tyr Ser Gly Ala
                   325                 330                 335
   Lys Asp Arg Gly Cys Gly Lys Glu Met Ser Glu Asn Lys Leu Asn Arg
               340                 345                 350
   His Val Trp Gly Glu Ile Phe Lys Phe Ile Thr Asn Pro Gln Lys Tyr
           355                 360                 365
   Val Ser Phe Lys Glu Ala Glu Gln Ser Asn His Leu Ser Asp Glu Leu
       370                 375                 380
   Glu Leu Ile Glu Lys Glu Ile Glu Lys Thr Lys Lys Gly Arg Lys Arg
   385                 390                 395                 400
   Leu Leu Thr Leu Ile Ser Leu Ser Asp Asp Asp Asp Leu Asp Ile Asp
                   405                 410                 415
   Glu Ile Lys Ala Gln Ile Ile Glu Leu Gln Lys Lys Gln Asn Gln Leu
               420                 425                 430
   Thr Glu Lys Cys Asn Arg Ile Gln Ser Lys Met Lys Val Leu Asp Asp
           435                 440                 445
   Thr Ser Ser Ser Glu Asn Ala Leu Lys Arg Ala Ile Asp Tyr Phe Gln
       450                 455                 460
   Ser Ile Gly Ala Asp Asn Leu Thr Leu Glu Asp Lys Lys Thr Ile Val
   465                 470                 475                 480
   Asn Phe Ile Val Lys Glu Val Thr Ile Val Asp Ser Asp Thr Ile Tyr
                   485                 490                 495
   Ile Glu Thr Tyr
               500
<210> 58
   <211> 1545
   <212> DNA
   <213> XisF recombinase
<220>
   <221> CDS
   <222> (1)..(1542)
<400> 58
   atg gaa aat tgg ggt tac gcg aga gtg agc ggt gag gaa cag caa aca    48
   Met Glu Asn Trp Gly Tyr Ala Arg Val Ser Gly Glu Glu Gln Gln Thr
    1               5                  10                  15
   gat aaa ggt gcg ttg cgt aaa caa ata gaa cgc ttg cgt aat gct gga      96
   Asp Lys Gly Ala Leu Arg Lys Gln Ile Glu Arg Leu Arg Asn Ala Gly
               20                  25                  30
   tgt tca aaa gtg tac tgg gat att caa tcg cgg aca act gaa gtc aga     144
   Cys Ser Lys Val Tyr Trp Asp Ile Gln Ser Arg Thr Thr Glu Val Arg
           35                  40                  45
   gaa ggg cta caa caa tta att aat gac tta aag aca tct tca aca ggt     192
   Glu Gly Leu Gln Gln Leu Ile Asn Asp Leu Lys Thr Ser Ser Thr Gly
       50                  55                  60
   aag gta aaa tca ctg caa ttt acc cgc att gat cgc atc ggc tca tca     240
   Lys Val Lys Ser Leu Gln Phe Thr Arg Ile Asp Arg Ile Gly Ser Ser
   65                  70                  75                  80
   tcg cgg ttg ttt tat tca ttg tta gag gta tta cgt tcc aag gga att     288
   Ser Arg Leu Phe Tyr Ser Leu Leu Glu Val Leu Arg Ser Lys Gly Ile
                   85                  90                  95
   aaa ctg ata gcc tta gat caa ggc gtt gac cca gac agc ctt ggc ggg     336
   Lys Leu Ile Ala Leu Asp Gln Gly Val Asp Pro Asp Ser Leu Gly Gly
               100                 105                 110
   gaa cta aca att gat atg tta ctg gct gct gcc aaa ttt gag gta aga     384
   Glu Leu Thr Ile Asp Met Leu Leu Ala Ala Ala Lys Phe Glu Val Arg
           115                 120                 125
   atg gtg acg gag agg tta aaa agc gaa cgt cgt cat agg gtg aac caa     432
   Met Val Thr Glu Arg Leu Lys Ser Glu Arg Arg His Arg Val Asn Gln
       130                 135                 140
   gga aaa agt cac cga gtt gcc cca tta gga tac cgc aaa gat aaa gat     480
   Gly Lys Ser His Arg Val Ala Pro Leu Gly Tyr Arg Lys Asp Lys Asp
   145                 150                 155                 160
   aaa tat ata cgc gat cgc tca cca tgt gtt tgc tta cta gaa gga cgc     528
   Lys Tyr Ile Arg Asp Arg Ser Pro Cys Val Cys Leu Leu Glu Gly Arg
                   165                 170                 175
   aga gaa tta acg gtg tct gac tta gcc cag tat att ttt cac act ttt     576
   Arg Glu Leu Thr Val Ser Asp Leu Ala Gln Tyr Ile Phe His Thr Phe
               180                 185                 190
   ttt gag tgc ggt tcc gtt gct gct act gtg cgt aag ctg cac tca gat     624
   Phe Glu Cys Gly Ser Val Ala Ala Thr Val Arg Lys Leu His Ser Asp
           195                 200                 205
   ttt ggt ata gaa aca aaa gtt ctg aat tgg aac aag cta gaa aaa tct     672
   Phe Gly Ile Glu Thr Lys Val Leu Asn Trp Asn Lys Leu Glu Lys Ser
       210                 215                 220
   tcc cgg att gtt ggc gac gac gac tta gat aaa att gca ttt aca cca     720
   Ser Arg Ile Val Gly Asp Asp Asp Leu Asp Lys Ile Ala Phe Thr Pro
   225                 230                 235                 240
   aat aaa act aac cac ccc ttg cgt tat ccc tgg tct ggg cta aga tgg     768
   Asn Lys Thr Asn His Pro Leu Arg Tyr Pro Trp Ser Gly Leu Arg Trp
                   245                 250                 255
   tca atc cct ggt tta aaa gcg tta tta gtt aac cct gtt tac gcc ggg     816
   Ser Ile Pro Gly Leu Lys Ala Leu Leu Val Asn Pro Val Tyr Ala Gly
               260                 265                 270
   ggt ttg ccc ttt gat act tac gtt aaa tca aaa gga aaa cgc aag cat     864
   Gly Leu Pro Phe Asp Thr Tyr Val Lys Ser Lys Gly Lys Arg Lys His
           275                 280                 285
   ttt gac gag tgg aaa gta aaa tgg gga acc cac gac gat gag gca atc      912
   Phe Asp Glu Trp Lys Val Lys Trp Gly Thr His Asp Asp Glu Ala Ile
       290                 295                 300
   att acc tgt gag gaa cat gaa aga ata aaa cag atg att cga gac aat      960
   Ile Thr Cys Glu Glu His Glu Arg Ile Lys Gln Met Ile Arg Asp Asn
   305                 310                 315                 320
   cgc aat aat cga tgg gct gca aga gaa gaa aac gaa gta aac cca ttt     1008
   Arg Asn Asn Arg Trp Ala Ala Arg Glu Glu Asn Glu Val Asn Pro Phe
                   325                 330                 335
   tct aat tta ctt aaa tgt acc cat tgc ggc ggc tca atg aca cgc cac     1056
   Ser Asn Leu Leu Lys Cys Thr His Cys Gly Gly Ser Met Thr Arg His
               340                 345                 350
   gcc aaa cgt gta gat aag agt gga caa gct atc tat tat tat cag tgc     1104
   Ala Lys Arg Val Asp Lys Ser Gly Gln Ala Ile Tyr Tyr Tyr Gln Cys
           355                 360                 365
   cga ttg tat aaa gct ggc aac tgt agc aat aaa aat atg att tca tcc     1152
   Arg Leu Tyr Lys Ala Gly Asn Cys Ser Asn Lys Asn Met Ile Ser Ser
       370                 375                 380
   aaa ata tta gat atc caa gta atg gat tta ttg gca caa gaa gcc gaa     1200
   Lys Ile Leu Asp Ile Gln Val Met Asp Leu Leu Ala Gln Glu Ala Glu
   385                 390                 395                 400
   cgt tta gca aat ttg gtg gaa aca gat gag ccg ctt att gta gaa gaa     1248
   Arg Leu Ala Asn Leu Val Glu Thr Asp Glu Pro Leu Ile Val Glu Glu
                   405                 410                 415
   ccc cca gaa gta aaa acg ctg cgc gca tcc ctg aat agt ctg gaa aca     1296
   Pro Pro Glu Val Lys Thr Leu Arg Ala Ser Leu Asn Ser Leu Glu Thr
               420                 425                 430
   ttg cca gca agt tca gca att gaa caa att aaa aat gac ctc aaa gaa     1344
   Leu Pro Ala Ser Ser Ala Ile Glu Gln Ile Lys Asn Asp Leu Lys Glu
           435                 440                 445
   cag att gcg atc gca cta gga gca acc aat aat gct tct aaa caa tct     1392
   Gln Ile Ala Ile Ala Leu Gly Ala Thr Asn Asn Ala Ser Lys Gln Ser
       450                 455                 460
   ctg att gcc aag gaa aga att ata caa gct ttt gct cat aaa agt tac     1440
   Leu Ile Ala Lys Glu Arg Ile Ile Gln Ala Phe Ala His Lys Ser Tyr
   465                 470                 475                 480
   tgg caa gga cta aac gct caa gat aaa cga gca atc ctc aat ggt tgc     1488
   Trp Gln Gly Leu Asn Ala Gln Asp Lys Arg Ala Ile Leu Asn Gly Cys
                   485                 490                 495
   gta aaa aaa atc tcc gta gat ggt aac ttt gtt aca gct att gag tat     1536
   Val Lys Lys Ile Ser Val Asp Gly Asn Phe Val Thr Ala Ile Glu Tyr
               500                 505                 510
   cgt tac tag                                                         1545
   Arg Tyr
<210> 59
   <211> 514
   <212> PRT
   <213> XisF recombinase
<400> 59
   Met Glu Asn Trp Gly Tyr Ala Arg Val Ser Gly Glu Glu Gln Gln Thr
    1               5                  10                  15
   Asp Lys Gly Ala Leu Arg Lys Gln Ile Glu Arg Leu Arg Asn Ala Gly
               20                  25                  30
   Cys Ser Lys Val Tyr Trp Asp Ile Gln Ser Arg Thr Thr Glu Val Arg
           35                  40                  45
   Glu Gly Leu Gln Gln Leu Ile Asn Asp Leu Lys Thr Ser Ser Thr Gly
       50                  55                  60
   Lys Val Lys Ser Leu Gln Phe Thr Arg Ile Asp Arg Ile Gly Ser Ser
   65                  70                  75                  80
   Ser Arg Leu Phe Tyr Ser Leu Leu Glu Val Leu Arg Ser Lys Gly Ile
                   85                  90                  95
   Lys Leu Ile Ala Leu Asp Gln Gly Val Asp Pro Asp Ser Leu Gly Gly
               100                 105                 110
   Glu Leu Thr Ile Asp Met Leu Leu Ala Ala Ala Lys Phe Glu Val Arg
           115                 120                 125
   Met Val Thr Glu Arg Leu Lys Ser Glu Arg Arg His Arg Val Asn Gln
       130                 135                 140
   Gly Lys Ser His Arg Val Ala Pro Leu Gly Tyr Arg Lys Asp Lys Asp
   145                 150                 155                 160
   Lys Tyr Ile Arg Asp Arg Ser Pro Cys Val Cys Leu Leu Glu Gly Arg
                   165                 170                 175
   Arg Glu Leu Thr Val Ser Asp Leu Ala Gln Tyr Ile Phe His Thr Phe
               180                 185                 190
   Phe Glu Cys Gly Ser Val Ala Ala Thr Val Arg Lys Leu His Ser Asp
           195                 200                 205
   Phe Gly Ile Glu Thr Lys Val Leu Asn Trp Asn Lys Leu Glu Lys Ser
       210                 215                 220
   Ser Arg Ile Val Gly Asp Asp Asp Leu Asp Lys Ile Ala Phe Thr Pro
   225                 230                 235                 240
   Asn Lys Thr Asn His Pro Leu Arg Tyr Pro Trp Ser Gly Leu Arg Trp
                   245                 250                 255
   Ser Ile Pro Gly Leu Lys Ala Leu Leu Val Asn Pro Val Tyr Ala Gly
               260                 265                 270
   Gly Leu Pro Phe Asp Thr Tyr Val Lys Ser Lys Gly Lys Arg Lys His
           275                 280                 285
   Phe Asp Glu Trp Lys Val Lys Trp Gly Thr His Asp Asp Glu Ala Ile
       290                 295                 300
   Ile Thr Cys Glu Glu His Glu Arg Ile Lys Gln Met Ile Arg Asp Asn
   305                 310                 315                 320
   Arg Asn Asn Arg Trp Ala Ala Arg Glu Glu Asn Glu Val Asn Pro Phe
                   325                 330                 335
   Ser Asn Leu Leu Lys Cys Thr His Cys Gly Gly Ser Met Thr Arg His
               340                 345                 350
   Ala Lys Arg Val Asp Lys Ser Gly Gln Ala Ile Tyr Tyr Tyr Gln Cys
           355                     360                     365
   Arg Leu Tyr Lys Ala Gly Asn Cys Ser Asn Lys Asn Met Ile Ser Ser
       370                     375                     380
   Lys Ile Leu Asp Ile Gln Val Met Asp Leu Leu Ala Gln Glu Ala Glu
   385                     390                     395                     400
   Arg Leu Ala Asn Leu Val Glu Thr Asp Glu Pro Leu Ile Val Glu Glu
                   405                     410                     415
   Pro Pro Glu Val Lys Thr Leu Arg Ala Ser Leu Asn Ser Leu Glu Thr
               420                     425                     430
   Leu Pro Ala Ser Ser Ala Ile Glu Gln Ile Lys Asn Asp Leu Lys Glu
           435                     440                     445
   Gln Ile Ala Ile Ala Leu Gly Ala Thr Asn Asn Ala Ser Lys Gln Ser
       450                     455                     460
   Leu Ile Ala Lys Glu Arg Ile Ile Gln Ala Phe Ala His Lys Ser Tyr
   465                     470                     475                     480
   Trp Gln Gly Leu Asn Ala Gln Asp Lys Arg Ala Ile Leu Asn Gly Cys
                   485                     490                     495
   Val Lys Lys Ile Ser Val Asp Gly Asn Phe Val Thr Ala Ile Glu Tyr
               500                     505                     510
   Arg Tyr
<210> 60
   <211> 2124
   <212> DNA
   <213> Transposon Tn4451
<220>
   <221> CDS
   <222> (1)..(2121)
<400> 60
   atg tca agg act tca aga att aca gca ctt tac gag cgt ttg tca aga      48
   Met Ser Arg Thr Ser Arg Ile Thr Ala Leu Tyr Glu Arg Leu Ser Arg
    1               5                  10                  15
   gat gat gac ctt act ggc gag agt aat tct att acc aat caa aag aaa      96
   Asp Asp Asp Leu Thr Gly Glu Ser Asn Ser Ile Thr Asn Gln Lys Lys
               20                  25                  30
   tac ctc gaa gat tat gcc cgt agg aat ggt ttt gag aac att cgc cat     144
   Tyr Leu Glu Asp Tyr Ala Arg Arg Asn Gly Phe Glu Asn Ile Arg His
           35                  40                  45
   ttt acc gat gac gga ttt tcg ggt gta aat ttc aat cgc cct ggc ttt     192
   Phe Thr Asp Asp Gly Phe Ser Gly Val Asn Phe Asn Arg Pro Gly Phe
       50                  55                  60
   caa tct ctg ata aaa gaa gtt gaa gca gga aat gta gaa acc ttg att     240
   Gln Ser Leu Ile Lys Glu Val Glu Ala Gly Asn Val Glu Thr Leu Ile
   65                  70                  75                  80
   gtt aag gat atg agc cga ttg ggg cga aat tat ctg caa gta ggt ttt     288
   Val Lys Asp Met Ser Arg Leu Gly Arg Asn Tyr Leu Gln Val Gly Phe
               85                  90                  95
   tat acg gaa gtt ctg ttt cca cag aaa aat gtc cgt ttc ctt gca att     336
   Tyr Thr Glu Val Leu Phe Pro Gln Lys Asn Val Arg Phe Leu Ala Ile
           100                 105                 110
   aac aac agt att gac agt aac aac gct tcg gat aat gac ttt gct ccg     384
   Asn Asn Ser Ile Asp Ser Asn Asn Ala Ser Asp Asn Asp Phe Ala Pro
           115                 120                 125
   ttt ttg aat att atg aac gaa tgg tat gcc aaa gac aca agc aac aaa     432
   Phe Leu Asn Ile Met Asn Glu Trp Tyr Ala Lys Asp Thr Ser Asn Lys
       130                 135                 140
   atc aag gct ata ttc gat gcc cgt atg aaa gac gga aag cgt tgt agc     480
   Ile Lys Ala Ile Phe Asp Ala Arg Met Lys Asp Gly Lys Arg Cys Ser
   145                 150                 155                 160
   ggt tct atc cct tat ggg tat aac cga ctg ccg agc gac aaa caa acg     528
   Gly Ser Ile Pro Tyr Gly Tyr Asn Arg Leu Pro Ser Asp Lys Gln Thr
                   165                 170                 175
   ctt gtg gtt gac cct gtg gct tcg gaa gtg gta aag cgt atc ttt act     576
   Leu Val Val Asp Pro Val Ala Ser Glu Val Val Lys Arg Ile Phe Thr
               180                 185                 190
   ctt gcc aat gat ggc aaa agt aca agg gca atc gca gaa ata ctg acc     624
   Leu Ala Asn Asp Gly Lys Ser Thr Arg Ala Ile Ala Glu Ile Leu Thr
           195                 200                 205
   gaa gaa aaa gtt tta acc cct gcg gca tac gca aag gaa tac cac ccc     672
   Glu Glu Lys Val Leu Thr Pro Ala Ala Tyr Ala Lys Glu Tyr His Pro
       210                 215                 220
   gaa cag tac aac ggc aac aag ttc aca aac cct tat ctt tgg gca atg     720
   Glu Gln Tyr Asn Gly Asn Lys Phe Thr Asn Pro Tyr Leu Trp Ala Met
   225                 230                 235                 240
   tca acg ata aga aat att tta ggc agg cag gaa tat ctc ggt cac acc     768
   Ser Thr Ile Arg Asn Ile Leu Gly Arg Gln Glu Tyr Leu Gly His Thr
                   245                 250                 255
   gtt ttg cga aag tcg gta agc aca aat ttc aaa ctt cac aag aga aaa     816
   Val Leu Arg Lys Ser Val Ser Thr Asn Phe Lys Leu His Lys Arg Lys
               260                 265                 270
   agc aca gac gaa gaa gaa cag tat gta ttt ccg aat aca cac gag cct     864
   Ser Thr Asp Glu Glu Glu Gln Tyr Val Phe Pro Asn Thr His Glu Pro
           275                 280                 285
   atc ata tcg cag gaa ctt tgg gac agc gtt caa aaa cgc aga agc aga     912
   Ile Ile Ser Gln Glu Leu Trp Asp Ser Val Gln Lys Arg Arg Ser Arg
       290                 295                 300
   gta aat cgt gcc tcg gct tgg gga acg cac agc aac cgt tta agc gga     960
   Val Asn Arg Ala Ser Ala Trp Gly Thr His Ser Asn Arg Leu Ser Gly
   305                 310                 315                 320
   tat ttg tac tgt gcc gat tgc gga aga aga atg act ttg cag aca cat     1008
   Tyr Leu Tyr Cys Ala Asp Cys Gly Arg Arg Met Thr Leu Gln Thr His
                   325                 330                 335
   tac agc aaa aaa gac ggt tct gtg cag tat tct tac cgt tgc ggt ggg     1056
   Tyr Ser Lys Lys Asp Gly Ser Val Gln Tyr Ser Tyr Arg Cys Gly Gly
               340                 345                 350
   tat gca agc aga gtg aac agt tgt acc agt cat tcg att agt acc gat     1104
   Tyr Ala Ser Arg Val Asn Ser Cys Thr Ser His Ser Ile Ser Thr Asp
           355                 360                 365
   aat gtt gaa gcc ttg ata tta tca tct gtc aaa cgc ttt tca agg ttt     1152
   Asn Val Glu Ala Leu Ile Leu Ser Ser Val Lys Arg Phe Ser Arg Phe
       370                 375                 380
   gtt ctg aat gat gaa caa gca ttt gct ttg gaa ctg caa tct ctt tgg     1200
   Val Leu Asn Asp Glu Gln Ala Phe Ala Leu Glu Leu Gln Ser Leu Trp
   385                 390                 395                 400
   aat gaa aaa cag gag gaa aag ccg aaa cac aat caa tcg gaa ctg caa     1248
   Asn Glu Lys Gln Glu Glu Lys Pro Lys His Asn Gln Ser Glu Leu Gln
                   405                 410                 415
   cgc tgt cag aaa cgc tat gac gaa ctc tct acc ctt gtt cgt ggc ttg     1296
   Arg Cys Gln Lys Arg Tyr Asp Glu Leu Ser Thr Leu Val Arg Gly Leu
               420                 425                 430
   tat gaa aat ctt atg tcg gga tta ctg ccc gaa aga cag tat aag caa     1344
   Tyr Glu Asn Leu Met Ser Gly Leu Leu Pro Glu Arg Gln Tyr Lys Gln
           435                 440                 445
   ctg atg aaa cag tat gat gac gag cag gca gag ttg gaa acg aaa atg     1392
   Leu Met Lys Gln Tyr Asp Asp Glu Gln Ala Glu Leu Glu Thr Lys Met
       450                 455                 460
   gaa acg atg aaa aca gaa ctt gcc gaa gaa aaa gta agt tcc gtt gat     1440
   Glu Thr Met Lys Thr Glu Leu Ala Glu Glu Lys Val Ser Ser Val Asp
   465                 470                 475                 480
   att aag cat ttc att tcg ctg ata cgc aag tgt aaa aat cct acg gaa     1488
   Ile Lys His Phe Ile Ser Leu Ile Arg Lys Cys Lys Asn Pro Thr Glu
                   485                 490                 495
   atc tcc gat aca atg ttt aat gaa ctt gtt gat aag ata gtg gtt tat     1536
   Ile Ser Asp Thr Met Phe Asn Glu Leu Val Asp Lys Ile Val Val Tyr
               500                 505                 510
   gaa gca gag ggt gtg gga aaa gca cga aca caa aag gtc gat att tat     1584
   Glu Ala Glu Gly Val Gly Lys Ala Arg Thr Gln Lys Val Asp Ile Tyr
           515                 520                 525
   ttt aac tat gtc ggt caa gtg gat att gcc tat acc gaa gaa gaa ctt     1632
   Phe Asn Tyr Val Gly Gln Val Asp Ile Ala Tyr Thr Glu Glu Glu Leu
       530                 535                 540
   gcc gag ata gaa aca cag aaa gag cag gag gaa cag caa cgc ttg gca     1680
   Ala Glu Ile Glu Thr Gln Lys Glu Gln Glu Glu Gln Gln Arg Leu Ala
   545                 550                 555                 560
   aga cag cgc aag cgt gaa aaa gcc tac cga gaa aag cga aag gca cag     1728
   Arg Gln Arg Lys Arg Glu Lys Ala Tyr Arg Glu Lys Arg Lys Ala Gln
                   565                 570                 575
   aaa atc gct gaa aac ggt ggc gaa atc gtt aag aca aag gtt tgc cct     1776
   Lys Ile Ala Glu Asn Gly Gly Glu Ile Val Lys Thr Lys Val Cys Pro
               580                 585                 590
   cat tgc aac aaa gag ttt atc ccg aca agc aac cga cag gtg ttc tgt     1824
   His Cys Asn Lys Glu Phe Ile Pro Thr Ser Asn Arg Gln Val Phe Cys
           595                 600                 605
   tcc aaa gag tgc tgc tat caa gca agg caa gac aaa aag aaa aca gac     1872
   Ser Lys Glu Cys Cys Tyr Gln Ala Arg Gln Asp Lys Lys Lys Thr Asp
       610                 615                 620
   cga gaa gca gaa cga gga aat cac tat tac cga cag cgt gta tgt gct      1920
   Arg Glu Ala Glu Arg Gly Asn His Tyr Tyr Arg Gln Arg Val Cys Ala
   625                 630                 635                 640
   gtg tgc ggc aat tcc tat tgg cct aca cac agc caa cag aaa ttc tgc      1968
   Val Cys Gly Asn Ser Tyr Trp Pro Thr His Ser Gln Gln Lys Phe Cys
                   645                 650                 655
   tcc gaa gaa tgt caa agg gta aat cac aat aag aaa aca ttg gaa ttt      2016
   Ser Glu Glu Cys Gln Arg Val Asn His Asn Lys Lys Thr Leu Glu Phe
               660                 665                 670
   tac cac cat aaa aaa gaa aag gag aag ctg caa tgc aaa gat tta tca      2064
   Tyr His His Lys Lys Glu Lys Glu Lys Leu Gln Cys Lys Asp Leu Ser
           675                 680                 685
   cag acg aaa gaa cgg gta tcc gat atg aac tta tcg ggg act att act      2112
   Gln Thr Lys Glu Arg Val Ser Asp Met Asn Leu Ser Gly Thr Ile Thr
       690                 695                 700
   acc cct gct taa                                                      2124
   Thr Pro Ala
   705
<210> 61
   <211> 707
   <212> PRT
   <213> Transposon Tn4451
<400> 61
   Met Ser Arg Thr Ser Arg Ile Thr Ala Leu Tyr Glu Arg Leu Ser Arg
    1               5                  10                  15
   Asp Asp Asp Leu Thr Gly Glu Ser Asn Ser Ile Thr Asn Gln Lys Lys
               20                  25                  30
   Tyr Leu Glu Asp Tyr Ala Arg Arg Asn Gly Phe Glu Asn Ile Arg His
           35                  40                  45
   Phe Thr Asp Asp Gly Phe Ser Gly Val Asn Phe Asn Arg Pro Gly Phe
       50                  55                  60
   Gln Ser Leu Ile Lys Glu Val Glu Ala Gly Asn Val Glu Thr Leu Ile
   65                  70                  75                  80
   Val Lys Asp Met Ser Arg Leu Gly Arg Asn Tyr Leu Gln Val Gly Phe
                   85                  90                  95
   Tyr Thr Glu Val Leu Phe Pro Gln Lys Asn Val Arg Phe Leu Ala Ile
               100                 105                 110
   Asn Asn Ser Ile Asp Ser Asn Asn Ala Ser Asp Asn Asp Phe Ala Pro
           115                 120                 125
   Phe Leu Asn Ile Met Asn Glu Trp Tyr Ala Lys Asp Thr Ser Asn Lys
       130                 135                 140
   Ile Lys Ala Ile Phe Asp Ala Arg Met Lys Asp Gly Lys Arg Cys Ser
   145                 150                 155                 160
   Gly Ser Ile Pro Tyr Gly Tyr Asn Arg Leu Pro Ser Asp Lys Gln Thr
                   165                 170                 175
   Leu Val Val Asp Pro Val Ala Ser Glu Val Val Lys Arg Ile Phe Thr
               180                 185                 190
   Leu Ala Asn Asp Gly Lys Ser Thr Arg Ala Ile Ala Glu Ile Leu Thr
           195                 200                 205
   Glu Glu Lys Val Leu Thr Pro Ala Ala Tyr Ala Lys Glu Tyr His Pro
       210                 215                 220
   Glu Gln Tyr Asn Gly Asn Lys Phe Thr Asn Pro Tyr Leu Trp Ala Met
   225                 230                 235                 240
   Ser Thr Ile Arg Asn Ile Leu Gly Arg Gln Glu Tyr Leu Gly His Thr
                   245                 250                 255
   Val Leu Arg Lys Ser Val Ser Thr Asn Phe Lys Leu His Lys Arg Lys
               260                 265                 270
   Ser Thr Asp Glu Glu Glu Gln Tyr Val Phe Pro Asn Thr His Glu Pro
           275                 280                 285
   Ile Ile Ser Gln Glu Leu Trp Asp Ser Val Gln Lys Arg Arg Ser Arg
       290                 295                 300
   Val Asn Arg Ala Ser Ala Trp Gly Thr His Ser Asn Arg Leu Ser Gly
   305                 310                 315                 320
   Tyr Leu Tyr Cys Ala Asp Cys Gly Arg Arg Met Thr Leu Gln Thr His
                   325                 330                 335
   Tyr Ser Lys Lys Asp Gly Ser Val Gln Tyr Ser Tyr Arg Cys Gly Gly
               340                 345                 350
   Tyr Ala Ser Arg Val Asn Ser Cys Thr Ser His Ser Ile Ser Thr Asp
           355                 360                 365
   Asn Val Glu Ala Leu Ile Leu Ser Ser Val Lys Arg Phe Ser Arg Phe
       370                 375                 380
   Val Leu Asn Asp Glu Gln Ala Phe Ala Leu Glu Leu Gln Ser Leu Trp
   385                 390                 395                 400
   Asn Glu Lys Gln Glu Glu Lys Pro Lys His Asn Gln Ser Glu Leu Gln
                   405                 410                 415
   Arg Cys Gln Lys Arg Tyr Asp Glu Leu Ser Thr Leu Val Arg Gly Leu
               420                 425                 430
   Tyr Glu Asn Leu Met Ser Gly Leu Leu Pro Glu Arg Gln Tyr Lys Gln
           435                 440                 445
   Leu Met Lys Gln Tyr Asp Asp Glu Gln Ala Glu Leu Glu Thr Lys Met
       450                 455                 460
   Glu Thr Met Lys Thr Glu Leu Ala Glu Glu Lys Val Ser Ser Val Asp
   465                 470                 475                 480
   Ile Lys His Phe Ile Ser Leu Ile Arg Lys Cys Lys Asn Pro Thr Glu
                   485                 490                 495
   Ile Ser Asp Thr Met Phe Asn Glu Leu Val Asp Lys Ile Val Val Tyr
               500                 505                 510
   Glu Ala Glu Gly Val Gly Lys Ala Arg Thr Gln Lys Val Asp Ile Tyr
           515                 520                 525
   Phe Asn Tyr Val Gly Gln Val Asp Ile Ala Tyr Thr Glu Glu Glu Leu
       530                 535                 540
   Ala Glu Ile Glu Thr Gln Lys Glu Gln Glu Glu Gln Gln Arg Leu Ala
   545                 550                 555                 560
   Arg Gln Arg Lys Arg Glu Lys Ala Tyr Arg Glu Lys Arg Lys Ala Gln
                   565                 570                 575
   Lys Ile Ala Glu Asn Gly Gly Glu Ile Val Lys Thr Lys Val Cys Pro
               580                 585                 590
   His Cys Asn Lys Glu Phe Ile Pro Thr Ser Asn Arg Gln Val Phe Cys
           595                 600                 605
   Ser Lys Glu Cys Cys Tyr Gln Ala Arg Gln Asp Lys Lys Lys Thr Asp
       610                 615                 620
   Arg Glu Ala Glu Arg Gly Asn His Tyr Tyr Arg Gln Arg Val Cys Ala
   625                 630                 635                 640
   Val Cys Gly Asn Ser Tyr Trp Pro Thr His Ser Gln Gln Lys Phe Cys
                   645                 650                 655
   Ser Glu Glu Cys Gln Arg Val Asn His Asn Lys Lys Thr Leu Glu Phe
               660                 665                 670
   Tyr His His Lys Lys Glu Lys Glu Lys Leu Gln Cys Lys Asp Leu Ser
           675                 680                 685
   Gln Thr Lys Glu Arg Val Ser Asp Met Asn Leu Ser Gly Thr Ile Thr
       690                 695                 700
   Thr Pro Ala
   705
<210> 62
   <211> 1420
   <212> DNA
   <213> XisA recombinase
<220>
   <221> CDS
   <222> (1)..(1416)
<400> 62
   atg caa aat cag ggt caa gac aaa tat caa caa gcc ttt gca gac tta    48
   Met Gln Asn Gln Gly Gln Asp Lys Tyr Gln Gln Ala Phe Ala Asp Leu
    1               5                  10                  15
   gag cca ctt tca tct acc gac ggc agt ttt ctc ggc tca agt ctg caa    96
   Glu Pro Leu Ser Ser Thr Asp Gly Ser Phe Leu Gly Ser Ser Leu Gln
               20                  25                  30
   gca cag cag caa aga gaa cac atg aga aca aaa gta cta caa gac cta   144
   Ala Gln Gln Gln Arg Glu His Met Arg Thr Lys Val Leu Gln Asp Leu
           35                  40                  45
   gac aag gta aat ctg cgt ttg aag tct gca aag acg aaa gtc tca gtt   192
   Asp Lys Val Asn Leu Arg Leu Lys Ser Ala Lys Thr Lys Val Ser Val
       50                  55                  60
   cga gaa tct aac gga agt ctg caa tta cga gca acg tta cca att aaa   240
   Arg Glu Ser Asn Gly Ser Leu Gln Leu Arg Ala Thr Leu Pro Ile Lys
   65                  70                  75                  80
   cct gga gat aag gac acc aac ggt aca ggc aga aag caa tac aat ctc   288
   Pro Gly Asp Lys Asp Thr Asn Gly Thr Gly Arg Lys Gln Tyr Asn Leu
                   85                  90                  95
   agc ttg aat atc cct gca aac ttg gat gga ctg aag acg gct gag gaa      336
   Ser Leu Asn Ile Pro Ala Asn Leu Asp Gly Leu Lys Thr Ala Glu Glu
           100                 105                 110
   gaa gct tat gaa tta ggt aaa tta atc gct cgg aaa acc ttt gaa tgg      384
   Glu Ala Tyr Glu Leu Gly Lys Leu Ile Ala Arg Lys Thr Phe Glu Trp
           115                 120                 125
   aat gat aaa tat tta ggc aaa gaa gcc act aaa aaa gat tca caa aca      432
   Asn Asp Lys Tyr Leu Gly Lys Glu Ala Thr Lys Lys Asp Ser Gln Thr
       130                 135                 140
   ata ggt gat tta cta gaa aaa ttt gca gaa gag tat ttt aaa acc cat      480
   Ile Gly Asp Leu Leu Glu Lys Phe Ala Glu Glu Tyr Phe Lys Thr His
   145                 150                 155                 160
   aaa cgc acc act aaa agc gaa cat acc ttt ttt tac tat ttt tcc cgc      528
   Lys Arg Thr Thr Lys Ser Glu His Thr Phe Phe Tyr Tyr Phe Ser Arg
                   165                 170                 175
   acc caa cga tat acc aat tcc aaa gat tta gca acg gcg gaa aat ctc      576
   Thr Gln Arg Tyr Thr Asn Ser Lys Asp Leu Ala Thr Ala Glu Asn Leu
               180                 185                 190
   atc aat tca att gag caa atc gat aaa gaa tgg gcg aga tat aat gcc      624
   Ile Asn Ser Ile Glu Gln Ile Asp Lys Glu Trp Ala Arg Tyr Asn Ala
           195                 200                 205
   gcc aga gcc ata tca gct ttt tgc ata aca ttc aat ata gaa att gat      672
   Ala Arg Ala Ile Ser Ala Phe Cys Ile Thr Phe Asn Ile Glu Ile Asp
       210                 215                 220
   ttg tcc cag tat tcc aaa atg cct gat cgc aat tcg cgc aac atc ccc      720
   Leu Ser Gln Tyr Ser Lys Met Pro Asp Arg Asn Ser Arg Asn Ile Pro
   225                 230                 235                 240
   aca gat gca gaa ata cta tca gga att acc aaa ttt gaa gac tat cta      768
   Thr Asp Ala Glu Ile Leu Ser Gly Ile Thr Lys Phe Glu Asp Tyr Leu
                   245                 250                 255
   gtt acc aga gga aat caa gtt aat gaa gat gta aaa gat agc tgg caa      816
   Val Thr Arg Gly Asn Gln Val Asn Glu Asp Val Lys Asp Ser Trp Gln
               260                 265                 270
   ctt tgg cgc tgg aca tat gga atg tta gca gtt ttt ggt tta cgc ccc      864
   Leu Trp Arg Trp Thr Tyr Gly Met Leu Ala Val Phe Gly Leu Arg Pro
           275                 280                 285
   agg gaa att ttt att aac cct aat att gat tgg tgg tta agc aaa gag      912
   Arg Glu Ile Phe Ile Asn Pro Asn Ile Asp Trp Trp Leu Ser Lys Glu
       290                 295                 300
   aat ata gac ctc aca tgg aaa gta gac aaa gaa tgt aaa act ggt gaa      960
   Asn Ile Asp Leu Thr Trp Lys Val Asp Lys Glu Cys Lys Thr Gly Glu
   305                 310                 315                 320
   aga caa gca tta ccc tta cat aaa gaa tgg att gat gag ttt gat tta     1008
   Arg Gln Ala Leu Pro Leu His Lys Glu Trp Ile Asp Glu Phe Asp Leu
                   325                 330                 335
   aga aat ccg aaa tat tta gaa atg ctg gca aca gca att agt aaa aaa     1056
   Arg Asn Pro Lys Tyr Leu Glu Met Leu Ala Thr Ala Ile Ser Lys Lys
               340                 345                 350
   gat aaa aca aat cat gct gaa ata aca gcc tta act cag cgt att agt     1104
   Asp Lys Thr Asn His Ala Glu Ile Thr Ala Leu Thr Gln Arg Ile Ser
               355                 360                 365
   tgg tgg ttt cgg aaa gtc gaa tta gat ttt aaa ccc tat gat tta cgt    1152
   Trp Trp Phe Arg Lys Val Glu Leu Asp Phe Lys Pro Tyr Asp Leu Arg
       370                 375                 380
   cac gcc tgg gca atc aga gcg cat att tta ggc ata cca atc aaa gcg    1200
   His Ala Trp Ala Ile Arg Ala His Ile Leu Gly Ile Pro Ile Lys Ala
   385                 390                 395                 400
   gcg gct gat aat ttg ggg cat agt atg cag gtt cat aca caa acc tat    1248
   Ala Ala Asp Asn Leu Gly His Ser Met Gln Val His Thr Gln Thr Tyr
                   405                 410                 415
   cag cgc tgg ttc tcg cta gat atg cgg aag tta gcg att aat cag gct    1296
   Gln Arg Trp Phe Ser Leu Asp Met Arg Lys Leu Ala Ile Asn Gln Ala
               420                 425                 430
   ttg act aag agg aat gaa ttt gag gtg att agg gag gag aat gct aaa    1344
   Leu Thr Lys Arg Asn Glu Phe Glu Val Ile Arg Glu Glu Asn Ala Lys
           435                 440                 445
   ttg cag ata gaa aat gaa agg ttg agg atg gaa att gag aag tta aag    1392
   Leu Gln Ile Glu Asn Glu Arg Leu Arg Met Glu Ile Glu Lys Leu Lys
       450                 455                 460
   atg gaa ata gct tat aag aat agt tgag                               1420
   Met Glu Ile Ala Tyr Lys Asn Ser
   465                 470
<210> 63
   <211> 472
   <212> PRT
   <213> XisA recombinase
<400> 63
   Met Gln Asn Gln Gly Gln Asp Lys Tyr Gln Gln Ala Phe Ala Asp Leu
    1               5                  10                  15
   Glu Pro Leu Ser Ser Thr Asp Gly Ser Phe Leu Gly Ser Ser Leu Gln
               20                  25                  30
   Ala Gln Gln Gln Arg Glu His Met Arg Thr Lys Val Leu Gln Asp Leu
           35                  40                  45
   Asp Lys Val Asn Leu Arg Leu Lys Ser Ala Lys Thr Lys Val Ser Val
       50                  55                  60
   Arg Glu Ser Asn Gly Ser Leu Gln Leu Arg Ala Thr Leu Pro Ile Lys
   65                  70                  75                  80
   Pro Gly Asp Lys Asp Thr Asn Gly Thr Gly Arg Lys Gln Tyr Asn Leu
                   85                  90                  95
   Ser Leu Asn Ile Pro Ala Asn Leu Asp Gly Leu Lys Thr Ala Glu Glu
               100                 105                 110
   Glu Ala Tyr Glu Leu Gly Lys Leu Ile Ala Arg Lys Thr Phe Glu Trp
           115                 120                 125
   Asn Asp Lys Tyr Leu Gly Lys Glu Ala Thr Lys Lys Asp Ser Gln Thr
       130                 135                 140
   Ile Gly Asp Leu Leu Glu Lys Phe Ala Glu Glu Tyr Phe Lys Thr His
   145                 150                 155                 160
   Lys Arg Thr Thr Lys Ser Glu His Thr Phe Phe Tyr Tyr Phe Ser Arg
   165 170 175
   Thr Gln Arg Tyr Thr Asn Ser Lys Asp Leu Ala Thr Ala Glu Asn Leu
   180 185 190
   Ile Asn Ser Ile Glu Gln Ile Asp Lys Glu Trp Ala Arg Tyr Asn Ala
   195 200 205
   Ala Arg Ala Ile Ser Ala Phe Cys Ile Thr Phe Asn Ile Glu Ile Asp
   210 215 220
   Leu Ser Gln Tyr Ser Lys Met Pro Asp Arg Asn Ser Arg Asn Ile Pro
   225 230 235 240
   Thr Asp Ala Glu Ile Leu Ser Gly Ile Thr Lys Phe Glu Asp Tyr Leu
   245 250 255
   Val Thr Arg Gly Asn Gln Val Asn Glu Asp Val Lys Asp Ser Trp Gln
   260 265 270
   Leu Trp Arg Trp Thr Tyr Gly Met Leu Ala Val Phe Gly Leu Arg Pro
   275 280 285
   Arg Glu Ile Phe Ile Asn Pro Asn Ile Asp Trp Trp Leu Ser Lys Glu
   290 295 300
   Asn Ile Asp Leu Thr Trp Lys Val Asp Lys Glu Cys Lys Thr Gly Glu
   305 310 315 320
   Arg Gln Ala Leu Pro Leu His Lys Glu Trp Ile Asp Glu Phe Asp Leu
   325 330 335
   Arg Asn Pro Lys Tyr Leu Glu Met Leu Ala Thr Ala Ile Ser Lys Lys
   340 345 350
   Asp Lys Thr Asn His Ala Glu Ile Thr Ala Leu Thr Gln Arg Ile Ser
   355 360 365
   Trp Trp Phe Arg Lys Val Glu Leu Asp Phe Lys Pro Tyr Asp Leu Arg
   370 375 380
   His Ala Trp Ala Ile Arg Ala His Ile Leu Gly Ile Pro Ile Lys Ala
   385 390 395 400
   Ala Ala Asp Asn Leu Gly His Ser Met Gln Val His Thr Gln Thr Tyr
   405 410 415
   Gln Arg Trp Phe Ser Leu Asp Met Arg Lys Leu Ala Ile Asn Gln Ala
   420 425 430
   Leu Thr Lys Arg Asn Glu Phe Glu Val Ile Arg Glu Glu Asn Ala Lys
   435 440 445
   Leu Gln Ile Glu Asn Glu Arg Leu Arg Met Glu Ile Glu Lys Leu Lys
   450 455 460
   Met Glu Ile Ala Tyr Lys Asn Ser
   465 470
<210> 64
   <211> 1008
   <212> DNA
   <213> Artificial Sequence
<220>
   <221> CDS
   <222> (1)..(1005)
<220>
   <223> Description of Artificial Sequence: vector pBS-SSV3
<400> 64
   atg acg aaa gat aag acg cgt tat aaa tac ggg gat tat att tta cgc     48
   Met Thr Lys Asp Lys Thr Arg Tyr Lys Tyr Gly Asp Tyr Ile Leu Arg
    1               5                  10                  15
   gag agg aaa ggg cgg tat tat gtt tac aag cta gag tat gaa aac ggt     96
   Glu Arg Lys Gly Arg Tyr Tyr Val Tyr Lys Leu Glu Tyr Glu Asn Gly
               20                  25                  30
   gag gta aaa gag cgt tac gtg ggt cct tta gct gac gtc gtt gaa tca    144
   Glu Val Lys Glu Arg Tyr Val Gly Pro Leu Ala Asp Val Val Glu Ser
           35                  40                  45
   tat cta aaa atg aaa tta ggg gtc gta ggg gat act ccc cta caa gcg    192
   Tyr Leu Lys Met Lys Leu Gly Val Val Gly Asp Thr Pro Leu Gln Ala
       50                  55                  60
   gat ccc ccc ggt ttc gag ccc ggg aca agc gga agc ggt ggt gga aaa    240
   Asp Pro Pro Gly Phe Glu Pro Gly Thr Ser Gly Ser Gly Gly Gly Lys
   65                  70                  75                  80
   gag gga act gaa cga cgt aaa ata gcg ttg gtt gcc aat ttg cgc caa    288
   Glu Gly Thr Glu Arg Arg Lys Ile Ala Leu Val Ala Asn Leu Arg Gln
                   85                  90                  95
   tac gcg acg gac ggc aac ata aag gcg ttc tac aac tat ctc atg aac    336
   Tyr Ala Thr Asp Gly Asn Ile Lys Ala Phe Tyr Asn Tyr Leu Met Asn
               100                 105                 110
   gaa agg ggg ata agc gaa aaa act gca aag gac tac atc aat gct ata    384
   Glu Arg Gly Ile Ser Glu Lys Thr Ala Lys Asp Tyr Ile Asn Ala Ile
           115                 120                 125
   tca aag ccg tat aaa gag acg aga gac gca cag aag gct tac cga ctc    432
   Ser Lys Pro Tyr Lys Glu Thr Arg Asp Ala Gln Lys Ala Tyr Arg Leu
       130                 135                 140
   ttt gca cgt ttc tta gcg tca cgc aat atc ata cat gat gaa ttt gcg    480
   Phe Ala Arg Phe Leu Ala Ser Arg Asn Ile Ile His Asp Glu Phe Ala
   145                 150                 155                 160
   gat aaa ata ttg aaa gcg gta aag gtg aag aag gcg aac gct gat atc    528
   Asp Lys Ile Leu Lys Ala Val Lys Val Lys Lys Ala Asn Ala Asp Ile
                   165                 170                 175
   tac att cca acg ttg gaa gag ata aaa agg acg tta caa tta gca aaa    576
   Tyr Ile Pro Thr Leu Glu Glu Ile Lys Arg Thr Leu Gln Leu Ala Lys
               180                 185                 190
   gac tat agc gaa aac gtc tac ttc atc tac cgt atc gct ctc gag tcg    624
   Asp Tyr Ser Glu Asn Val Tyr Phe Ile Tyr Arg Ile Ala Leu Glu Ser
           195                 200                 205
   ggc gtt agg ctg agc gaa ata ctg aaa gtg ctg aag gaa ccc gaa agg    672
   Gly Val Arg Leu Ser Glu Ile Leu Lys Val Leu Lys Glu Pro Glu Arg
       210                 215                 220
   gac att tgc ggt aac gac gtc tgt tat tat ccg ctt agt tgg act agg    720
   Asp Ile Cys Gly Asn Asp Val Cys Tyr Tyr Pro Leu Ser Trp Thr Arg
   225                 230                 235                 240
   gga tat aag ggc gtc ttc tat gta ttc cac ata acg cct ctg aag aga    768
   Gly Tyr Lys Gly Val Phe Tyr Val Phe His Ile Thr Pro Leu Lys Arg
                   245                 250                 255
   gta gag gtg acg aag tgg gca ata gcg gac ttt gaa cga cgt cat aag    816
   Val Glu Val Thr Lys Trp Ala Ile Ala Asp Phe Glu Arg Arg His Lys
               260                 265                 270
   gac gct ata gcg ata aag tac ttc cgc aaa ttc gta gcg tct aag atg    864
   Asp Ala Ile Ala Ile Lys Tyr Phe Arg Lys Phe Val Ala Ser Lys Met
           275                 280                 285
   gct gag cta agc gta ccg tta gat att atc gat ttt att caa ggg cgt    912
   Ala Glu Leu Ser Val Pro Leu Asp Ile Ile Asp Phe Ile Gln Gly Arg
       290                 295                 300
   aaa ccg aca cgc gtt tta acg caa cat tac gta tcg ctc ttc ggc ata    960
   Lys Pro Thr Arg Val Leu Thr Gln His Tyr Val Ser Leu Phe Gly Ile
   305                 310                 315                 320
   gcg aaa gag caa tat aaa aag tat gcg gaa tgg cta aaa ggg gtc tga    1008
   Ala Lys Glu Gln Tyr Lys Lys Tyr Ala Glu Trp Leu Lys Gly Val
                   325                 330                 335
<210> 65
   <211> 335
   <212> PRT
   <213> Artificial Sequence
   <223> Description of Artificial Sequence: vector pBS-SSV3
<400> 65
   Met Thr Lys Asp Lys Thr Arg Tyr Lys Tyr Gly Asp Tyr Ile Leu Arg
    1               5                  10                  15
   Glu Arg Lys Gly Arg Tyr Tyr Val Tyr Lys Leu Glu Tyr Glu Asn Gly
               20                  25                  30
   Glu Val Lys Glu Arg Tyr Val Gly Pro Leu Ala Asp Val Val Glu Ser
           35                  40                  45
   Tyr Leu Lys Met Lys Leu Gly Val Val Gly Asp Thr Pro Leu Gln Ala
       50                  55                  60
   Asp Pro Pro Gly Phe Glu Pro Gly Thr Ser Gly Ser Gly Gly Gly Lys
   65                  70                  75                  80
   Glu Gly Thr Glu Arg Arg Lys Ile Ala Leu Val Ala Asn Leu Arg Gln
                   85                  90                  95
   Tyr Ala Thr Asp Gly Asn Ile Lys Ala Phe Tyr Asn Tyr Leu Met Asn
               100                 105                 110
   Glu Arg Gly Ile Ser Glu Lys Thr Ala Lys Asp Tyr Ile Asn Ala Ile
           115                 120                 125
   Ser Lys Pro Tyr Lys Glu Thr Arg Asp Ala Gln Lys Ala Tyr Arg Leu
       130                 135                 140
   Phe Ala Arg Phe Leu Ala Ser Arg Asn Ile Ile His Asp Glu Phe Ala
   145                 150                 155                 160
   Asp Lys Ile Leu Lys Ala Val Lys Val Lys Lys Ala Asn Ala Asp Ile
                   165                 170                 175
   Tyr Ile Pro Thr Leu Glu Glu Ile Lys Arg Thr Leu Gln Leu Ala Lys
   180 185 190
   Asp Tyr Ser Glu Asn Val Tyr Phe Ile Tyr Arg Ile Ala Leu Glu Ser
   195 200 205
   Gly Val Arg Leu Ser Glu Ile Leu Lys Val Leu Lys Glu Pro Glu Arg
   210 215 220
   Asp Ile Cys Gly Asn Asp Val Cys Tyr Tyr Pro Leu Ser Trp Thr Arg
   225 230 235 240
   Gly Tyr Lys Gly Val Phe Tyr Val Phe His Ile Thr Pro Leu Lys Arg
   245 250 255
   Val Glu Val Thr Lys Trp Ala Ile Ala Asp Phe Glu Arg Arg His Lys
   260 265 270
   Asp Ala Ile Ala Ile Lys Tyr Phe Arg Lys Phe Val Ala Ser Lys Met
   275 280 285
   Ala Glu Leu Ser Val Pro Leu Asp Ile Ile Asp Phe Ile Gln Gly Arg
   290 295 300
   Lys Pro Thr Arg Val Leu Thr Gln His Tyr Val Ser Leu Phe Gly Ile
   305 310 315 320
   Ala Lys Glu Gln Tyr Lys Lys Tyr Ala Glu Trp Leu Lys Gly Val
   325 330 335
<210> 66
   <211> 1441
   <212> DNA
   <213> Artificial Sequence
<220>
   <223> Description of Artificial Sequence: DNA sequence coding for fusion protein NLS-XisA
<220>
   <221> CDS
   <222> (1)..(1437)
<400> 66
   atg ccc aag aag aag agg aag gtg caa aat cag ggt caa gac aaa tat    48
   Met Pro Lys Lys Lys Arg Lys Val Gln Asn Gln Gly Gln Asp Lys Tyr
    1               5                  10                  15
   caa caa gcc ttt gca gac tta gag cca ctt tca tct acc gac ggc agt    96
   Gln Gln Ala Phe Ala Asp Leu Glu Pro Leu Ser Ser Thr Asp Gly Ser
               20                  25                  30
   ttt ctc ggc tca agt ctg caa gca cag cag caa aga gaa cac atg aga   144
   Phe Leu Gly Ser Ser Leu Gln Ala Gln Gln Gln Arg Glu His Met Arg
           35                  40                  45
   aca aaa gta cta caa gac cta gac aag gta aat ctg cgt ttg aag tct   192
   Thr Lys Val Leu Gln Asp Leu Asp Lys Val Asn Leu Arg Leu Lys Ser
       50                  55                  60
   gca aag acg aaa gtc tca gtt cga gaa tct aac gga agt ctg caa tta   240
   Ala Lys Thr Lys Val Ser Val Arg Glu Ser Asn Gly Ser Leu Gln Leu
   65                  70                  75                  80
   cga gca acg tta cca att aaa cct gga gat aag gac acc aac ggt aca     288
   Arg Ala Thr Leu Pro Ile Lys Pro Gly Asp Lys Asp Thr Asn Gly Thr
                   85                  90                  95
   ggc aga aag caa tac aat ctc agc ttg aat atc cct gca aac ttg gat     336
   Gly Arg Lys Gln Tyr Asn Leu Ser Leu Asn Ile Pro Ala Asn Leu Asp
               100                 105                 110
   gga ctg aag acg gct gag gaa gaa gct tat gaa tta ggt aaa tta atc     384
   Gly Leu Lys Thr Ala Glu Glu Glu Ala Tyr Glu Leu Gly Lys Leu Ile
           115                 120                 125
   gct cgg aaa acc ttt gaa tgg aat gat aaa tat tta ggc aaa gaa gcc     432
   Ala Arg Lys Thr Phe Glu Trp Asn Asp Lys Tyr Leu Gly Lys Glu Ala
       130                 135                 140
   act aaa aaa gat tca caa aca ata ggt gat tta cta gaa aaa ttt gca     480
   Thr Lys Lys Asp Ser Gln Thr Ile Gly Asp Leu Leu Glu Lys Phe Ala
   145                 150                 155                 160
   gaa gag tat ttt aaa acc cat aaa cgc acc act aaa agc gaa cat acc     528
   Glu Glu Tyr Phe Lys Thr His Lys Arg Thr Thr Lys Ser Glu His Thr
                   165                 170                 175
   ttt ttt tac tat ttt tcc cgc acc caa cga tat acc aat tcc aaa gat     576
   Phe Phe Tyr Tyr Phe Ser Arg Thr Gln Arg Tyr Thr Asn Ser Lys Asp
               180                 185                 190
   tta gca acg gcg gaa aat ctc atc aat tca att gag caa atc gat aaa     624
   Leu Ala Thr Ala Glu Asn Leu Ile Asn Ser Ile Glu Gln Ile Asp Lys
           195                 200                 205
   gaa tgg gcg aga tat aat gcc gcc aga gcc ata tca gct ttt tgc ata     672
   Glu Trp Ala Arg Tyr Asn Ala Ala Arg Ala Ile Ser Ala Phe Cys Ile
       210                 215                 220
   aca ttc aat ata gaa att gat ttg tcc cag tat tcc aaa atg cct gat     720
   Thr Phe Asn Ile Glu Ile Asp Leu Ser Gln Tyr Ser Lys Met Pro Asp
   225                 230                 235                 240
   cgc aat tcg cgc aac atc ccc aca gat gca gaa ata cta tca gga att     768
   Arg Asn Ser Arg Asn Ile Pro Thr Asp Ala Glu Ile Leu Ser Gly Ile
                   245                 250                 255
   acc aaa ttt gaa gac tat cta gtt acc aga gga aat caa gtt aat gaa     816
   Thr Lys Phe Glu Asp Tyr Leu Val Thr Arg Gly Asn Gln Val Asn Glu
               260                 265                 270
   gat gta aaa gat agc tgg caa ctt tgg cgc tgg aca tat gga atg tta     864
   Asp Val Lys Asp Ser Trp Gln Leu Trp Arg Trp Thr Tyr Gly Met Leu
           275                 280                 285
   gca gtt ttt ggt tta cgc ccc agg gaa att ttt att aac cct aat att     912
   Ala Val Phe Gly Leu Arg Pro Arg Glu Ile Phe Ile Asn Pro Asn Ile
       290                 295                 300
   gat tgg tgg tta agc aaa gag aat ata gac ctc aca tgg aaa gta gac     960
   Asp Trp Trp Leu Ser Lys Glu Asn Ile Asp Leu Thr Trp Lys Val Asp
   305                 310                 315                 320
   aaa gaa tgt aaa act ggt gaa aga caa gca tta ccc tta cat aaa gaa     1008
   Lys Glu Cys Lys Thr Gly Glu Arg Gln Ala Leu Pro Leu His Lys Glu
                   325                 330                 335
   tgg att gat gag ttt gat tta aga aat ccg aaa tat tta gaa atg ctg     1056
   Trp Ile Asp Glu Phe Asp Leu Arg Asn Pro Lys Tyr Leu Glu Met Leu
               340                 345                 350
   gca aca gca att agt aaa aaa gat aaa aca aat cat gct gaa ata aca    1104
   Ala Thr Ala Ile Ser Lys Lys Asp Lys Thr Asn His Ala Glu Ile Thr
           355                 360                 365
   gcc tta act cag cgt att agt tgg tgg ttt cgg aaa gtc gaa tta gat    1152
   Ala Leu Thr Gln Arg Ile Ser Trp Trp Phe Arg Lys Val Glu Leu Asp
       370                 375                 380
   ttt aaa ccc tat gat tta cgt cac gcc tgg gca atc aga gcg cat att    1200
   Phe Lys Pro Tyr Asp Leu Arg His Ala Trp Ala Ile Arg Ala His Ile
   385                 390                 395                 400
   tta ggc ata cca atc aaa gcg gcg gct gat aat ttg ggg cat agt atg    1248
   Leu Gly Ile Pro Ile Lys Ala Ala Ala Asp Asn Leu Gly His Ser Met
                   405                 410                 415
   cag gtt cat aca caa acc tat cag cgc tgg ttc tcg cta gat atg cgg    1296
   Gln Val His Thr Gln Thr Tyr Gln Arg Trp Phe Ser Leu Asp Met Arg
               420                 425                 430
   aag tta gcg att aat cag gct ttg act aag agg aat gaa ttt gag gtg    1344
   Lys Leu Ala Ile Asn Gln Ala Leu Thr Lys Arg Asn Glu Phe Glu Val
           435                 440                 445
   att agg gag gag aat gct aaa ttg cag ata gaa aat gaa agg ttg agg    1392
   Ile Arg Glu Glu Asn Ala Lys Leu Gln Ile Glu Asn Glu Arg Leu Arg
       450                 455                 460
   atg gaa att gag aag tta aag atg gaa ata gct tat aag aat agt tgag   1441
   Met Glu Ile Glu Lys Leu Lys Met Glu Ile Ala Tyr Lys Asn Ser
   465                 470                 475
<210> 67
   <211> 479
   <212> PRT
   <213> Artificial Sequence
   <223> Description of Artificial Sequence: DNA sequence coding for fusion protein NLS-XisA
<400> 67
   Met Pro Lys Lys Lys Arg Lys Val Gln Asn Gln Gly Gln Asp Lys Tyr
   1 5 10 15
   Gln Gln Ala Phe Ala Asp Leu Glu Pro Leu Ser Ser Thr Asp Gly Ser
   20 25 30
   Phe Leu Gly Ser Ser Leu Gln Ala Gln Gln Gln Arg Glu His Met Arg
   35 40 45
   Thr Lys Val Leu Gln Asp Leu Asp Lys Val Asn Leu Arg Leu Lys Ser
   50 55 60
   Ala Lys Thr Lys Val Ser Val Arg Glu Ser Asn Gly Ser Leu Gln Leu
   65 70 75 80
   Arg Ala Thr Leu Pro Ile Lys Pro Gly Asp Lys Asp Thr Asn Gly Thr
   85 90 95
   Gly Arg Lys Gln Tyr Asn Leu Ser Leu Asn Ile Pro Ala Asn Leu Asp
   100 105 110
   Gly Leu Lys Thr Ala Glu Glu Glu Ala Tyr Glu Leu Gly Lys Leu Ile
   115 120 125
   Ala Arg Lys Thr Phe Glu Trp Asn Asp Lys Tyr Leu Gly Lys Glu Ala
       130                 135                 140
   Thr Lys Lys Asp Ser Gln Thr Ile Gly Asp Leu Leu Glu Lys Phe Ala
   145                 150                 155                 160
   Glu Glu Tyr Phe Lys Thr His Lys Arg Thr Thr Lys Ser Glu His Thr
                   165                 170                 175
   Phe Phe Tyr Tyr Phe Ser Arg Thr Gln Arg Tyr Thr Asn Ser Lys Asp
               180                 185                 190
   Leu Ala Thr Ala Glu Asn Leu Ile Asn Ser Ile Glu Gln Ile Asp Lys
           195                 200                 205
   Glu Trp Ala Arg Tyr Asn Ala Ala Arg Ala Ile Ser Ala Phe Cys Ile
       210                 215                 220
   Thr Phe Asn Ile Glu Ile Asp Leu Ser Gln Tyr Ser Lys Met Pro Asp
   225                 230                 235                 240
   Arg Asn Ser Arg Asn Ile Pro Thr Asp Ala Glu Ile Leu Ser Gly Ile
                   245                 250                 255
   Thr Lys Phe Glu Asp Tyr Leu Val Thr Arg Gly Asn Gln Val Asn Glu
               260                 265                 270
   Asp Val Lys Asp Ser Trp Gln Leu Trp Arg Trp Thr Tyr Gly Met Leu
           275                 280                 285
   Ala Val Phe Gly Leu Arg Pro Arg Glu Ile Phe Ile Asn Pro Asn Ile
       290                 295                 300
   Asp Trp Trp Leu Ser Lys Glu Asn Ile Asp Leu Thr Trp Lys Val Asp
   305                 310                 315                 320
   Lys Glu Cys Lys Thr Gly Glu Arg Gln Ala Leu Pro Leu His Lys Glu
                   325                 330                 335
   Trp Ile Asp Glu Phe Asp Leu Arg Asn Pro Lys Tyr Leu Glu Met Leu
               340                 345                 350
   Ala Thr Ala Ile Ser Lys Lys Asp Lys Thr Asn His Ala Glu Ile Thr
           355                 360                 365
   Ala Leu Thr Gln Arg Ile Ser Trp Trp Phe Arg Lys Val Glu Leu Asp
       370                 375                 380
   Phe Lys Pro Tyr Asp Leu Arg His Ala Trp Ala Ile Arg Ala His Ile
   385                 390                 395                 400
   Leu Gly Ile Pro Ile Lys Ala Ala Ala Asp Asn Leu Gly His Ser Met
                   405                 410                 415
   Gln Val His Thr Gln Thr Tyr Gln Arg Trp Phe Ser Leu Asp Met Arg
               420                 425                 430
   Lys Leu Ala Ile Asn Gln Ala Leu Thr Lys Arg Asn Glu Phe Glu Val
           435                 440                 445
   Ile Arg Glu Glu Asn Ala Lys Leu Gln Ile Glu Asn Glu Arg Leu Arg
       450                 455                 460
   Met Glu Ile Glu Lys Leu Lys Met Glu Ile Ala Tyr Lys Asn Ser
   465                 470                 475
<210> 68
   <211> 1029
   <212> DNA
   <213> Artificial Sequence
<220>
   <223> Description of Artificial Sequence: DNA sequence coding for fusion protein NLS-Ssv
<220>
   <221> CDS
   <222> (1)..(1026)
<400> 68
   atg ccc aag aag aag agg aag gtg acg aaa gat aag acg cgt tat aaa      48
   Met Pro Lys Lys Lys Arg Lys Val Thr Lys Asp Lys Thr Arg Tyr Lys
    1               5                  10                  15
   tac ggg gat tat att tta cgc gag agg aaa ggg cgg tat tat gtt tac      96
   Tyr Gly Asp Tyr Ile Leu Arg Glu Arg Lys Gly Arg Tyr Tyr Val Tyr
               20                  25                  30
   aag cta gag tat gaa aac ggt gag gta aaa gag cgt tac gtg ggt cct     144
   Lys Leu Glu Tyr Glu Asn Gly Glu Val Lys Glu Arg Tyr Val Gly Pro
           35                  40                  45
   tta gct gac gtc gtt gaa tca tat cta aaa atg aaa tta ggg gtc gta     192
   Leu Ala Asp Val Val Glu Ser Tyr Leu Lys Met Lys Leu Gly Val Val
       50                  55                  60
   ggg gat act ccc cta caa gcg gat ccc ccc ggt ttc gag ccc ggg aca     240
   Gly Asp Thr Pro Leu Gln Ala Asp Pro Pro Gly Phe Glu Pro Gly Thr
   65                  70                  75                  80
   agc gga agc ggt ggt gga aaa gag gga act gaa cga cgt aaa ata gcg     288
   Ser Gly Ser Gly Gly Gly Lys Glu Gly Thr Glu Arg Arg Lys Ile Ala
                   85                  90                  95
   ttg gtt gcc aat ttg cgc caa tac gcg acg gac ggc aac ata aag gcg     336
   Leu Val Ala Asn Leu Arg Gln Tyr Ala Thr Asp Gly Asn Ile Lys Ala
               100                 105                 110
   ttc tac aac tat ctc atg aac gaa agg ggg ata agc gaa aaa act gca     384
   Phe Tyr Asn Tyr Leu Met Asn Glu Arg Gly Ile Ser Glu Lys Thr Ala
           115                 120                 125
   aag gac tac atc aat gct ata tca aag ccg tat aaa gag acg aga gac     432
   Lys Asp Tyr Ile Asn Ala Ile Ser Lys Pro Tyr Lys Glu Thr Arg Asp
       130                 135                 140
   gca cag aag gct tac cga ctc ttt gca cgt ttc tta gcg tca cgc aat     480
   Ala Gln Lys Ala Tyr Arg Leu Phe Ala Arg Phe Leu Ala Ser Arg Asn
   145                 150                 155                 160
   atc ata cat gat gaa ttt gcg gat aaa ata ttg aaa gcg gta aag gtg     528
   Ile Ile His Asp Glu Phe Ala Asp Lys Ile Leu Lys Ala Val Lys Val
                   165                 170                 175
   aag aag gcg aac gct gat atc tac att cca acg ttg gaa gag ata aaa     576
   Lys Lys Ala Asn Ala Asp Ile Tyr Ile Pro Thr Leu Glu Glu Ile Lys
               180                 185                 190
   agg acg tta caa tta gca aaa gac tat agc gaa aac gtc tac ttc atc     624
   Arg Thr Leu Gln Leu Ala Lys Asp Tyr Ser Glu Asn Val Tyr Phe Ile
           195                 200                 205
   tac cgt atc gct ctc gag tcg ggc gtt agg ctg agc gaa ata ctg aaa     672
   Tyr Arg Ile Ala Leu Glu Ser Gly Val Arg Leu Ser Glu Ile Leu Lys
       210                 215                 220
   gtg ctg aag gaa ccc gaa agg gac att tgc ggt aac gac gtc tgt tat      720
   Val Leu Lys Glu Pro Glu Arg Asp Ile Cys Gly Asn Asp Val Cys Tyr
   225                 230                 235                 240
   tat ccg ctt agt tgg act agg gga tat aag ggc gtc ttc tat gta ttc      768
   Tyr Pro Leu Ser Trp Thr Arg Gly Tyr Lys Gly Val Phe Tyr Val Phe
                   245                 250                 255
   cac ata acg cct ctg aag aga gta gag gtg acg aag tgg gca ata gcg      816
   His Ile Thr Pro Leu Lys Arg Val Glu Val Thr Lys Trp Ala Ile Ala
               260                 265                 270
   gac ttt gaa cga cgt cat aag gac gct ata gcg ata aag tac ttc cgc      864
   Asp Phe Glu Arg Arg His Lys Asp Ala Ile Ala Ile Lys Tyr Phe Arg
           275                 280                 285
   aaa ttc gta gcg tct aag atg gct gag cta agc gta ccg tta gat att      912
   Lys Phe Val Ala Ser Lys Met Ala Glu Leu Ser Val Pro Leu Asp Ile
       290                 295                 300
   atc gat ttt att caa ggg cgt aaa ccg aca cgc gtt tta acg caa cat      960
   Ile Asp Phe Ile Gln Gly Arg Lys Pro Thr Arg Val Leu Thr Gln His
   305                 310                 315                 320
   tac gta tcg ctc ttc ggc ata gcg aaa gag caa tat aaa aag tat gcg      1008
   Tyr Val Ser Leu Phe Gly Ile Ala Lys Glu Gln Tyr Lys Lys Tyr Ala
                   325                 330                 335
   gaa tgg cta aaa ggg gtc tga                                          1029
   Glu Trp Leu Lys Gly Val
               340
<210> 69
   <211> 342
   <212> PRT
   <213> Artificial Sequence
   <223> Description of Artificial Sequence: DNA sequence coding for fusion protein NLS-Ssv
<400> 69
   Met Pro Lys Lys Lys Arg Lys Val Thr Lys Asp Lys Thr Arg Tyr Lys
   1 5 10 15
   Tyr Gly Asp Tyr Ile Leu Arg Glu Arg Lys Gly Arg Tyr Tyr Val Tyr
   20 25 30
   Lys Leu Glu Tyr Glu Asn Gly Glu Val Lys Glu Arg Tyr Val Gly Pro
   35 40 45
   Leu Ala Asp Val Val Glu Ser Tyr Leu Lys Met Lys Leu Gly Val Val
   50 55 60
   Gly Asp Thr Pro Leu Gln Ala Asp Pro Pro Gly Phe Glu Pro Gly Thr
   65 70 75 80
   Ser Gly Ser Gly Gly Gly Lys Glu Gly Thr Glu Arg Arg Lys Ile Ala
   85 90 95
   Leu Val Ala Asn Leu Arg Gln Tyr Ala Thr Asp Gly Asn Ile Lys Ala
   100 105 110
   Phe Tyr Asn Tyr Leu Met Asn Glu Arg Gly Ile Ser Glu Lys Thr Ala
   115 120 125
   Lys Asp Tyr Ile Asn Ala Ile Ser Lys Pro Tyr Lys Glu Thr Arg Asp
       130                 135                 140
   Ala Gln Lys Ala Tyr Arg Leu Phe Ala Arg Phe Leu Ala Ser Arg Asn
   145                 150                 155                 160
   Ile Ile His Asp Glu Phe Ala Asp Lys Ile Leu Lys Ala Val Lys Val
                   165                 170                 175
   Lys Lys Ala Asn Ala Asp Ile Tyr Ile Pro Thr Leu Glu Glu Ile Lys
               180                 185                 190
   Arg Thr Leu Gln Leu Ala Lys Asp Tyr Ser Glu Asn Val Tyr Phe Ile
           195                 200                 205
   Tyr Arg Ile Ala Leu Glu Ser Gly Val Arg Leu Ser Glu Ile Leu Lys
       210                 215                 220
   Val Leu Lys Glu Pro Glu Arg Asp Ile Cys Gly Asn Asp Val Cys Tyr
   225                 230                 235                 240
   Tyr Pro Leu Ser Trp Thr Arg Gly Tyr Lys Gly Val Phe Tyr Val Phe
                   245                 250                 255
   His Ile Thr Pro Leu Lys Arg Val Glu Val Thr Lys Trp Ala Ile Ala
               260                 265                 270
   Asp Phe Glu Arg Arg His Lys Asp Ala Ile Ala Ile Lys Tyr Phe Arg
           275                 280                 285
   Lys Phe Val Ala Ser Lys Met Ala Glu Leu Ser Val Pro Leu Asp Ile
       290                 295                 300
   Ile Asp Phe Ile Gln Gly Arg Lys Pro Thr Arg Val Leu Thr Gln His
   305                 310                 315                 320
   Tyr Val Ser Leu Phe Gly Ile Ala Lys Glu Gln Tyr Lys Lys Tyr Ala
                   325                 330                 335
   Glu Trp Leu Lys Gly Val
               340
<210> 70
   <211> 3908
   <212> DNA
   <213> Artificial Sequence
<220>
   <223> Description of Artificial Sequence: vector pBS-SSV3
<400> 70
   cacctaaatt gtaagcgtta atattttgtt aaaattcgcg ttaaattttt gttaaatcag 60
   ctcatttttt aaccaatagg ccgaaatcgg caaaatccct tataaatcaa aagaatagac 120
   cgagataggg ttgagtgttg ttccagtttg gaacaagagt ccactattaa agaacgtgga 180
   ctccaacgtc aaagggcgaa aaaccgtcta tcagggcgat ggcccactac gtgaaccatc 240
   accctaatca agttttttgg ggtcgaggtg ccgtaaagca ctaaatcgga accctaaagg 300
   gagcccccga tttagagctt gacggggaaa gccggcgaac gtggcgagaa aggaagggaa 360
   gaaagcgaaa ggagcgggcg ctagggcgct ggcaagtgta gcggtcacgc tgcgcgtaac 420
   caccacaccc gccgcgctta atgcgccgct acagggcgcg tcccattcgc cattcaggct 480
   gcgcaactgt tgggaagggc gatcggtgcg ggcctcttcg ctattacgcc agctggcgaa 540
   agggggatgt gctgcaaggc gattaagttg ggtaacgcca gggttttccc agtcacgacg 600
   ttgtaaaacg acggccagtg aattgtaata cgactcacta tagggcgaat tggagctcca 660
   ccgcggtggc ggccgcccga tatgacgaaa gataagacgc gttataaata cggggattat 720
   attttacgcg agaggaaagg gcggtattat gtttacaagc tagagtatga aaacggtgag 780
   gtaaaagagc gttacgtggg tcctttagct gacgtcgttg aatcatatct aaaaatgaaa 840
   ttaggggtcg taggggatac tcccctacaa gcggatcccc ccggtttcga gcccgggaca 900
   agcggaagcg gtggtggaaa agagggaact gaacgacgta aaatagcgtt ggttgccaat 960
   ttgcgccaat acgcgacgga cggcaacata aaggcgttct acaactatct catgaacgaa 1020
   agggggataa gcgaaaaaac tgcaaaggac tacatcaatg ctatatcaaa gccgtataaa 1080
   gagacgagag acgcacagaa ggcttaccga ctctttgcac gtttcttagc gtcacgcaat 1140
   atcatacatg atgaatttgc ggataaaata ttgaaagcgg taaaggtgaa gaaggcgaac 1200
   gctgatatct acattccaac gttggaagag ataaaaagga cgttacaatt agcaaaagac 1260
   tatagcgaaa acgtctactt catctaccgt atcgctctcg agtcgggcgt taggctgagc 1320
   gaaatactga aagtgctgaa ggaacccgaa agggacattt gcggtaacga cgtctgttat 1380
   tatccgctta gttggactag gggatataag ggcgtcttct atgtattcca cataacgcct 1440
   ctgaagagag tagaggtgac gaagtgggca atagcggact ttgaacgacg tcataaggac 1500
   gctatagcga taaagtactt ccgcaaattc gtagcgtcta agatggctga gctaagcgta 1560
   ccgttagata ttatcgattt tattcaaggg cgtaaaccga cacgcgtttt aacgcaacat 1620
   tacgtatcgc tcttcggcat agcgaaagag caatataaaa agtatgcgga atggctaaaa 1680
   ggggtctgac tcgagggggg gcccggtacc cagcttttgt tccctttagt gagggttaat 1740
   ttcgagcttg gcgtaatcat ggtcatagct gtttcctgtg tgaaattgtt atccgctcac 1800
   aattccacac aacatacgag ccggaagcat aaagtgtaaa gcctggggtg cctaatgagt 1860
   gagctaactc acattaattg cgttgcgctc actgcccgct ttccagtcgg gaaacctgtc 1920
   gtgccagctg cattaatgaa tcggccaacg cgcggggaga ggcggtttgc gtattgggcg 1980
   ctcttccgct tcctcgctca ctgactcgct gcgctcggtc gttcggctgc ggcgagcggt 2040
   atcagctcac tcaaaggcgg taatacggtt atccacagaa tcaggggata acgcaggaaa 2100
   gaacatgtga gcaaaaggcc agcaaaaggc caggaaccgt aaaaaggccg cgttgctggc 2160
   gtttttccat aggctccgcc cccctgacga gcatcacaaa aatcgacgct caagtcagag 2220
   gtggcgaaac ccgacaggac tataaagata ccaggcgttt ccccctggaa gctccctcgt 2280
   gcgctctcct gttccgaccc tgccgcttac cggatacctg tccgcctttc tcccttcggg 2340
   aagcgtggcg ctttctcata gctcacgctg taggtatctc agttcggtgt aggtcgttcg 2400
   ctccaagctg ggctgtgtgc acgaaccccc cgttcagccc gaccgctgcg ccttatccgg 2460
   taactatcgt cttgagtcca acccggtaag acacgactta tcgccactgg cagcagccac 2520
   tggtaacagg attagcagag cgaggtatgt aggcggtgct acagagttct tgaagtggtg 2580
   gcctaactac ggctacacta gaaggacagt atttggtatc tgcgctctgc tgaagccagt 2640
   taccttcgga aaaagagttg gtagctcttg atccggcaaa caaaccaccg ctggtagcgg 2700
   tggttttttt gtttgcaagc agcagattac gcgcagaaaa aaaggatctc aagaagatcc 2760
   tttgatcttt tctacggggt ctgacgctca gtggaacgaa aactcacgtt aagggatttt 2820
   ggtcatgaga ttatcaaaaa ggatcttcac ctagatcctt ttaaattaaa aatgaagttt 2880
   taaatcaatc taaagtatat atgagtaaac ttggtctgac agttaccaat gcttaatcag 2940
   tgaggcacct atctcagcga tctgtctatt tcgttcatcc atagttgcct gactccccgt 3000
   cgtgtagata actacgatac gggagggctt accatctggc cccagtgctg caatgatacc 3060
   gcgagaccca cgctcaccgg ctccagattt atcagcaata aaccagccag ccggaagggc 3120
   cgagcgcaga agtggtcctg caactttatc cgcctccatc cagtctatta attgttgccg 3180
   ggaagctaga gtaagtagtt cgccagttaa tagtttgcgc aacgttgttg ccattgctac 3240
   aggcatcgtg gtgtcacgct cgtcgtttgg tatggcttca ttcagctccg gttcccaacg 3300
   atcaaggcga gttacatgat cccccatgtt gtgcaaaaaa gcggttagct ccttcggtcc 3360
   tccgatcgtt gtcagaagta agttggccgc agtgttatca ctcatggtta tggcagcact 3420
   gcataattct cttactgtca tgccatccgt aagatgcttt tctgtgactg gtgagtactc 3480
   aaccaagtca ttctgagaat agtgtatgcg gcgaccgagt tgctcttgcc cggcgtcaat 3540
   acgggataat accgcgccac atagcagaac tttaaaagtg ctcatcattg gaaaacgttc 3600
   ttcggggcga aaactctcaa ggatcttacc gctgttgaga tccagttcga tgtaacccac 3660
   tcgtgcaccc aactgatctt cagcatcttt tactttcacc agcgtttctg ggtgagcaaa 3720
   aacaggaagg caaaatgccg caaaaaaggg aataagggcg acacggaaat gttgaatact 3780
   catactcttc ctttttcaat attattgaag catttatcag ggttattgtc tcatgagcgg 3840
   atacatattt gaatgtattt agaaaaataa acaaataggg gttccgcgca catttccccg 3900
   aaaagtgc 3908
<210> 71
   <211> 3927
   <212> DNA
   <213> Artificial Sequence
<220>
   <223> Description of Artificial Sequence: vector pBS-SSV4
<400> 71
   cacctaaatt gtaagcgtta atattttgtt aaaattcgcg ttaaattttt gttaaatcag 60
   ctcatttttt aaccaatagg ccgaaatcgg caaaatccct tataaatcaa aagaatagac 120
   cgagataggg ttgagtgttg ttccagtttg gaacaagagt ccactattaa agaacgtgga 180
   ctccaacgtc aaagggcgaa aaaccgtcta tcagggcgat ggcccactac gtgaaccatc 240
   accctaatca agttttttgg ggtcgaggtg ccgtaaagca ctaaatcgga accctaaagg 300
   gagcccccga tttagagctt gacggggaaa gccggcgaac gtggcgagaa aggaagggaa 360
   gaaagcgaaa ggagcgggcg ctagggcgct ggcaagtgta gcggtcacgc tgcgcgtaac 420
   caccacaccc gccgcgctta atgcgccgct acagggcgcg tcccattcgc cattcaggct 480
   gcgcaactgt tgggaagggc gatcggtgcg ggcctcttcg ctattacgcc agctggcgaa 540
   agggggatgt gctgcaaggc gattaagttg ggtaacgcca gggttttccc agtcacgacg 600
   ttgtaaaacg acggccagtg aattgtaata cgactcacta tagggcgaat tggagctcca 660
   ccgcggtggc ggccgcacca tgcccaagaa gaagaggaag gtgacgaaag ataagacgcg 720
   ttataaatac ggggattata ttttacgcga gaggaaaggg cggtattatg tttacaagct 780
   agagtatgaa aacggtgagg taaaagagcg ttacgtgggt cctttagctg acgtcgttga 840
   atcatatcta aaaatgaaat taggggtcgt aggggatact cccctacaag cggatccccc 900
   cggtttcgag cccgggacaa gcggaagcgg tggtggaaaa gagggaactg aacgacgtaa 960
   aatagcgttg gttgccaatt tgcgccaata cgcgacggac ggcaacataa aggcgttcta 1020
   caactatctc atgaacgaaa gggggataag cgaaaaaact gcaaaggact acatcaatgc 1080
   tatatcaaag ccgtataaag agacgagaga cgcacagaag gcttaccgac tctttgcacg 1140
   tttcttagcg tcacgcaata tcatacatga tgaatttgcg gataaaatat tgaaagcggt 1200
   aaaggtgaag aaggcgaacg ctgatatcta cattccaacg ttggaagaga taaaaaggac 1260
   gttacaatta gcaaaagact atagcgaaaa cgtctacttc atctaccgta tcgctctcga 1320
   gtcgggcgtt aggctgagcg aaatactgaa agtgctgaag gaacccgaaa gggacatttg 1380
   cggtaacgac gtctgttatt atccgcttag ttggactagg ggatataagg gcgtcttcta 1440
   tgtattccac ataacgcctc tgaagagagt agaggtgacg aagtgggcaa tagcggactt 1500
   tgaacgacgt cataaggacg ctatagcgat aaagtacttc cgcaaattcg tagcgtctaa 1560
   gatggctgag ctaagcgtac cgttagatat tatcgatttt attcaagggc gtaaaccgac 1620
   acgcgtttta acgcaacatt acgtatcgct cttcggcata gcgaaagagc aatataaaaa 1680
   gtatgcggaa tggctaaaag ggtctgactt cgaggggggg cccggtaccc agcttttgtt 1740
   ccctttagtg agggttaatt tcgagcttgg cgtaatcatg gtcatagctg tttcctgtgt 1800
   gaaattgtta tccgctcaca attccacaca acatacgagc cggaagcata aagtgtaaag 1860
   cctggggtgc ctaatgagtg agctaactca cattaattgc gttgcgctca ctgcccgctt 1920
   tccagtcggg aaacctgtcg tgccagctgc attaatgaat cggccaacgc gcggggagag 1980
   gcggtttgcg tattgggcgc tcttccgctt cctcgctcac tgactcgctg cgctcggtcg 2040
   ttcggctgcg gcgagcggta tcagctcact caaaggcggt aatacggtta tccacagaat 2100
   caggggataa cgcaggaaag aacatgtgag caaaaggcca gcaaaaggcc aggaaccgta 2160
   aaaaggccgc gttgctggcg tttttccata ggctccgccc ccctgacgag catcacaaaa 2220
   atcgacgctc aagtcagagg tggcgaaacc cgacaggact ataaagatac caggcgtttc 2280
   cccctggaag ctccctcgtg cgctctcctg ttccgaccct gccgcttacc ggatacctgt 2340
   ccgcctttct cccttcggga agcgtggcgc tttctcatag ctcacgctgt aggtatctca 2400
   gttcggtgta ggtcgttcgc tccaagctgg gctgtgtgca cgaacccccc gttcagcccg 2460
   accgctgcgc cttatccggt aactatcgtc ttgagtccaa cccggtaaga cacgacttat 2520
   cgccactggc agcagccact ggtaacagga ttagcagagc gaggtatgta ggcggtgcta 2580
   cagagttctt gaagtggtgg cctaactacg gctacactag aaggacagta tttggtatct 2640
   gcgctctgct gaagccagtt accttcggaa aaagagttgg tagctcttga tccggcaaac 2700
   aaaccaccgc tggtagcggt ggtttttttg tttgcaagca gcagattacg cgcagaaaaa 2760
   aaggatctca agaagatcct ttgatctttt ctacggggtc tgacgctcag tggaacgaaa 2820
   actcacgtta agggattttg gtcatgagat tatcaaaaag gatcttcacc tagatccttt 2880
   taaattaaaa atgaagtttt aaatcaatct aaagtatata tgagtaaact tggtctgaca 2940
   gttaccaatg cttaatcagt gaggcaccta tctcagcgat ctgtctattt cgttcatcca 3000
   tagttgcctg actccccgtc gtgtagataa ctacgatacg ggagggctta ccatctggcc 3060
   ccagtgctgc aatgataccg cgagacccac gctcaccggc tccagattta tcagcaataa 3120
   accagccagc cggaagggcc gagcgcagaa gtggtcctgc aactttatcc gcctccatcc 3180
   agtctattaa ttgttgccgg gaagctagag taagtagttc gccagttaat agtttgcgca 3240
   acgttgttgc cattgctaca ggcatcgtgg tgtcacgctc gtcgtttggt atggcttcat 3300
   tcagctccgg ttcccaacga tcaaggcgag ttacatgatc ccccatgttg tgcaaaaaag 3360
   cggttagctc cttcggtcct ccgatcgttg tcagaagtaa gttggccgca gtgttatcac 3420
   tcatggttat ggcagcactg cataattctc ttactgtcat gccatccgta agatgctttt 3480
   ctgtgactgg tgagtactca accaagtcat tctgagaata gtgtatgcgg cgaccgagtt 3540
   gctcttgccc ggcgtcaata cgggataata ccgcgccaca tagcagaact ttaaaagtgc 3600
   tcatcattgg aaaacgttct tcggggcgaa aactctcaag gatcttaccg ctgttgagat 3660
   ccagttcgat gtaacccact cgtgcaccca actgatcttc agcatctttt actttcacca 3720
   gcgtttctgg gtgagcaaaa acaggaaggc aaaatgccgc aaaaaaggga ataagggcga 3780
   cacggaaatg ttgaatactc atactcttcc tttttcaata ttattgaagc atttatcagg 3840
   gttattgtct catgagcgga tacatatttg aatgtattta gaaaaataaa caaatagggg 3900
   ttccgcgcac atttccccga aaagtgc                                     3927
<210> 72
   <211> 3351
   <212> DNA
   <213> Artificial Sequence
<220>
   <223> Description of Artificial Sequence: vector pBS-SSVs
<400> 72
   cgacctcgag tcagacccct tttagccatt ccgcatactt tttatattgc tctttcgcta 60
   tgccgaagag cgatacgtaa tgttgcgtta aaacgcgtgt cggtttacgc ccttgaataa 120
   aatcgataat atctaacggt acgcttagct cagccatctt agacgctacg aatttgcgga 180
   agtactttat cgctatagcg tccttatgac gtcgttcaaa gtccgctatt gcccacttcg 240
   tcacctctac tctcttcaga ggcgttatgt ggaatacata gaagacgccc ttatatcccc 300
   tagtccaact aagcggataa taacagacgt cgttaccgca aatgtccctt tcgggttcct 360
   tcagcacttt cagtatttcg ctcagcctaa cgcccgactc gagggggggc ccggtaccca 420
   gcttttgttc cctttagtga gggttaattt cgagcttggc gtaatcatgg tcatagctgt 480
   ttcctgtgtg aaattgttat ccgctcacaa ttccacacaa catacgagcc ggaagcataa 540
   agtgtaaagc ctggggtgcc taatgagtga gctaactcac attaattgcg ttgcgctcac 600
   tgcccgcttt ccagtcggga aacctgtcgt gccagctgca ttaatgaatc ggccaacgcg 660
   cggggagagg cggtttgcgt attgggcgct cttccgcttc ctcgctcact gactcgctgc 720
   gctcggtcgt tcggctgcgg cgagcggtat cagctcactc aaaggcggta atacggttat 780
   ccacagaatc aggggataac gcaggaaaga acatgtgagc aaaaggccag caaaaggcca 840
   ggaaccgtaa aaaggccgcg ttgctggcgt ttttccatag gctccgcccc cctgacgagc 900
   atcacaaaaa tcgacgctca agtcagaggt ggcgaaaccc gacaggacta taaagatacc 960
   aggcgtttcc ccctggaagc tccctcgtgc gctctcctgt tccgaccctg ccgcttaccg 1020
   gatacctgtc cgcctttctc ccttcgggaa gcgtggcgct ttctcatagc tcacgctgta 1080
   ggtatctcag ttcggtgtag gtcgttcgct ccaagctggg ctgtgtgcac gaaccccccg 1140
   ttcagcccga ccgctgcgcc ttatccggta actatcgtct tgagtccaac ccggtaagac 1200
   acgacttatc gccactggca gcagccactg gtaacaggat tagcagagcg aggtatgtag 1260
   gcggtgctac agagttcttg aagtggtggc ctaactacgg ctacactaga aggacagtat 1320
   ttggtatctg cgctctgctg aagccagtta ccttcggaaa aagagttggt agctcttgat 1380
   ccggcaaaca aaccaccgct ggtagcggtg gtttttttgt ttgcaagcag cagattacgc 1440
   gcagaaaaaa aggatctcaa gaagatcctt tgatcttttc tacggggtct gacgctcagt 1500
   ggaacgaaaa ctcacgttaa gggattttgg tcatgagatt atcaaaaagg atcttcacct 1560
   agatcctttt aaattaaaaa tgaagtttta aatcaatcta aagtatatat gagtaaactt 1620
   ggtctgacag ttaccaatgc ttaatcagtg aggcacctat ctcagcgatc tgtctatttc 1680
   gttcatccat agttgcctga ctccccgtcg tgtagataac tacgatacgg gagggcttac 1740
   catctggccc cagtgctgca atgataccgc gagacccacg ctcaccggct ccagatttat 1800
   cagcaataaa ccagccagcc ggaagggccg agcgcagaag tggtcctgca actttatccg 1860
   cctccatcca gtctattaat tgttgccggg aagctagagt aagtagttcg ccagttaata 1920
   gtttgcgcaa cgttgttgcc attgctacag gcatcgtggt gtcacgctcg tcgtttggta 1980
   tggcttcatt cagctccggt tcccaacgat caaggcgagt tacatgatcc cccatgttgt 2040
   gcaaaaaagc ggttagctcc ttcggtcctc cgatcgttgt cagaagtaag ttggccgcag 2100
   tgttatcact catggttatg gcagcactgc ataattctct tactgtcatg ccatccgtaa 2160
   gatgcttttc tgtgactggt gagtactcaa ccaagtcatt ctgagaatag tgtatgcggc 2220
   gaccgagttg ctcttgcccg gcgtcaatac gggataatac cgcgccacat agcagaactt 2280
   taaaagtgct catcattgga aaacgttctt cggggcgaaa actctcaagg atcttaccgc 2340
   tgttgagatc cagttcgatg taacccactc gtgcacccaa ctgatcttca gcatctttta 2400
   ctttcaccag cgtttctggg tgagcaaaaa caggaaggca aaatgccgca aaaaagggaa 2460
   taagggcgac acggaaatgt tgaatactca tactcttcct ttttcaatat tattgaagca 2520
   tttatcaggg ttattgtctc atgagcggat acatatttga atgtatttag aaaaataaac 2580
   aaataggggt tccgcgcaca tttccccgaa aagtgccacc taaattgtaa gcgttaatat 2640
   tttgttaaaa ttcgcgttaa atttttgtta aatcagctca ttttttaacc aataggccga 2700
   aatcggcaaa atcccttata aatcaaaaga atagaccgag atagggttga gtgttgttcc 2760
   agtttggaac aagagtccac tattaaagaa cgtggactcc aacgtcaaag ggcgaaaaac 2820
   cgtctatcag ggcgatggcc cactacgtga accatcaccc taatcaagtt ttttggggtc 2880
   gaggtgccgt aaagcactaa atcggaaccc taaagggagc ccccgattta gagcttgacg 2940
   gggaaagccg gcgaacgtgg cgagaaagga agggaagaaa gcgaaaggag cgggcgctag 3000
   ggcgctggca agtgtagcgg tcacgctgcg cgtaaccacc acacccgccg cgcttaatgc 3060
   gccgctacag ggcgcgtccc attcgccatt caggctgcgc aactgttggg aagggcgatc 3120
   ggtgcgggcc tcttcgctat tacgccagct ggcgaaaggg ggatgtgctg caaggcgatt 3180
   aagttgggta acgccagggt tttcccagtc acgacgttgt aaaacgacgg ccagtgaatt 3240
   gtaatacgac tcactatagg gcgaattgga gctccaccgc ggtggcggcc gctctagaac 3300
   tagtggatcc cccgggctgc aggaattcga tatcaagctt atcgataccg t          3351
<210> 73
   <211> 5730
   <212> DNA
   <213> Artificial Sequence
<220>
   <223> Description of Artificial Sequence: vector pCMVC31(NNLS)
<400> 73
   aaacagtccg atgtacgggc cagatatacg cgttgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggac 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catggtgatg 420
   cggttttggc agtacatcaa tgggcgtgga tagcggtttg actcacgggg atttccaagt 480
   ctccacccca ttgacgtcaa tgggagtttg ttttggcacc aaaatcaacg ggactttcca 540
   aaatgtcgta acaactccgc cccattgacg caaatgggcg gtaggcgtgt acggtgggag 600
   gtctatataa gcagagctct ctggctaact agagaaccca ctgcttactg gcttatcgaa 660
   attaatacga ctcactatag ggagacccaa gctgactcta gacttaatta agcgttgggg 720
   tgagtactcc ctctcaaaag cgggcatgac ttctgcgcta agattgtcag tttccaaaaa 780
   cgaggaggat ttgatattca cctggcccgc ggtgatgcct ttgagggtgg ccgcgtccat 840
   ctggtcagaa aagacaatct ttttgttgtc aagcttgagg tgtggcaggc ttgagatctg 900
   gccatacact tgagtgacat tgacatccac tttgcctttc tctccacagg tgtccactcc 960
   cagggcggcc gcaccatgcc caagaagaag aggaaggtga cacaaggggt tgtgaccggg 1020
   gtggacacgt acgcgggtgc ttacgaccgt cagtcgcgcg agcgcgagaa ttcgagcgca 1080
   gcaagcccag cgacacagcg tagcgccaac gaagacaagg cggccgacct tcagcgcgaa 1140
   gtcgagcgcg acgggggccg gttcaggttc gtcgggcatt tcagcgaagc gccgggcacg 1200
   tcggcgttcg ggacggcgga gcgcccggag ttcgaacgca tcctgaacga atgccgcgcc 1260
   gggcggctca acatgatcat tgtctatgac gtgtcgcgct tctcgcgcct gaaggtcatg 1320
   gacgcgattc cgattgtctc ggaattgctc gccctgggcg tgacgattgt ttccactcag 1380
   gaaggcgtct tccggcaggg aaacgtcatg gacctgattc acctgattat gcggctcgac 1440
   gcgtcgcaca aagaatcttc gctgaagtcg gcgaagattc tcgacacgaa gaaccttcag 1500
   cgcgaattgg gcgggtacgt cggcgggaag gcgccttacg gcttcgagct tgtttcggag 1560
   acgaaggaga tcacgcgcaa cggccgaatg gtcaatgtcg tcatcaacaa gcttgcgcac 1620
   tcgaccactc cccttaccgg acccttcgag ttcgagcccg acgtaatccg gtggtggtgg 1680
   cgtgagatca agacgcacaa acaccttccc ttcaagccgg gcagtcaagc cgccattcac 1740
   ccgggcagca tcacggggct ttgtaagcgc atggacgctg acgccgtgcc gacccggggc 1800
   gagacgattg ggaagaagac cgcttcaagc gcctgggacc cggcaaccgt tatgcgaatc 1860
   cttcgggacc cgcgtattgc gggcttcgcc gctgaggtga tctacaagaa gaagccggac 1920
   ggcacgccga ccacgaagat tgagggttac cgcattcagc gcgacccgat cacgctccgg 1980
   ccggtcgagc ttgattgcgg accgatcatc gagcccgctg agtggtatga gcttcaggcg 2040
   tggttggacg gcagggggcg cggcaagggg ctttcccggg ggcaagccat tctgtccgcc 2100
   atggacaagc tgtactgcga gtgtggcgcc gtcatgactt cgaagcgcgg ggaagaatcg 2160
   atcaaggact cttaccgctg ccgtcgccgg aaggtggtcg acccgtccgc acctgggcag 2220
   cacgaaggca cgtgcaacgt cagcatggcg gcactcgaca agttcgttgc ggaacgcatc 2280
   ttcaacaaga tcaggcacgc cgaaggcgac gaagagacgt tggcgcttct gtgggaagcc 2340
   gcccgacgct tcggcaagct cactgaggcg cctgagaaga gcggcgaacg ggcgaacctt 2400
   gttgcggagc gcgccgacgc cctgaacgcc cttgaagagc tgtacgaaga ccgcgcggca 2460
   ggcgcgtacg acggacccgt tggcaggaag cacttccgga agcaacaggc agcgctgacg 2520
   ctccggcagc aaggggcgga agagcggctt gccgaacttg aagccgccga agccccgaag 2580
   cttccccttg accaatggtt ccccgaagac gccgacgctg acccgaccgg ccctaagtcg 2640
   tggtgggggc gcgcgtcagt agacgacaag cgcgtgttcg tcgggctctt cgtagacaag 2700
   atcgttgtca cgaagtcgac tacgggcagg gggcagggaa cgcccatcga gaagcgcgct 2760
   tcgatcacgt gggcgaagcc gccgaccgac gacgacgaag acgacgccca ggacggcacg 2820
   gaagacgtag cggcgtaggc ggcgcccggg ctcgagatcc aggcgcggat caataaaaga 2880
   tcattatttt caatagatct gtgtgttggt tttttgtgtg ccttggggga gggggaggcc 2940
   agaatgaggc gcggccaagg gggaggggga ggccagaatg accttggggg agggggaggc 3000
   cagaatgacc ttgggggagg gggaggccag aatgaggcgc gcccccgggt accgagctcg 3060
   aattcactgg ccgtcgtttt acaacgtcgt gactgggaaa accctggcgt tacccaactt 3120
   aatcgccttg cagcacatcc ccctttcgcc agctggcgta atagcgaaga ggcccgcacc 3180
   gatcgccctt cccaacagtt gcgcagcctg aatggcgaat ggcgcctgat gcggtatttt 3240
   ctccttacgc atctgtgcgg tatttcacac cgcatatggt gcactctcag tacaatctgc 3300
   tctgatgccg catagttaag ccagccccga cacccgccaa cacccgctga cgcgccctga 3360
   cgggcttgtc tgctcccggc atccgcttac agacaagctg tgaccgtctc cgggagctgc 3420
   atgtgtcaga ggttttcacc gtcatcaccg aaacgcgcga gacgaaaggg cctcgtgata 3480
   cgcctatttt tataggttaa tgtcatgata ataatggttt cttagacgtc aggtggcact 3540
   tttcggggaa atgtgcgcgg aacccctatt tgtttatttt tctaaataca ttcaaatatg 3600
   tatccgctca tgagacaata accctgataa atgcttcaat aatattgaaa aaggaagagt 3660
   atgagtattc aacatttccg tgtcgccctt attccctttt ttgcggcatt ttgccttcct 3720
   gtttttgctc acccagaaac gctggtgaaa gtaaaagatg ctgaagatca gttgggtgca 3780
   cgagtgggtt acatcgaact ggatctcaac agcggtaaga tccttgagag ttttcgcccc 3840
   gaagaacgtt ttccaatgat gagcactttt aaagttctgc tatgtggcgc ggtattatcc 3900
   cgtattgacg ccgggcaaga gcaactcggt cgccgcatac actattctca gaatgacttg 3960
   gttgagtact caccagtcac agaaaagcat cttacggatg gcatgacagt aagagaatta 4020
   tgcagtgctg ccataaccat gagtgataac actgcggcca acttacttct gacaacgatc 4080
   ggaggaccga aggagctaac cgcttttttg cacaacatgg gggatcatgt aactcgcctt 4140
   gatcgttggg aaccggagct gaatgaagcc ataccaaacg acgagcgtga caccacgatg 4200
   cctgtagcaa tggcaacaac gttgcgcaaa ctattaactg gcgaactact tactctagct 4260
   tcccggcaac aattaataga ctggatggag gcggataaag ttgcaggacc acttctgcgc 4320
   tcggcccttc cggctggctg gtttattgct gataaatctg gagccggtga gcgtgggtct 4380
   cgcggtatca ttgcagcact ggggccagat ggtaagccct cccgtatcgt agttatctac 4440
   acgacgggga gtcaggcaac tatggatgaa cgaaatagac agatcgctga gataggtgcc 4500
   tcactgatta agcattggta actgtcagac caagtttact catatatact ttagattgat 4560
   ttaaaacttc atttttaatt taaaaggatc taggtgaaga tcctttttga taatctcatg 4620
   accaaaatcc cttaacgtga gttttcgttc cactgagcgt cagaccccgt agaaaagatc 4680
   aaaggatctt cttgagatcc tttttttctg cgcgtaatct gctgcttgca aacaaaaaaa 4740
   ccaccgctac cagcggtggt ttgtttgccg gatcaagagc taccaactct ttttccgaag 4800
   gtaactggct tcagcagagc gcagatacca aatactgtcc ttctagtgta gccgtagtta 4860
   ggccaccact tcaagaactc tgtagcaccg cctacatacc tcgctctgct aatcctgtta 4920
   ccagtggctg ctgccagtgg cgataagtcg tgtcttaccg ggttggactc aagacgatag 4980
   ttaccggata aggcgcagcg gtcgggctga acggggggtt cgtgcacaca gcccagcttg 5040
   gagcgaacga cctacaccga actgagatac ctacagcgtg agctatgaga aagcgccacg 5100
   cttcccgaag ggagaaaggc ggacaggtat ccggtaagcg gcagggtcgg aacaggagag 5160
   cgcacgaggg agcttccagg gggaaacgcc tggtatcttt atagtcctgt cgggtttcgc 5220
   cacctctgac ttgagcgtcg atttttgtga tgctcgtcag gggggcggag cctatggaaa 5280
   aacgccagca acgcggcctt tttacggttc ctggcctttt gctggccttt tgctcacatg 5340
   ttctttcctg cgttatcccc tgattctgtg gataaccgta ttaccgcctt tgagtgagct 5400
   gataccgctc gccgcagccg aacgaccgag cgcagcgagt cagtgagcga ggaagcggaa 5460
   gagcgcccaa tacgcaaacc gcctctcccc gcgcgttggc cgattcatta atgcagctgg 5520
   cacgacaggt ttcccgactg gaaagcgggc agtgagcgca acgcaattaa tgtgagttag 5580
   ctcactcatt aggcacccca ggctttacac tttatgcttc cggctcgtat gttgtgtgga 5640
   attgtgagcg gataacaatt tcacacagga aacagctatg accatgatta cgccaagcta 5700
   gcccgggcta gcttgcatgc ctgcaggttt                                  5730
<210> 74
   <211> 4886
   <212> DNA
   <213> Artificial Sequence
<220>
   <223> Description of Artificial Sequence: vector pCMV-SSV
<400> 74
   aaacagtccg atgtacgggc cagatatacg cgttgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggac 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catggtgatg 420
   cggttttggc agtacatcaa tgggcgtgga tagcggtttg actcacgggg atttccaagt 480
   ctccacccca ttgacgtcaa tgggagtttg ttttggcacc aaaatcaacg ggactttcca 540
   aaatgtcgta acaactccgc cccattgacg caaatgggcg gtaggcgtgt acggtgggag 600
   gtctatataa gcagagctct ctggctaact agagaaccca ctgcttactg gcttatcgaa 660
   attaatacga ctcactatag ggagacccaa gctgactcta gacttaatta agcgttgggg 720
   tgagtactcc ctctcaaaag cgggcatgac ttctgcgcta agattgtcag tttccaaaaa 780
   cgaggaggat ttgatattca cctggcccgc ggtgatgcct ttgagggtgg ccgcgtccat 840
   ctggtcagaa aagacaatct ttttgttgtc aagcttgagg tgtggcaggc ttgagatctg 900
   gccatacact tgagtgacat tgacatccac tttgcctttc tctccacagg tgtccactcc 960
   cagggcggcc gcccgatatg acgaaagata agacgcgtta taaatacggg gattatattt 1020
   tacgcgagag gaaagggcgg tattatgttt acaagctaga gtatgaaaac ggtgaggtaa 1080
   aagagcgtta cgtgggtcct ttagctgacg tcgttgaatc atatctaaaa atgaaattag 1140
   gggtcgtagg ggatactccc ctacaagcgg atccccccgg tttcgagccc gggacaagcg 1200
   gaagcggtgg tggaaaagag ggaactgaac gacgtaaaat agcgttggtt gccaatttgc 1260
   gccaatacgc gacggacggc aacataaagg cgttctacaa ctatctcatg aacgaaaggg 1320
   ggataagcga aaaaactgca aaggactaca tcaatgctat atcaaagccg tataaagaga 1380
   cgagagacgc acagaaggct taccgactct ttgcacgttt cttagcgtca cgcaatatca 1440
   tacatgatga atttgcggat aaaatattga aagcggtaaa ggtgaagaag gcgaacgctg 1500
   atatctacat tccaacgttg gaagagataa aaaggacgtt acaattagca aaagactata 1560
   gcgaaaacgt ctacttcatc taccgtatcg ctctcgagtc gggcgttagg ctgagcgaaa 1620
   tactgaaagt gctgaaggaa cccgaaaggg acatttgcgg taacgacgtc tgttattatc 1680
   cgcttagttg gactagggga tataagggcg tcttctatgt attccacata acgcctctga 1740
   agagagtaga ggtgacgaag tgggcaatag cggactttga acgacgtcat aaggacgcta 1800
   tagcgataaa gtacttccgc aaattcgtag cgtctaagat ggctgagcta agcgtaccgt 1860
   tagatattat cgattttatt caagggcgta aaccgacacg cgttttaacg caacattacg 1920
   tatcgctctt cggcatagcg aaagagcaat ataaaaagta tgcggaatgg ctaaaagggg 1980
   tctgactcga gggggggccc gtcgacctcg agatccaggc gcggatcaat aaaagatcat 2040
   tattttcaat agatctgtgt gttggttttt tgtgtgcctt gggggagggg gaggccagaa 2100
   tgaggcgcgg ccaaggggga gggggaggcc agaatgacct tgggggaggg ggaggccaga 2160
   atgaccttgg gggaggggga ggccagaatg aggcgcgccc ccgggtaccg agctcgaatt 2220
   cactggccgt cgttttacaa cgtcgtgact gggaaaaccc tggcgttacc caacttaatc 2280
   gccttgcagc acatccccct ttcgccagct ggcgtaatag cgaagaggcc cgcaccgatc 2340
   gcccttccca acagttgcgc agcctgaatg gcgaatggcg cctgatgcgg tattttctcc 2400
   ttacgcatct gtgcggtatt tcacaccgca tatggtgcac tctcagtaca atctgctctg 2460
   atgccgcata gttaagccag ccccgacacc cgccaacacc cgctgacgcg ccctgacggg 2520
   cttgtctgct cccggcatcc gcttacagac aagctgtgac cgtctccggg agctgcatgt 2580
   gtcagaggtt ttcaccgtca tcaccgaaac gcgcgagacg aaagggcctc gtgatacgcc 2640
   tatttttata ggttaatgtc atgataataa tggtttctta gacgtcaggt ggcacttttc 2700
   ggggaaatgt gcgcggaacc cctatttgtt tatttttcta aatacattca aatatgtatc 2760
   cgctcatgag acaataaccc tgataaatgc ttcaataata ttgaaaaagg aagagtatga 2820
   gtattcaaca tttccgtgtc gcccttattc ccttttttgc ggcattttgc cttcctgttt 2880
   ttgctcaccc agaaacgctg gtgaaagtaa aagatgctga agatcagttg ggtgcacgag 2940
   tgggttacat cgaactggat ctcaacagcg gtaagatcct tgagagtttt cgccccgaag 3000
   aacgttttcc aatgatgagc acttttaaag ttctgctatg tggcgcggta ttatcccgta 3060
   ttgacgccgg gcaagagcaa ctcggtcgcc gcatacacta ttctcagaat gacttggttg 3120
   agtactcacc agtcacagaa aagcatctta cggatggcat gacagtaaga gaattatgca 3180
   gtgctgccat aaccatgagt gataacactg cggccaactt acttctgaca acgatcggag 3240
   gaccgaagga gctaaccgct tttttgcaca acatggggga tcatgtaact cgccttgatc 3300
   gttgggaacc ggagctgaat gaagccatac caaacgacga gcgtgacacc acgatgcctg 3360
   tagcaatggc aacaacgttg cgcaaactat taactggcga actacttact ctagcttccc 3420
   ggcaacaatt aatagactgg atggaggcgg ataaagttgc aggaccactt ctgcgctcgg 3480
   cccttccggc tggctggttt attgctgata aatctggagc cggtgagcgt gggtctcgcg 3540
   gtatcattgc agcactgggg ccagatggta agccctcccg tatcgtagtt atctacacga 3600
   cggggagtca ggcaactatg gatgaacgaa atagacagat cgctgagata ggtgcctcac 3660
   tgattaagca ttggtaactg tcagaccaag tttactcata tatactttag attgatttaa 3720
   aacttcattt ttaatttaaa aggatctagg tgaagatcct ttttgataat ctcatgacca 3780
   aaatccctta acgtgagttt tcgttccact gagcgtcaga ccccgtagaa aagatcaaag 3840
   gatcttcttg agatcctttt tttctgcgcg taatctgctg cttgcaaaca aaaaaaccac 3900
   cgctaccagc ggtggtttgt ttgccggatc aagagctacc aactcttttt ccgaaggtaa 3960
   ctggcttcag cagagcgcag ataccaaata ctgtccttct agtgtagccg tagttaggcc 4020
   accacttcaa gaactctgta gcaccgccta catacctcgc tctgctaatc ctgttaccag 4080
   tggctgctgc cagtggcgat aagtcgtgtc ttaccgggtt ggactcaaga cgatagttac 4140
   cggataaggc gcagcggtcg ggctgaacgg ggggttcgtg cacacagccc agcttggagc 4200
   gaacgaccta caccgaactg agatacctac agcgtgagct atgagaaagc gccacgcttc 4260
   ccgaagggag aaaggcggac aggtatccgg taagcggcag ggtcggaaca ggagagcgca 4320
   cgagggagct tccaggggga aacgcctggt atctttatag tcctgtcggg tttcgccacc 4380
   tctgacttga gcgtcgattt ttgtgatgct cgtcaggggg gcggagccta tggaaaaacg 4440
   ccagcaacgc ggccttttta cggttcctgg ccttttgctg gccttttgct cacatgttct 4500
   ttcctgcgtt atcccctgat tctgtggata accgtattac cgcctttgag tgagctgata 4560
   ccgctcgccg cagccgaacg accgagcgca gcgagtcagt gagcgaggaa gcggaagagc 4620
   gcccaatacg caaaccgcct ctccccgcgc gttggccgat tcattaatgc agctggcacg 4680
   acaggtttcc cgactggaaa gcgggcagtg agcgcaacgc aattaatgtg agttagctca 4740
   ctcattaggc accccaggct ttacacttta tgcttccggc tcgtatgttg tgtggaattg 4800
   tgagcggata acaatttcac acaggaaaca gctatgacca tgattacgcc aagctagccc 4860
   gggctagctt gcatgcctgc aggttt                                     4886
<210> 75
   <211> 4905
   <212> DNA
   <213> Artificial Sequence
<220>
   <223> Description of Artificial Sequence: vector pCMV-SSV(NNLS)
<400> 75
   aaacagtccg atgtacgggc cagatatacg cgttgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggac 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catggtgatg 420
   cggttttggc agtacatcaa tgggcgtgga tagcggtttg actcacgggg atttccaagt 480
   ctccacccca ttgacgtcaa tgggagtttg ttttggcacc aaaatcaacg ggactttcca 540
   aaatgtcgta acaactccgc cccattgacg caaatgggcg gtaggcgtgt acggtgggag 600
   gtctatataa gcagagctct ctggctaact agagaaccca ctgcttactg gcttatcgaa 660
   attaatacga ctcactatag ggagacccaa gctgactcta gacttaatta agcgttgggg 720
   tgagtactcc ctctcaaaag cgggcatgac ttctgcgcta agattgtcag tttccaaaaa 780
   cgaggaggat ttgatattca cctggcccgc ggtgatgcct ttgagggtgg ccgcgtccat 840
   ctggtcagaa aagacaatct ttttgttgtc aagcttgagg tgtggcaggc ttgagatctg 900
   gccatacact tgagtgacat tgacatccac tttgcctttc tctccacagg tgtccactcc 960
   cagggcggcc gcaccatgcc caagaagaag aggaaggtga cgaaagataa gacgcgttat 1020
   aaatacgggg attatatttt acgcgagagg aaagggcggt attatgttta caagctagag 1080
   tatgaaaacg gtgaggtaaa agagcgttac gtgggtcctt tagctgacgt cgttgaatca 1140
   tatctaaaaa tgaaattagg ggtcgtaggg gatactcccc tacaagcgga tccccccggt 1200
   ttcgagcccg ggacaagcgg aagcggtggt ggaaaagagg gaactgaacg acgtaaaata 1260
   gcgttggttg ccaatttgcg ccaatacgcg acggacggca acataaaggc gttctacaac 1320
   tatctcatga acgaaagggg gataagcgaa aaaactgcaa aggactacat caatgctata 1380
   tcaaagccgt ataaagagac gagagacgca cagaaggctt accgactctt tgcacgtttc 1440
   ttagcgtcac gcaatatcat acatgatgaa tttgcggata aaatattgaa agcggtaaag 1500
   gtgaagaagg cgaacgctga tatctacatt ccaacgttgg aagagataaa aaggacgtta 1560
   caattagcaa aagactatag cgaaaacgtc tacttcatct accgtatcgc tctcgagtcg 1620
   ggcgttaggc tgagcgaaat actgaaagtg ctgaaggaac ccgaaaggga catttgcggt 1680
   aacgacgtct gttattatcc gcttagttgg actaggggat ataagggcgt cttctatgta 1740
   ttccacataa cgcctctgaa gagagtagag gtgacgaagt gggcaatagc ggactttgaa 1800
   cgacgtcata aggacgctat agcgataaag tacttccgca aattcgtagc gtctaagatg 1860
   gctgagctaa gcgtaccgtt agatattatc gattttattc aagggcgtaa accgacacgc 1920
   gttttaacgc aacattacgt atcgctcttc ggcatagcga aagagcaata taaaaagtat 1980
   gcggaatggc taaaaggggt ctgactcgag ggggggcccg tcgacctcga gatccaggcg 2040
   cggatcaata aaagatcatt attttcaata gatctgtgtg ttggtttttt gtgtgccttg 2100
   ggggaggggg aggccagaat gaggcgcggc caagggggag gggaggccca gaatgacctt 2160
   gggggagggg gaggccagaa tgaccttggg ggagggggag gccagaatga ggcgcgcccc 2220
   cgggtaccga gctcgaattc actggccgtc gttttacaac gtcgtgactg ggaaaaccct 2280
   ggcgttaccc aacttaatcg ccttgcagca catccccctt tcgccagctg gcgtaatagc 2340
   gaagaggccc gcaccgatcg cccttcccaa cagttgcgca gcctgaatgg cgaatggcgc 2400
   ctgatgcggt attttctcct tacgcatctg tgcggtattt cacaccgcat atggtgcact 2460
   ctcagtacaa tctgctctga tgccgcatag ttaagccagc cccgacaccc gccaacaccc 2520
   gctgacgcgc cctgacgggc ttgtctgctc ccggcatccg cttacagaca agctgtgacc 2580
   gtctccggga gctgcatgtg tcagaggttt tcaccgtcat caccgaaacg cgcgagacga 2640
   aagggcctcg tgatacgcct atttttatag gttaatgtca tgataataat ggtttcttag 2700
   acgtcaggtg gcacttttcg gggaaatgtg cgcggaaccc ctatttgttt atttttctaa 2760
   atacattcaa atatgtatcc gctcatgaga caataaccct gataaatgct tcaataatat 2820
   tgaaaaagga agagtatgag tattcaacat ttccgtgtcg cccttattcc cttttttgcg 2880
   gcattttgcc ttcctgtttt tgctcaccca gaaacgctgg tgaaagtaaa agatgctgaa 2940
   gatcagttgg gtgcacgagt gggttacatc gaactggatc tcaacagcgg taagatcctt 3000
   gagagttttc gccccgaaga acgttttcca atgatgagca cttttaaagt tctgctatgt 3060
   ggcgcggtat tatcccgtat tgacgccggg caagagcaac tcggtcgccg catacactat 3120
   tctcagaatg acttggttga gtactcacca gtcacagaaa agcatcttac ggatggcatg 3180
   acagtaagag aattatgcag tgctgccata accatgagtg ataacactgc ggccaactta 3240
   cttctgacaa cgatcggagg accgaaggag ctaaccgctt ttttgcacaa catgggggat 3300
   catgtaactc gccttgatcg ttgggaaccg gagctgaatg aagccatacc aaacgacgag 3360
   cgtgacacca cgatgcctgt agcaatggca acaacgttgc gcaaactatt aactggcgaa 3420
   ctacttactc tagcttcccg gcaacaatta atagactgga tggaggcgga taaagttgca 3480
   ggaccacttc tgcgctcggc ccttccggct ggctggttta ttgctgataa atctggagcc 3540
   ggtgagcgtg ggtctcgcgg tatcattgca gcactggggc cagatggtaa gccctcccgt 3600
   atcgtagtta tctacacgac ggggagtcag gcaactatgg atgaacgaaa tagacagatc 3660
   gctgagatag gtgcctcact gattaagcat tggtaactgt cagaccaagt ttactcatat 3720
   atactttaga ttgatttaaa acttcatttt taatttaaaa ggatctaggt gaagatcctt 3780
   tttgataatc tcatgaccaa aatcccttaa cgtgagtttt cgttccactg agcgtcagac 3840
   cccgtagaaa agatcaaagg atcttcttga gatccttttt ttctgcgcgt aatctgctgc 3900
   ttgcaaacaa aaaaaccacc gctaccagcg gtggtttgtt tgccggatca agagctacca 3960
   actctttttc cgaaggtaac tggcttcagc agagcgcaga taccaaatac tgtccttcta 4020
   gtgtagccgt agttaggcca ccacttcaag aactctgtag caccgcctac atacctcgct 4080
   ctgctaatcc tgttaccagt ggctgctgcc agtggcgata agtcgtgtct taccgggttg 4140
   gactcaagac gatagttacc ggataaggcg cagcggtcgg gctgaacggg gggttcgtgc 4200
   acacagccca gcttggagcg aacgacctac accgaactga gatacctaca gcgtgagcta 4260
   tgagaaagcg ccacgcttcc cgaagggaga aaggcggaca ggtatccggt aagcggcagg 4320
   gtcggaacag gagagcgcac gagggagctt ccagggggaa acgcctggta tctttatagt 4380
   cctgtcgggt ttcgccacct ctgacttgag cgtcgatttt tgtgatgctc gtcagggggg 4440
   cggagcctat ggaaaaacgc cagcaacgcg gcctttttac ggttcctggc cttttgctgg 4500
   ccttttgctc acatgttctt tcctgcgtta tcccctgatt ctgtggataa ccgtattacc 4560
   gcctttgagt gagctgatac cgctcgccgc agccgaacga ccgagcgcag cgagtcagtg 4620
   agcgaggaag cggaagagcg cccaatacgc aaaccgcctc tccccgcgcg ttggccgatt 4680
   cattaatgca gctggcacga caggtttccc gactggaaag cgggcagtga gcgcaacgca 4740
   attaatgtga gttagctcac tcattaggca ccccaggctt tacactttat gcttccggct 4800
   cgtatgttgt gtggaattgt gagcggataa caatttcaca caggaaacag ctatgaccat 4860
   gattacgcca agctagcccg ggctagcttg catgcctgca ggttt                4905
<210> 76
   <211> 5290
   <212> DNA
   <213> Artificial Sequence
<220>
   <223> Description of Artificial Sequence: vector pCMVXisA
<400> 76
   agtccgatgt acgggccaga tatacgcgtt gacattgatt attgactagt tattaatagt 60
   aatcaattac ggggtcatta gttcatagcc catatatgga gttccgcgtt acataactta 120
   cggtaaatgg cccgcctggc tgaccgccca acgacccccg cccattgacg tcaataatga 180
   cgtatgttcc catagtaacg ccaataggga ctttccattg acgtcaatgg gtggactatt 240
   tacggtaaac tgcccacttg gcagtacatc aagtgtatca tatgccaagt acgcccccta 300
   ttgacgtcaa tgacggtaaa tggcccgcct ggcattatgc ccagtacatg accttatggg 360
   actttcctac ttggcagtac atctacgtat tagtcatcgc tattaccatg gtgatgcggt 420
   tttggcagta catcaatggg cgtggatagc ggtttgactc acggggattt ccaagtctcc 480
   accccattga cgtcaatggg agtttgtttt ggcaccaaaa tcaacgggac tttccaaaat 540
   gtcgtaacaa ctccgcccca ttgacgcaaa tgggcggtag gcgtgtacgg tgggaggtct 600
   atataagcag agctctctgg ctaactagag aacccactgc ttactggctt atcgaaatta 660
   atacgactca ctatagggag acccaagctg actctagact taattaagcg ttggggtgag 720
   tactccctct caaaagcggg catgacttct gcgctaagat tgtcagtttc caaaaacgag 780
   gaggatttga tattcacctg gcccgcggtg atgcctttga gggtggccgc gtccatctgg 840
   tcagaaaaga caatcttttt gttgtcaagc ttgaggtgtg gcaggcttga gatctggcca 900
   tacacttgag tgacattgac atccactttg cctttctctc cacaggtgtc cactcccagg 960
   gcggccgccc gatatgcaaa atcagggtca agacaaatat caacaagcct ttgcagactt 1020
   agagccactc tcatctaccg acggcagttt tctcggctca agtctgcaag cacagcagca 1080
   aagagaacac atgagaacaa aagtactaca agacctagac aaggtaaatc tgcgtttgaa 1140
   gtctgcaaag acgaaagtct cagttcgaga atctaacgga agtctgcaat tacgagcaac 1200
   gttaccaatt aaacctggag ataaggacac caacggtaca ggcagaaagc aatacaatct 1260
   cagcttgaat atccctgcaa acttggatgg actgaagacg gctgaggaag aagcttatga 1320
   attaggtaaa ttaatcgctc ggaaaacctt tgaatggaat gataaatatt taggcaaaga 1380
   agccactaaa aaagattcac aaacaatagg tgatttacta gaaaaatttg cagaagagta 1440
   ttttaaaacc cataaacgca ccactaaaag cgaacatacc tttttttact atttttcccg 1500
   cacccaacga tataccaatt ccaaagattt agcaacggcg gaaaatctca tcaattcaat 1560
   tgagcaaatc gataaagaat gggcgagata taatgccgcc agagccatat cagctttttg 1620
   cataacattc aatatagaaa ttgatttgtc ccagtattcc aaaatgcctg atcgcaattc 1680
   gcgcaacatc cccacagatg cagaaatact atcaggaatt accaaatttg aagactatct 1740
   agttaccaga ggaaatcaag ttaatgaaga tgtaaaagat agctggcaac tttggcgctg 1800
   gacatatgga atgttagcag tttttggttt acgccccagg gaaattttta ttaaccctaa 1860
   tattgattgg tggttaagca aagagaatat agacctcaca tggaaagtag acaaagaatg 1920
   taaaactggt gaaagacaag cattaccctt acataaagaa tggattgatg agtttgattt 1980
   aagaaatccg aaatatttag aaatgctggc aacagcaatt agtaaaaaag ataaaacaaa 2040
   tcatgctgaa ataacagcct taactcagcg tattagttgg tggtttcgga aagtcgaatt 2100
   agattttaaa ccctatgatt tacgtcacgc ctgggcaatt agagcgcata ttttaggcat 2160
   accaatcaaa gcggcggctg ataatttggg gcatagtatg caggttcata cacaaaccta 2220
   tcagcgctgg ttctcgctag atatgcggaa gttagcgatt aatcaggctt tgactaagag 2280
   gaatgaattt gaggtgatta gggaggagaa tgctaaattg cagatagaaa atgaaaggtt 2340
   gaggatggaa attgagaagt taaagatgga aatagcttat aagaatagtt gagcggccgc 2400
   gtcgacctcg agatccaggc gcggatcaat aaaagatcat tattttcaat agatctgtgt 2460
   gttggttttt tgtgtgcctt gggggagggg gaggccagaa tgaggcgcgg ccaaggggga 2520
   gggggaggcc agaatgacct tgggggaggg ggaggccaga atgaccttgg gggaggggga 2580
   ggccagaatg aggcgcgccc ccgggtaccg agctcgaatt cactggccgt cgttttacaa 2640
   cgtcgtgact gggaaaaccc tggcgttacc caacttaatc gccttgcagc acatccccct 2700
   ttcgccagct ggcgtaatag cgaagaggcc cgcaccgatc gcccttccca acagttgcgc 2760
   agcctgaatg gcgaatggcg cctgatgcgg tattttctcc ttacgcatct gtgcggtatt 2820
   tcacaccgca tatggtgcac tctcagtaca atctgctctg atgccgcata gttaagccag 2880
   ccccgacacc cgccaacacc cgctgacgcg ccctgacggg cttgtctgct cccggcatcc 2940
   gcttacagac aagctgtgac cgtctccggg agctgcatgt gtcagaggtt ttcaccgtca 3000
   tcaccgaaac gcgcgagacg aaagggcctc gtgatacgcc tatttttata ggttaatgtc 3060
   atgataataa tggtttctta gacgtcaggt ggcacttttc ggggaaatgt gcgcggaacc 3120
   cctatttgtt tatttttcta aatacattca aatatgtatc cgctcatgag acaataaccc 3180
   tgataaatgc ttcaataata ttgaaaaagg aagagtatga gtattcaaca tttccgtgtc 3240
   gcccttattc ccttttttgc ggcattttgc cttcctgttt ttgctcaccc agaaacgctg 3300
   gtgaaagtaa aagatgctga agatcagttg ggtgcacgag tgggttacat cgaactggat 3360
   ctcaacagcg gtaagatcct tgagagtttt cgccccgaag aacgttttcc aatgatgagc 3420
   acttttaaag ttctgctatg tggcgcggta ttatcccgta ttgacgccgg gcaagagcaa 3480
   ctcggtcgcc gcatacacta ttctcagaat gacttggttg agtactcacc agtcacagaa 3540
   aagcatctta cggatggcat gacagtaaga gaattatgca gtgctgccat aaccatgagt 3600
   gataacactg cggccaactt acttctgaca acgatcggag gaccgaagga gctaaccgct 3660
   tttttgcaca acatggggga tcatgtaact cgccttgatc gttgggaacc ggagctgaat 3720
   gaagccatac caaacgacga gcgtgacacc acgatgcctg tagcaatggc aacaacgttg 3780
   cgcaaactat taactggcga actacttact ctagcttccc ggcaacaatt aatagactgg 3840
   atggaggcgg ataaagttgc aggaccactt ctgcgctcgg cccttccggc tggctggttt 3900
   attgctgata aatctggagc cggtgagcgt gggtctcgcg gtatcattgc agcactgggg 3960
   ccagatggta agccctcccg tatcgtagtt atctacacga cggggagtca ggcaactatg 4020
   gatgaacgaa atagacagat cgctgagata ggtgcctcac tgattaagca ttggtaactg 4080
   tcagaccaag tttactcata tatactttag attgatttaa aacttcattt ttaatttaaa 4140
   aggatctagg tgaagatcct ttttgataat ctcatgacca aaatccctta acgtgagttt 4200
   tcgttccact gagcgtcaga ccccgtagaa aagatcaaag gatcttcttg agatcctttt 4260
   tttctgcgcg taatctgctg cttgcaaaca aaaaaaccac cgctaccagc ggtggtttgt 4320
   ttgccggatc aagagctacc aactcttttt ccgaaggtaa ctggcttcag cagagcgcag 4380
   ataccaaata ctgtccttct agtgtagccg tagttaggcc accacttcaa gaactctgta 4440
   gcaccgccta catacctcgc tctgctaatc ctgttaccag tggctgctgc cagtggcgat 4500
   aagtcgtgtc ttaccgggtt ggactcaaga cgatagttac cggataaggc gcagcggtcg 4560
   ggctgaacgg ggggttcgtg cacacagccc agcttggagc gaacgaccta caccgaactg 4620
   agatacctac agcgtgagct atgagaaagc gccacgcttc ccgaagggag aaaggcggac 4680
   aggtatccgg taagcggcag ggtcggaaca ggagagcgca cgagggagct tccaggggga 4740
   aacgcctggt atctttatag tcctgtcggg tttcgccacc tctgacttga gcgtcgattt 4800
   ttgtgatgct cgtcaggggg gcggagccta tggaaaaacg ccagcaacgc ggccttttta 4860
   cggttcctgg ccttttgctg gccttttgct cacatgttct ttcctgcgtt atcccctgat 4920
   tctgtggata accgtattac cgcctttgag tgagctgata ccgctcgccg cagccgaacg 4980
   accgagcgca gcgagtcagt gagcgaggaa gcggaagagc gcccaatacg caaaccgcct 5040
   ctccccgcgc gttggccgat tcattaatgc agctggcacg acaggtttcc cgactggaaa 5100
   gcgggcagtg agcgcaacgc aattaatgtg agttagctca ctcattaggc accccaggct 5160
   ttacacttta tgcttccggc tcgtatgttg tgtggaattg tgagcggata acaatttcac 5220
   acaggaaaca gctatgacca tgattacgcc aagctagccc gggctagctt gcatgcctgc 5280
   aggtttaaac 5290
<210> 77
   <211> 5309
   <212> DNA
   <213> Artificial Sequence
<220>
   <223> Description of Artificial Sequence: vector pCMVXisANNLS
<400> 77
   aaacagtccg atgtacgggc cagatatacg cgttgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggac 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catggtgatg 420
   cggttttggc agtacatcaa tgggcgtgga tagcggtttg actcacgggg atttccaagt 480
   ctccacccca ttgacgtcaa tgggagtttg ttttggcacc aaaatcaacg ggactttcca 540
   aaatgtcgta acaactccgc cccattgacg caaatgggcg gtaggcgtgt acggtgggag 600
   gtctatataa gcagagctct ctggctaact agagaaccca ctgcttactg gcttatcgaa 660
   attaatacga ctcactatag ggagacccaa gctgactcta gacttaatta agcgttgggg 720
   tgagtactcc ctctcaaaag cgggcatgac ttctgcgcta agattgtcag tttccaaaaa 780
   cgaggaggat ttgatattca cctggcccgc ggtgatgcct ttgagggtgg ccgcgtccat 840
   ctggtcagaa aagacaatct ttttgttgtc aagcttgagg tgtggcaggc ttgagatctg 900
   gccatacact tgagtgacat tgacatccac tttgcctttc tctccacagg tgtccactcc 960
   cagggcggcc gcaccatgcc caagaagaag aggaaggtgc aaaatcaggg tcaagacaaa 1020
   tatcaacaag cctttgcaga cttagagcca ctttcatcta ccgacggcag ttttctcggc 1080
   tcaagtctgc aagcacagca gcaaagagaa cacatgagaa caaaagtact acaagaccta 1140
   gacaaggtaa atctgcgttt gaagtctgca aagacgaaag tctcagttcg agaatctaac 1200
   ggaagtctgc aattacgagc aacgttacca attaaacctg gagataagga caccaacggt 1260
   acaggcagaa agcaatacaa tctcagcttg aatatccctg caaacttgga tggactgaag 1320
   acggctgagg aagaagctta tgaattaggt aaattaatcg ctcggaaaac ctttgaatgg 1380
   aatgataaat atttaggcaa agaagccact aaaaaagatt cacaaacaat aggtgattta 1440
   ctagaaaaat ttgcagaaga gtattttaaa acccataaac gcaccactaa aagcgaacat 1500
   accttttttt actatttttc ccgcacccaa cgatatacca attccaaaga tttagcaacg 1560
   gcggaaaatc tcatcaattc aattgagcaa atcgataaag aatgggcgag atataatgcc 1620
   gccagagcca tatcagcttt ttgcataaca ttcaatatag aaattgattt gtcccagtat 1680
   tccaaaatgc ctgatcgcaa ttcgcgcaac atccccacag atgcagaaat actatcagga 1740
   attaccaaat ttgaagacta tctagttacc agaggaaatc aagttaatga agatgtaaaa 1800
   gatagctggc aactttggcg ctggacatat ggaatgttag cagtttttgg tttacgcccc 1860
   agggaaattt ttattaaccc taatattgat tggtggttaa gcaaagagaa tatagacctc 1920
   acatggaaag tagacaaaga atgtaaaact ggtgaaagac aagcattacc cttacataaa 1980
   gaatggattg atgagtttga tttaagaaat ccgaaatatt tagaaatgct ggcaacagca 2040
   attagtaaaa aagataaaac aaatcatgct gaaataacag ccttaactca gcgtattagt 2100
   tggtggtttc ggaaagtcga attagatttt aaaccctatg atttacgtca cgcctgggca 2160
   atcagagcgc atattttagg cataccaatc aaagcggcgg ctgataattt ggggcatagt 2220
   atgcaggttc atacacaaac ctatcagcgc tggttctcgc tagatatgcg gaagttagcg 2280
   attaatcagg ctttgactaa gaggaatgaa tttgaggtga ttagggagga gaatgctaaa 2340
   ttgcagatag aaaatgaaag gttgaggatg gaaattgaga agttaaagat ggaaatagct 2400
   tataagaata gttgagcggc cgcgtcgacc tcgagatcca ggcgcggatc aataaaagat 2460
   cattattttc aatagatctg tgtgttggtt ttttgtgtgc cttgggggag ggggaggcca 2520
   gaatgaggcg cggccaaggg ggagggggag gccagaatga ccttggggga gggggaggcc 2580
   agaatgacct tgggggaggg ggaggccaga atgaggcgcg cccccgggta ccgagctcga 2640
   attcactggc cgtcgtttta caacgtcgtg actgggaaaa ccctggcgtt acccaactta 2700
   atcgccttgc agcacatccc cctttcgcca gctggcgtaa tagcgaagag gcccgcaccg 2760
   atcgcccttc ccaacagttg cgcagcctga atggcgaatg gcgcctgatg cggtattttc 2820
   tccttacgca tctgtgcggt atttcacacc gcatatggtg cactctcagt acaatctgct 2880
   ctgatgccgc atagttaagc cagccccgac acccgccaac acccgctgac gcgccctgac 2940
   gggcttgtct gctcccggca tccgcttaca gacaagctgt gaccgtctcc gggagctgca 3000
   tgtgtcagag gttttcaccg tcatcaccga aacgcgcgag acgaaagggc ctcgtgatac 3060
   gcctattttt ataggttaat gtcatgataa taatggtttc ttagacgtca ggtggcactt 3120
   ttcggggaaa tgtgcgcgga acccctattt gtttattttt ctaaatacat tcaaatatgt 3180
   atccgctcat gagacaataa ccctgataaa tgcttcaata atattgaaaa aggaagagta 3240
   tgagtattca acatttccgt gtcgccctta ttcccttttt tgcggcattt tgccttcctg 3300
   tttttgctca cccagaaacg ctggtgaaag taaaagatgc tgaagatcag ttgggtgcac 3360
   gagtgggtta catcgaactg gatctcaaca gcggtaagat ccttgagagt tttcgccccg 3420
   aagaacgttt tccaatgatg agcactttta aagttctgct atgtggcgcg gtattatccc 3480
   gtattgacgc cgggcaagag caactcggtc gccgcataca ctattctcag aatgacttgg 3540
   ttgagtactc accagtcaca gaaaagcatc ttacggatgg catgacagta agagaattat 3600
   gcagtgctgc cataaccatg agtgataaca ctgcggccaa cttacttctg acaacgatcg 3660
   gaggaccgaa ggagctaacc gcttttttgc acaacatggg ggatcatgta actcgccttg 3720
   atcgttggga accggagctg aatgaagcca taccaaacga cgagcgtgac accacgatgc 3780
   ctgtagcaat ggcaacaacg ttgcgcaaac tattaactgg cgaactactt actctagctt 3840
   cccggcaaca attaatagac tggatggagg cggataaagt tgcaggacca cttctgcgct 3900
   cggcccttcc ggctggctgg tttattgctg ataaatctgg agccggtgag cgtgggtctc 3960
   gcggtatcat tgcagcactg gggccagatg gtaagccctc ccgtatcgta gttatctaca 4020
   cgacggggag tcaggcaact atggatgaac gaaatagaca gatcgctgag ataggtgcct 4080
   cactgattaa gcattggtaa ctgtcagacc aagtttactc atatatactt tagattgatt 4140
   taaaacttca tttttaattt aaaaggatct aggtgaagat cctttttgat aatctcatga 4200
   ccaaaatccc ttaacgtgag ttttcgttcc actgagcgtc agaccccgta gaaaagatca 4260
   aaggatcttc ttgagatcct ttttttctgc gcgtaatctg ctgcttgcaa acaaaaaaac 4320
   caccgctacc agcggtggtt tgtttgccgg atcaagagct accaactctt tttccgaagg 4380
   taactggctt cagcagagcg cagataccaa atactgtcct tctagtgtag ccgtagttag 4440
   gccaccactt caagaactct gtagcaccgc ctacatacct cgctctgcta atcctgttac 4500
   cagtggctgc tgccagtggc gataagtcgt gtcttaccgg gttggactca agacgatagt 4560
   taccggataa ggcgcagcgg tcgggctgaa cggggggttc gtgcacacag cccagcttgg 4620
   agcgaacgac ctacaccgaa ctgagatacc tacagcgtga gctatgagaa agcgccacgc 4680
   ttcccgaagg gagaaaggcg gacaggtatc cggtaagcgg cagggtcgga acaggagagc 4740
   gcacgaggga gcttccaggg ggaaacgcct ggtatcttta tagtcctgtc gggtttcgcc 4800
   acctctgact tgagcgtcga tttttgtgat gctcgtcagg ggggcggagc ctatggaaaa 4860
   acgccagcaa cgcggccttt ttacggttcc tggccttttg ctggcctttt gctcacatgt 4920
   tctttcctgc gttatcccct gattctgtgg ataaccgtat taccgccttt gagtgagctg 4980
   ataccgctcg ccgcagccga acgaccgagc gcagcgagtc agtgagcgag gaagcggaag 5040
   agcgcccaat acgcaaaccg cctctccccg cgcgttggcc gattcattaa tgcagctggc 5100
   acgacaggtt tcccgactgg aaagcgggca gtgagcgcaa cgcaattaat gtgagttagc 5160
   tcactcatta ggcaccccag gctttacact ttatgcttcc ggctcgtatg ttgtgtggaa 5220
   ttgtgagcgg ataacaattt cacacaggaa acagctatga ccatgattac gccaagctag 5280
   cccgggctag cttgcatgcc tgcaggttt 5309
<210> 78
   <211> 7608
   <212> DNA
   <213> Artificial Sequence
<220>
   <223> Description of Artificial Sequence: vector pPGKnifD
<400> 78
   tcgaggaatt ctaccgggta ggggaggcgc ttttcccaag gcagtctgga gcatgcgctt 60
   tagcagcccc gctgggcact tggcgctaca caagtggcct ctggctcgca cacattccac 120
   atccaccggt aggcgccaac cggctccgtt ctttggtggc cccttcgcgc caccttctac 180
   tcctccccta gtcaggaagt tcccccccgc cccgcagctc gcgtcgtgca ggacgtgaca 240
   aatggaagta gcacgtctca ctagtctcgt gcagatggac agcaccgctg agcaatggaa 300
   gcgggtaggc ctttggggca gcggccaata gcagctttgc tccttcgctt tctgggctca 360
   gaggctggga aggggtgggt ccgggggcgg gctcaggggc gggctcaggg gcggggcggg 420
   cgcccgaagg tcctccggag gcccggcatt ctgcacgctt caaaagcgca cgtctgccgc 480
   gctgttctcc tcttcctcat ctccgggcct ttcgacctgc agcccggtac agttcgaatg 540
   gctcttccct tccgtcaaat gcactcttgg gattactccg aacctagcga tggggtgcaa 600
   atgtcagatc agataagttc gaataacttc gtatagcata cattatacga agttataagc 660
   ttgcatgcct gcaggtcggc cgccacgacc ggccggccgg tgccgccacc atcccctgac 720
   ccacgcccct gacccctcac aaggagacga ccttccatga ccgagtacaa gcccacggtg 780
   cgcctcgcca cccgcgacga cgtcccccgg gccgtacgca ccctcgccgc cgcgttcgcc 840
   gactaccccg ccacgcgcca caccgtcgac ccggaccgcc acatcgagcg ggtcaccgag 900
   ctgcaagaac tcttcctcac gcgcgtcggg ctcgacatcg gcaaggtgtg ggtcgcggac 960
   gacggcgccg cggtggcggt ctggaccacg ccggagagcg tcgaagcggg ggcggtgttc 1020
   gccgagatcg gcccgcgcat ggccgagttg agcggttccc ggctggccgc gcagcaacag 1080
   atggaaggcc tcctggcgcc gcaccggccc aaggagcccg cgtggttcct ggccaccgtc 1140
   ggcgtctcgc ccgaccacca gggcaagggt ctgggcagcg ccgtcgtgct ccccggagtg 1200
   gaggcggccg agcgcgccgg ggtgcccgcc ttcctggaga cctccgcgcc ccgcaacctc 1260
   cccttctacg agcggctcgg cttcaccgtc accgccgacg tcgagtgccc gaaggaccgc 1320
   gcgacctggt gcatgacccg caagcccggt gcctgacgcc cgccccacga cccgcagcgc 1380
   ccgaccgaaa ggagcgcacg accccatggc tccgaccgaa gccgacccgg gcggccccgc 1440
   cgaccccgca cccgcccccg aggcccaccg actctagagg atcataatca gccataccac 1500
   atttgtagag gttttacttg ctttaaaaaa cctcccacac ctccccctga acctgaaaca 1560
   taaaatgaat gcaattgttg ttgttaactt gtttattgca gcttataatg gttacaaata 1620
   aagcaatagc atcacaaatt tcacaaataa agcatttttt tcactgcatt ctagttgtgg 1680
   tttgtccaaa ctcatcaatg tatcttatca tgtctggatc cagctgttga aagctattaa 1740
   accacaaaaa ggattactcc ggcccttatc acggttacga cggatttgga tccataactt 1800
   cgtatagcat acattatacg aagttatacc gggccaccat ggtcgcgagt agcttggcac 1860
   tggccgtcgt tttacaacgt cgtgactggg aaaaccctgg cgttacccaa cttaatcgcc 1920
   ttgcagcaca tccccctttc gccagctggc gtaatagcga agaggcccgc accgatcgcc 1980
   cttcccaaca gttgcgcagc ctgaatggcg aatggcgctt tgcctggttt ccggcaccag 2040
   aagcggtgcc ggaaagctgg ctggagtgcg atcttcctga ggccgatact gtcgtcgtcc 2100
   cctcaaactg gcagatgcac ggttacgatg cgcccatcta caccaacgta acctatccca 2160
   ttacggtcaa tccgccgttt gttcccacgg agaatccgac gggttgttac tcgctcacat 2220
   ttaatgttga tgaaagctgg ctacaggaag gccagacgcg aattattttt gatggcgtta 2280
   actcggcgtt tcatctgtgg tgcaacgggc gctgggtcgg ttacggccag gacagtcgtt 2340
   tgccgtctga atttgacctg agcgcatttt tacgcgccgg agaaaaccgc ctcgcggtga 2400
   tggtgctgcg ttggagtgac ggcagttatc tggaagatca ggatatgtgg cggatgagcg 2460
   gcattttccg tgacgtctcg ttgctgcata aaccgactac acaaatcagc gatttccatg 2520
   ttgccactcg ctttaatgat gatttcagcc gcgctgtact ggaggctgaa gttcagatgt 2580
   gcggcgagtt gcgtgactac ctacgggtaa cagtttcttt atggcagggt gaaacgcagg 2640
   tcgccagcgg caccgcgcct ttcggcggtg aaattatcga tgagcgtggt ggttatgccg 2700
   atcgcgtcac actacgtctg aacgtcgaaa acccgaaact gtggagcgcc gaaatcccga 2760
   atctctatcg tgcggtggtt gaactgcaca ccgccgacgg cacgctgatt gaagcagaag 2820
   cctgcgatgt cggtttccgc gaggtgcgga ttgaaaatgg tctgctgctg ctgaacggca 2880
   agccgttgct gattcgaggc gttaaccgtc acgagcatca tcctctgcat ggtcaggtca 2940
   tggatgagca gacgatggtg caggatatcc tgctgatgaa gcagaacaac tttaacgccg 3000
   tgcgctgttc gcattatccg aaccatccgc tgtggtacac gctgtgcgac cgctacggcc 3060
   tgtatgtggt ggatgaagcc aatattgaaa cccacggcat ggtgccaatg aatcgtctga 3120
   ccgatgatcc gcgctggcta ccggcgatga gcgaacgcgt aacgcgaatg gtgcagcgcg 3180
   atcgtaatca cccgagtgtg atcatctggt cgctggggaa tgaatcaggc cacggcgcta 3240
   atcacgacgc gctgtatcgc tggatcaaat ctgtcgatcc ttcccgcccg gtgcagtatg 3300
   aaggcggcgg agccgacacc acggccaccg atattatttg cccgatgtac gcgcgcgtgg 3360
   atgaagacca gcccttcccg gctgtgccga aatggtccat caaaaaatgg ctttcgctac 3420
   ctggagagac gcgcccgctg atcctttgcg aatacgccca cgcgatgggt aacagtcttg 3480
   gcggtttcgc taaatactgg caggcgtttc gtcagtatcc ccgtttacag ggcggcttcg 3540
   tctgggactg ggtggatcag tcgctgatta aatatgatga aaacggcaac ccgtggtcgg 3600
   cttacggcgg tgattttggc gatacgccga acgatcgcca gttctgtatg aacggtctgg 3660
   tctttgccga ccgcacgccg catccagcgc tgacggaagc aaaacaccag cagcagtttt 3720
   tccagttccg tttatccggg caaaccatcg aagtgaccag cgaatacctg ttccgtcata 3780
   gcgataacga gctcctgcac tggatggtgg cgctggatgg taagccgctg gcaagcggtg 3840
   aagtgcctct ggatgtcgct ccacaaggta aacagttgat tgaactgcct gaactaccgc 3900
   agccggagag cgccgggcaa ctctggctca cagtacgcgt agtgcaaccg aacgcgaccg 3960
   catggtcaga agccgggcac atcagcgcct ggcagcagtg gcgtctggcg gaaaacctca 4020
   gtgtgacgct ccccgccgcg tcccacgcca tcccgcatct gaccaccagc gaaatggatt 4080
   tttgcatcga gctgggtaat aagcgttggc aatttaaccg ccagtcaggc tttctttcac 4140
   agatgtggat tggcgataaa aaacaactgc tgacgccgct gcgcgatcag ttcacccgtg 4200
   caccgctgga taacgacatt ggcgtaagtg aagcgacccg cattgaccct aacgcctggg 4260
   tcgaacgctg gaaggcggcg ggccattacc aggccgaagc agcgttgttg cagtgcacgg 4320
   cagatacact tgctgatgcg gtgctgatta cgaccgctca cgcgtggcag catcagggga 4380
   aaaccttatt tatcagccgg aaaacctacc ggattgatgg tagtggtcaa atggcgatta 4440
   ccgttgatgt tgaagtggcg agcgatacac cgcatccggc gcggattggc ctgaactgcc 4500
   agctggcgca ggtagcagag cgggtaaact ggctcggatt agggccgcaa gaaaactatc 4560
   ccgaccgcct tactgccgcc tgttttgacc gctgggatct gccattgtca gacatgtata 4620
   ccccgtacgt cttcccgagc gaaaacggtc tgcgctgcgg gacgcgcgaa ttgaattatg 4680
   gcccacacca gtggcgcggc gacttccagt tcaacatcag ccgctacagt caacagcaac 4740
   tgatggaaac cagccatcgc catctgctgc acgcggaaga aggcacatgg ctgaatatcg 4800
   acggtttcca tatggggatt ggtggcgacg actcctggag cccgtcagta tcggcggaat 4860
   tccagctgag cgccggtcgc taccattacc agttggtctg gtgtcaaaaa taataataac 4920
   cgggcagggg ggatctttgt gaaggaacct tacttctgtg gtgtgacata attggacaaa 4980
   ctacctacag agatttaaag ctctaaggta aatataaaat ttttaagtgt ataatgtgtt 5040
   aaactactga ttctaattgt ttgtgtattt tagattccaa cctatggaac tgatgaatgg 5100
   gagcagtggt ggaatgccag atccagacat gataagatac attgatgagt ttggacaaac 5160
   cacaactaga atgcagtgaa aaaaatgctt tatttgtgaa atttgtgatg ctattgcttt 5220
   atttgtaacc attataagct gcaataaaca agttaacaac aacaattgca ttcattttat 5280
   gtttcaggtt cagggggagg tgtgggaggt tttttaaagc aagtaaaacc tctacaaatg 5340
   tggtatggct gattatgatc tgcggccgca gggcctcgtg atacgcctat ttttataggt 5400
   taatgtcatg ataataatgg tttcttagac gtcaggtggc acttttcggg gaaatgtgcg 5460
   cggaacccct atttgtttat ttttctaaat acattcaaat atgtatccgc tcatgagaca 5520
   ataaccctga taaatgcttc aataatattg aaaaaggaag agtatgagta ttcaacattt 5580
   ccgtgtcgcc cttattccct tttttgcggc attttgcctt cctgtttttg ctcacccaga 5640
   aacgctggtg aaagtaaaag atgctgaaga tcagttgggt gcacgagtgg gttacatcga 5700
   actggatctc aacagcggta agatccttga gagttttcgc cccgaagaac gttttccaat 5760
   gatgagcact tttaaagttc tgctatgtgg cgcggtatta tcccgtattg acgccgggca 5820
   agagcaactc ggtcgccgca tacactattc tcagaatgac ttggttgagt actcaccagt 5880
   cacagaaaag catcttacgg atggcatgac agtaagagaa ttatgcagtg ctgccataac 5940
   catgagtgat aacactgcgg ccaacttact tctgacaacg atcggaggac cgaaggagct 6000
   aaccgctttt ttgcacaaca tgggggatca tgtaactcgc cttgatcgtt gggaaccgga 6060
   gctgaatgaa gccataccaa acgacgagcg tgacaccacg atgcctgtag caatggcaac 6120
   aacgttgcgc aaactattaa ctggcgaact acttactcta gcttcccggc aacaattaat 6180
   agactggatg gaggcggata aagttgcagg accacttctg cgctcggccc ttccggctgg 6240
   ctggtttatt gctgataaat ctggagccgg tgagcgtggg tctcgcggta tcattgcagc 6300
   actggggcca gatggtaagc cctcccgtat cgtagttatc tacacgacgg ggagtcaggc 6360
   aactatggat gaacgaaata gacagatcgc tgagataggt gcctcactga ttaagcattg 6420
   gtaactgtca gaccaagttt actcatatat actttagatt gatttaaaac ttcattttta 6480
   atttaaaagg atctaggtga agatcctttt tgataatctc atgaccaaaa tcccttaacg 6540
   tgagttttcg ttccactgag cgtcagaccc cgtagaaaag atcaaaggat cttcttgaga 6600
   tccttttttt ctgcgcgtaa tctgctgctt gcaaacaaaa aaaccaccgc taccagcggt 6660
   ggtttgtttg ccggatcaag agctaccaac tctttttccg aaggtaactg gcttcagcag 6720
   agcgcagata ccaaatactg tccttctagt gtagccgtag ttaggccacc acttcaagaa 6780
   ctctgtagca ccgcctacat acctcgctct gctaatcctg ttaccagtgg ctgctgccag 6840
   tggcgataag tcgtgtctta ccgggttgga ctcaagacga tagttaccgg ataaggcgca 6900
   gcggtcgggc tgaacggggg gttcgtgcac acagcccagc ttggagcgaa cgacctacac 6960
   cgaactgaga tacctacagc gtgagctatg agaaagcgcc acgcttcccg aagggagaaa 7020
   ggcggacagg tatccggtaa gcggcagggt cggaacagga gagcgcacga gggagcttcc 7080
   agggggaaac gcctggtatc tttatagtcc tgtcgggttt cgccacctct gacttgagcg 7140
   tcgatttttg tgatgctcgt caggggggcg gagcctatgg aaaaacgcca gcaacgcggc 7200
   ctttttacgg ttcctggcct tttgctggcc ttttgctcac atgttctttc ctgcgttatc 7260
   ccctgattct gtggataacc gtattaccgc ctttgagtga gctgataccg ctcgccgcag 7320
   ccgaacgacc gagcgcagcg agtcagtgag cgaggaagcg gaagagcgcc caatacgcaa 7380
   accgcctctc cccgcgcgtt ggccgattca ttaatgcagc tggcacgaca ggtttcccga 7440
   ctggaaagcg ggcagtgagc gcaacgcaat taatgtgagt tagctcactc attaggcacc 7500
   ccaggcttta cactttatgc ttccggctcg tatgttgtgt ggaattgtga gcggataaca 7560
   atttcacaca ggaaacagct atgaccatga ttacgccaag ctggcgcg 7608
<210> 79
   <211> 7523
   <212> DNA
   <213> Artificial Sequence
<220>
   <223> Description of Artificial Sequence: vector pPGKnifD3'
<400> 79
   tcgaggaatt ctaccgggta ggggaggcgc ttttcccaag gcagtctgga gcatgcgctt 60
   tagcagcccc gctgggcact tggcgctaca caagtggcct ctggctcgca cacattccac 120
   atccaccggt aggcgccaac cggctccgtt ctttggtggc cccttcgcgc caccttctac 180
   tcctccccta gtcaggaagt tcccccccgc cccgcagctc gcgtcgtgca ggacgtgaca 240
   aatggaagta gcacgtctca ctagtctcgt gcagatggac agcaccgctg agcaatggaa 300
   gcgggtaggc ctttggggca gcggccaata gcagctttgc tccttcgctt tctgggctca 360
   gaggctggga aggggtgggt ccgggggcgg gctcaggggc gggctcaggg gcggggcggg 420
   cgcccgaagg tcctccggag gcccggcatt ctgcacgctt caaaagcgca cgtctgccgc 480
   gctgttctcc tcttcctcat ctccgggcct ttcgacctgc agcccggtac agttcgaata 540
   acttcgtata gcatacatta tacgaagtta taagcttgca tgcctgcagg tcggccgcca 600
   cgaccggccg gccggtgccg ccaccatccc ctgacccacg cccctgaccc ctcacaagga 660
   gacgaccttc catgaccgag tacaagccca cggtgcgcct cgccacccgc gacgacgtcc 720
   cccgggccgt acgcaccctc gccgccgcgt tcgccgacta ccccgccacg cgccacaccg 780
   tcgacccgga ccgccacatc gagcgggtca ccgagctgca agaactcttc ctcacgcgcg 840
   tcgggctcga catcggcaag gtgtgggtcg cggacgacgg cgccgcggtg gcggtctgga 900
   ccacgccgga gagcgtcgaa gcgggggcgg tgttcgccga gatcggcccg cgcatggccg 960
   agttgagcgg ttcccggctg gccgcgcagc aacagatgga aggcctcctg gcgccgcacc 1020
   ggcccaagga gcccgcgtgg ttcctggcca ccgtcggcgt ctcgcccgac caccagggca 1080
   agggtctggg cagcgccgtc gtgctccccg gagtggaggc ggccgagcgc gccggggtgc 1140
   ccgccttcct ggagacctcc gcgccccgca acctcccctt ctacgagcgg ctcggcttca 1200
   ccgtcaccgc cgacgtcgag tgcccgaagg accgcgcgac ctggtgcatg acccgcaagc 1260
   ccggtgcctg acgcccgccc cacgacccgc agcgcccgac cgaaaggagc gcacgacccc 1320
   atggctccga ccgaagccga cccgggcggc cccgccgacc ccgcacccgc ccccgaggcc 1380
   caccgactct agaggatcat aatcagccat accacatttg tagaggtttt acttgcttta 1440
   aaaaacctcc cacacctccc cctgaacctg aaacataaaa tgaatgcaat tgttgttgtt 1500
   aacttgttta ttgcagctta taatggttac aaataaagca atagcatcac aaatttcaca 1560
   aataaagcat ttttttcact gcattctagt tgtggtttgt ccaaactcat caatgtatct 1620
   tatcatgtct ggatccagct gttgaaagct attaaaccac aaaaaggatt actccggccc 1680
   ttatcacggt tacgacggat ttggatccat aacttcgtat agcatacatt atacgaagtt 1740
   ataccgggcc accatggtcg cgagtagctt ggcactggcc gtcgttttac aacgtcgtga 1800
   ctgggaaaac cctggcgtta cccaacttaa tcgccttgca gcacatcccc ctttcgccag 1860
   ctggcgtaat agcgaagagg cccgcaccga tcgcccttcc caacagttgc gcagcctgaa 1920
   tggcgaatgg cgctttgcct ggtttccggc accagaagcg gtgccggaaa gctggctgga 1980
   gtgcgatctt cctgaggccg atactgtcgt cgtcccctca aactggcaga tgcacggtta 2040
   cgatgcgccc atctacacca acgtaaccta tcccattacg gtcaatccgc cgtttgttcc 2100
   cacggagaat ccgacgggtt gttactcgct cacatttaat gttgatgaaa gctggctaca 2160
   ggaaggccag acgcgaatta tttttgatgg cgttaactcg gcgtttcatc tgtggtgcaa 2220
   cgggcgctgg gtcggttacg gccaggacag tcgtttgccg tctgaatttg acctgagcgc 2280
   atttttacgc gccggagaaa accgcctcgc ggtgatggtg ctgcgttgga gtgacggcag 2340
   ttatctggaa gatcaggata tgtggcggat gagcggcatt ttccgtgacg tctcgttgct 2400
   gcataaaccg actacacaaa tcagcgattt ccatgttgcc actcgcttta atgatgattt 2460
   cagccgcgct gtactggagg ctgaagttca gatgtgcggc gagttgcgtg actacctacg 2520
   ggtaacagtt tctttatggc agggtgaaac gcaggtcgcc agcggcaccg cgcctttcgg 2580
   cggtgaaatt atcgatgagc gtggtggtta tgccgatcgc gtcacactac gtctgaacgt 2640
   cgaaaacccg aaactgtgga gcgccgaaat cccgaatctc tatcgtgcgg tggttgaact 2700
   gcacaccgcc gacggcacgc tgattgaagc agaagcctgc gatgtcggtt tccgcgaggt 2760
   gcggattgaa aatggtctgc tgctgctgaa cggcaagccg ttgctgattc gaggcgttaa 2820
   ccgtcacgag catcatcctc tgcatggtca ggtcatggat gagcagacga tggtgcagga 2880
   tatcctgctg atgaagcaga acaactttaa cgccgtgcgc tgttcgcatt atccgaacca 2940
   tccgctgtgg tacacgctgt gcgaccgcta cggcctgtat gtggtggatg aagccaatat 3000
   tgaaacccac ggcatggtgc caatgaatcg tctgaccgat gatccgcgct ggctaccggc 3060
   gatgagcgaa cgcgtaacgc gaatggtgca gcgcgatcgt aatcacccga gtgtgatcat 3120
   ctggtcgctg gggaatgaat caggccacgg cgctaatcac gacgcgctgt atcgctggat 3180
   caaatctgtc gatccttccc gcccggtgca gtatgaaggc ggcggagccg acaccacggc 3240
   caccgatatt atttgcccga tgtacgcgcg cgtggatgaa gaccagccct tcccggctgt 3300
   gccgaaatgg tccatcaaaa aatggctttc gctacctgga gagacgcgcc cgctgatcct 3360
   ttgcgaatac gcccacgcga tgggtaacag tcttggcggt ttcgctaaat actggcaggc 3420
   gtttcgtcag tatccccgtt tacagggcgg cttcgtctgg gactgggtgg atcagtcgct 3480
   gattaaatat gatgaaaacg gcaacccgtg gtcggcttac ggcggtgatt ttggcgatac 3540
   gccgaacgat cgccagttct gtatgaacgg tctggtcttt gccgaccgca cgccgcatcc 3600
   agcgctgacg gaagcaaaac accagcagca gtttttccag ttccgtttat ccgggcaaac 3660
   catcgaagtg accagcgaat acctgttccg tcatagcgat aacgagctcc tgcactggat 3720
   ggtggcgctg gatggtaagc cgctggcaag cggtgaagtg cctctggatg tcgctccaca 3780
   aggtaaacag ttgattgaac tgcctgaact accgcagccg gagagcgccg ggcaactctg 3840
   gctcacagta cgcgtagtgc aaccgaacgc gaccgcatgg tcagaagccg ggcacatcag 3900
   cgcctggcag cagtggcgtc tggcggaaaa cctcagtgtg acgctccccg ccgcgtccca 3960
   cgccatcccg catctgacca ccagcgaaat ggatttttgc atcgagctgg gtaataagcg 4020
   ttggcaattt aaccgccagt caggctttct ttcacagatg tggattggcg ataaaaaaca 4080
   actgctgacg ccgctgcgcg atcagttcac ccgtgcaccg ctggataacg acattggcgt 4140
   aagtgaagcg acccgcattg accctaacgc ctgggtcgaa cgctggaagg cggcgggcca 4200
   ttaccaggcc gaagcagcgt tgttgcagtg cacggcagat acacttgctg atgcggtgct 4260
   gattacgacc gctcacgcgt ggcagcatca ggggaaaacc ttatttatca gccggaaaac 4320
   ctaccggatt gatggtagtg gtcaaatggc gattaccgtt gatgttgaag tggcgagcga 4380
   tacaccgcat ccggcgcgga ttggcctgaa ctgccagctg gcgcaggtag cagagcgggt 4440
   aaactggctc ggattagggc cgcaagaaaa ctatcccgac cgccttactg ccgcctgttt 4500
   tgaccgctgg gatctgccat tgtcagacat gtataccccg tacgtcttcc cgagcgaaaa 4560
   cggtctgcgc tgcgggacgc gcgaattgaa ttatggccca caccagtggc gcggcgactt 4620
   ccagttcaac atcagccgct acagtcaaca gcaactgatg gaaaccagcc atcgccatct 4680
   gctgcacgcg gaagaaggca catggctgaa tatcgacggt ttccatatgg ggattggtgg 4740
   cgacgactcc tggagcccgt cagtatcggc ggaattccag ctgagcgccg gtcgctacca 4800
   ttaccagttg gtctggtgtc aaaaataata ataaccgggc aggggggatc tttgtgaagg 4860
   aaccttactt ctgtggtgtg acataattgg acaaactacc tacagagatt taaagctcta 4920
   aggtaaatat aaaattttta agtgtataat gtgttaaact actgattcta attgtttgtg 4980
   tattttagat tccaacctat ggaactgatg aatgggagca gtggtggaat gccagatcca 5040
   gacatgataa gatacattga tgagtttgga caaaccacaa ctagaatgca gtgaaaaaaa 5100
   tgctttattt gtgaaatttg tgatgctatt gctttatttg taaccattat aagctgcaat 5160
   aaacaagtta acaacaacaa ttgcattcat tttatgtttc aggttcaggg ggaggtgtgg 5220
   gaggtttttt aaagcaagta aaacctctac aaatgtggta tggctgatta tgatctgcgg 5280
   ccgcagggcc tcgtgatacg cctattttta taggttaatg tcatgataat aatggtttct 5340
   tagacgtcag gtggcacttt tcggggaaat gtgcgcggaa cccctatttg tttatttttc 5400
   taaatacatt caaatatgta tccgctcatg agacaataac cctgataaat gcttcaataa 5460
   tattgaaaaa ggaagagtat gagtattcaa catttccgtg tcgcccttat tccctttttt 5520
   gcggcatttt gccttcctgt ttttgctcac ccagaaacgc tggtgaaagt aaaagatgct 5580
   gaagatcagt tgggtgcacg agtgggttac atcgaactgg atctcaacag cggtaagatc 5640
   cttgagagtt ttcgccccga agaacgtttt ccaatgatga gcacttttaa agttctgcta 5700
   tgtggcgcgg tattatcccg tattgacgcc gggcaagagc aactcggtcg ccgcatacac 5760
   tattctcaga atgacttggt tgagtactca ccagtcacag aaaagcatct tacggatggc 5820
   atgacagtaa gagaattatg cagtgctgcc ataaccatga gtgataacac tgcggccaac 5880
   ttacttctga caacgatcgg aggaccgaag gagctaaccg cttttttgca caacatgggg 5940
   gatcatgtaa ctcgccttga tcgttgggaa ccggagctga atgaagccat accaaacgac 6000
   gagcgtgaca ccacgatgcc tgtagcaatg gcaacaacgt tgcgcaaact attaactggc 6060
   gaactactta ctctagcttc ccggcaacaa ttaatagact ggatggaggc ggataaagtt 6120
   gcaggaccac ttctgcgctc ggcccttccg gctggctggt ttattgctga taaatctgga 6180
   gccggtgagc gtgggtctcg cggtatcatt gcagcactgg ggccagatgg taagccctcc 6240
   cgtatcgtag ttatctacac gacggggagt caggcaacta tggatgaacg aaatagacag 6300
   atcgctgaga taggtgcctc actgattaag cattggtaac tgtcagacca agtttactca 6360
   tatatacttt agattgattt aaaacttcat ttttaattta aaaggatcta ggtgaagatc 6420
   ctttttgata atctcatgac caaaatccct taacgtgagt tttcgttcca ctgagcgtca 6480
   gaccccgtag aaaagatcaa aggatcttct tgagatcctt tttttctgcg cgtaatctgc 6540
   tgcttgcaaa caaaaaaacc accgctacca gcggtggttt gtttgccgga tcaagagcta 6600
   ccaactcttt ttccgaaggt aactggcttc agcagagcgc agataccaaa tactgtcctt 6660
   ctagtgtagc cgtagttagg ccaccacttc aagaactctg tagcaccgcc tacatacctc 6720
   gctctgctaa tcctgttacc agtggctgct gccagtggcg ataagtcgtg tcttaccggg 6780
   ttggactcaa gacgatagtt accggataag gcgcagcggt cgggctgaac ggggggttcg 6840
   tgcacacagc ccagcttgga gcgaacgacc tacaccgaac tgagatacct acagcgtgag 6900
   ctatgagaaa gcgccacgct tcccgaaggg agaaaggcgg acaggtatcc ggtaagcggc 6960
   agggtcggaa caggagagcg cacgagggag cttccagggg gaaacgcctg gtatctttat 7020
   agtcctgtcg ggtttcgcca cctctgactt gagcgtcgat ttttgtgatg ctcgtcaggg 7080
   gggcggagcc tatggaaaaa cgccagcaac gcggcctttt tacggttcct ggccttttgc 7140
   tggccttttg ctcacatgtt ctttcctgcg ttatcccctg attctgtgga taaccgtatt 7200
   accgcctttg agtgagctga taccgctcgc cgcagccgaa cgaccgagcg cagcgagtca 7260
   gtgagcgagg aagcggaaga gcgcccaata cgcaaaccgc ctctccccgc gcgttggccg 7320
   attcattaat gcagctggca cgacaggttt cccgactgga aagcgggcag tgagcgcaac 7380
   gcaattaatg tgagttagct cactcattag gcaccccagg ctttacactt tatgcttccg 7440
   gctcgtatgt tgtgtggaat tgtgagcgga taacaatttc acacaggaaa cagctatgac 7500
   catgattacg ccaagctggc gcg 7523
<210> 80
   <211> 4754
   <212> DNA
   <213> Artificial Sequence
<220>
   <223> Description of Artificial Sequence: vector pRK55pbsC31-Re
<400> 80
   ggccgcccga tatgacacaa ggggttgtga ccggggtgga cacgtacgcg ggtgcttacg 60
   accgtcagtc gcgcgagcgc gagaattcga gcgcagcaag cccagcgaca cagcgtagcg 120
   ccaacgaaga caaggcggcc gaccttcagc gcgaagtcga gcgcgacggg ggccggttca 180
   ggttcgtcgg gcatttcagc gaagcgccgg gcacgtcggc gttcgggacg gcggagcgcc 240
   cggagttcga acgcatcctg aacgaatgcc gcgccgggcg gctcaacatg atcattgtct 300
   atgacgtgtc gcgcttctcg cgcctgaagg tcatggacgc gattccgatt gtctcggaat 360
   tgctcgccct gggcgtgacg attgtttcca ctcaggaagg cgtcttccgg cagggaaacg 420
   tcatggacct gattcacctg attatgcggc tcgacgcgtc gcacaaagaa tcttcgctga 480
   agtcggcgaa gattctcgac acgaagaacc ttcagcgcga attgggcggg tacgtcggcg 540
   ggaaggcgcc ttacggcttc gagcttgttt cggagacgaa ggagatcacg cgcaacggcc 600
   gaatggtcaa tgtcgtcatc aacaagcttg cgcactcgac cactcccctt accggaccct 660
   tcgagttcga gcccgacgta atccggtggt ggtggcgtga gatcaagacg cacaaacacc 720
   ttcccttcaa gccgggcagt caagccgcca ttcacccggg cagcatcacg gggctttgta 780
   agcgcatgga cgctgacgcc gtgccgaccc ggggcgagac gattgggaag aagaccgctt 840
   caagcgcctg ggacccggca accgttatgc gaatccttcg ggacccgcgt attgcgggct 900
   tcgccgctga ggtgatctac aagaagaagc cggacggcac gccgaccacg aagattgagg 960
   gttaccgcat tcagcgcgac ccgatcacgc tccggccggt cgagcttgat tgcggaccga 1020
   tcatcgagcc cgctgagtgg tatgagcttc aggcgtggtt ggacggcagg gggcgcggca 1080
   aggggctttc ccgggggcaa gccattctgt ccgccatgga caagctgtac tgcgagtgtg 1140
   gcgccgtcat gacttcgaag cgcggggaag aatcgatcaa ggactcttac cgctgccgtc 1200
   gccggaaggt ggtcgacccg tccgcacctg ggcagcacga aggcacgtgc aacgtcagca 1260
   tggcggcact cgacaagttc gttgcggaac gcatcttcaa caagatcagg cacgccgaag 1320
   gcgacgaaga gacgttggcg cttctgtggg aagccgcccg acgcttcggc aagctcactg 1380
   aggcgcctga gaagagcggc gaacgggcga accttgttgc ggagcgcgcc gacgccctga 1440
   acgcccttga agagctgtac gaagaccgcg cggcaggcgc gtacgacgga cccgttggca 1500
   ggaagcactt ccggaagcaa caggcagcgc tgacgctccg gcagcaaggg gcggaagagc 1560
   ggcttgccga acttgaagcc gccgaagccc cgaagcttcc ccttgaccaa tggttccccg 1620
   aagacgccga cgctgacccg accggcccta agtcgtggtg ggggcgcgcg tcagtagacg 1680
   acaagcgcgt gttcgtcggg ctcttcgtag acaagatcgt tgtcacgaag tcgactacgg 1740
   gcagggggca gggaacgccc atcgagaagc gcgcttcgat cacgtgggcg aagccgccga 1800
   ccgacgacga cgaagacgac gcccaggacg gcacggaaga cgtagcggcg taggcggcgc 1860
   ccgggctcga gggggggccc ggtacccagc ttttgttccc tttagtgagg gttaatttcg 1920
   agcttggcgt aatcatggtc atagctgttt cctgtgtgaa attgttatcc gctcacaatt 1980
   ccacacaaca tacgagccgg aagcataaag tgtaaagcct ggggtgccta atgagtgagc 2040
   taactcacat taattgcgtt gcgctcactg cccgctttcc agtcgggaaa cctgtcgtgc 2100
   cagctgcatt aatgaatcgg ccaacgcgcg gggagaggcg gtttgcgtat tgggcgctct 2160
   tccgcttcct cgctcactga ctcgctgcgc tcggtcgttc ggctgcggcg agcggtatca 2220
   gctcactcaa aggcggtaat acggttatcc acagaatcag gggataacgc aggaaagaac 2280
   atgtgagcaa aaggccagca aaaggccagg aaccgtaaaa aggccgcgtt gctggcgttt 2340
   ttccataggc tccgcccccc tgacgagcat cacaaaaatc gacgctcaag tcagaggtgg 2400
   cgaaacccga caggactata aagataccag gcgtttcccc ctggaagctc cctcgtgcgc 2460
   tctcctgttc cgaccctgcc gcttaccgga tacctgtccg cctttctccc ttcgggaagc 2520
   gtggcgcttt ctcatagctc acgctgtagg tatctcagtt cggtgtaggt cgttcgctcc 2580
   aagctgggct gtgtgcacga accccccgtt cagcccgacc gctgcgcctt atccggtaac 2640
   tatcgtcttg agtccaaccc ggtaagacac gacttatcgc cactggcagc agccactggt 2700
   aacaggatta gcagagcgag gtatgtaggc ggtgctacag agttcttgaa gtggtggcct 2760
   aactacggct acactagaag gacagtattt ggtatctgcg ctctgctgaa gccagttacc 2820
   ttcggaaaaa gagttggtag ctcttgatcc ggcaaacaaa ccaccgctgg tagcggtggt 2880
   ttttttgttt gcaagcagca gattacgcgc agaaaaaaag gatctcaaga agatcctttg 2940
   atcttttcta cggggtctga cgctcagtgg aacgaaaact cacgttaagg gattttggtc 3000
   atgagattat caaaaaggat cttcacctag atccttttaa attaaaaatg aagttttaaa 3060
   tcaatctaaa gtatatatga gtaaacttgg tctgacagtt accaatgctt aatcagtgag 3120
   gcacctatct cagcgatctg tctatttcgt tcatccatag ttgcctgact ccccgtcgtg 3180
   tagataacta cgatacggga gggcttacca tctggcccca gtgctgcaat gataccgcga 3240
   gacccacgct caccggctcc agatttatca gcaataaacc agccagccgg aagggccgag 3300
   cgcagaagtg gtcctgcaac tttatccgcc tccatccagt ctattaattg ttgccgggaa 3360
   gctagagtaa gtagttcgcc agttaatagt ttgcgcaacg ttgttgccat tgctacaggc 3420
   atcgtggtgt cacgctcgtc gtttggtatg gcttcattca gctccggttc ccaacgatca 3480
   aggcgagtta catgatcccc catgttgtgc aaaaaagcgg ttagctcctt cggtcctccg 3540
   atcgttgtca gaagtaagtt ggccgcagtg ttatcactca tggttatggc agcactgcat 3600
   aattctctta ctgtcatgcc atccgtaaga tgcttttctg tgactggtga gtactcaacc 3660
   aagtcattct gagaatagtg tatgcggcga ccgagttgct cttgcccggc gtcaatacgg 3720
   gataataccg cgccacatag cagaacttta aaagtgctca tcattggaaa acgttcttcg 3780
   gggcgaaaac tctcaaggat cttaccgctg ttgagatcca gttcgatgta acccactcgt 3840
   gcacccaact gatcttcagc atcttttact ttcaccagcg tttctgggtg agcaaaaaca 3900
   ggaaggcaaa atgccgcaaa aaagggaata agggcgacac ggaaatgttg aatactcata 3960
   ctcttccttt ttcaatatta ttgaagcatt tatcagggtt attgtctcat gagcggatac 4020
   atatttgaat gtatttagaa aaataaacaa ataggggttc cgcgcacatt tccccgaaaa 4080
   gtgccaccta aattgtaagc gttaatattt tgttaaaatt cgcgttaaat ttttgttaaa 4140
   tcagctcatt ttttaaccaa taggccgaaa tcggcaaaat cccttataaa tcaaaagaat 4200
   agaccgagat agggttgagt gttgttccag tttggaacaa gagtccacta ttaaagaacg 4260
   tggactccaa cgtcaaaggg cgaaaaaccg tctatcaggg cgatggccca ctacgtgaac 4320
   catcacccta atcaagtttt ttggggtcga ggtgccgtaa agcactaaat cggaacccta 4380
   aagggagccc ccgatttaga gcttgacggg gaaagccggc gaacgtggcg agaaaggaag 4440
   ggaagaaagc gaaaggagcg ggcgctaggg cgctggcaag tgtagcggtc acgctgcgcg 4500
   taaccaccac acccgccgcg cttaatgcgc cgctacaggg cgcgtcccat tcgccattca 4560
   ggctgcgcaa ctgttgggaa gggcgatcgg tgcgggcctc ttcgctatta cgccagctgg 4620
   cgaaaggggg atgtgctgca aggcgattaa gttgggtaac gccagggttt tcccagtcac 4680
   gacgttgtaa aacgacggcc agtgaattgt aatacgactc actatagggc gaattggagc 4740
   tccaccgcgg tggc                                                   4754
<210> 81
   <211> 4773
   <212> DNA
   <213> Artificial Sequence
<220>
   <223> Description of Artificial Sequence: vector pRK63pbsC31NLS-Re
<400> 81
   ggccgcacca tgcccaagaa gaagaggaag gtgacacaag gggttgtgac cggggtggac 60
   acgtacgcgg gtgcttacga ccgtcagtcg cgcgagcgcg agaattcgag cgcagcaagc 120
   ccagcgacac agcgtagcgc caacgaagac aaggcggccg accttcagcg cgaagtcgag 180
   cgcgacgggg gccggttcag gttcgtcggg catttcagcg aagcgccggg cacgtcggcg 240
   ttcgggacgg cggagcgccc ggagttcgaa cgcatcctga acgaatgccg cgccgggcgg 300
   ctcaacatga tcattgtcta tgacgtgtcg cgcttctcgc gcctgaaggt catggacgcg 360
   attccgattg tctcggaatt gctcgccctg ggcgtgacga ttgtttccac tcaggaaggc 420
   gtcttccggc agggaaacgt catggacctg attcacctga ttatgcggct cgacgcgtcg 480
   cacaaagaat cttcgctgaa gtcggcgaag attctcgaca cgaagaacct tcagcgcgaa 540
   ttgggcgggt acgtcggcgg gaaggcgcct tacggcttcg agcttgtttc ggagacgaag 600
   gagatcacgc gcaacggccg aatggtcaat gtcgtcatca acaagcttgc gcactcgacc 660
   actcccctta ccggacccct cgagttcgag cccgacgtaa tccggtggtg gtggcgtgag 720
   atcaagacgc acaaacacct tcccttcaag ccgggcagtc aagccgccat tcacccgggc 780
   agcatcacgg ggctttgtaa gcgcatggac gctgacgccg tgccgacccg gggcgagacg 840
   attgggaaga agaccgcttc aagcgcctgg gacccggcaa ccgttatgcg aatccttcgg 900
   gacccgcgta ttgcgggctt cgccgctgag gtgatctaca agaagaagcc ggacggcacg 960
   ccgaccacga agattgaggg ttaccgcatt cagcgcgacc cgatcacgct ccggccggtc 1020
   gagcttgatt gcggaccgat catcgagccc gctgagtggt atgagcttca ggcgtggttg 1080
   gacggcaggg ggcgcggcaa ggggctttcc cgggggcaag ccattctgtc cgccatggac 1140
   aagctgtact gcgagtgtgg cgccgtcatg acttcgaagc gcggggaaga atcgatcaag 1200
   gactcttacc gctgccgtcg ccggaaggtg gtcgacccgt ccgcacctgg gcagcacgaa 1260
   ggcacgtgca acgtcagcat ggcggcactc gacaagttcg ttgcggaacg catcttcaac 1320
   aagatcaggc acgccgaagg cgacgaagag acgttggcgc ttctgtggga agccgcccga 1380
   cgcttcggca agctcactga ggcgcctgag aagagcggcg aacgggcgaa ccttgttgcg 1440
   gagcgcgccg acgccctgaa cgcccttgaa gagctgtacg aagaccgcgc ggcaggcgcg 1500
   tacgacggac ccgttggcag gaagcacttc cggaagcaac aggcagcgct gacgctccgg 1560
   cagcaagggg cggaagagcg gcttgccgaa cttgaagccg ccgaagcccc gaagcttccc 1620
   cttgaccaat ggttccccga agacgccgac gctgacccga ccggccctaa gtcgtggtgg 1680
   gggcgcgcgt cagtagacga caagcgcgtg ttcgtcgggc tcttcgtaga caagatcgtt 1740
   gtcacgaagt cgactacggg cagggggcag ggaacgccca tcgagaagcg cgcttcgatc 1800
   acgtgggcga agccgccgac cgacgacgac gaagacgacg cccaggacgg cacggaagac 1860
   gtagcggcgt aggcggcgcc cgggctcgag ggggggcccg gtacccagct tttgttccct 1920
   ttagtgaggg ttaatttcga gcttggcgta atcatggtca tagctgtttc ctgtgtgaaa 1980
   ttgttatccg ctcacaattc cacacaacat acgagccgga agcataaagt gtaaagcctg 2040
   gggtgcctaa tgagtgagct aactcacatt aattgcgttg cgctcactgc ccgctttcca 2100
   gtcgggaaac ctgtcgtgcc agctgcatta atgaatcggc caacgcgcgg ggagaggcgg 2160
   tttgcgtatt gggcgctctt ccgcttcctc gctcactgac tcgctgcgct cggtcgttcg 2220
   gctgcggcga gcggtatcag ctcactcaaa ggcggtaata cggttatcca cagaatcagg 2280
   ggataacgca ggaaagaaca tgtgagcaaa aggccagcaa aaggccagga accgtaaaaa 2340
   ggccgcgttg ctggcgtttt tccataggct ccgcccccct gacgagcatc acaaaaatcg 2400
   acgctcaagt cagaggtggc gaaacccgac aggactataa agataccagg cgtttccccc 2460
   tggaagctcc ctcgtgcgct ctcctgttcc gaccctgccg cttaccggat acctgtccgc 2520
   ctttctccct tcgggaagcg tggcgctttc tcatagctca cgctgtaggt atctcagttc 2580
   ggtgtaggtc gttcgctcca agctgggctg tgtgcacgaa ccccccgttc agcccgaccg 2640
   ctgcgcctta tccggtaact atcgtcttga gtccaacccg gtaagacacg acttatcgcc 2700
   actggcagca gccactggta acaggattag cagagcgagg tatgtaggcg gtgctacaga 2760
   gttcttgaag tggtggccta actacggcta cactagaagg acagtatttg gtatctgcgc 2820
   tctgctgaag ccagttacct tcggaaaaag agttggtagc tcttgatccg gcaaacaaac 2880
   caccgctggt agcggtggtt tttttgtttg caagcagcag attacgcgca gaaaaaaagg 2940
   atctcaagaa gatcctttga tcttttctac ggggtctgac gctcagtgga acgaaaactc 3000
   acgttaaggg attttggtca tgagattatc aaaaaggatc ttcacctaga tccttttaaa 3060
   ttaaaaatga agttttaaat caatctaaag tatatatgag taaacttggt ctgacagtta 3120
   ccaatgctta atcagtgagg cacctatctc agcgatctgt ctatttcgtt catccatagt 3180
   tgcctgactc cccgtcgtgt agataactac gatacgggag ggcttaccat ctggccccag 3240
   tgctgcaatg ataccgcgag acccacgctc accggctcca gatttatcag caataaacca 3300
   gccagccgga agggccgagc gcagaagtgg tcctgcaact ttatccgcct ccatccagtc 3360
   tattaattgt tgccgggaag ctagagtaag tagttcgcca gttaatagtt tgcgcaacgt 3420
   tgttgccatt gctacaggca tcgtggtgtc acgctcgtcg tttggtatgg cttcattcag 3480
   ctccggttcc caacgatcaa ggcgagttac atgatccccc atgttgtgca aaaaagcggt 3540
   tagctccttc ggtcctccga tcgttgtcag aagtaagttg gccgcagtgt tatcactcat 3600
   ggttatggca gcactgcata attctcttac tgtcatgcca tccgtaagat gcttttctgt 3660
   gactggtgag tactcaacca agtcattctg agaatagtgt atgcggcgac cgagttgctc 3720
   ttgcccggcg tcaatacggg ataataccgc gccacatagc agaactttaa aagtgctcat 3780
   cattggaaaa cgttcttcgg ggcgaaaact ctcaaggatc ttaccgctgt tgagatccag 3840
   ttcgatgtaa cccactcgtg cacccaactg atcttcagca tcttttactt tcaccagcgt 3900
   ttctgggtga gcaaaaacag gaaggcaaaa tgccgcaaaa aagggaataa gggcgacacg 3960
   gaaatgttga atactcatac tcttcctttt tcaatattat tgaagcattt atcagggtta 4020
   ttgtctcatg agcggataca tatttgaatg tatttagaaa aataaacaaa taggggttcc 4080
   gcgcacattt ccccgaaaag tgccacctaa attgtaagcg ttaatatttt gttaaaattc 4140
   gcgttaaatt tttgttaaat cagctcattt tttaaccaat aggccgaaat cggcaaaatc 4200
   ccttataaat caaaagaata gaccgagata gggttgagtg ttgttccagt ttggaacaag 4260
   agtccactat taaagaacgt ggactccaac gtcaaagggc gaaaaaccgt ctatcagggc 4320
   gatggcccac tacgtgaacc atcaccctaa tcaagttttt tggggtcgag gtgccgtaaa 4380
   gcactaaatc ggaaccctaa agggagcccc cgatttagag cttgacgggg aaagccggcg 4440
   aacgtggcga gaaaggaagg gaagaaagcg aaaggagcgg gcgctagggc gctggcaagt 4500
   gtagcggtca cgctgcgcgt aaccaccaca cccgccgcgc ttaatgcgcc gctacagggc 4560
   gcgtcccatt cgccattcag gctgcgcaac tgttgggaag ggcgatcggt gcgggcctct 4620
   tcgctattac gccagctggc gaaaggggga tgtgctgcaa ggcgattaag ttgggtaacg 4680
   ccagggtttt cccagtcacg acgttgtaaa acgacggcca gtgaattgta atacgactca 4740
   ctatagggcg aattggagct ccaccgcggt ggc                               4773
<210> 82
   <211> 7803
   <212> DNA
   <213> Artificial Sequence
<220>
   <223> Description of Artificial Sequence: vector pPGKattA1
<400> 82
   tatcatgtct ggatccgcgt taacacctaa gaaggcgaag ttttccttac accttgcaga 60
   tataaagtgt ctaacagttt aaaatatccg atgaggcata tttatgttgg acccgtagct 120
   cagccaggat agagcactgg cctccggagc cggaggtccc gggttcaaat cccggcgggt 180
   ccgtatatta ctttttgatt cagattagat ttgtaaatct ttattacaag gataatttga 240
   tcttgtatat tggtaactct ctactctata atttttatga gaaattcaca gtcgtccctt 300
   tataccataa atagctaagt ttgtcaaagt tcttattaaa ctctccatgt agagattaaa 360
   tcggatccat aacttcgtat agcatacatt atacgaagtt ataccgggcc accatggtcg 420
   cgagtagctt ggcactggcc gtcgttttac aacgtcgtga ctgggaaaac cctggcgtta 480
   cccaacttaa tcgccttgca gcacatcccc ctttcgccag ctggcgtaat agcgaagagg 540
   cccgcaccga tcgcccttcc caacagttgc gcagcctgaa tggcgaatgg cgctttgcct 600
   ggtttccggc accagaagcg gtgccggaaa gctggctgga gtgcgatctt cctgaggccg 660
   atactgtcgt cgtcccctca aactggcaga tgcacggtta cgatgcgccc atctacacca 720
   acgtaaccta tcccattacg gtcaatccgc cgtttgttcc cacggagaat ccgacgggtt 780
   gttactcgct cacatttaat gttgatgaaa gctggctaca ggaaggccag acgcgaatta 840
   tttttgatgg cgttaactcg gcgtttcatc tgtggtgcaa cgggcgctgg gtcggttacg 900
   gccaggacag tcgtttgccg tctgaatttg acctgagcgc atttttacgc gccggagaaa 960
   accgcctcgc ggtgatggtg ctgcgttgga gtgacggcag ttatctggaa gatcaggata 1020
   tgtggcggat gagcggcatt ttccgtgacg tctcgttgct gcataaaccg actacacaaa 1080
   tcagcgattt ccatgttgcc actcgcttta atgatgattt cagccgcgct gtactggagg 1140
   ctgaagttca gatgtgcggc gagttgcgtg actacctacg ggtaacagtt tctttatggc 1200
   agggtgaaac gcaggtcgcc agcggcaccg cgcctttcgg cggtgaaatt atcgatgagc 1260
   gtggtggtta tgccgatcgc gtcacactac gtctgaacgt cgaaaacccg aaactgtgga 1320
   gcgccgaaat cccgaatctc tatcgtgcgg tggttgaact gcacaccgcc gacggcacgc 1380
   tgattgaagc agaagcctgc gatgtcggtt tccgcgaggt gcggattgaa aatggtctgc 1440
   tgctgctgaa cggcaagccg ttgctgattc gaggcgttaa ccgtcacgag catcatcctc 1500
   tgcatggtca ggtcatggat gagcagacga tggtgcagga tatcctgctg atgaagcaga 1560
   acaactttaa cgccgtgcgc tgttcgcatt atccgaacca tccgctgtgg tacacgctgt 1620
   gcgaccgcta cggcctgtat gtggtggatg aagccaatat tgaaacccac ggcatggtgc 1680
   caatgaatcg tctgaccgat gatccgcgct ggctaccggc gatgagcgaa cgcgtaacgc 1740
   gaatggtgca gcgcgatcgt aatcacccga gtgtgatcat ctggtcgctg gggaatgaat 1800
   caggccacgg cgctaatcac gacgcgctgt atcgctggat caaatctgtc gatccttccc 1860
   gcccggtgca gtatgaaggc ggcggagccg acaccacggc caccgatatt atttgcccga 1920
   tgtacgcgcg cgtggatgaa gaccagccct tcccggctgt gccgaaatgg tccatcaaaa 1980
   aatggctttc gctacctgga gagacgcgcc cgctgatcct ttgcgaatac gcccacgcga 2040
   tgggtaacag tcttggcggt ttcgctaaat actggcaggc gtttcgtcag tatccccgtt 2100
   tacagggcgg cttcgtctgg gactgggtgg atcagtcgct gattaaatat gatgaaaacg 2160
   gcaacccgtg gtcggcttac ggcggtgatt ttggcgatac gccgaacgat cgccagttct 2220
   gtatgaacgg tctggtcttt gccgaccgca cgccgcatcc agcgctgacg gaagcaaaac 2280
   accagcagca gtttttccag ttccgtttat ccgggcaaac catcgaagtg accagcgaat 2340
   acctgttccg tcatagcgat aacgagctcc tgcactggat ggtggcgctg gatggtaagc 2400
   cgctggcaag cggtgaagtg cctctggatg tcgctccaca aggtaaacag ttgattgaac 2460
   tgcctgaact accgcagccg gagagcgccg ggcaactctg gctcacagta cgcgtagtgc 2520
   aaccgaacgc gaccgcatgg tcagaagccg ggcacatcag cgcctggcag cagtggcgtc 2580
   tggcggaaaa cctcagtgtg acgctccccg ccgcgtccca cgccatcccg catctgacca 2640
   ccagcgaaat ggatttttgc atcgagctgg gtaataagcg ttggcaattt aaccgccagt 2700
   caggctttct ttcacagatg tggattggcg ataaaaaaca actgctgacg ccgctgcgcg 2760
   atcagttcac ccgtgcaccg ctggataacg acattggcgt aagtgaagcg acccgcattg 2820
   accctaacgc ctgggtcgaa cgctggaagg cggcgggcca ttaccaggcc gaagcagcgt 2880
   tgttgcagtg cacggcagat acacttgctg atgcggtgct gattacgacc gctcacgcgt 2940
   ggcagcatca ggggaaaacc ttatttatca gccggaaaac ctaccggatt gatggtagtg 3000
   gtcaaatggc gattaccgtt gatgttgaag tggcgagcga tacaccgcat ccggcgcgga 3060
   ttggcctgaa ctgccagctg gcgcaggtag cagagcgggt aaactggctc ggattagggc 3120
   cgcaagaaaa ctatcccgac cgccttactg ccgcctgttt tgaccgctgg gatctgccat 3180
   tgtcagacat gtataccccg tacgtcttcc cgagcgaaaa cggtctgcgc tgcgggacgc 3240
   gcgaattgaa ttatggccca caccagtggc gcggcgactt ccagttcaac atcagccgct 3300
   acagtcaaca gcaactgatg gaaaccagcc atcgccatct gctgcacgcg gaagaaggca 3360
   catggctgaa tatcgacggt ttccatatgg ggattggtgg cgacgactcc tggagcccgt 3420
   cagtatcggc ggaattccag ctgagcgccg gtcgctacca ttaccagttg gtctggtgtc 3480
   aaaaataata ataaccgggc aggggggatc tttgtgaagg aaccttactt ctgtggtgtg 3540
   acataattgg acaaactacc tacagagatt taaagctcta aggtaaatat aaaattttta 3600
   agtgtataat gtgttaaact actgattcta attgtttgtg tattttagat tccaacctat 3660
   ggaactgatg aatgggagca gtggtggaat gccagatcca gacatgataa gatacattga 3720
   tgagtttgga caaaccacaa ctagaatgca gtgaaaaaaa tgctttattt gtgaaatttg 3780
   tgatgctatt gctttatttg taaccattat aagctgcaat aaacaagtta acaacaacaa 3840
   ttgcattcat tttatgtttc aggttcaggg ggaggtgtgg gaggtttttt aaagcaagta 3900
   aaacctctac aaatgtggta tggctgatta tgatctgcgg ccgcagggcc tcgtgatacg 3960
   cctattttta taggttaatg tcatgataat aatggtttct tagacgtcag gtggcacttt 4020
   tcggggaaat gtgcgcggaa cccctatttg tttatttttc taaatacatt caaatatgta 4080
   tccgctcatg agacaataac cctgataaat gcttcaataa tattgaaaaa ggaagagtat 4140
   gagtattcaa catttccgtg tcgcccttat tccctttttt gcggcatttt gccttcctgt 4200
   ttttgctcac ccagaaacgc tggtgaaagt aaaagatgct gaagatcagt tgggtgcacg 4260
   agtgggttac atcgaactgg atctcaacag cggtaagatc cttgagagtt ttcgccccga 4320
   agaacgtttt ccaatgatga gcacttttaa agttctgcta tgtggcgcgg tattatcccg 4380
   tattgacgcc gggcaagagc aactcggtcg ccgcatacac tattctcaga atgacttggt 4440
   tgagtactca ccagtcacag aaaagcatct tacggatggc atgacagtaa gagaattatg 4500
   cagtgctgcc ataaccatga gtgataacac tgcggccaac ttacttctga caacgatcgg 4560
   aggaccgaag gagctaaccg cttttttgca caacatgggg gatcatgtaa ctcgccttga 4620
   tcgttgggaa ccggagctga atgaagccat accaaacgac gagcgtgaca ccacgatgcc 4680
   tgtagcaatg gcaacaacgt tgcgcaaact attaactggc gaactactta ctctagcttc 4740
   ccggcaacaa ttaatagact ggatggaggc ggataaagtt gcaggaccac ttctgcgctc 4800
   ggcccttccg gctggctggt ttattgctga taaatctgga gccggtgagc gtgggtctcg 4860
   cggtatcatt gcagcactgg ggccagatgg taagccctcc cgtatcgtag ttatctacac 4920
   gacggggagt caggcaacta tggatgaacg aaatagacag atcgctgaga taggtgcctc 4980
   actgattaag cattggtaac tgtcagacca agtttactca tatatacttt agattgattt 5040
   aaaacttcat ttttaattta aaaggatcta ggtgaagatc ctttttgata atctcatgac 5100
   caaaatccct taacgtgagt tttcgttcca ctgagcgtca gaccccgtag aaaagatcaa 5160
   aggatcttct tgagatcctt tttttctgcg cgtaatctgc tgcttgcaaa caaaaaaacc 5220
   accgctacca gcggtggttt gtttgccgga tcaagagcta ccaactcttt ttccgaaggt 5280
   aactggcttc agcagagcgc agataccaaa tactgtcctt ctagtgtagc cgtagttagg 5340
   ccaccacttc aagaactctg tagcaccgcc tacatacctc gctctgctaa tcctgttacc 5400
   agtggctgct gccagtggcg ataagtcgtg tcttaccggg ttggactcaa gacgatagtt 5460
   accggataag gcgcagcggt cgggctgaac ggggggttcg tgcacacagc ccagcttgga 5520
   gcgaacgacc tacaccgaac tgagatacct acagcgtgag ctatgagaaa gcgccacgct 5580
   tcccgaaggg agaaaggcgg acaggtatcc ggtaagcggc agggtcggaa caggagagcg 5640
   cacgagggag cttccagggg gaaacgcctg gtatctttat agtcctgtcg ggtttcgcca 5700
   cctctgactt gagcgtcgat ttttgtgatg ctcgtcaggg gggcggagcc tatggaaaaa 5760
   cgccagcaac gcggcctttt tacggttcct ggccttttgc tggccttttg ctcacatgtt 5820
   ctttcctgcg ttatcccctg attctgtgga taaccgtatt accgcctttg agtgagctga 5880
   taccgctcgc cgcagccgaa cgaccgagcg cagcgagtca gtgagcgagg aagcggaaga 5940
   gcgcccaata cgcaaaccgc ctctccccgc gcgttggccg attcattaat gcagctggca 6000
   cgacaggttt cccgactgga aagcgggcag tgagcgcaac gcaattaatg tgagttagct 6060
   cactcattag gcaccccagg ctttacactt tatgcttccg gctcgtatgt tgtgtggaat 6120
   tgtgagcgga taacaatttc acacaggaaa cagctatgac catgattacg ccaagctggc 6180
   gcgtcgagga attctaccgg gtaggggagg cgcttttccc aaggcagtct ggagcatgcg 6240
   ctttagcagc cccgctgggc acttggcgct acacaagtgg cctctggctc gcacacattc 6300
   cacatccacc ggtaggcgcc aaccggctcc gttctttggt ggccccttcg cgccaccttc 6360
   tactcctccc ctagtcagga agttcccccc cgccccgcag ctcgcgtcgt gcaggacgtg 6420
   acaaatggaa gtagcacgtc tcactagtct cgtgcagatg gacagcaccg ctgagcaatg 6480
   gaagcgggta ggcctttggg gcagcggcca atagcagctt tgctccttcg ctttctgggc 6540
   tcagaggctg ggaaggggtg ggtccggggg cgggctcagg ggcgggctca ggggcggggc 6600
   gggcgcccga aggtcctccg gaggcccggc attctgcacg cttcaaaagc gcacgtctgc 6660
   cgcgctgttc tcctcttcct catctccggg cctttcgacc tgcagcccgg tacagttcga 6720
   ataacttcgt atagcataca ttatacgaag ttataagctt gcatgcctgc aggtcggccg 6780
   ccacgaccgg ccggccggtg ccgccaccat cccctgaccc acgcccctga cccctcacaa 6840
   ggagacgacc ttccatgacc gagtacaagc ccacggtgcg cctcgccacc cgcgacgacg 6900
   tcccccgggc cgtacgcacc ctcgccgccg cgttcgccga ctaccccgcc acgcgccaca 6960
   ccgtcgaccc ggaccgccac atcgagcggg tcaccgagct gcaagaactc ttcctcacgc 7020
   gcgtcgggct cgacatcggc aaggtgtggg tcgcggacga cggcgccgcg gtggcggtct 7080
   ggaccacgcc ggagagcgtc gaagcggggg cggtgttcgc cgagatcggc ccgcgcatgg 7140
   ccgagttgag cggttcccgg ctggccgcgc agcaacagat ggaaggcctc ctggcgccgc 7200
   accggcccaa ggagcccgcg tggttcctgg ccaccgtcgg cgtctcgccc gaccaccagg 7260
   gcaagggtct gggcagcgcc gtcgtgctcc ccggagtgga ggcggccgag cgcgccgggg 7320
   tgcccgcctt cctggagacc tccgcgcccc gcaacctccc cttctacgag cggctcggct 7380
   tcaccgtcac cgccgacgtc gagtgcccga aggaccgcgc gacctggtgc atgacccgca 7440
   agcccggtgc ctgacgcccg ccccacgacc cgcagcgccc gaccgaaagg agcgcacgac 7500
   cccatggctc cgaccgaagc cgacccgggc ggccccgccg accccgcacc cgcccccgag 7560
   gcccaccgac tctagaggat cataatcagc cataccacat ttgtagaggt tttacttgct 7620
   ttaaaaaacc tcccacacct ccccctgaac ctgaaacata aaatgaatgc aattgttgtt 7680
   gttaacttgt ttattgcagc ttataatggt tacaaataaa gcaatagcat cacaaatttc 7740
   acaaataaag catttttttc actgcattct agttgtggtt tgtccaaact catcaatgta 7800
   tct 7803
<210> 83
   <211> 8167
   <212> DNA
   <213> Artificial Sequence
<220>
   <223> Description of Artificial Sequence: vector pPGKattA2
<400> 83
   tatcatgtct ggatccgcgt taacacctaa gaaggcgaag ttttccttac accttgcaga 60
   tataaagtgt ctaacagttt aaaatatccg atgaggcata tttatgttgg acccgtagct 120
   cagccaggat agagcactgg cctccggagc cggaggtccc gggttcaaat cccggcgggt 180
   ccgtatatta ctttttgatt cagattagat ttgtaaatct ttattacaag gataatttga 240
   tcttgtatat tggtaactct ctactctata atttttatga gaaattcaca gtcgtccctt 300
   tataccataa atagctaagt ttgtcaaagt tcttattaaa ctctccatgt agagattaaa 360
   tcggatccat aacttcgtat agcatacatt atacgaagtt ataccgggcc accatggtcg 420
   cgagtagctt ggcactggcc gtcgttttac aacgtcgtga ctgggaaaac cctggcgtta 480
   cccaacttaa tcgccttgca gcacatcccc ctttcgccag ctggcgtaat agcgaagagg 540
   cccgcaccga tcgcccttcc caacagttgc gcagcctgaa tggcgaatgg cgctttgcct 600
   ggtttccggc accagaagcg gtgccggaaa gctggctgga gtgcgatctt cctgaggccg 660
   atactgtcgt cgtcccctca aactggcaga tgcacggtta cgatgcgccc atctacacca 720
   acgtaaccta tcccattacg gtcaatccgc cgtttgttcc cacggagaat ccgacgggtt 780
   gttactcgct cacatttaat gttgatgaaa gctggctaca ggaaggccag acgcgaatta 840
   tttttgatgg cgttaactcg gcgtttcatc tgtggtgcaa cgggcgctgg gtcggttacg 900
   gccaggacag tcgtttgccg tctgaatttg acctgagcgc atttttacgc gccggagaaa 960
   accgcctcgc ggtgatggtg ctgcgttgga gtgacggcag ttatctggaa gatcaggata 1020
   tgtggcggat gagcggcatt ttccgtgacg tctcgttgct gcataaaccg actacacaaa 1080
   tcagcgattt ccatgttgcc actcgcttta atgatgattt cagccgcgct gtactggagg 1140
   ctgaagttca gatgtgcggc gagttgcgtg actacctacg ggtaacagtt tctttatggc 1200
   agggtgaaac gcaggtcgcc agcggcaccg cgcctttcgg cggtgaaatt atcgatgagc 1260
   gtggtggtta tgccgatcgc gtcacactac gtctgaacgt cgaaaacccg aaactgtgga 1320
   gcgccgaaat cccgaatctc tatcgtgcgg tggttgaact gcacaccgcc gacggcacgc 1380
   tgattgaagc agaagcctgc gatgtcggtt tccgcgaggt gcggattgaa aatggtctgc 1440
   tgctgctgaa cggcaagccg ttgctgattc gaggcgttaa ccgtcacgag catcatcctc 1500
   tgcatggtca ggtcatggat gagcagacga tggtgcagga tatcctgctg atgaagcaga 1560
   acaactttaa cgccgtgcgc tgttcgcatt atccgaacca tccgctgtgg tacacgctgt 1620
   gcgaccgcta cggcctgtat gtggtggatg aagccaatat tgaaacccac ggcatggtgc 1680
   caatgaatcg tctgaccgat gatccgcgct ggctaccggc gatgagcgaa cgcgtaacgc 1740
   gaatggtgca gcgcgatcgt aatcacccga gtgtgatcat ctggtcgctg gggaatgaat 1800
   caggccacgg cgctaatcac gacgcgctgt atcgctggat caaatctgtc gatccttccc 1860
   gcccggtgca gtatgaaggc ggcggagccg acaccacggc caccgatatt atttgcccga 1920
   tgtacgcgcg cgtggatgaa gaccagccct tcccggctgt gccgaaatgg tccatcaaaa 1980
   aatggctttc gctacctgga gagacgcgcc cgctgatcct ttgcgaatac gcccacgcga 2040
   tgggtaacag tcttggcggt ttcgctaaat actggcaggc gtttcgtcag tatccccgtt 2100
   tacagggcgg cttcgtctgg gactgggtgg atcagtcgct gattaaatat gatgaaaacg 2160
   gcaacccgtg gtcggcttac ggcggtgatt ttggcgatac gccgaacgat cgccagttct 2220
   gtatgaacgg tctggtcttt gccgaccgca cgccgcatcc agcgctgacg gaagcaaaac 2280
   accagcagca gtttttccag ttccgtttat ccgggcaaac catcgaagtg accagcgaat 2340
   acctgttccg tcatagcgat aacgagctcc tgcactggat ggtggcgctg gatggtaagc 2400
   cgctggcaag cggtgaagtg cctctggatg tcgctccaca aggtaaacag ttgattgaac 2460
   tgcctgaact accgcagccg gagagcgccg ggcaactctg gctcacagta cgcgtagtgc 2520
   aaccgaacgc gaccgcatgg tcagaagccg ggcacatcag cgcctggcag cagtggcgtc 2580
   tggcggaaaa cctcagtgtg acgctccccg ccgcgtccca cgccatcccg catctgacca 2640
   ccagcgaaat ggatttttgc atcgagctgg gtaataagcg ttggcaattt aaccgccagt 2700
   caggctttct ttcacagatg tggattggcg ataaaaaaca actgctgacg ccgctgcgcg 2760
   atcagttcac ccgtgcaccg ctggataacg acattggcgt aagtgaagcg acccgcattg 2820
   accctaacgc ctgggtcgaa cgctggaagg cggcgggcca ttaccaggcc gaagcagcgt 2880
   tgttgcagtg cacggcagat acacttgctg atgcggtgct gattacgacc gctcacgcgt 2940
   ggcagcatca ggggaaaacc ttatttatca gccggaaaac ctaccggatt gatggtagtg 3000
   gtcaaatggc gattaccgtt gatgttgaag tggcgagcga tacaccgcat ccggcgcgga 3060
   ttggcctgaa ctgccagctg gcgcaggtag cagagcgggt aaactggctc ggattagggc 3120
   cgcaagaaaa ctatcccgac cgccttactg ccgcctgttt tgaccgctgg gatctgccat 3180
   tgtcagacat gtataccccg tacgtcttcc cgagcgaaaa cggtctgcgc tgcgggacgc 3240
   gcgaattgaa ttatggccca caccagtggc gcggcgactt ccagttcaac atcagccgct 3300
   acagtcaaca gcaactgatg gaaaccagcc atcgccatct gctgcacgcg gaagaaggca 3360
   catggctgaa tatcgacggt ttccatatgg ggattggtgg cgacgactcc tggagcccgt 3420
   cagtatcggc ggaattccag ctgagcgccg gtcgctacca ttaccagttg gtctggtgtc 3480
   aaaaataata ataaccgggc aggggggatc tttgtgaagg aaccttactt ctgtggtgtg 3540
   acataattgg acaaactacc tacagagatt taaagctcta aggtaaatat aaaattttta 3600
   agtgtataat gtgttaaact actgattcta attgtttgtg tattttagat tccaacctat 3660
   ggaactgatg aatgggagca gtggtggaat gccagatcca gacatgataa gatacattga 3720
   tgagtttgga caaaccacaa ctagaatgca gtgaaaaaaa tgctttattt gtgaaatttg 3780
   tgatgctatt gctttatttg taaccattat aagctgcaat aaacaagtta acaacaacaa 3840
   ttgcattcat tttatgtttc aggttcaggg ggaggtgtgg gaggtttttt aaagcaagta 3900
   aaacctctac aaatgtggta tggctgatta tgatctgcgg ccgcagggcc tcgtgatacg 3960
   cctattttta taggttaatg tcatgataat aatggtttct tagacgtcag gtggcacttt 4020
   tcggggaaat gtgcgcggaa cccctatttg tttatttttc taaatacatt caaatatgta 4080
   tccgctcatg agacaataac cctgataaat gcttcaataa tattgaaaaa ggaagagtat 4140
   gagtattcaa catttccgtg tcgcccttat tccctttttt gcggcatttt gccttcctgt 4200
   ttttgctcac ccagaaacgc tggtgaaagt aaaagatgct gaagatcagt tgggtgcacg 4260
   agtgggttac atcgaactgg atctcaacag cggtaagatc cttgagagtt ttcgccccga 4320
   agaacgtttt ccaatgatga gcacttttaa agttctgcta tgtggcgcgg tattatcccg 4380
   tattgacgcc gggcaagagc aactcggtcg ccgcatacac tattctcaga atgacttggt 4440
   tgagtactca ccagtcacag aaaagcatct tacggatggc atgacagtaa gagaattatg 4500
   cagtgctgcc ataaccatga gtgataacac tgcggccaac ttacttctga caacgatcgg 4560
   aggaccgaag gagctaaccg cttttttgca caacatgggg gatcatgtaa ctcgccttga 4620
   tcgttgggaa ccggagctga atgaagccat accaaacgac gagcgtgaca ccacgatgcc 4680
   tgtagcaatg gcaacaacgt tgcgcaaact attaactggc gaactactta ctctagcttc 4740
   ccggcaacaa ttaatagact ggatggaggc ggataaagtt gcaggaccac ttctgcgctc 4800
   ggcccttccg gctggctggt ttattgctga taaatctgga gccggtgagc gtgggtctcg 4860
   cggtatcatt gcagcactgg ggccagatgg taagccctcc cgtatcgtag ttatctacac 4920
   gacggggagt caggcaacta tggatgaacg aaatagacag atcgctgaga taggtgcctc 4980
   actgattaag cattggtaac tgtcagacca agtttactca tatatacttt agattgattt 5040
   aaaacttcat ttttaattta aaaggatcta ggtgaagatc ctttttgata atctcatgac 5100
   caaaatccct taacgtgagt tttcgttcca ctgagcgtca gaccccgtag aaaagatcaa 5160
   aggatcttct tgagatcctt tttttctgcg cgtaatctgc tgcttgcaaa caaaaaaacc 5220
   accgctacca gcggtggttt gtttgccgga tcaagagcta ccaactcttt ttccgaaggt 5280
   aactggcttc agcagagcgc agataccaaa tactgtcctt ctagtgtagc cgtagttagg 5340
   ccaccacttc aagaactctg tagcaccgcc tacatacctc gctctgctaa tcctgttacc 5400
   agtggctgct gccagtggcg ataagtcgtg tcttaccggg ttggactcaa gacgatagtt 5460
   accggataag gcgcagcggt cgggctgaac ggggggttcg tgcacacagc ccagcttgga 5520
   gcgaacgacc tacaccgaac tgagatacct acagcgtgag ctatgagaaa gcgccacgct 5580
   tcccgaaggg agaaaggcgg acaggtatcc ggtaagcggc agggtcggaa caggagagcg 5640
   cacgagggag cttccagggg gaaacgcctg gtatctttat agtcctgtcg ggtttcgcca 5700
   cctctgactt gagcgtcgat ttttgtgatg ctcgtcaggg gggcggagcc tatggaaaaa 5760
   cgccagcaac gcggcctttt tacggttcct ggccttttgc tggccttttg ctcacatgtt 5820
   ctttcctgcg ttatcccctg attctgtgga taaccgtatt accgcctttg agtgagctga 5880
   taccgctcgc cgcagccgaa cgaccgagcg cagcgagtca gtgagcgagg aagcggaaga 5940
   gcgcccaata cgcaaaccgc ctctccccgc gcgttggccg attcattaat gcagctggca 6000
   cgacaggttt cccgactgga aagcgggcag tgagcgcaac gcaattaatg tgagttagct 6060
   cactcattag gcaccccagg ctttacactt tatgcttccg gctcgtatgt tgtgtggaat 6120
   tgtgagcgga taacaatttc acacaggaaa cagctatgac catgattacg ccaagctggc 6180
   gcgtcgagga attctaccgg gtaggggagg cgcttttccc aaggcagtct ggagcatgcg 6240
   ctttagcagc cccgctgggc acttggcgct acacaagtgg cctctggctc gcacacattc 6300
   cacatccacc ggtaggcgcc aaccggctcc gttctttggt ggccccttcg cgccaccttc 6360
   tactcctccc ctagtcagga agttcccccc cgccccgcag ctcgcgtcgt gcaggacgtg 6420
   acaaatggaa gtagcacgtc tcactagtct cgtgcagatg gacagcaccg ctgagcaatg 6480
   gaagcgggta ggcctttggg gcagcggcca atagcagctt tgctccttcg ctttctgggc 6540
   tcagaggctg ggaaggggtg ggtccggggg cgggctcagg ggcgggctca ggggcggggc 6600
   gggcgcccga aggtcctccg gaggcccggc attctgcacg cttcaaaagc gcacgtctgc 6660
   cgcgctgttc tcctcttcct catctccggg cctttcgacc tgcagcccgg tacagttcga 6720
   aggatccgcg ttaacaccta agaaggcgaa gttttcctta caccttgcag atataaagtg 6780
   tctaacagtt taaaatatcc gatgaggcat atttatgttg gacccgtagc tcagccagga 6840
   tagagcactg gcctccggag ccggaggtcc cgggttcaaa tcccggcggg tccgtatatt 6900
   actttttgat tcagattaga tttgtaaatc tttattacaa ggataatttg atcttgtata 6960
   ttggtaactc tctactctat aatttttatg agaaattcac agtcgtccct ttataccata 7020
   aatagctaag tttgtcaaag ttcttattaa actctccatg tagagattaa atcggatcct 7080
   tcgaataact tcgtatagca tacattatac gaagttataa gcttgcatgc ctgcaggtcg 7140
   gccgccacga ccggccggcc ggtgccgcca ccatcccctg acccacgccc ctgacccctc 7200
   acaaggagac gaccttccat gaccgagtac aagcccacgg tgcgcctcgc cacccgcgac 7260
   gacgtccccc gggccgtacg caccctcgcc gccgcgttcg ccgactaccc cgccacgcgc 7320
   cacaccgtcg acccggaccg ccacatcgag cgggtcaccg agctgcaaga actcttcctc 7380
   acgcgcgtcg ggctcgacat cggcaaggtg tgggtcgcgg acgacggcgc cgcggtggcg 7440
   gtctggacca cgccggagag cgtcgaagcg ggggcggtgt tcgccgagat cggcccgcgc 7500
   atggccgagt tgagcggttc ccggctggcc gcgcagcaac agatggaagg cctcctggcg 7560
   ccgcaccggc ccaaggagcc cgcgtggttc ctggccaccg tcggcgtctc gcccgaccac 7620
   cagggcaagg gtctgggcag cgccgtcgtg ctccccggag tggaggcggc cgagcgcgcc 7680
   ggggtgcccg ccttcctgga gacctccgcg ccccgcaacc tccccttcta cgagcggctc 7740
   ggcttcaccg tcaccgccga cgtcgagtgc ccgaaggacc gcgcgacctg gtgcatgacc 7800
   cgcaagcccg gtgcctgacg cccgccccac gacccgcagc gcccgaccga aaggagcgca 7860
   cgaccccatg gctccgaccg aagccgaccc gggcggcccc gccgaccccg cacccgcccc 7920
   cgaggcccac cgactctaga ggatcataat cagccatacc acatttgtag aggttttact 7980
   tgctttaaaa aacctcccac acctccccct gaacctgaaa cataaaatga atgcaattgt 8040
   tgttgttaac ttgtttattg cagcttataa tggttacaaa taaagcaata gcatcacaaa 8100
   tttcacaaat aaagcatttt tttcactgca ttctagttgt ggtttgtcca aactcatcaa 8160
   tgtatct                                                           8167
<210> 84
   <211> 51
   <212> DNA
   <213> Artificial Sequence
<220>
   <223> Description of Artificial Sequence: primer XisA1
<400> 84
   ataagaatgc ggccgcccga tatgcaaaat cagggtcaag acaaatatca a          51
<210> 85
   <211> 76
   <212> DNA
   <213> Artificial Sequence
<220>
   <223> Description of Artificial Sequence: primer XisA2
<400> 85
   ataagaatgc ggccgcacca tgcccaagaa gaagaggaag gtgcaaaatc agggtcaaga 60
   caaatatcaa caagcc 76
<210> 86
   <211> 44
   <212> DNA
   <213> Artificial Sequence
<220>
   <223> Description of Artificial Sequence: primer XisA3
<400> 86
   ataagaatgc ggccgctcaa ctattcttat aagctatttc catc 44
<210> 87
   <211> 82
   <212> DNA
   <213> Artificial Sequence
<220>
   <223> Description of Artificial Sequence: primer nifD1
<400> 87
   cgatggctct tcccttccgt caaatgcact cttgggatta ctccgaacct agcgatgggg 60
   tgcaaatgtc agatcagata ag 82
<210> 88
   <211> 82
   <212> DNA
   <213> Artificial Sequence
<220>
   <223> Description of Artificial Sequence: primer nifD2
<400> 88
   cgcttatctg atctgacatt tgcaccccat cgctaggttc ggagtaatcc caagagtgca 60
   tttgacggaa gggaagagcc at 82
<210> 89
   <211> 74
   <212> DNA
   <213> Artificial Sequence
<220>
   <223> Description of Artificial Sequence: primer nifD3
<400> 89
   gatcagctgt tgaaagctat taaaccacaa aaaggattac tccggccctt atcacggtta 60
   cgacggattt gcta 74
<210> 90
   <211> 74
   <212> DNA
   <213> Artificial Sequence
<220>
   <223> Description of Artificial Sequence: primer nifD4
<400> 90
   gatctagcaa atccgtcgta accgtgataa gggccggagt aatccttttt gtggtttaat 60
   agctttcaac agct 74
<210> 91
   <211> 41
   <212> DNA
   <213> Artificial Sequence
<220>
   <223> Description of Artificial Sequence: primerSSV1-1
<400> 91
   ataagaatgc ggccgcccga tatgacgaaa gataagacgc g 41
<210> 92
   <211> 70
   <212> DNA
   <213> Artificial Sequence
<220>
   <223> Description of Artificial Sequence: primer SSV1-2
<400> 92
   ataagaatgc ggccgcacca tgcccaagaa gaagaggaag gtgacgaaag ataagacgcg 60
   ttataaatac 70
<210> 93
   <211> 47
   <212> DNA
   <213> Artificial Sequence
<220>
   <223> Description of Artificial Sequence: primer SSV2
<400> 93
   tgtcccgggc tcgaaaccgg ggggatccgc ttgtagggga gtatccc 47
<210> 94
   <211> 47
   <212> DNA
   <213> Artificial Sequence
<220>
   <223> Description of Artificial Sequence: primer SSV3
<400> 94
   gagcccggga caagcggaag cggtggtgga aaagagggaa ctgaacg 47
<210> 95
   <211> 33
   <212> DNA
   <213> Artificial Sequence
<220>
   <223> Description of Artificial Sequence: primer SSV4
<400> 95
   atcgctcgag tcagacccct tttagccatt ccg 33
<210> 96
   <211> 40
   <212> DNA
   <213> Artificial Sequence
<220>
   <223> Description of Artificial Sequence: primer SSV5
<400> 96
   atcgttcgaa ggatccgcgt taacacctaa gaaggcgaag 40
<210> 97
   <211> 38
   <212> DNA
   <213> Artificial Sequence
<220>
   <223> Description of Artificial Sequence: primer SSV6
<400> 97
   atcgttcgaa ggatccgatt taatctctac atggagag 38
<210> 98
   <211> 64
   <212> DNA
   <213> Artificial Sequence
<220>
   <223> Description of Artificial Sequence: primer C31-2
<400> 98
   ataagaatgc ggccgcacca tgcccaagaa gaagaggaag gtgacacaag gggttgtgac 60
   cggg 64
<210> 99
   <211> 4831
   <212> DNA
   <213> Artificial Sequence
<220>
   <223> Description of Artificial Sequence: vector pRK41
<400> 99
   ggccgcacca tgcccaagaa gaagaggaag gtgacacaag gggttgtgac cggggtggac 60
   acgtacgcgg gtgcttacga ccgtcagtcg cgcgagcgcg agaattcgag cgcagcaagc 120
   ccagcgacac agcgtagcgc caacgaagac aaggcggccg accttcagcg cgaagtcgag 180
   cgcgacgggg gccggttcag gttcgtcggg catttcagcg aagcgccggg cacgtcggcg 240
   ttcgggacgg cggagcgccc ggagttcgaa cgcatcctga acgaatgccg cgccgggcgg 300
   ctcaacatga tcattgtcta tgacgtgtcg cgcttctcgc gcctgaaggt catggacgcg 360
   attccgattg tctcggaatt gctcgccctg ggcgtgacga ttgtttccac tcaggaaggc 420
   gtcttccggc agggaaacgt catggacctg attcacctga ttatgcggct cgacgcgtcg 480
   cacaaagaat cttcgctgaa gtcggcgaag attctcgaca cgaagaacct tcagcgcgaa 540
   ttgggcgggt acgtcggcgg gaaggcgcct tacggcttcg agcttgtttc ggagacgaag 600
   gagatcacgc gcaacggccg aatggtcaat gtcgtcatca acaagcttgc gcactcgacc 660
   actcccctta ccggaccctt cgagttcgag cccgacgtaa tccggtggtg gtggcgtgag 720
   atcaagacgc acaaacacct tcccttcaag ccgggcagtc aagccgccat tcacccgggc 780
   agcatcacgg ggctttgtaa gcgcatggac gctgacgccg tgccgacccg gggcgagacg 840
   attgggaaga agaccgcttc aagcgcctgg gacccggcaa ccgttatgcg aatccttcgg 900
   gacccgcgta ttgcgggctt cgccgctgag gtgatctaca agaagaagcc ggacggcacg 960
   ccgaccacga agattgaggg ttaccgcatt cagcgcgacc cgatcacgct ccggccggtc 1020
   gagcttgatt gcggaccgat catcgagccc gctgagtggt atgagcttca ggcgtggttg 1080
   gacggcaggg ggcgcggcaa ggggctttcc cgggggcaag ccattctgtc cgccatggac 1140
   aagctgtact gcgagtgtgg cgccgtcatg acttcgaagc gcggggaaga atcgatcaag 1200
   gactcttacc gctgccgtcg ccggaaggtg gtcgacccgt ccgcacctgg gcagcacgaa 1260
   ggcacgtgca acgtcagcat ggcggcactc gacaagttcg ttgcggaacg catcttcaac 1320
   aagatcaggc acgccgaagg cgacgaagag acgttggcgc ttctgtggga agccgcccga 1380
   cgcttcggca agctcactga ggcgcctgag aagagcggcg aacgggcgaa ccttgttgcg 1440
   gagcgcgccg acgccctgaa cgcccttgaa gagctgtacg aagaccgcgc ggcaggcgcg 1500
   tacgacggac ccgttggcag gaagcacttc cggaagcaac aggcagcgct gacgctccgg 1560
   cagcaagggg cggaagagcg gcttgccgaa cttgaagccg ccgaagcccc gaagcttccc 1620
   cttgaccaat ggttccccga agacgccgac gctgacccga ccggccctaa gtcgtggtgg 1680
   gggcgcgcgt cagtagacga caagcgcgtg ttcgtcgggc tcttcgtaga caagatcgtt 1740
   gtcacgaagt cgactacggg cagggggcag ggaacgccca tcgagaagcg cgcttcgatc 1800
   acgtgggcga agccgccgac cgacgacgac gaagacgacg cccaggacgg cacggaagac 1860
   gtagcggcgt agcggccgct ctagaactag tggatccccc gggctgcagg aattcgatat 1920
   caagcttatc gataccgtcg acctcgaggg ggggcccggt acccagcttt tgttcccttt 1980
   agtgagggtt aatttcgagc ttggcgtaat catggtcata gctgtttcct gtgtgaaatt 2040
   gttatccgct cacaattcca cacaacatac gagccggaag cataaagtgt aaagcctggg 2100
   gtgcctaatg agtgagctaa ctcacattaa ttgcgttgcg ctcactgccc gctttccagt 2160
   cgggaaacct gtcgtgccag ctgcattaat gaatcggcca acgcgcgggg agaggcggtt 2220
   tgcgtattgg gcgctcttcc gcttcctcgc tcactgactc gctgcgctcg gtcgttcggc 2280
   tgcggcgagc ggtatcagct cactcaaagg cggtaatacg gttatccaca gaatcagggg 2340
   ataacgcagg aaagaacatg tgagcaaaag gccagcaaaa ggccaggaac cgtaaaaagg 2400
   ccgcgttgct ggcgtttttc cataggctcc gcccccctga cgagcatcac aaaaatcgac 2460
   gctcaagtca gaggtggcga aacccgacag gactataaag ataccaggcg tttccccctg 2520
   gaagctccct cgtgcgctct cctgttccga ccctgccgct taccggatac ctgtccgcct 2580
   ttctcccttc gggaagcgtg gcgctttctc atagctcacg ctgtaggtat ctcagttcgg 2640
   tgtaggtcgt tcgctccaag ctgggctgtg tgcacgaacc ccccgttcag cccgaccgct 2700
   gcgccttatc cggtaactat cgtcttgagt ccaacccggt aagacacgac ttatcgccac 2760
   tggcagcagc cactggtaac aggattagca gagcgaggta tgtaggcggt gctacagagt 2820
   tcttgaagtg gtggcctaac tacggctaca ctagaaggac agtatttggt atctgcgctc 2880
   tgctgaagcc agttaccttc ggaaaaagag ttggtagctc ttgatccggc aaacaaacca 2940
   ccgctggtag cggtggtttt tttgtttgca agcagcagat tacgcgcaga aaaaaaggat 3000
   ctcaagaaga tcctttgatc ttttctacgg ggtctgacgc tcagtggaac gaaaactcac 3060
   gttaagggat tttggtcatg agattatcaa aaaggatctt cacctagatc cttttaaatt 3120
   aaaaatgaag ttttaaatca atctaaagta tatatgagta aacttggtct gacagttacc 3180
   aatgcttaat cagtgaggca cctatctcag cgatctgtct atttcgttca tccatagttg 3240
   cctgactccc cgtcgtgtag ataactacga tacgggaggg cttaccatct ggccccagtg 3300
   ctgcaatgat accgcgagac ccacgctcac cggctccaga tttatcagca ataaaccagc 3360
   cagccggaag ggccgagcgc agaagtggtc ctgcaacttt atccgcctcc atccagtcta 3420
   ttaattgttg ccgggaagct agagtaagta gttcgccagt taatagtttg cgcaacgttg 3480
   ttgccattgc tacaggcatc gtggtgtcac gctcgtcgtt tggtatggct tcattcagct 3540
   ccggttccca acgatcaagg cgagttacat gatcccccat gttgtgcaaa aaagcggtta 3600
   gctccttcgg tcctccgatc gttgtcagaa gtaagttggc cgcagtgtta tcactcatgg 3660
   ttatggcagc actgcataat tctcttactg tcatgccatc cgtaagatgc ttttctgtga 3720
   ctggtgagta ctcaaccaag tcattctgag aatagtgtat gcggcgaccg agttgctctt 3780
   gcccggcgtc aatacgggat aataccgcgc cacatagcag aactttaaaa gtgctcatca 3840
   ttggaaaacg ttcttcgggg cgaaaactct caaggatctt accgctgttg agatccagtt 3900
   cgatgtaacc cactcgtgca cccaactgat cttcagcatc ttttactttc accagcgttt 3960
   ctgggtgagc aaaaacagga aggcaaaatg ccgcaaaaaa gggaataagg gcgacacgga 4020
   aatgttgaat actcatactc ttcctttttc aatattattg aagcatttat cagggttatt 4080
   gtctcatgag cggatacata tttgaatgta tttagaaaaa taaacaaata ggggttccgc 4140
   gcacatttcc ccgaaaagtg ccacctaaat tgtaagcgtt aatattttgt taaaattcgc 4200
   gttaaatttt tgttaaatca gctcattttt taaccaatag gccgaaatcg gcaaaatccc 4260
   ttataaatca aaagaataga ccgagatagg gttgagtgtt gttccagttt ggaacaagag 4320
   tccactatta aagaacgtgg actccaacgt caaagggcga aaaaccgtct atcagggcga 4380
   tggcccacta cgtgaaccat caccctaatc aagttttttg gggtcgaggt gccgtaaagc 4440
   actaaatcgg aaccctaaag ggagcccccg atttagagct tgacggggaa agccggcgaa 4500
   cgtggcgaga aaggaaggga agaaagcgaa aggagcgggc gctagggcgc tggcaagtgt 4560
   agcggtcacg ctgcgcgtaa ccaccacacc cgccgcgctt aatgcgccgc tacagggcgc 4620
   gtcccattcg ccattcaggc tgcgcaactg ttgggaaggg cgatcggtgc gggcctcttc 4680
   gctattacgc cagctggcga aagggggatg tgctgcaagg cgattaagtt gggtaacgcc 4740
   agggttttcc cagtcacgac gttgtaaaac gacggccagt gaattgtaat acgactcact 4800
   atagggcgaa ttggagctcc accgcggtgg c                                4831
<210> 100
   <211> 20
   <212> DNA
   <213> Artificial Sequence
<220>
   <223> Description of Artificial Sequence: primer C31-screen 1
<400> 100
   gcgtgagatc aagacgcaca 20
<210> 101
   <211> 21
   <212> DNA
   <213> Artificial Sequence
<220>
   <223> Description of Artificial Sequence: primer C31-screen 2
<400> 101
   gcagcggtaa gagtccttga t 21
<210> 102
   <211> 20
   <212> DNA
   <213> Artificial Sequence
<220>
   <223> Description of Artificial Sequence: primer beta-Gal 3
<400> 102
   atcctctgca tggtcaggtc 20
<210> 103
   <211> 18
   <212> DNA
   <213> Artificial Sequence
<220>
   <223> Description of Artificial Sequence: primer beta-Gal 4
<400> 103
   cgtggcctga ttcattcc 18
<210> 104
   <211> 5878
   <212> DNA
   <213> Artificial Sequence
<220>
   <223> Description of Artificial Sequence: vector pCAGGS-Cre-pA
<400> 104
<210> 105
   <211> 6641
   <212> DNA
   <213> Artificial Sequence
<220>
   <223> Description of Artificial Sequence: vector pCAGGSC31CNLS-pA
<400> 105
   attgattatt gactagttat taatagtaat caattacggg gtcattagtt catagcccat 60
   atatggagtt ccgcgttaca taacttacgg taaatggccc gcctggctga ccgcccaacg 120
   acccccgccc attgacgtca ataatgacgt atgttcccat agtaacgcca atagggactt 180
   tccattgacg tcaatgggtg gactatttac ggtaaactgc ccacttggca gtacatcaag 240
   tgtatcatat gccaagtacg ccccctattg acgtcaatga cggtaaatgg cccgcctggc 300
   attatgccca gtacatgacc ttatgggact ttcctacttg gcagtacatc tacgtattag 360
   tcatcgctat taccatgggt cgaggtgagc cccacgttct gcttcactct ccccatctcc 420
   cccccctccc cacccccaat tttgtattta tttatttttt aattattttg tgcagcgatg 480
   ggggcggggg gggggggggc gcgcgccagg cggggcgggg cggggcgagg ggcggggcgg 540
   ggcgaggcgg agaggtgcgg cggcagccaa tcagagcggc gcgctccgaa agtttccttt 600
   tatggcgagg cggcggcggc ggcggcccta taaaaagcga agcgcgcggc gggcgggagt 660
   cgctgcgttg ccttcgcccc gtgccccgct ccgcgccgcc tcgcgccgcc cgccccggct 720
   ctgactgacc gcgttactcc cacaggtgag cgggcgggac ggcccttctc ctccgggctg 780
   taattagcgc ttggtttaat gacggctcgt ttcttttctg tggctgcgtg aaagccttaa 840
   agggctccgg gagggccctt tgtgcggggg ggagcggctc ggggggtgcg tgcgtgtgtg 900
   tgtgcgtggg gagcgccgcg tgcggcccgc gctgcccggc ggctgtgagc gctgcgggcg 960
   cggcgcgggg ctttgtgcgc tccgcgtgtg cgcgagggga gcgcggccgg gggcggtgcc 1020
   ccgcggtgcg ggggggctgc gaggggaaca aaggctgcgt gcggggtgtg tgcgtggggg 1080
   ggtgagcagg gggtgtgggc gcggcggtcg ggctgtaacc cccccctgca cccccctccc 1140
   cgagttgctg agcacggccc ggcttcgggt gcggggctcc gtgcggggcg tggcgcgggg 1200
   ctcgccgtgc cgggcggggg gtggcggcag gtgggggtgc cgggcggggc ggggccgcct 1260
   cgggccgggg agggctcggg ggaggggcgc ggcggccccg gagcgccggc ggctgtcgag 1320
   gcgcggcgag ccgcagccat tgccttttat ggtaatcgtg cgagagggcg cagggacttc 1380
   ctttgtccca aatctggcgg agccgaaatc tgggaggcgc cgccgcaccc cctctagcgg 1440
   gcgcgggcga agcggtgcgg cgccggcagg aaggaaatgg gcggggaggg ccttcgtgcg 1500
   tcgccgcgcc gccgtcccct tctccatctc cagcctcggg gctgccgcag ggggacggct 1560
   gccttcgggg gggacggggc agggcggggt tcggcttctg gcgtgtgacc ggcggctcta 1620
   gtaagcgttg gggtgagtac tccctctcaa aagcgggcat gacttctgcg ctaagattgt 1680
   cagtttccaa aaacgaggag gatttgatat tcacctggcc cgcggtgatg cctttgaggg 1740
   tggccgcgtc catctggtca gaaaagacaa tctttttgtt gtcaagcttg aggtgtggca 1800
   ggcttgagat ctggccatac acttgagtga cattgacatc cactttgcct ttctctccac 1860
   aggtgtccac tcccagggcg gccgcccgat atgacacaag gggttgtgac cggggtggac 1920
   acgtacgcgg gtgcttacga ccgtcagtcg cgcgagcgcg agaattcgag cgcagcaagc 1980
   ccagcgacac agcgtagcgc caacgaagac aaggcggccg accttcagcg cgaagtcgag 2040
   cgcgacgggg gccggttcag gttcgtcggg catttcagcg aagcgccggg cacgtcggcg 2100
   ttcgggacgg cggagcgccc ggagttcgaa cgcatcctga acgaatgccg cgccgggcgg 2160
   ctcaacatga tcattgtcta tgacgtgtcg cgcttctcgc gcctgaaggt catggacgcg 2220
   attccgattg tctcggaatt gctcgccctg ggcgtgacga ttgtttccac tcaggaaggc 2280
   gtcttccggc agggaaacgt catggacctg attcacctga ttatgcggct cgacgcgtcg 2340
   cacaaagaat cttcgctgaa gtcggcgaag attctcgaca cgaagaacct tcagcgcgaa 2400
   ttgggcgggt acgtcggcgg gaaggcgcct tacggcttcg agcttgtttc ggagacgaag 2460
   gagatcacgc gcaacggccg aatggtcaat gtcgtcatca acaagcttgc gcactcgacc 2520
   actcccctta ccggaccctt cgagttcgag cccgacgtaa tccggtggtg gtggcgtgag 2580
   atcaagacgc acaaacacct tcccttcaag ccgggcagtc aagccgccat tcacccgggc 2640
   agcatcacgg ggctttgtaa gcgcatggac gctgacgccg tgccgacccg gggcgagacg 2700
   attgggaaga agaccgcttc aagcgcctgg gacccggcaa ccgttatgcg aatccttcgg 2760
   gacccgcgta ttgcgggctt cgccgctgag gtgatctaca agaagaagcc ggacggcacg 2820
   ccgaccacga agattgaggg ttaccgcatt cagcgcgacc cgatcacgct ccggccggtc 2880
   gagcttgatt gcggaccgat catcgagccc gctgagtggt atgagcttca ggcgtggttg 2940
   gacggcaggg ggcgcggcaa ggggctttcc cgggggcaag ccattctgtc cgccatggac 3000
   aagctgtact gcgagtgtgg cgccgtcatg acttcgaagc gcggggaaga atcgatcaag 3060
   gactcttacc gctgccgtcg ccggaaggtg gtcgacccgt ccgcacctgg gcagcacgaa 3120
   ggcacgtgca acgtcagcat ggcggcactc gacaagttcg ttgcggaacg catcttcaac 3180
   aagatcaggc acgccgaagg cgacgaagag acgttggcgc ttctgtggga agccgcccga 3240
   cgcttcggca agctcactga ggcgcctgag aagagcggcg aacgggcgaa ccttgttgcg 3300
   gagcgcgccg acgccctgaa cgcccttgaa gagctgtacg aagaccgcgc ggcaggcgcg 3360
   tacgacggac ccgttggcag gaagcacttc cggaagcaac aggcagcgct gacgctccgg 3420
   cagcaagggg cggaagagcg gcttgccgaa cttgaagccg ccgaagcccc gaagcttccc 3480
   cttgaccaat ggttccccga agacgccgac gctgacccga ccggccctaa gtcgtggtgg 3540
   gggcgcgcgt cagtagacga caagcgcgtg ttcgtcgggc tcttcgtaga caagatcgtt 3600
   gtcacgaagt cgactacggg cagggggcag ggaacgccca tcgagaagcg cgcttcgatc 3660
   acgtgggcga agccgccgac cgacgacgac gaagacgacg cccaggacgg cacggaagac 3720
   gtagcggcgc ctaagaagaa gaggaaggtt tagactctcg agatccaggc gcggatcaat 3780
   aaaagatcat tattttcaat agatctgtgt gttggttttt tgtgtgcctt gggggagggg 3840
   gaggccagaa tgaggcgcgg ccaaggggga gggggaggcc agaatgacct tgggggaggg 3900
   ggaggccaga atgaccttgg gggaggggga ggccagaatg aggcgcgccc ccgggtaccg 3960
   agctcgaatt cactggccgt cgttttacaa cgtcgtgact gggaaaaccc tggcgttacc 4020
   caacttaatc gccttgcagc acatccccct ttcgccagct ggcgtaatag cgaagaggcc 4080
   cgcaccgatc gcccttccca acagttgcgc agcctgaatg gcgaatggcg cctgatgcgg 4140
   tattttctcc ttacgcatct gtgcggtatt tcacaccgca tatggtgcac tctcagtaca 4200
   atctgctctg atgccgcata gttaagccag ccccgacacc cgccaacacc cgctgacgcg 4260
   ccctgacggg cttgtctgct cccggcatcc gcttacagac aagctgtgac cgtctccggg 4320
   agctgcatgt gtcagaggtt ttcaccgtca tcaccgaaac gcgcgagacg aaagggcctc 4380
   gtgatacgcc tatttttata ggttaatgtc atgataataa tggtttctta gacgtcaggt 4440
   ggcacttttc ggggaaatgt gcgcggaacc cctatttgtt tatttttcta aatacattca 4500
   aatatgtatc cgctcatgag acaataaccc tgataaatgc ttcaataata ttgaaaaagg 4560
   aagagtatga gtattcaaca tttccgtgtc gcccttattc ccttttttgc ggcattttgc 4620
   cttcctgttt ttgctcaccc agaaacgctg gtgaaagtaa aagatgctga agatcagttg 4680
   ggtgcacgag tgggttacat cgaactggat ctcaacagcg gtaagatcct tgagagtttt 4740
   cgccccgaag aacgttttcc aatgatgagc acttttaaag ttctgctatg tggcgcggta 4800
   ttatcccgta ttgacgccgg gcaagagcaa ctcggtcgcc gcatacacta ttctcagaat 4860
   gacttggttg agtactcacc agtcacagaa aagcatctta cggatggcat gacagtaaga 4920
   gaattatgca gtgctgccat aaccatgagt gataacactg cggccaactt acttctgaca 4980
   acgatcggag gaccgaagga gctaaccgct tttttgcaca acatggggga tcatgtaact 5040
   cgccttgatc gttgggaacc ggagctgaat gaagccatac caaacgacga gcgtgacacc 5100
   acgatgcctg tagcaatggc aacaacgttg cgcaaactat taactggcga actacttact 5160
   ctagcttccc ggcaacaatt aatagactgg atggaggcgg ataaagttgc aggaccactt 5220
   ctgcgctcgg cccttccggc tggctggttt attgctgata aatctggagc cggtgagcgt 5280
   gggtctcgcg gtatcattgc agcactgggg ccagatggta agccctcccg tatcgtagtt 5340
   atctacacga cggggagtca ggcaactatg gatgaacgaa atagacagat cgctgagata 5400
   ggtgcctcac tgattaagca ttggtaactg tcagaccaag tttactcata tatactttag 5460
   attgatttaa aacttcattt ttaatttaaa aggatctagg tgaagatcct ttttgataat 5520
   ctcatgacca aaatccctta acgtgagttt tcgttccact gagcgtcaga ccccgtagaa 5580
   aagatcaaag gatcttcttg agatcctttt tttctgcgcg taatctgctg cttgcaaaca 5640
   aaaaaaccac cgctaccagc ggtggtttgt ttgccggatc aagagctacc aactcttttt 5700
   ccgaaggtaa ctggcttcag cagagcgcag ataccaaata ctgtccttct agtgtagccg 5760
   tagttaggcc accacttcaa gaactctgta gcaccgccta catacctcgc tctgctaatc 5820
   ctgttaccag tggctgctgc cagtggcgat aagtcgtgtc ttaccgggtt ggactcaaga 5880
   cgatagttac cggataaggc gcagcggtcg ggctgaacgg ggggttcgtg cacacagccc 5940
   agcttggagc gaacgaccta caccgaactg agatacctac agcgtgagct atgagaaagc 6000
   gccacgcttc ccgaagggag aaaggcggac aggtatccgg taagcggcag ggtcggaaca 6060
   ggagagcgca cgagggagct tccaggggga aacgcctggt atctttatag tcctgtcggg 6120
   tttcgccacc tctgacttga gcgtcgattt ttgtgatgct cgtcaggggg gcggagccta 6180
   tggaaaaacg ccagcaacgc ggccttttta cggttcctgg ccttttgctg gccttttgct 6240
   cacatgttct ttcctgcgtt atcccctgat tctgtggata accgtattac cgcctttgag 6300
   tgagctgata ccgctcgccg cagccgaacg accgagcgca gcgagtcagt gagcgaggaa 6360
   gcggaagagc gcccaatacg caaaccgcct ctccccgcgc gttggccgat tcattaatgc 6420
   agctggcacg acaggtttcc cgactggaaa gcgggcagtg agcgcaacgc aattaatgtg 6480
   agttagctca ctcattaggc accccaggct ttacacttta tgcttccggc tcgtatgttg 6540
   tgtggaattg tgagcggata acaatttcac acaggaaaca gctatgacca tgattacgcc 6600
   aagctagccc gggctagctt gcatgcctgc aggttttcga c 6641
<210> 106
   <211> 11784
   <212> DNA
   <213> Artificial Sequence
<220>
   <223> Description of Artificial Sequence: modified ROSA26 locus
<400> 106
   ggcaggccct ccgagcgtgg tggagccgtt ctgtgagaca gccgggtacg agtcgtgacg 60
   ctggaagggg caagcgggtg gtgggcagga atgcggtccg ccctgcagca accggagggg 120
   gagggagaag ggagcggaaa agtctccacc ggacgcggcc atggctcggg gggggggggg 180
   cagcggagga gcgcttccgg ccgacgtctc gtcgctgatt ggcttctttt cctcccgccg 240
   tgtgtgaaaa cacaaatggc gtgttttggt tggcgtaagg cgcctgtcag ttaacggcag 300
   ccggagtgcg cagccgccgg cagcctcgct ctgcccactg ggtggggcgg gaggtaggtg 360
   gggtgaggcg agctggacgt gcgggcgcgg tcggcctctg gcggggcggg ggaggggagg 420
   gagggtcagc gaaagtagct cgcgcgcgag cggccgccca ccctcccctt cctctggggg 480
   agtcgtttta cccgccgccg gccgggcctc gtcgtctgat tggctctcgg ggcccagaaa 540
   actggccctt gccattggct cgtgttcgtg caagttgagt ccatccgccg gccagcgggg 600
   gcggcgagga ggcgctccca ggttccggcc ctcccctcgg ccccgcgccg cagagtctgg 660
   ccgcgcgccc ctgcgcaacg tggcaggaag cgcgcgctgg gggcggggac gggcagtagg 720
   gctgagcggc tgcgggcggg gtgcaagcac gtttccgact tgagttgcct caagaggggc 780
   gtgctgagcc agacctccat cgcgcactcc ggggagtgga gggaaggagc gagggctcag 840
   ttgggctgtt ttggaggcag gaagcacttg ctctcccaaa gtcgctctga gttgttatca 900
   gtaagggagc tgcagtggag taggcgggga gaaggccgca cccttctccg gaggggggag 960
   gggagtgttg caataccttt ctgggagttc tctgctgcct cctggcttct gaggaccgcc 1020
   ctgggcctgg gagaatccct tccccctctt ccctcgtgat ctgcaactcc agtctttcgc 1080
   ctaggtaacc gatatccctg caggggtgac ctgcacgtct agggcgcagt agtccagggt 1140
   ttccttgatg atgtcatact tatcctgtcc cttttttttc cacagctcgc ggttgaggac 1200
   aaactcttcg cggtctttcc agtactcctg caggtgactg actgagtcga cgacactgca 1260
   gagacctact tcactaacaa ccggtacagt tcgtggacca gatgggtgag gtggagtacg 1320
   cgcccgggga gcccaagggc acgccctggc acccgcaccg cggcttcgag accgtcacga 1380
   ataacttcgt atagcataca ttatacgaag ttataagctc gatgaattct accgggtagg 1440
   ggaggcgctt ttcccaaggc agtctggagc atgcgcttta gcagccccgc tggcacttgg 1500
   cgctacacaa gtggcctctg gcctcgcaca cattccacat ccaccggtag cgccaaccgg 1560
   ctccgttctt tggtggcccc ttcgcgccac cttctactcc tcccctagtc aggaagttcc 1620
   cccccgcccc gcagctcgcg tcgtgcagga cgtgacaaat ggaagtagca cgtctcacta 1680
   gtctcgtgca gatggacagc accgctgagc aatggaagcg ggtaggcctt tggggcagcg 1740
   gccaatagca gctttgctcc ttcgctttct gggctcagag gctgggaagg ggtgggtccg 1800
   ggggcgggct caggggcggg ctcaggggcg gggcgggcgc gaaggtcctc ccgaggcccg 1860
   gcattctcgc acgcttcaaa agcgcacgtc tgccgcgctg ttctcctctt cctcatctcc 1920
   gggcctttcg acgatccagc cgccaccatg aaaaagcctg aactcaccgc gacgtctgtc 1980
   gagaagtttc tgatcgaaaa gttcgacagc gtctccgacc tgatgcagct ctcggagggc 2040
   gaagaatctc gtgctttcag cttcgatgta ggagggcgtg gatatgtcct gcgggtaaat 2100
   agctgcgccg atggtttcta caaagatcgt tatgtttatc ggcactttgc atcggccgcg 2160
   ctcccgattc cggaagtgct tgacattggg gaattcagcg agagcctgac ctattgcatc 2220
   tcccgccgtg cacagggtgt cacgttgcaa gacctgcctg aaaccgaact gcccgctgtt 2280
   ctgcagccgg tcgcggaggc catggatgcg atcgctgcgg ccgatcttag ccagacgagc 2340
   gggttcggcc cattcggacc gcaaggaatc ggtcaataca ctacatggcg tgatttcata 2400
   tgcgcgattg ctgatcccca tgtgtatcac tggcaaactg tgatggacga caccgtcagt 2460
   gcgtccgtcg cgcaggctct cgatgagctg atgctttggg ccgaggactg ccccgaagtc 2520
   cggcacctcg tgcacgcgga tttcggctcc aacaatgtcc tgacggacaa tggccgcata 2580
   acagcggtca ttgactggag cgaggcgatg ttcggggatt cccaatacga ggtcgccaac 2640
   atcttcttct ggaggccgtg gttggcttgt atggagcagc agacgcgcta cttcgagcgg 2700
   aggcatccgg agcttgcagg atcgccgcgg ctccgggcgt atatgctccg cattggtctt 2760
   gaccaactct atcagagctt ggttgacggc aatttcgatg atgcagcttg ggcgcagggt 2820
   cgatgcgacg caatcgtccg atccggagcc gggactgtcg ggcgtacaca aatcgcccgc 2880
   agaagcgcgg ccgtctggac cgatggctgt gtagaagtac tcgccgatag tggaaaccga 2940
   cgccccagca ctcgtccgag ggcaaaggaa tagtcgatgc agaaattgat gatctattaa 3000
   acaataaaga tgtccactaa aatggaagtt tttcctgtca tactttgtta agaagggtga 3060
   gaacagagta cctacatttt gaatggaagg attggagcta cgggggtggg ggtggggtgg 3120
   gattagataa atgcctgctc tttactgaag gctctttact attgctttat gataatgttt 3180
   catagttgga tatcataatt taaacaagca aaaccaaatt aagggccagc tcattcctcc 3240
   cactcatgat ctatagatct atagatctct cgtgggatca ttgtttttct cttgattccc 3300
   actttgtggt tctaagtact gtggtttcca aatgtgtcag tttcatagcc tgaagaacga 3360
   gatcagcagc ctctgttcca catacacttc attctcagta ttgttttgcc aagttctaat 3420
   tccatcagaa gcttcagctg ctcgactaga ggatcataat cagccatacc acatttgtag 3480
   aggttttact tgctttaaaa aacctcccac acctccccct gaacctgaaa cataaaatga 3540
   atgcaattgt tgttgttaac ttgtttattg cagcttataa tggttacaaa taaagcaata 3600
   gcatcacaaa tttcacaaat aaagcatttt tttcactgca ttctagttgt ggtttgtcca 3660
   aactcatcaa tgtatcttat catgtctgga tccgtgtcat gtcggcgacc ctacgccccc 3720
   aactgagaga actcaaaggt taccccagtt ggggcactac tcccgaaaac cgcttctgga 3780
   tccataactt cgtatagcat acattatacg aagttatacc gggccaccat ggtcgcgagt 3840
   agcttggcac tggccgtcgt tttacaacgt cgtgactggg aaaaccctgg cgttacccaa 3900
   cttaatcgcc ttgcagcaca tccccctttc gccagctggc gtaatagcga agaggcccgc 3960
   accgatcgcc cttcccaaca gttgcgcagc ctgaatggcg aatggcgctt tgcctggttt 4020
   ccggcaccag aagcggtgcc ggaaagctgg ctggagtgcg atcttcctga ggccgatact 4080
   gtcgtcgtcc cctcaaactg gcagatgcac ggttacgatg cgcccatcta caccaacgta 4140
   acctatccca ttacggtcaa tccgccgttt gttcccacgg agaatccgac gggttgttac 4200
   tcgctcacat ttaatgttga tgaaagctgg ctacaggaag gccagacgcg aattattttt 4260
   gatggcgtta actcggcgtt tcatctgtgg tgcaacgggc gctgggtcgg ttacggccag 4320
   gacagtcgtt tgccgtctga atttgacctg agcgcatttt tacgcgccgg agaaaaccgc 4380
   ctcgcggtga tggtgctgcg ttggagtgac ggcagttatc tggaagatca ggatatgtgg 4440
   cggatgagcg gcattttccg tgacgtctcg ttgctgcata aaccgactac acaaatcagc 4500
   gatttccatg ttgccactcg ctttaatgat gatttcagcc gcgctgtact ggaggctgaa 4560
   gttcagatgt gcggcgagtt gcgtgactac ctacgggtaa cagtttcttt atggcagggt 4620
   gaaacgcagg tcgccagcgg caccgcgcct ttcggcggtg aaattatcga tgagcgtggt 4680
   ggttatgccg atcgcgtcac actacgtctg aacgtcgaaa acccgaaact gtggagcgcc 4740
   gaaatcccga atctctatcg tgcggtggtt gaactgcaca ccgccgacgg cacgctgatt 4800
   gaagcagaag cctgcgatgt cggtttccgc gaggtgcgga ttgaaaatgg tctgctgctg 4860
   ctgaacggca agccgttgct gattcgaggc gttaaccgtc acgagcatca tcctctgcat 4920
   ggtcaggtca tggatgagca gacgatggtg caggatatcc tgctgatgaa gcagaacaac 4980
   tttaacgccg tgcgctgttc gcattatccg aaccatccgc tgtggtacac gctgtgcgac 5040
   cgctacggcc tgtatgtggt ggatgaagcc aatattgaaa cccacggcat ggtgccaatg 5100
   aatcgtctga ccgatgatcc gcgctggcta ccggcgatga gcgaacgcgt aacgcgaatg 5160
   gtgcagcgcg atcgtaatca cccgagtgtg atcatctggt cgctggggaa tgaatcaggc 5220
   cacggcgcta atcacgacgc gctgtatcgc tggatcaaat ctgtcgatcc ttcccgcccg 5280
   gtgcagtatg aaggcggcgg agccgacacc acggccaccg atattatttg cccgatgtac 5340
   gcgcgcgtgg atgaagacca gcccttcccg gctgtgccga aatggtccat caaaaaatgg 5400
   ctttcgctac ctggagagac gcgcccgctg atcctttgcg aatacgccca cgcgatgggt 5460
   aacagtcttg gcggtttcgc taaatactgg caggcgtttc gtcagtatcc ccgtttacag 5520
   ggcggcttcg tctgggactg ggtggatcag tcgctgatta aatatgatga aaacggcaac 5580
   ccgtggtcgg cttacggcgg tgattttggc gatacgccga acgatcgcca gttctgtatg 5640
   aacggtctgg tctttgccga ccgcacgccg catccagcgc tgacggaagc aaaacaccag 5700
   cagcagtttt tccagttccg tttatccggg caaaccatcg aagtgaccag cgaatacctg 5760
   ttccgtcata gcgataacga gctcctgcac tggatggtgg cgctggatgg taagccgctg 5820
   gcaagcggtg aagtgcctct ggatgtcgct ccacaaggta aacagttgat tgaactgcct 5880
   gaactaccgc agccggagag cgccgggcaa ctctggctca cagtacgcgt agtgcaaccg 5940
   aacgcgaccg catggtcaga agccgggcac atcagcgcct ggcagcagtg gcgtctggcg 6000
   gaaaacctca gtgtgacgct ccccgccgcg tcccacgcca tcccgcatct gaccaccagc 6060
   gaaatggatt tttgcatcga gctgggtaat aagcgttggc aatttaaccg ccagtcaggc 6120
   tttctttcac agatgtggat tggcgataaa aaacaactgc tgacgccgct gcgcgatcag 6180
   ttcacccgtg caccgctgga taacgacatt ggcgtaagtg aagcgacccg cattgaccct 6240
   aacgcctggg tcgaacgctg gaaggcggcg ggccattacc aggccgaagc agcgttgttg 6300
   cagtgcacgg cagatacact tgctgatgcg gtgctgatta cgaccgctca cgcgtggcag 6360
   catcagggga aaaccttatt tatcagccgg aaaacctacc ggattgatgg tagtggtcaa 6420
   atggcgatta ccgttgatgt tgaagtggcg agcgatacac cgcatccggc gcggattggc 6480
   ctgaactgcc agctggcgca ggtagcagag cgggtaaact ggctcggatt agggccgcaa 6540
   gaaaactatc ccgaccgcct tactgccgcc tgttttgacc gctgggatct gccattgtca 6600
   gacatgtata ccccgtacgt cttcccgagc gaaaacggtc tgcgctgcgg gacgcgcgaa 6660
   ttgaattatg gcccacacca gtggcgcggc gacttccagt tcaacatcag ccgctacagt 6720
   caacagcaac tgatggaaac cagccatcgc catctgctgc acgcggaaga aggcacatgg 6780
   ctgaatatcg acggtttcca tatggggatt ggtggcgacg actcctggag cccgtcagta 6840
   tcggcggaat tccagctgag cgccggtcgc taccattacc agttggtctg gtgtcaaaaa 6900
   taataataac cgggcagggg ggatctttgt gaaggaacct tacttctgtg gtgtgacata 6960
   attggacaaa ctacctacag agatttaaag ctctaaggta aatataaaat ttttaagtgt 7020
   ataatgtgtt aaactactga ttctaattgt ttgtgtattt tagattccaa cctatggaac 7080
   tgatgaatgg gagcagtggt ggaatgccag atccagacat gataagatac attgatgagt 7140
   ttggacaaac cacaactaga atgcagtgaa aaaaatgctt tatttgtgaa atttgtgatg 7200
   ctattgcttt atttgtaacc attataagct gcaataaaca agttaacaac aacaattgca 7260
   ttcattttat gtttcaggtt cagggggagg tgtgggaggt tttttaaagc aagtaaaacc 7320
   tctacaaatg tggtatggct gattatgatc tgcggccaaa tcggccggcc taggcgcgcc 7380
   ggtaaccgaa gttcctatac tttctagaga ataggaactt cggaatagga acttcaagct 7440
   taagcgctag cctagaagat gggcgggagt cttctgggca ggcttaaagg ctaacctggt 7500
   gtgtgggcgt tgtcctgcag gggaattgaa caggtgtaaa attggaggga caagacttcc 7560
   cacagatttt cggttttgtc gggaagtttt ttaatagggg caaataagga aaatgggagg 7620
   ataggtagtc atctggggtt ttatgcagca aaactacagg ttattattgc ttgtgatccg 7680
   cctcggagta ttttccatcg aggtagatta aagacatgct cacccgagtt ttatactctc 7740
   ctgcttgaga tccttactac agtatgaaat tacagtgtcg cgagttagac tatgtaagca 7800
   gaattttaat catttttaaa gagcccagta cttcatatcc atttctcccg ctccttctgc 7860
   agccttatca aaaggtattt tagaacactc attttagccc cattttcatt tattatactg 7920
   gcttatccaa cccctagaca gagcattggc attttccctt tcctgatctt agaagtctga 7980
   tgactcatga aaccagacag attagttaca tacaccacaa atcgaggctg tagctggggc 8040
   ctcaacactg cagttctttt ataactcctt agtacacttt ttgttgatcc tttgccttga 8100
   tccttaattt tcagtgtcta tcacctctcc cgtcagtggt gttccacatt tgggcctatt 8160
   ctcagtccag ggagttttac aacaatagat gtattgagaa tccaacctaa agcttaactt 8220
   tccactccca tgaatgcctc tctccttttt ctccatttat aaactgagct attaaccatt 8280
   aatggttcca ggtggatgtc tcctccccat attacctgat gtatcttaca tattgccagg 8340
   ctgatatttt aagacattaa aaggtatatt tcattattga gccacatggt attgattact 8400
   gcttactaaa attttgtcat tgtacacatc tgtaaaaggt ggttcctttt ggaatgcaaa 8460
   gttcaggtgt ttgttgtctt tcctgaccta aggtcttgtg agcttgtatt ttttctattt 8520
   aagcagtgct ttctcttgga ctggcttgac tcatggcatt ctacacgtta ttgctggtct 8580
   aaatgtgatt ttgccaagct tcttcaggac ctataatttt gcttgacttg tagccaaaca 8640
   caagtaaaat gattaagcaa caaatgtatt tgtgaagctt ggtttttagg ttgttgtgtt 8700
   gtgtgtgctt gtgctctata ataatactat ccaggggctg gagaggtggc tcggagttca 8760
   agagcacaga ctgctcttcc agaagtcctg agttcaattc ccagcaacca catggtggct 8820
   cacaaccatc tgtaatggga tctgatgccc tcttctggtg tgtctgaaga ccacaagtgt 8880
   attcacatta aataaataaa tcctccttct tcttcttttt ttttttttta aagagaatac 8940
   tgtctccagt agaatttact gaagtaatga aatactttgt gtttgttcca atatggtagc 9000
   caataatcaa attactcttt aagcactgga aatgttacca aggaactaat ttttatttga 9060
   agtgtaactg tggacagagg agccataact gcagacttgt gggatacaga agaccaatgc 9120
   agactttaat gtcttttctc ttacactaag caataaagaa ataaaaattg aacttctagt 9180
   atcctatttg tttaaactgc tagctttact taacttttgt gcttcatcta tacaaagctg 9240
   aaagctaagt ctgcagccat tactaaacat gaaagcaagt aatgataatt ttggatttca 9300
   aaaatgtagg gccagagttt agccagccag tggtggtgct tgcctttatg cctttaatcc 9360
   cagcactctg gaggcagaga caggcagatc tctgagtttg agcccagcct ggtctacaca 9420
   tcaagttcta tctaggatag ccaggaatac acacagaaac cctgttgggg aggggggctc 9480
   tgagatttca taaaattata attgaagcat tccctaatga gccactatgg atgtggctaa 9540
   atccgtctac ctttctgatg agatttgggt attatttttt ctgtctctgc tgttggttgg 9600
   gtcttttgac actgtgggct ttctttaaag cctccttcct gccatgtggt ctcttgtttg 9660
   ctactaactt cccatggctt aaatggcatg gctttttgcc ttctaagggc agctgctgag 9720
   atttgcagcc tgatttccag ggtggggttg ggaaatcttt caaacactaa aattgtcctt 9780
   taattttttt tttaaaaaat gggttatata ataaacctca taaaatagtt atgaggagtg 9840
   aggtggacta atattaaatg agtccctccc ctataaaaga gctattaagg ctttttgtct 9900
   tatacttaac ttttttttta aatgtggtat ctttagaacc aagggtctta gagttttagt 9960
   atacagaaac tgttgcatcg cttaatcaga ttttctagtt tcaaatccag agaatccaaa 10020
   ttcttcacag ccaaagtcaa attaagaatt tctgactttt aatgttaatt tgcttactgt 10080
   gaatataaaa atgatagctt ttcctgaggc agggtctcac tatgtatctc tgcctgatct 10140
   gcaacaagat atgtagacta aagttctgcc tgcttttgtc tcctgaatac taaggttaaa 10200
   atgtagtaat acttttggaa cttgcaggtc agattctttt ataggggaca cactaaggga 10260
   gcttgggtga tagttggtaa aatgtgtttc aagtgatgaa aacttgaatt attatcaccg 10320
   caacctactt tttaaaaaaa aaagccaggc ctgttagagc atgcttaagg gatccctagg 10380
   acttgctgag cacacaagag tagttacttg gcaggctcct ggtgagagca tatttcaaaa 10440
   aacaaggcag acaaccaaga aactacagtt aaggttacct gtctttaaac catctgcata 10500
   tacacaggga tattaaaata ttccaaataa tatttcattc aagttttccc ccatcaaatt 10560
   gggacatgga tttctccggt gaataggcag agttggaaac taaacaaatg ttggttttgt 10620
   gatttgtgaa attgttttca agtgatagtt aaagcccatg agatacagaa caaagctgct 10680
   atttcgaggt ctcttggttt atactcagaa gcacttcttt gggtttccct gcactatcct 10740
   gatcatgtgc taggcctacc ttaggctgat tgttgttcaa ataaacttaa gtttcctgtc 10800
   aggtgatgtc atatgatttc atatatcaag gcaaaacatg ttatatatgt taaacatttg 10860
   tacttaatgt gaaagttagg tctttgtggg tttgattttt aattttcaaa acctgagcta 10920
   aataagtcat ttttacatgt cttacatttg gtggaattgt ataattgtgg tttgcaggca 10980
   agactctctg acctagtaac cctacctata gagcactttg ctgggtcaca agtctaggag 11040
   tcaagcattt caccttgaag ttgagacgtt ttgttagtgt atactagttt atatgttgga 11100
   ggacatgttt atccagaaga tattcaggac tatttttgac tgggctaagg aattgattct 11160
   gattagcact gttagtgagc attgagtggc ctttaggctt gaattggagt cacttgtata 11220
   tctcaaataa tgctggcctt ttttaaaaag cccttgttct ttatcaccct gttttctaca 11280
   taatttttgt tcaaagaaat acttgtttgg atctcctttt gacaacaata gcatgttttc 11340
   aagccatatt ttttttcctt tttttttttt tttttggttt ttcgagacag ggtttctctg 11400
   tatagccctg gctgtcctgg aactcacttt gtagaccagg ctggcctcga actcagaaat 11460
   ccgcctgcct ctgcctcctg agtgccggga ttaaaggcgt gcaccaccac gcctggctaa 11520
   gttggatatt ttgttatata actataacca atactaactc cactgggtgg atttttaatt 11580
   cagtcagtag tcttaagtgg tctttattgg cccttcatta aaatctactg ttcactctaa 11640
   cagaggctgt tggtactagt ggcacttaag caacttccta cggatatact agcagattaa 11700
   gggtcaggga tagaaactag tctagcgttt tgtataccta ccagctttat actaccttgt 11760
   tctgatagaa atatttcagg acat                                        11784
<210> 107
   <211> 1458
   <212> DNA
   <213> Bacteriophage TP901-1
<220>
   <221> CDS
   <222> (1)..(1455)
<400> 107
   atg act aag aaa gta gca atc tat aca cga gta tcc act act aac caa      48
   Met Thr Lys Lys Val Ala Ile Tyr Thr Arg Val Ser Thr Thr Asn Gln
    1               5                  10                  15
   gca gag gaa ggg ttc tca att gat gag caa att gac cgt tta aca aaa      96
   Ala Glu Glu Gly Phe Ser Ile Asp Glu Gln Ile Asp Arg Leu Thr Lys
               20                  25                  30
   tat gct gaa gca atg ggg tgg caa gta tct gat act tat act gat gct     144
   Tyr Ala Glu Ala Met Gly Trp Gln Val Ser Asp Thr Tyr Thr Asp Ala
           35                  40                  45
   ggt ttt tca ggg gcc aaa ctt gaa cgc cca gca atg caa aga tta atc     192
   Gly Phe Ser Gly Ala Lys Leu Glu Arg Pro Ala Met Gln Arg Leu Ile
       50                  55                  60
   aac gat atc gag aat aaa gct ttt gat aca gtt ctt gta tat aag cta     240
   Asn Asp Ile Glu Asn Lys Ala Phe Asp Thr Val Leu Val Tyr Lys Leu
   65                  70                  75                  80
   gac cgc ctt tca cgt agt gta aga gat act ctt tat ctt gtt aag gat     288
   Asp Arg Leu Ser Arg Ser Val Arg Asp Thr Leu Tyr Leu Val Lys Asp
                   85                  90                  95
   gtg ttc aca aaa aat aaa ata gac ttt atc tcg ctt aat gaa agt att     336
   Val Phe Thr Lys Asn Lys Ile Asp Phe Ile Ser Leu Asn Glu Ser Ile
               100                 105                 110
   gat act tct tct gct atg ggt agc ttg ttt ctc act att ctt tct gca     384
   Asp Thr Ser Ser Ala Met Gly Ser Leu Phe Leu Thr Ile Leu Ser Ala
           115                 120                 125
   att aat gag ttt gaa aga gag aat ata aaa gaa cgc atg act atg ggt     432
   Ile Asn Glu Phe Glu Arg Glu Asn Ile Lys Glu Arg Met Thr Met Gly
       130                 135                 140
   aaa cta ggg cga gcg aaa tct ggt aag tct atg atg tgg act aag aca     480
   Lys Leu Gly Arg Ala Lys Ser Gly Lys Ser Met Met Trp Thr Lys Thr
   145                 150                 155                 160
   gct ttt ggg tat tac cac aac aga aag aca ggt ata tta gaa att gtt     528
   Ala Phe Gly Tyr Tyr His Asn Arg Lys Thr Gly Ile Leu Glu Ile Val
               165                 170                 175
   cct tta caa gct aca ata gtt gaa caa ata ttc act gat tat tta tca    576
   Pro Leu Gln Ala Thr Ile Val Glu Gln Ile Phe Thr Asp Tyr Leu Ser
               180                 185                 190
   gga ata tca ctt aca aaa tta aga gat aaa ctc aat gaa tct gga cac    624
   Gly Ile Ser Leu Thr Lys Leu Arg Asp Lys Leu Asn Glu Ser Gly His
           195                 200                 205
   atc ggt aaa gat ata ccg tgg tct tat cgt acc cta aga caa aca ctt    672
   Ile Gly Lys Asp Ile Pro Trp Ser Tyr Arg Thr Leu Arg Gln Thr Leu
       210                 215                 220
   gat aat cca gtt tac tgt ggt tat atc aaa ttt aag gac agc cta ttt    720
   Asp Asn Pro Val Tyr Cys Gly Tyr Ile Lys Phe Lys Asp Ser Leu Phe
   225                 230                 235                 240
   gaa ggt atg cac aaa cca att atc cct tat gag act tat tta aaa gtt    768
   Glu Gly Met His Lys Pro Ile Ile Pro Tyr Glu Thr Tyr Leu Lys Val
                   245                 250                 255
   caa aaa gag cta gaa gaa aga caa cag cag act tat gaa aga aat aac    816
   Gln Lys Glu Leu Glu Glu Arg Gln Gln Gln Thr Tyr Glu Arg Asn Asn
               260                 265                 270
   aac cct aga cct ttc caa gct aaa tat atg ctg tca ggg atg gca agg    864
   Asn Pro Arg Pro Phe Gln Ala Lys Tyr Met Leu Ser Gly Met Ala Arg
           275                 280                 285
   tgc ggt tac tgt gga gca cct tta aaa att gtt ctt ggc cac aaa aga    912
   Cys Gly Tyr Cys Gly Ala Pro Leu Lys Ile Val Leu Gly His Lys Arg
       290                 295                 300
   aaa gat gga agc cgc act atg aaa tat cac tgt gca aat aga ttt cct    960
   Lys Asp Gly Ser Arg Thr Met Lys Tyr His Cys Ala Asn Arg Phe Pro
   305                 310                 315                 320
   cga aaa aca aaa gga att aca gta tat aat gac aat aaa aag tgt gat    1008
   Arg Lys Thr Lys Gly Ile Thr Val Tyr Asn Asp Asn Lys Lys Cys Asp
                   325                 330                 335
   tca gga act tat gat tta agt aat tta gaa aat act gtt att gac aac    1056
   Ser Gly Thr Tyr Asp Leu Ser Asn Leu Glu Asn Thr Val Ile Asp Asn
               340                 345                 350
   ctg att gga ttt caa gaa aat aat gac tcc tta ttg aaa att atc aat    1104
   Leu Ile Gly Phe Gln Glu Asn Asn Asp Ser Leu Leu Lys Ile Ile Asn
           355                 360                 365
   ggc aac aac caa cct att ctt gat act tcg tca ttt aaa aag caa att    1152
   Gly Asn Asn Gln Pro Ile Leu Asp Thr Ser Ser Phe Lys Lys Gln Ile
       370                 375                 380
   tca cag atc gat aaa aaa ata caa aag aac tct gat ttg tac cta aat    1200
   Ser Gln Ile Asp Lys Lys Ile Gln Lys Asn Ser Asp Leu Tyr Leu Asn
   385                 390                 395                 400
   gat ttt atc act atg gat gag ttg aaa gat cgt act gat tcc ctt cag    1248
   Asp Phe Ile Thr Met Asp Glu Leu Lys Asp Arg Thr Asp Ser Leu Gln
                   405                 410                 415
   gct gag aaa aag ctg ctt aaa gct aag att agc gaa aat aaa ttt aat    1296
   Ala Glu Lys Lys Leu Leu Lys Ala Lys Ile Ser Glu Asn Lys Phe Asn
               420                 425                 430
   gac tct act gat gtt ttt gag tta gtt aaa act cag ttg ggc tca att    1344
   Asp Ser Thr Asp Val Phe Glu Leu Val Lys Thr Gln Leu Gly Ser Ile
           435                 440                 445
   ccg att aat gaa cta tca tat gat aat aaa aag aaa atc gtc aac aac    1392
   Pro Ile Asn Glu Leu Ser Tyr Asp Asn Lys Lys Lys Ile Val Asn Asn
       450                 455                 460
   ctt gta tca aag gtt gat gtt act gct gat aat gta gat atc ata ttt    1440
   Leu Val Ser Lys Val Asp Val Thr Ala Asp Asn Val Asp Ile Ile Phe
   465                 470                 475                 480
   aaa ttc caa ctc gct taa                                            1458
   Lys Phe Gln Leu Ala
                   485
<210> 108
   <211> 485
   <212> PRT
   <213> Bacteriophage TP901-1
<400> 108
   Met Thr Lys Lys Val Ala Ile Tyr Thr Arg Val Ser Thr Thr Asn Gln
   1               5                   10                  15
   Ala Glu Glu Gly Phe Ser Ile Asp Glu Gln Ile Asp Arg Leu Thr Lys
               20                  25                  30
   Tyr Ala Glu Ala Met Gly Trp Gln Val Ser Asp Thr Tyr Thr Asp Ala
           35                  40                  45
   Gly Phe Ser Gly Ala Lys Leu Glu Arg Pro Ala Met Gln Arg Leu Ile
       50                  55                  60
   Asn Asp Ile Glu Asn Lys Ala Phe Asp Thr Val Leu Val Tyr Lys Leu
   65                  70                  75                  80
   Asp Arg Leu Ser Arg Ser Val Arg Asp Thr Leu Tyr Leu Val Lys Asp
                   85                  90                  95
   Val Phe Thr Lys Asn Lys Ile Asp Phe Ile Ser Leu Asn Glu Ser Ile
               100                 105                 110
   Asp Thr Ser Ser Ala Met Gly Ser Leu Phe Leu Thr Ile Leu Ser Ala
           115                 120                 125
   Ile Asn Glu Phe Glu Arg Glu Asn Ile Lys Glu Arg Met Thr Met Gly
       130                 135                 140
   Lys Leu Gly Arg Ala Lys Ser Gly Lys Ser Met Met Trp Thr Lys Thr
   145                 150                 155                 160
   Ala Phe Gly Tyr Tyr His Asn Arg Lys Thr Gly Ile Leu Glu Ile Val
                   165                 170                 175
   Pro Leu Gln Ala Thr Ile Val Glu Gln Ile Phe Thr Asp Tyr Leu Ser
               180                 185                 190
   Gly Ile Ser Leu Thr Lys Leu Arg Asp Lys Leu Asn Glu Ser Gly His
           195                 200                 205
   Ile Gly Lys Asp Ile Pro Trp Ser Tyr Arg Thr Leu Arg Gln Thr Leu
       210                 215                 220
   Asp Asn Pro Val Tyr Cys Gly Tyr Ile Lys Phe Lys Asp Ser Leu Phe
   225                 230                 235                 240
   Glu Gly Met His Lys Pro Ile Ile Pro Tyr Glu Thr Tyr Leu Lys Val
                   245                 250                 255
   Gln Lys Glu Leu Glu Glu Arg Gln Gln Gln Thr Tyr Glu Arg Asn Asn
               260                 265                 270
   Asn Pro Arg Pro Phe Gln Ala Lys Tyr Met Leu Ser Gly Met Ala Arg
           275                 280                 285
   Cys Gly Tyr Cys Gly Ala Pro Leu Lys Ile Val Leu Gly His Lys Arg
       290                 295                 300
   Lys Asp Gly Ser Arg Thr Met Lys Tyr His Cys Ala Asn Arg Phe Pro
   305                 310                 315                 320
   Arg Lys Thr Lys Gly Ile Thr Val Tyr Asn Asp Asn Lys Lys Cys Asp
                   325                 330                 335
   Ser Gly Thr Tyr Asp Leu Ser Asn Leu Glu Asn Thr Val Ile Asp Asn
               340                 345                 350
   Leu Ile Gly Phe Gln Glu Asn Asn Asp Ser Leu Leu Lys Ile Ile Asn
           355                 360                 365
   Gly Asn Asn Gln Pro Ile Leu Asp Thr Ser Ser Phe Lys Lys Gln Ile
       370                 375                 380
   Ser Gln Ile Asp Lys Lys Ile Gln Lys Asn Ser Asp Leu Tyr Leu Asn
   385                 390                 395                 400
   Asp Phe Ile Thr Met Asp Glu Leu Lys Asp Arg Thr Asp Ser Leu Gln
                   405                 410                 415
   Ala Glu Lys Lys Leu Leu Lys Ala Lys Ile Ser Glu Asn Lys Phe Asn
               420                 425                 430
   Asp Ser Thr Asp Val Phe Glu Leu Val Lys Thr Gln Leu Gly Ser Ile
           435                 440                 445
   Pro Ile Asn Glu Leu Ser Tyr Asp Asn Lys Lys Lys Ile Val Asn Asn
       450                 455                 460
   Leu Val Ser Lys Val Asp Val Thr Ala Asp Asn Val Asp Ile Ile Phe
   465                 470                 475                 480
   Lys Phe Gln Leu Ala
                   485